# EUROPEAN PATENT APPLICATION

(11) **EP 4 596 563 A2**
(43) Date of publication of application: **06.08.2025**
(21) Application number: 25155196.6
(22) Date of filing: 31.01.2025
(51) Int. Cl.: C07F 15/00, A61B 5/145, G01N 33/66, C08F 8/18, C08F 8/30, C08F 8/42, C08F 120/24, C08F 120/34, G01N 27/327, G01N 33/543

(54) **ELECTROCHEMICAL BIOSENSOR COMPRISING TRANSITION METAL COMPLEX OR OXIDATION-REDUCTION POLYMER**

(30) Priority: 31.01.2024 KR 20240014707
(71) Applicant: i-Sens, Inc., Seoul 06646 (KR)
(72) Inventor: SHIN, Hyun Seo, 04138 Seoul (KR); YANG, Bo Na, 02055 Seoul (KR); KANG, Geun Hee, 25774 Donghae-si, Gangwon-do (KR); YU, Ar Eum, 03782 Seoul (KR)
(74) Representative: Isarpatent

(57) **Abstract**

The present invention relates to a transition metal complex or oxidation-reduction polymer including a phenyl-pyridine derivative, and an electrochemical biosensor including the same. The transition metal complex according to the present invention can be easily linked to various types of polymers. In addition, the transition metal complex according to one aspect of the present invention is electrochemically and structurally stable. Furthermore, in accordance with a method for preparing the transition metal complex according to one aspect of the present invention, various types of functional groups can be easily linked to a ligand.

## Description

### BACKGROUND OF THE INVENTION

### Field of the Invention

The present invention relates to a transition metal complex or oxidation-reduction polymer including a phenyl-pyridine derivative, and an electrochemical biosensor including the same.

### Description of the Related Art

Periodic measurement of blood glucose is very important in the management of diabetes, and therefore, various blood glucose level measuring devices are being developed to allow easy measurement of blood glucose using portable measuring devices. The operating principle of such a biosensor is based on an optical method or an electrochemical method. An electrochemical biosensor can reduce the influence of oxygen, unlike a biosensor using an optical method in the related art, and has the advantage that it can be used without any separate pretreatment even if the sample becomes turbid. Therefore, various types of electrochemical biosensors with accuracy and precision are widely used.

Currently commercialized electrochemical blood glucose sensors mainly use enzyme electrodes, and have a structure in which a glucose oxidase is immobilized on an electrode capable of converting an electrical signal by a chemical or physical method. These electrochemical blood glucose sensors are based on the principle of measuring the electric current generated by transferring electrons generated by the enzymatic oxidation of glucose in analytes such as blood to the electrodes, thereby measuring the concentration of glucose in the analyte. In the case of a biosensor that uses an enzyme electrode, the distance from the active center of the enzyme is so great that there occurs a problem in that it is difficult to directly transfer electrons generated by the oxidation of a substrate to the electrode. Therefore, in order to easily perform such an electron transfer reaction, an oxidation reduction mediator, that is, an electron transfer mediator, is essentially required. Therefore, what most significantly determines the characteristics of an electrochemical biosensor that measures blood glucose is the type of enzyme used and the characteristics of the electron transfer mediator.

The development trend of blood glucose sensors is shifting to the use of GDH, which excludes oxygen in an enzymatic reaction, instead of GOx, in which oxygen participates in the enzymatic reaction with glucose in the blood, in order to block changes in measurement values due to differences in oxygen partial pressure (pO₂) that vary depending on the blood (venous blood, capillary blood, and the like). In addition, in the case of an electron transfer mediator, ferricyanide, which has sensitive stability depending on humidity, is being replaced with organic compounds such as quinone derivatives (phenanthroline quinone, quineonediimine, and the like), which are highly stable under temperature and humidity, and organometallic compounds such as Ru complexes (ruthenium hexamine, and the like) and osmium complexes.

The most commonly used electron transfer mediator is potassium ferricyanide [K₃Fe(CN)₆], and is useful for all sensors using flavin adenine dinucleotide-glucose oxidase (FAD-GOx), glucose dehydrogenase-pyrrolo quinoline quinone (PQQ-GDH) or flavin adenine dinucleotide-glucose dehydrogenase (FAD-GDH) because it is inexpensive and has good reactivity. However, sensors using the aforementioned electron transfer mediator cause a measurement error due to interfering materials such as uric acid or gentisic acid present in the blood, and are easily deteriorated due to temperature and humidity, and thus, special care must be taken during preparation and storage, and it is difficult to accurately detect low concentration of glucose due to changes in background current after long-term storage.

Meanwhile, among the electron transfer mediators, hexaamine ruthenium chloride [Ru(NH₃)₆Cl₃] has higher oxidation reduction stability than ferricyanide, so that biosensors using the electron transfer mediator have advantages in easy manufacturing and storage, and being highly stable due to small change in background current even when they are stored for a long period of time. However, hexaamine ruthenium chloride has limitations in that it is difficult for hexaamine ruthenium chloride to be manufactured as a commercially useful sensor because it is not compatible with FAD-GDH in reactivity.

Further, in using such a biosensor, obtaining accurate and rapid measurement values with a small amount of samples is a very important problem in terms of maximizing convenience for users.

Therefore, there is still a need for developing a new electron transfer mediator capable of achieving the reduction of the disadvantages and measurement time of the electron transfer mediator in the related art as described above.

Meanwhile, a continuous glucose monitoring (CGM) system is used to continuously monitor blood glucose levels and manage diseases such as diabetes, and existing enzyme sensors are difficult to use in CGM due to the pain caused by collecting blood. In order to solve these problems, an enzyme sensor that can adhere to the body and thus minimize invasion has recently been developed.

In the case of such a continuous blood glucose monitoring enzyme sensor, since a part of the sensor is inserted into the human body, an electron transfer mediator used in the continuous blood glucose monitoring enzyme sensor is required to have the following characteristics, unlike existing electron transfer mediators.

First, the electrochemical characteristics should be excellent, such as appropriate oxidation reduction potential difference, stable oxidation reduction ability, and fast electron transfer ability between oxide and reducing material. These characteristics are key elements that can affect rapid electron transfer in the sensor and can also affect its selectivity and sensitivity. In particular, the oxidation reduction potential of the electron transfer mediator should be set to a potential at which redox shuttling, which is a phenomenon in which in addition to electron transfer among an enzyme, an electron transfer mediator, and a working electrode, electron transfer between a counter electrode and the electron transfer mediator occurs to generate additional background current, does not occur. This is because the generation of background current reduces sensitivity and decreases analytical accuracy when measuring low concentrations of blood glucose. Therefore, in order for an electron transfer mediator to perform its function efficiently, it is ideal for the electron transfer mediator to have a potential value that is appropriately lower than the oxidation reduction potential value of a working electrode used. In addition, it is very important to set an appropriate oxidation reduction potential for the electron transfer mediator in order to prevent an increase in background current due to the generation of current by chemical species capable of undergoing oxidation and reduction in the body (interfering factors: vitamin C, oxygen, and the like).

Secondly, in the case of a continuous blood glucose monitoring enzyme sensor, a part of the sensor is inserted into the human body, but problems should be solved in which electron transfer mediators including transition metals are lost inside the human body to cause toxicity, reduce signals, and shorten the service life of the sensor. To solve this problem, it is required to select an appropriate polymer, link a polymer and an electron transfer mediator through a linker, form a stable oxidation-reduction polymer film using a crosslinker, and the like. For this purpose, in the related art, a transition metal electron transfer mediator including a bipyridine ligand is immobilized on a polymer backbone such as polyvinylpyridine or polyvinylimidazole to prepare an oxidation-reduction polymer (polymer) for an enzyme sensor and use the polymer. However, there is a limitation in that it is difficult to synthesize an electron transfer mediators with stable and various oxidation reduction potentials through existing limited ligands. Furthermore, existing oxidation-reduction polymers have limitations such as long and complicated synthesis steps, low immobilization rates of transition metal complexes, and very tricky crosslinking conditions for the formation of a polymer film.

Therefore, there is a need for developing new types of oxidation-reduction polymers to overcome the limitations of existing materials.

### [Related Art Documents]

### [Patent Documents]

(Patent Document 1) EP 1973983 B1

### SUMMARY OF THE INVENTION

In one aspect, an object of the present invention is to provide a transition metal complex, an electron transfer mediator, and an oxidation-reduction polymer including the same, which are suitable for a continuous blood glucose monitoring enzyme sensor.

In one aspect, an object of the present invention is to provide an electrochemically and structurally stable transition metal complex.

In one aspect, an object of the present invention is to provide a transition metal complex capable of stably binding to various polymers.

In one aspect, an object of the present invention is to provide an electron transfer mediator having a fast self-exchange rate.

In one aspect, an object of the present invention is to provide an electron transfer mediator that does not generate background current.

In one aspect, an object of the present invention is to provide an electron transfer mediator having excellent binding ability and binding strength with various polymers.

In one aspect, an object of the present invention is to provide an oxidation-reduction polymer which is easily formed into a polymer film because crosslinking conditions are simple during the formation of the polymer film.

In one aspect, an object of the present invention is to solve a problem in that transition metals used in continuous blood glucose monitoring enzyme sensors are leaving from a sensor membrane.

In one aspect, an object of the present invention is to enhance the measurement accuracy of an electrochemical biosensor that uses an enzyme.

In one aspect, an object of the present invention is to enhance the signal intensity of an electrochemical biosensor that uses carbon nanotubes (CNTs).

In one aspect, an object of the present invention is to easily introduce a phenyl-pyridine derivative into a ligand of a transition metal complex.

In order to achieve the above objects, in one aspect, the present invention provides a transition metal complex, which is a compound represented by the following Chemical Formula 1, or a salt compound thereof, and an oxidation-reduction polymer in a form in which the transition metal complex and a polymer are bound to each other:
a transition metal complex, which is a compound represented by the following Chemical Formula 1, or a salt compound thereof:

   [Chemical Formula 1] [M(L)ₐ(X₁)_{b}]^{c} d(X₂)
wherein,
M is a transition metal selected from the group consisting of Fe, Ru, and Os,
L is a bidentate ligand including pyridine; and one structure selected from the group consisting of pyrazole, triazole, tetrazole, oxadiazole and thiadiazole;
the pyridine is
an unsubstituted pyridine, or a pyridine substituted with one to four selected from the group consisting of a C₁₋₄ alkyl group, a C₁₋₄ alkoxy group, a -(CH₂)-O-C₁₋₄ alkyl group, a - (CH₂CH₂)-O-C₁₋₄ alkyl group, and C₁₋₄ alkylamino group,
one selected from the group consisting of the pyrazole, triazole, tetrazole, and oxadiazole is each independently unsubstituted or substituted with one to three selected from the group consisting of a C₁₋₄ alkyl group, a C₁₋₄ alkoxy group, a -(CH₂)-O-C₁₋₄ alkyl group, a -(CH₂CH₂)-O-C₁₋₄ alkyl group, 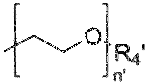 , and a C₁₋₄ alkylamino group,
R'4 is hydrogen or a substituted or unsubstituted C₁₋₄ alkyl,
n' is an integer selected from 1 to 4,
a is 2,
the C₁₋₄ alkyl group, C₁₋₄ alkoxy group, -(CH₂)-O-C₁₋₄ alkyl group, -(CH₂CH₂)-O-C₁₋₄ alkyl group, and C₁₋₄ alkylamino group are each substituted or unsubstituted,
the substituted C₁₋₄ alkyl group, C₁₋₄ alkoxy group, -(CH₂)-O-C₁₋₄ alkyl group, - (CH₂CH₂)-O-C₁₋₄ alkyl group, or C₁₋₄ alkylamino group is one in which a substituted hydrogen atom is substituted with a halogen atom of F, Cl, Br, or I, a cyano group, a hydroxyl group, a thiol group, a nitro group, an amino group, an imino group, an azido group, an amidino group, a hydrazino group, a hydrazono group, an oxo group, a carbonyl group, a carbamyl group, an ester group, an ether group, a carboxyl group or a salt thereof, a sulfonic acid group or a salt thereof, or phosphoric acid or a salt thereof,
b is 1,
c is an integer selected from 1 to 3,
X₁ is a compound of the following Chemical Formula 2 or 3, 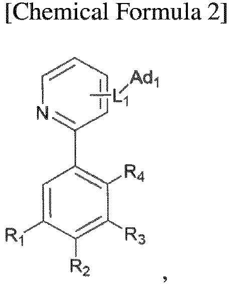 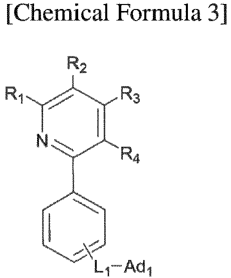
X₂ is one counter ion selected from the group consisting of F, Cl, Br, I, and PF₆,
d is 0, 1, 2, or 3,
in Chemical Formulae 2 and 3,
L₁ is selected from the group consisting of a substituted or unsubstituted C₁₋₁₂ alkylene group, a substituted or unsubstituted C₁₋₁₅ alkoxy group, a substituted or unsubstituted C₂₋₁₀ ethylene glycol group, an amine group (-NH₂), and an acyl group (-CO);
Ad₁ is selected from the group consisting of a primary amine group, a secondary amine group, an ammonium group, a halogen group, an epoxy group, an azide group, an alkenyl group, an alkynyl group, a thiol group, and an alcohol group,
Ri and R₄ are each independently hydrogen, deuterium, a methyl group, an ethyl group, or a methoxy group, and
R₂ and R₃ are each independently hydrogen, a C₁₋₄ alkyl group, a C₁₋₄ alkoxy group, or a C₁₋₄ alkylamino group.

Further, in one aspect, the present invention provides an oxidation-reduction polymer.

In one aspect, the present invention provides an oxidation-reduction polymer, wherein the oxidation-reduction polymer includes a combination of a transition metal complex of Chemical Formula 1 or a salt compound thereof and a polymer, and the polymer includes one or more selected from the group consisting of polyvinylimidazole, polyvinylpyridine, poly(allyl glycidyl ether), poly(dimethylaminoethyl methacrylate), poly(pentafluorophenyl acrylate), poly(pentafluorophenyl methacrylate), a poly(pentafluorophenyl acrylate)-polyacrylamide copolymer, a poly(dimethylaminoethyl methacrylate)-poly(pentafluorophenyl methacrylate) copolymer, and polymers thereof.

In addition, in one aspect, the present invention provides an oxidation-reduction polymer, wherein the oxidation-reduction polymer is for use in an electrochemical biosensor.

Furthermore, in one aspect, the present invention provides an oxidation-reduction polymer, wherein the electrochemical biosensor includes an electrochemical biosensor which is capable of being inserted into the body.

Further, in one aspect, the present invention provides an oxidation-reduction polymer, wherein the electrochemical biosensor includes an electrochemical biosensor for measuring blood glucose.

In addition, in one aspect, the present invention provides a sensing layer for an electrochemical biosensor, including an enzyme capable of oxidizing-reducing a biological sample, and the transition metal complex or the salt compound thereof, or the oxidation-reduction polymer as an electron transfer mediator.

Furthermore, in one aspect, the present invention provides a sensing layer for an electrochemical biosensor, further including single-walled carbon nanotubes, multi-walled carbon nanotubes, and functionalized carbon nanotubes.

Further, in one aspect, the present invention provides a continuous blood glucose monitoring sensor including the sensing layer for an electrochemical biosensor.

The objects of the present invention are not limited to the aforementioned technical problems, and other technical problems can be derived from the following description.

The transition metal complex according to the present invention can be easily linked to various types of polymers. In addition, the transition metal complex according to one aspect of the present invention is electrochemically and structurally stable. Furthermore, in accordance with a method for preparing the transition metal complex according to one aspect of the present invention, various types of functional groups can be easily linked to a ligand. Further, when a transition metal complex according to one aspect of the present invention is prepared, a phenyl-pyridine ligand can be introduced very easily.

The effects of the present invention are not limited to those mentioned above, and other effects, which have not been mentioned, will be able to be clearly understood by those skilled in the art from the following description.

### BRIEF DESCRIPTION OF THE DRAWINGS

FIG. 1 is a cyclic voltammetry curves showing the electrochemical characteristics of a transition metal complex, which is one aspect of the present invention.
FIG. 2 is a cyclic voltammetry curves showing the electrochemical characteristics of an oxidation-reduction polymer including a transition metal complex, which is one aspect of the present invention.
FIG. 3 is a graph showing the sensitivity of an electrode to a change in glucose concentration, to which an oxidation-reduction polymer, which is one aspect of the present invention, is applied, using a chronoamperometry.
FIG. 4 is a graph showing the sensitivity of electrodes according to a change in glucose concentration, to which an oxidation-reduction polymer which is one aspect of the present invention is applied, using a multistep voltammetry.

### DETAILED DESCRIPTION OF THE INVENTION

The terms or words used in the specification and the claims of the present invention should not be construed as being limited to typical or dictionary meanings, and should be construed as meanings and concepts conforming to the technical spirit of the present invention on the basis of the principle that an inventor can appropriately define concepts of the terms in order to describe his or her own invention in the best way.

Throughout the specification of the present invention, when one part "includes" one constituent element, unless otherwise specifically described, this does not mean that another constituent element is excluded, but means that another constituent element may be further included.

Hereinafter, the present invention will be described in detail.

The following description is merely an example, and the scope of the present invention is not limited by the following description.

In one aspect, the present invention is a transition metal complex, which is a compound of the following Chemical Formula 1, or a salt compound thereof:

[Chemical Formula 1] [M(L)ₐ(X₁)_{b}]^{c} d(X₂)

The definitions of residues used in the present specification will be described in detail. Unless otherwise specified, each residue has the following definition and is used in the sense commonly understood by one of ordinary skill in the art.

As used herein, "halo" or "halogen" refers to, for example, fluoro, chloro, bromo, and iodo.

As used herein, "alkyl" refers to an aliphatic hydrocarbon radical and includes all straight-chain or branched hydrocarbon radicals. For example, an aliphatic hydrocarbon having 1 to 6 carbon atoms includes methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl, but is not limited thereto. Unless otherwise defined, alkyl may refer to an alkyl having 1 to 6 carbon atoms, 1 to 5 carbon atoms, 1 to 4 carbon atoms, 1 to 3 carbon atoms, or 1 to 2 carbon atoms, or an alkyl having 2 to 6 carbon atoms, 2 to 5 carbon atoms, 2 to 4 carbon atoms, 2 to 3 carbon atoms, 3 to 6 carbon atoms, 3 to 5 carbon atoms, 3 to 4 carbon atoms, 1 carbon atom, 2 carbon atoms, 3 carbon atoms, 4 carbon atoms, 5 carbon atoms, or 6 carbon atoms.

As used herein, "alkoxy" refers to an -O-alkyl or alkyl-O- group, where the alkyl group is as defined above. Examples thereof include methoxy, ethoxy, n-propoxy, n-butoxy, t-butoxy and the like, but are not limited thereto. The alkoxy group may be unsubstituted or substituted with one or more suitable groups.

As used herein, the term "hydroxy" or "hydroxyl", alone or in combination with other terms, refers to -OH.

As used herein, "amino" refers to -NH2; and "nitro" refers to -NO2.

As used herein, "aryl" refers to a monovalent aromatic hydrocarbon, for example, having 6 to 20 carbon atoms, 6 to 12 carbon atoms, or 6 to 10 carbon atoms, derived by removing one hydrogen atom from one carbon atom in a parent aromatic ring system. The aryl may include a double ring radical including an aromatic ring fused with a saturated or partially unsaturated ring. Exemplary aryl groups include radicals derived from benzene (phenyl), substituted phenyl, biphenyl, naphthyl, tetrahydronaphthyl, fluorenyl, toluyl, naphthalenyl, anthracenyl, indenyl, indanyl, and the like, but are not limited thereto. The aryl group may be unsubstituted or substituted with one or more suitable groups.

As used herein, unless otherwise specified, the "substituted" may mean that at least one atom or atomic group is replaced with and bonded to another type of atom or atomic group, or the like.

In the formula, M may be a transition metal selected from the group consisting of ferrite (Fe), ruthenium (Ru), and osmium (Os).

Further, in the formula, L may be a bidentate ligand including pyridine and one structure selected from the group consisting of pyrazole, triazole, tetrazole, oxadiazole, and thiadiazole.

In one aspect, the pyridine may be unsubstituted pyridine or include pyridine substituted with one to four groups selected from the group consisting of a C₁₋₄ alkyl group, a C₁₋₄ alkoxy group, a -(CH₂)-O-C₁₋₄ alkyl group, a -(CH₂CH₂)-O-C₁₋₄ alkyl group, and a C₁₋₄ alkylamino group, but is not limited thereto.

In addition, the one selected from the group consisting of pyrazole, triazole, tetrazole, and oxadiazole may each independently be unsubstituted or include those substituted with one to three groups selected from the group consisting of a C₁₋₄ alkyl group, a C₁₋₄ alkoxy group, a -(CH₂)-O-C₁₋₄ alkyl group, a -(CH₂CH₂)-O-C₁₋₄ alkyl group, 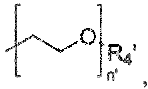 and a C₁₋₄ alkylamino group, but is not limited thereto.

Furthermore, in Chemical Formula 1, R'4 may include hydrogen or a substituted or unsubstituted C₁₋₄ alkyl.

Further, n' may be an integer from 1 to 4.

In addition, in Chemical Formula 1, a may be 2 or 3, b may be 0, 1, or 2, and c may be an integer selected from 1 to 3. Furthermore, d may be 0, 1, 2, or 3.

The C₁₋₄ alkyl group, C₁₋₄ alkoxy group, -(CH₂)-O-C₁₋₄ alkyl group, -(CH₂CH₂)-O-C₁₋₄ alkyl group, and C₁₋₄ alkylamino group may each be substituted or unsubstituted, but are not limited thereto.

In one aspect, the substituted C₁₋₄ alkyl group, C₁₋₄ alkoxy group, -(CH₂)-O-C₁₋₄ alkyl group, -(CH₂CH₂)-O-C₁₋₄ alkyl group, or C₁₋₄ alkylamino group may include one in which a substituted hydrogen atom is substituted with a halogen atom of F, Cl, Br, or I, a cyano group, a hydroxyl group, a thiol group, a nitro group, an amino group, an imino group, an azido group, an amidino group, a hydrazino group, a hydrazono group, an oxo group, a carbonyl group, a carbamyl group, an ester group, an ether group, a carboxyl group or a salt thereof, a sulfonic acid group or a salt thereof, or phosphoric acid or a salt thereof, but is not limited thereto.

X₁ may be a compound of the following Chemical Formula 2 or 3.

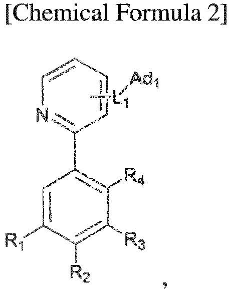

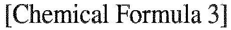

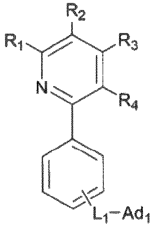

Further, in Chemical Formula 1, X₂ may include one counter ion selected from the group consisting of F, Cl, Br, I, and PF₆.

In Chemical Formulae 2 and 3, L₁ may be selected from the group consisting of a substituted or unsubstituted C₁₋₁₂ alkylene group, a substituted or unsubstituted C₁₋₁₅ alkoxy group, a substituted or unsubstituted C₂₋₁₀ ethylene glycol group, but is not limited thereto.

In addition, in Chemical Formulae 2 and 3, Ad₁ may be selected from the group consisting of a primary amine group, a secondary amine group, an ammonium group, a halogen group, an epoxy group, an azide group, an alkenyl group, an alkynyl group, a thiol group, and an alcohol group, but is not limited thereto.

Furthermore, R₁ and R₄ may each independently be hydrogen, deuterium, a methyl group, an ethyl group, or a methoxy group, but are not limited thereto.

Further, R₂ and R₃ may each independently be hydrogen, a C₁₋₄ alkyl group, a C₁₋₄ alkoxy group, or a C₁₋₄ alkylamino group, but are not limited thereto.

In one example, the transition metal complex may include a transition metal composite in an oxidized state, specifically a trivalent osmium composite or a divalent osmium composite. As an oxidizing agent used in the oxidation treatment, a commonly used oxidizing agent may be used, and examples of the oxidizing agent may be one or more selected from the group consisting of NaOCl, H₂O₂, O₂, O₃, PbO₂, MnO₂, KMnO₄, ClO₂, F₂, Cl₂, H₂CrO₄, N₂O, Ag₂O, OsO₄, H₂S₂O₈, ceric ammonium nitrate (CAN), pyridinium chlorochromate, and 2,2'-dipyridyldisulfide. In addition, when the transition metal complex includes a compound in an oxidized state and a compound in a reduced state, an oxidation treatment may be carried out to provide a transition metal complex in an oxidized state or a salt compound thereof.

In one aspect, the transition metal complex may be in the form of a salt compound having an appropriate counterion and/or ion, and the salt compound may have high solubility in water, an aqueous solution, or an organic solvent. Among the salt compounds, those consisting of small counter negative ions such as F, Cl⁻, and Br⁻ tend to be dissolved well in water or aqueous solutions, while those consisting of large counter negative ions such as I⁻, hexafluorophosphate (PF₆⁻) and tetrafluoroborate (BF₄⁻) tend to be dissolved well in organic solvents. Examples of counter negative ions may be one or more selected from halides selected from the group consisting of F, Cl, Br and I, hexafluorophosphate, and tetrafluoroborate.

In one aspect, the transition metal complex may be a compound of the following Chemical Formula 4.

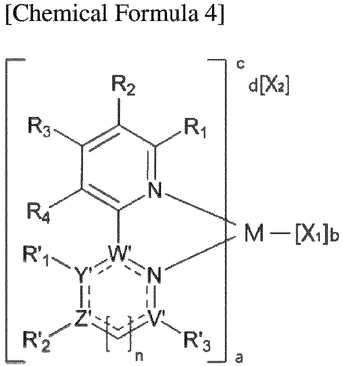

In Chemical Formula 4, R'₁, R'₂, and R'₃ may each independently be hydrogen, deuterium, a methyl group, or an ethyl group, but are not limited thereto.

Furthermore, in Chemical Formula 4, at least one of W', Y', Z', and V' may be nitrogen (N).

Further, at the same time, W' may be nitrogen (N) or carbon (C).

In addition, Y', Z', and V' may each independently be nitrogen (N), sulfur (S), oxygen (O), or carbon (C).

Furthermore, in Chemical Formula 4, n may be 0 (zero).

Further, in one aspect, when W' in Chemical Formula 4 is carbon, Y' may be sulfur (S), oxygen (O), or carbon (C).

In one aspect, the compound of Chemical Formula 4 may include a transition metal complex or a salt compound thereof, having phenyl-pyridine and/or a derivative thereof as an auxiliary ligand.

In one aspect, the transition metal complex or the salt compound thereof may exhibit a low level of oxidation reduction potential by including phenyl-pyridine as an auxiliary ligand. Specifically, when the transition metal complex is used as an electron transfer mediator in an electrochemical biosensor, the transition metal complex may exhibit a lower oxidation reduction potential than the oxidation reduction potential of a working electrode. Specifically, by introducing a phenyl-pyridine ligand, the oxidation-reduction potential may be lowered through carbon-metal bonds in the transition metal complex. In addition, the transition metal complex or the salt compound thereof may be expected to have a stabilizing effect due to chelation through carbon-metal bonds, and has a fast self-exchange rate due to small structural changes caused by oxidation-reduction (small size difference between oxidized species and reduced species).

In one aspect, the compound of Chemical Formula 4 may be selected from the group consisting of compounds of the following Chemical Formulae 5 to 274.

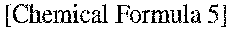

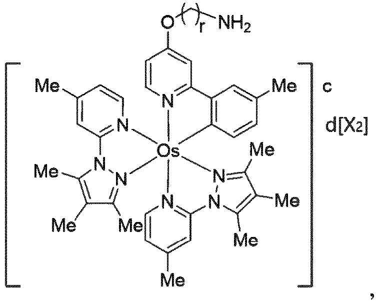

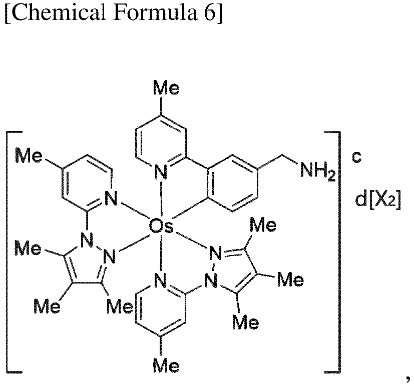

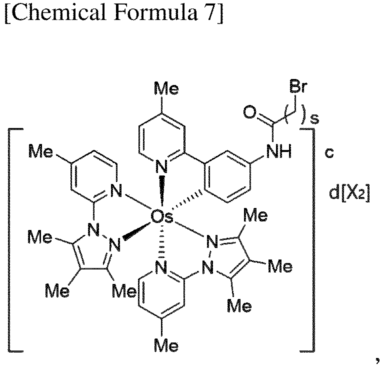

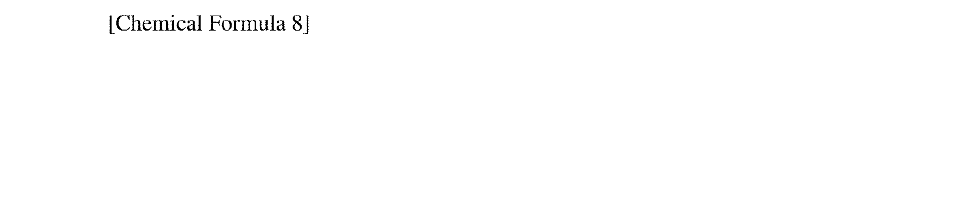

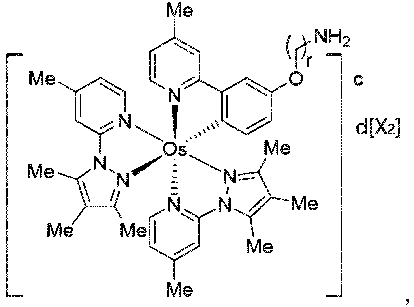

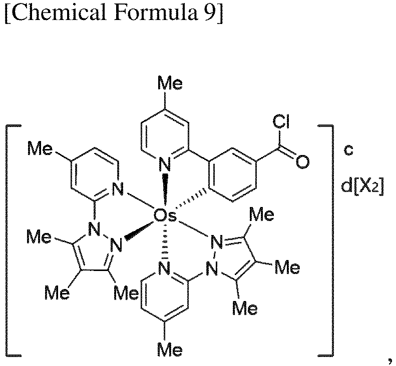

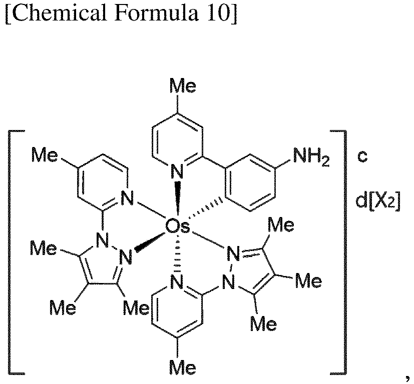

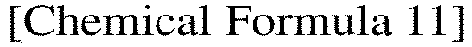

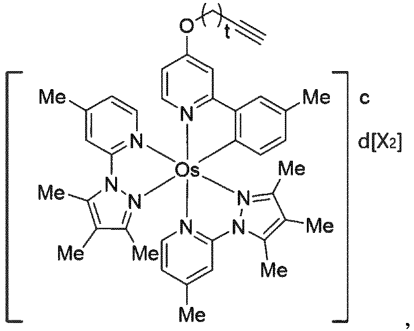

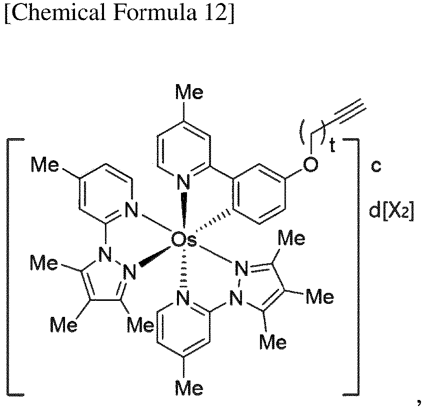

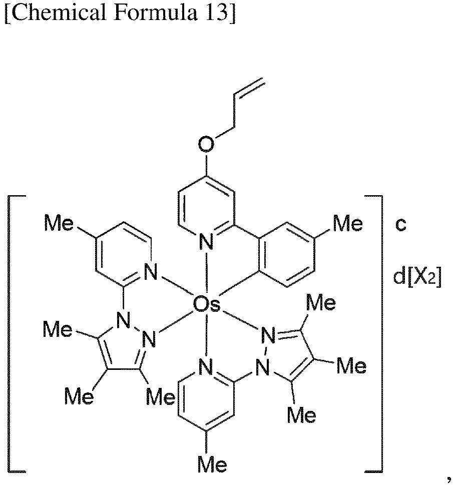

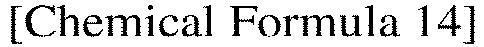

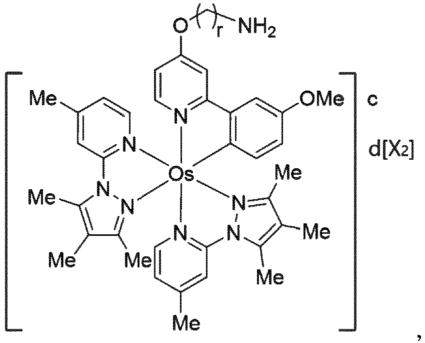

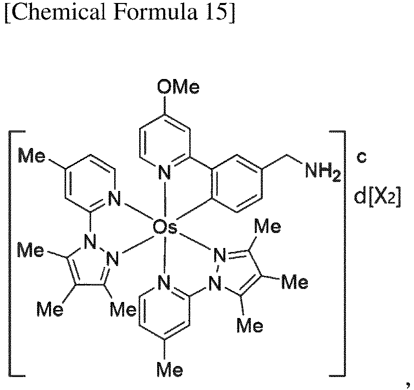

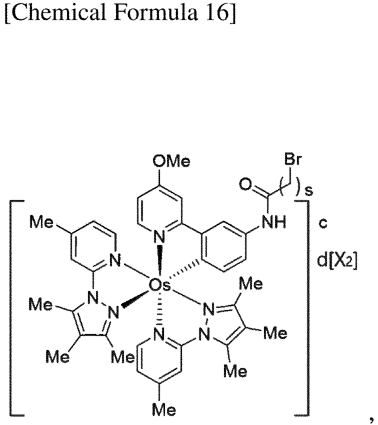

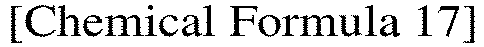

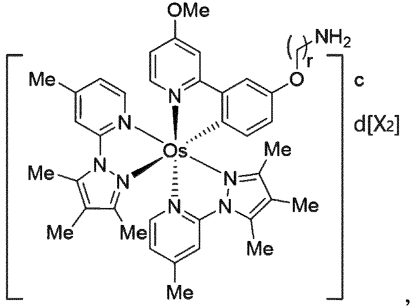

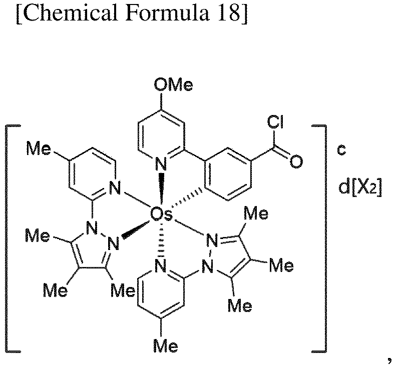

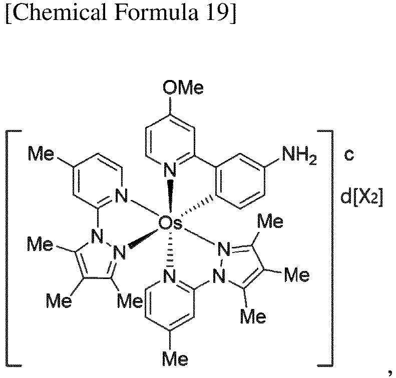

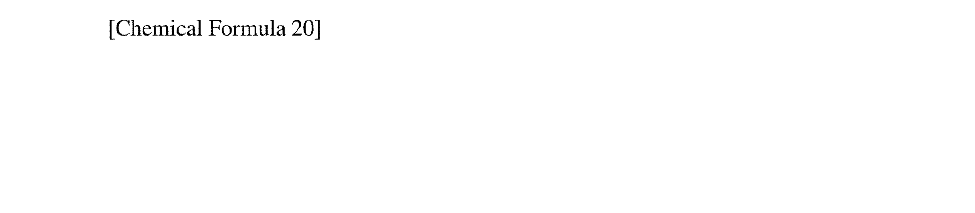

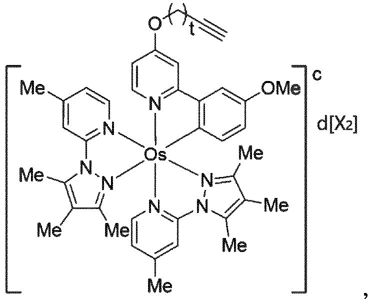

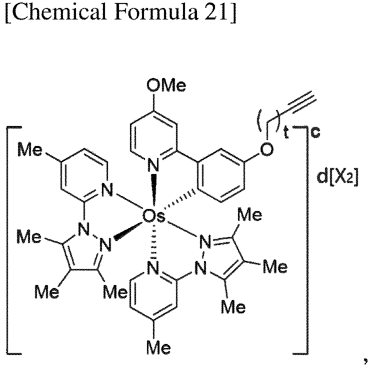

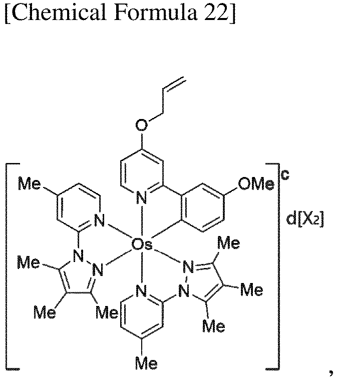

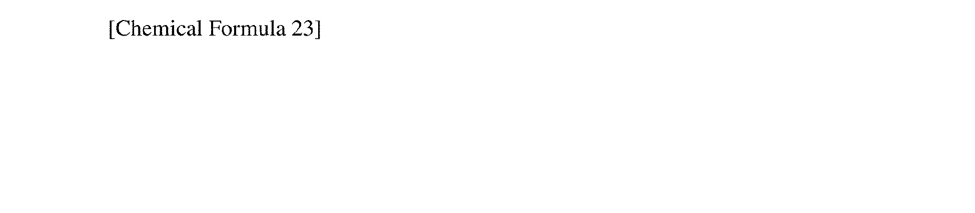

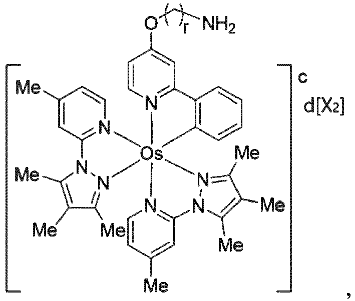

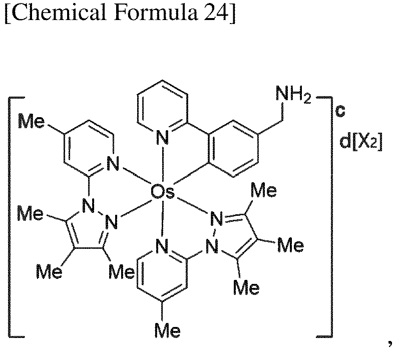

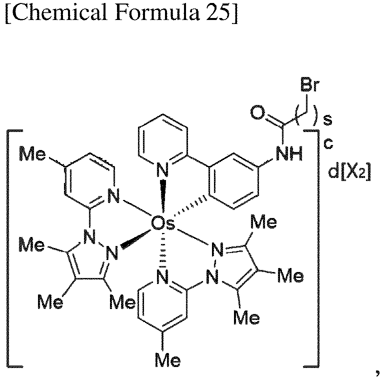

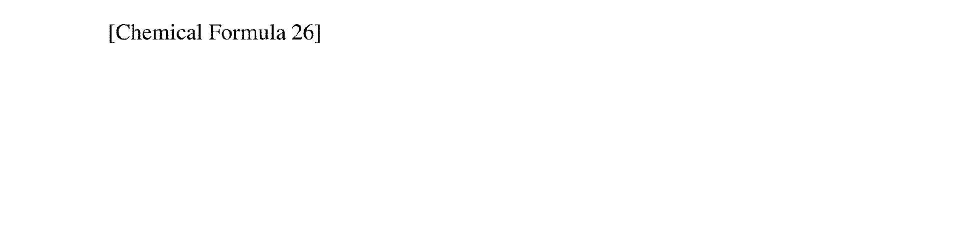

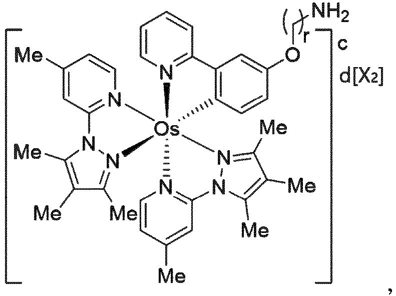

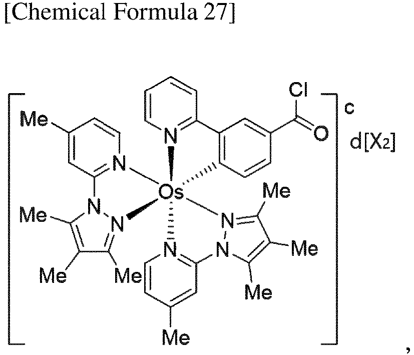

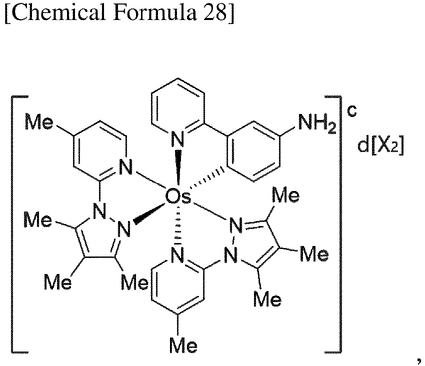

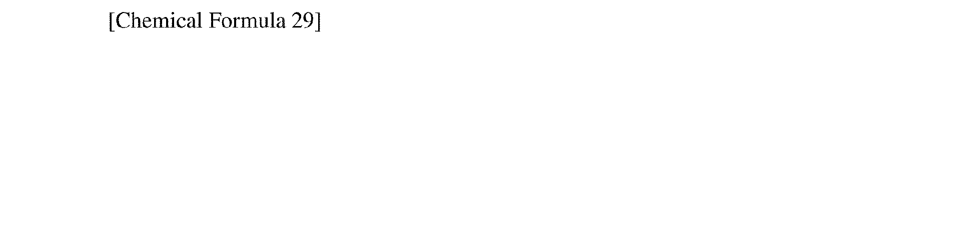

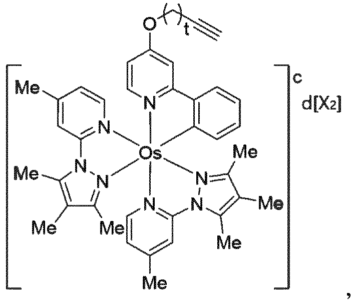

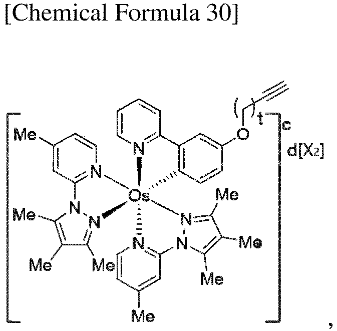

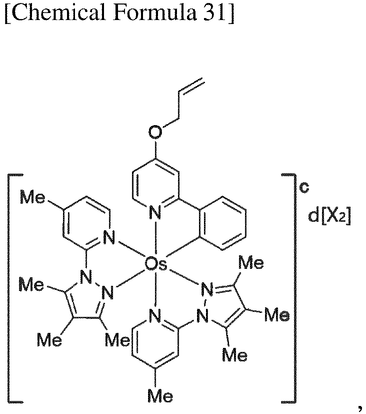

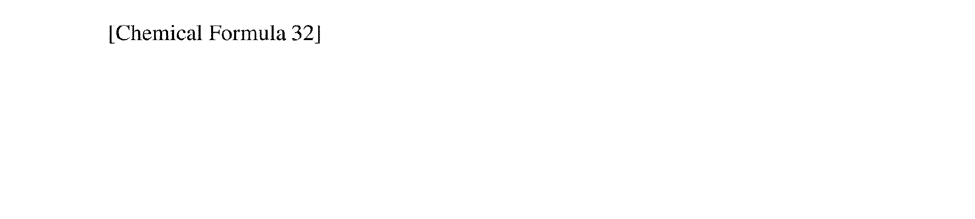

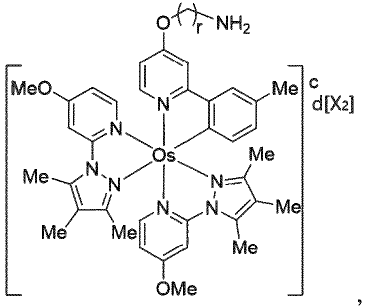

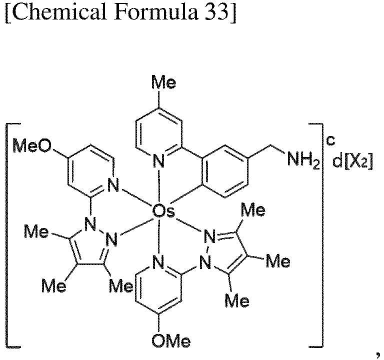

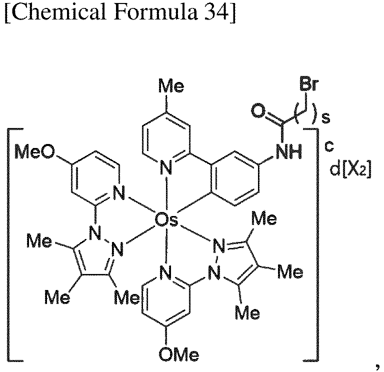

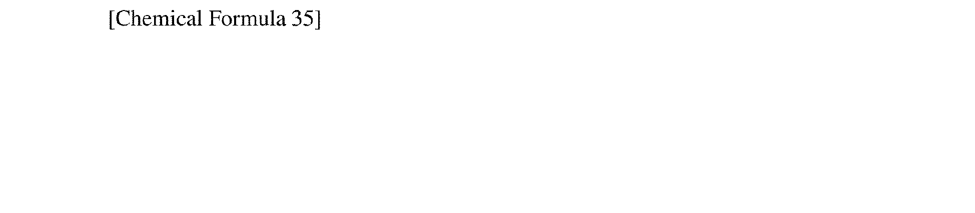

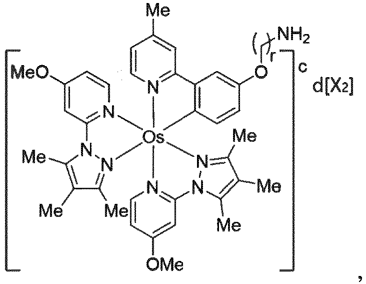

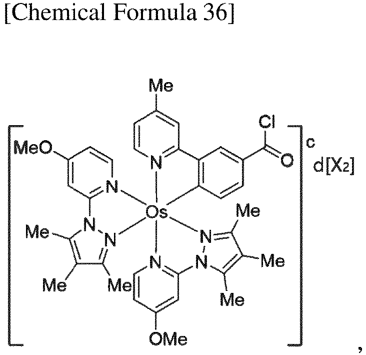

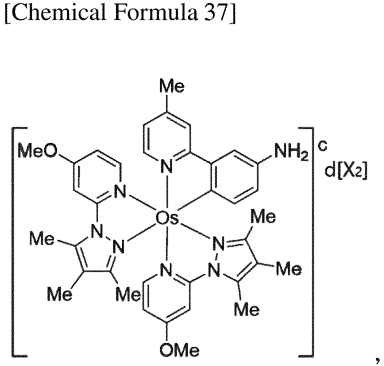

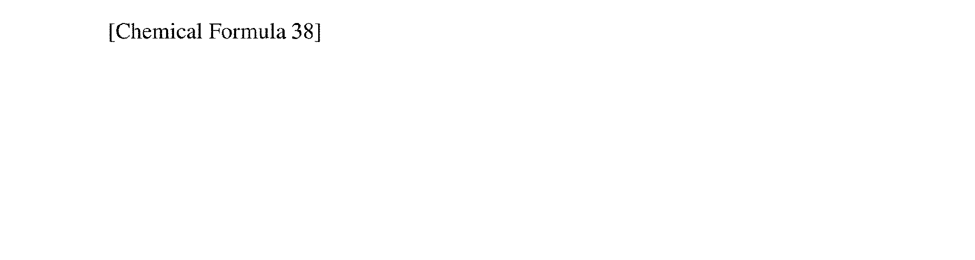

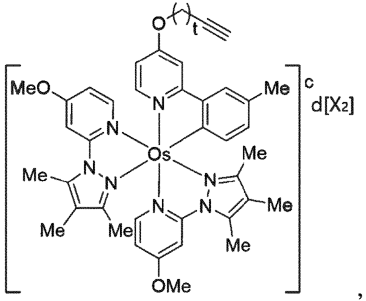

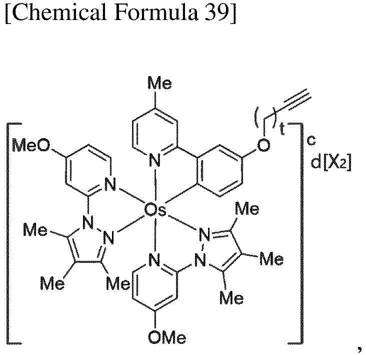

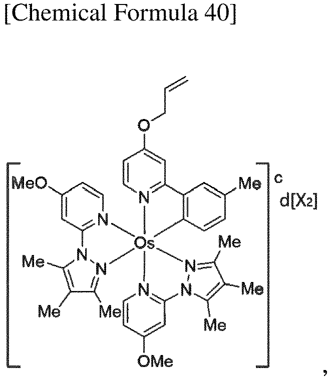

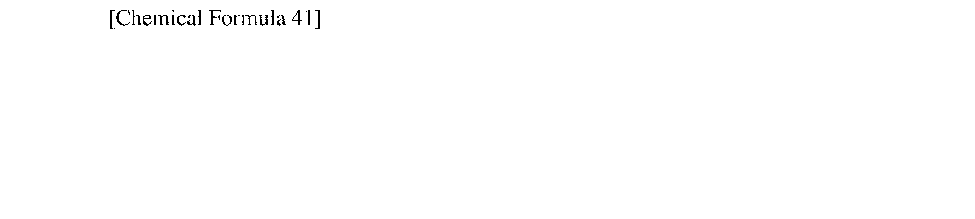

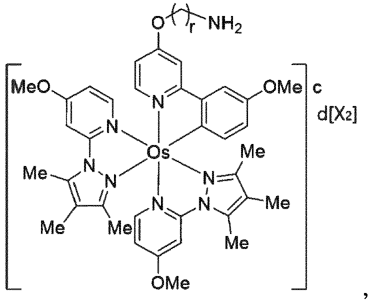

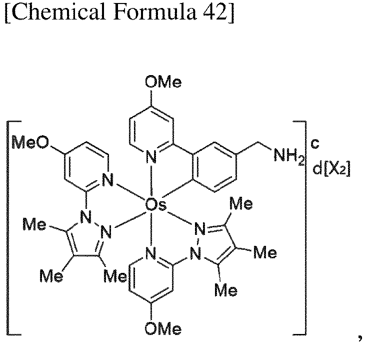

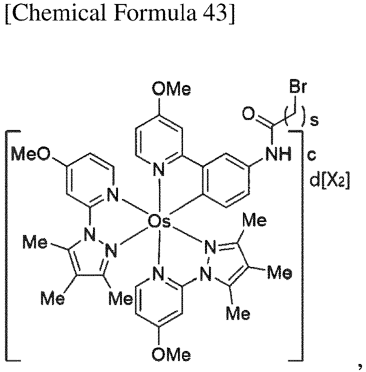

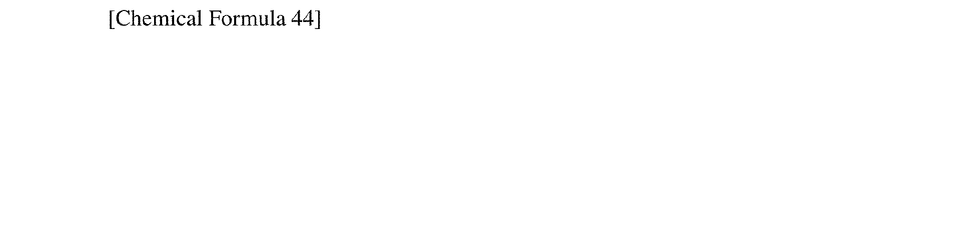

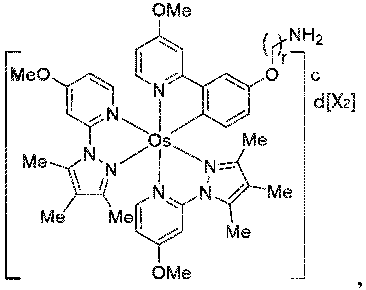

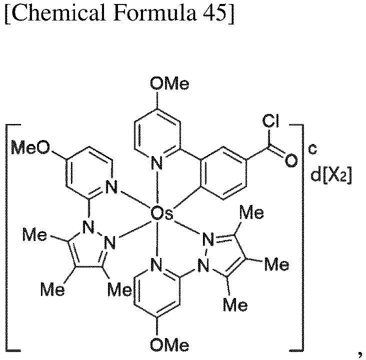

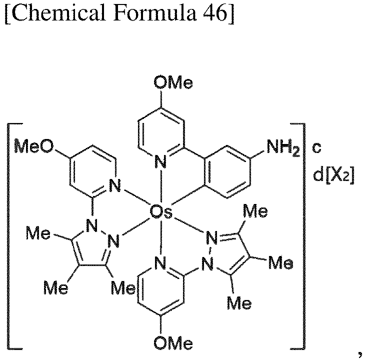

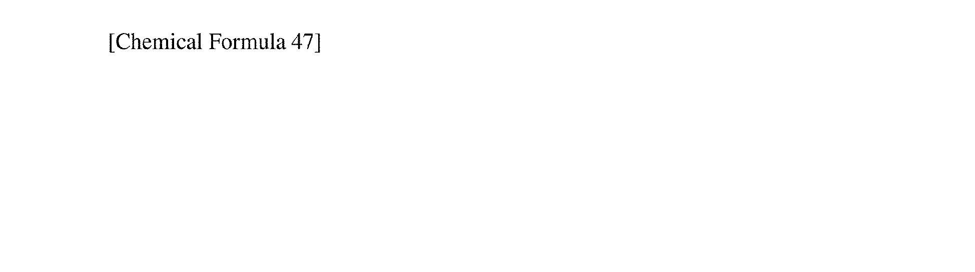

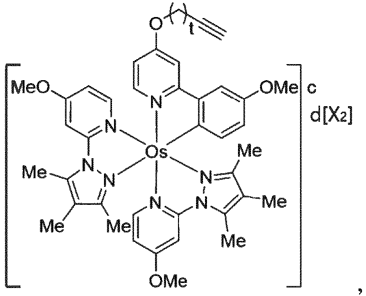

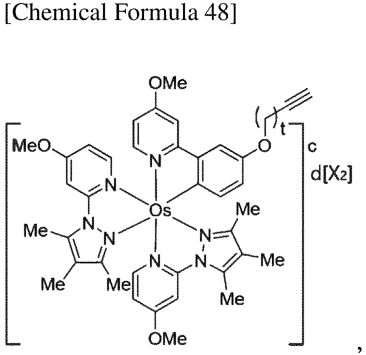

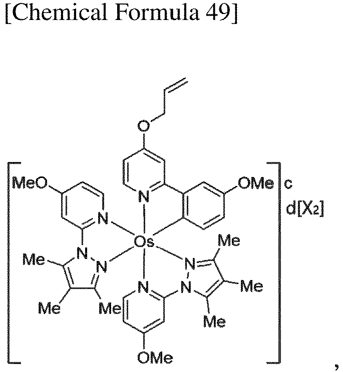

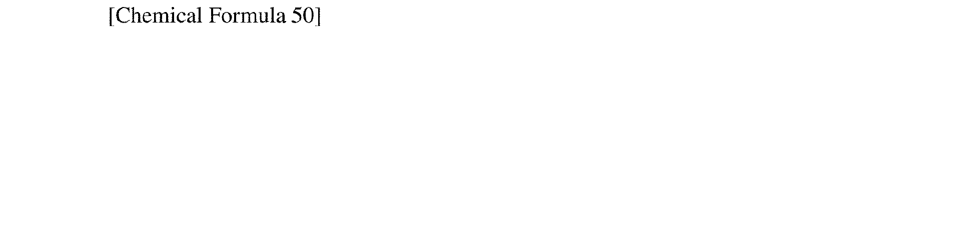

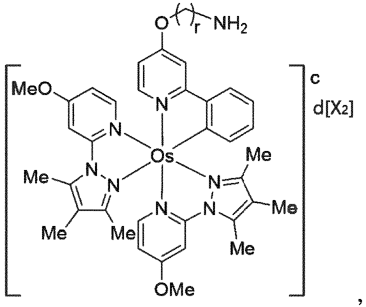

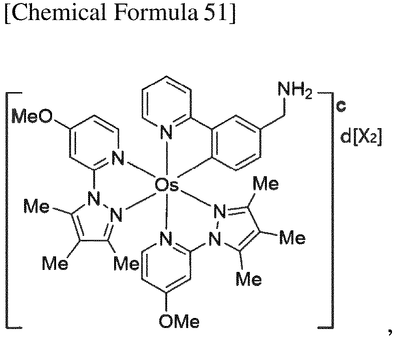

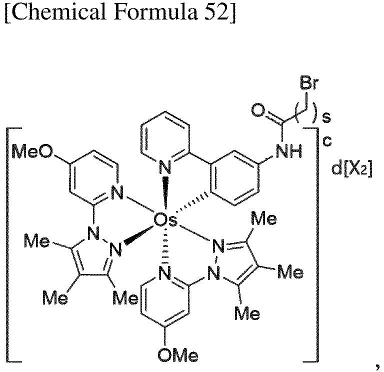

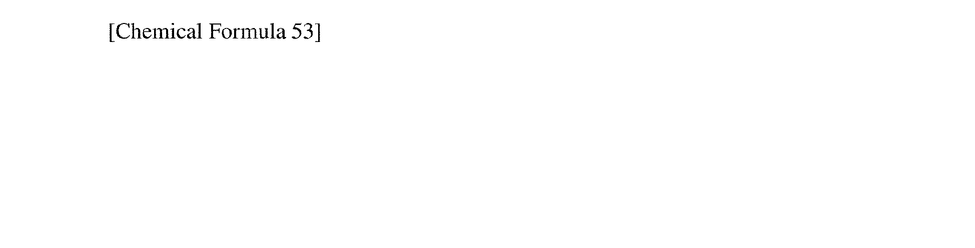

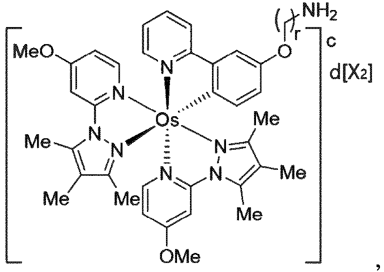

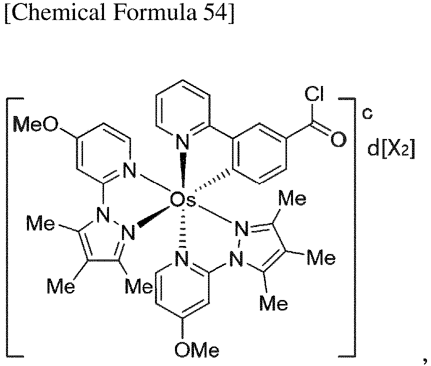

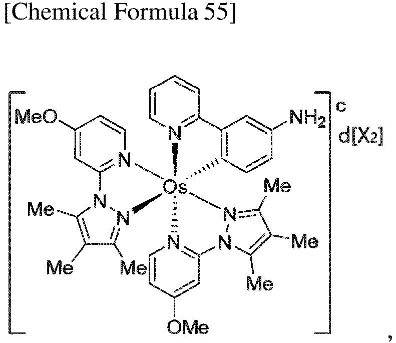

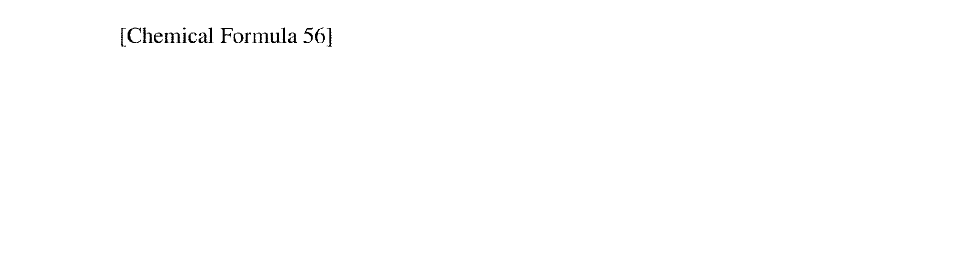

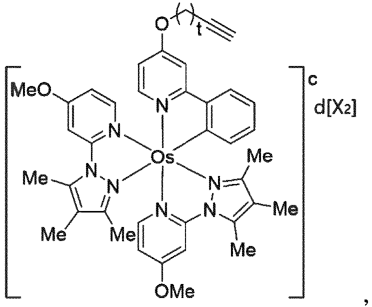

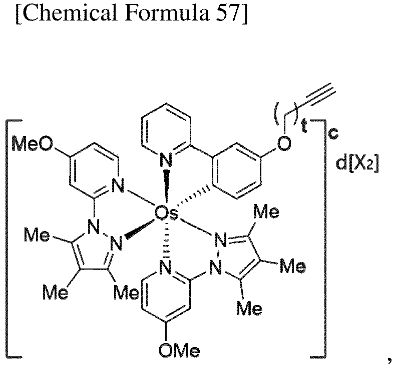

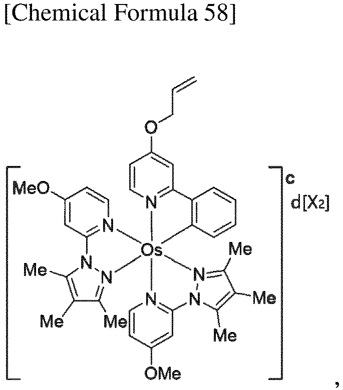

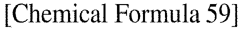

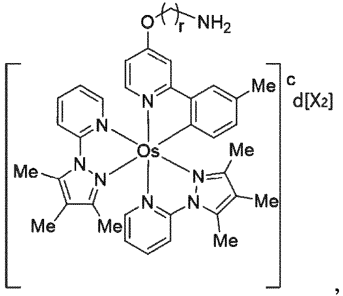

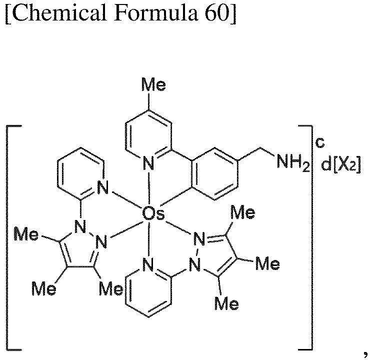

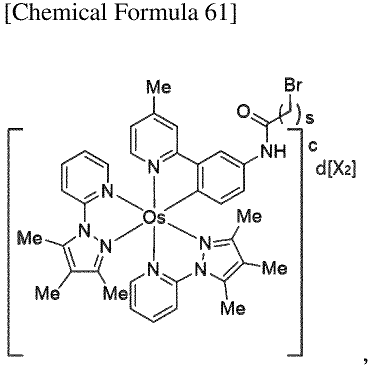

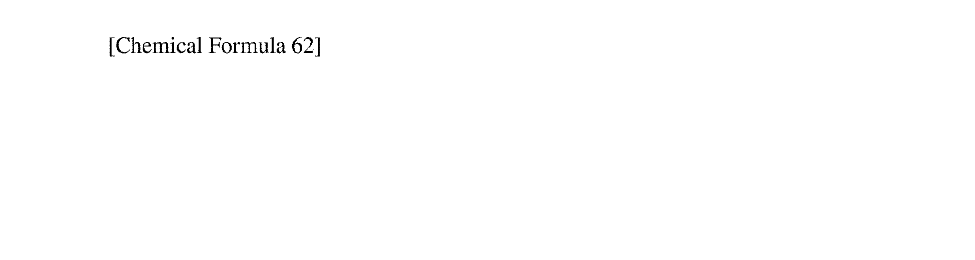

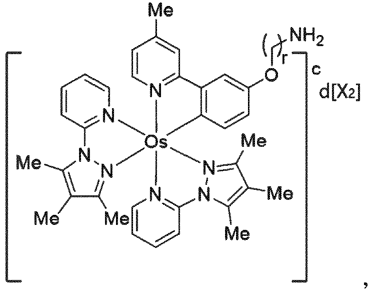

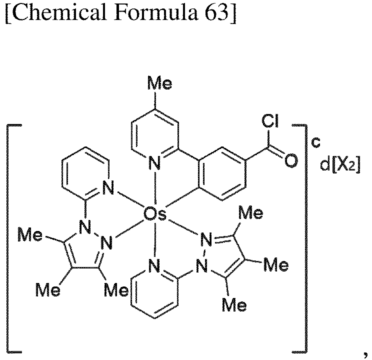

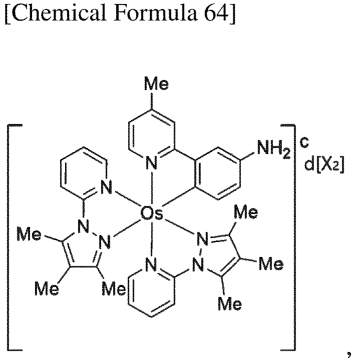

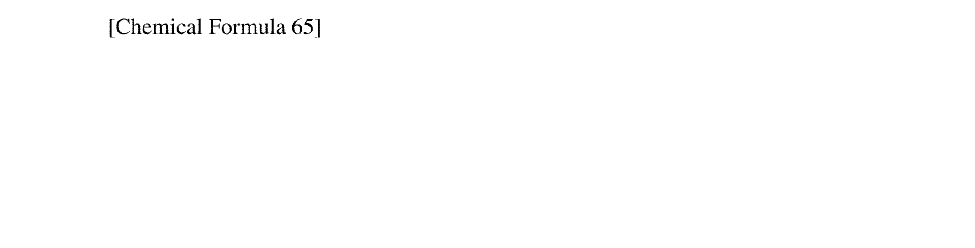

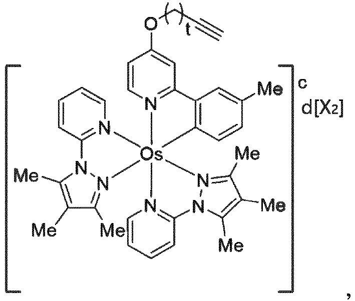

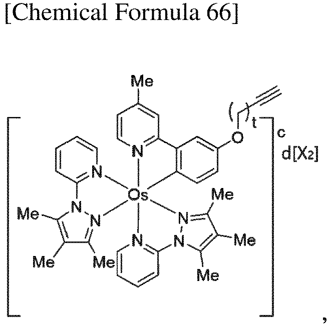

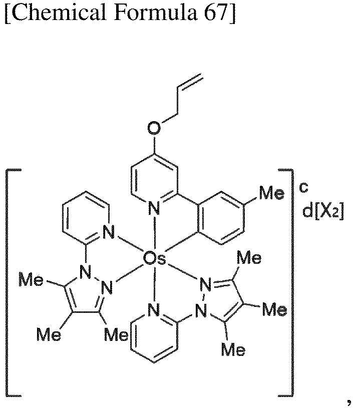

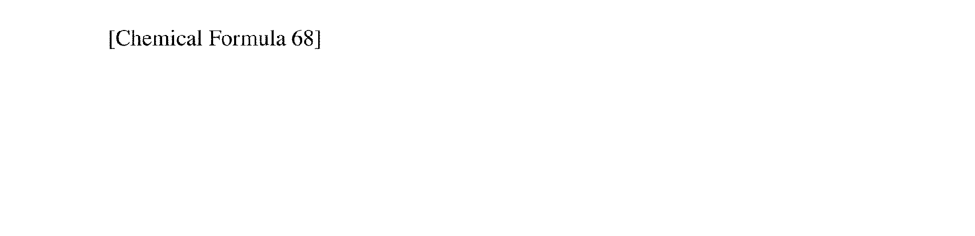

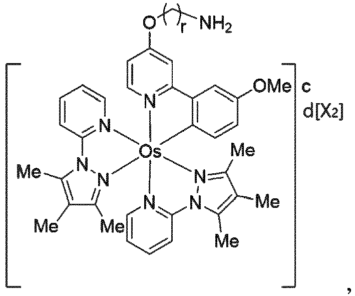

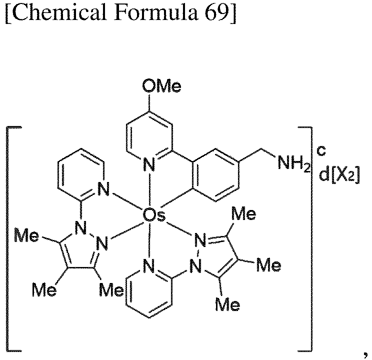

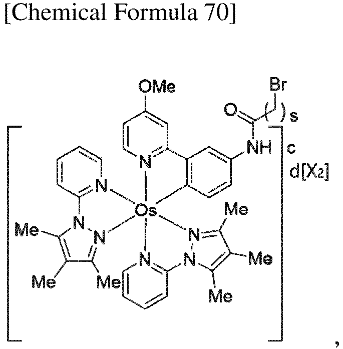

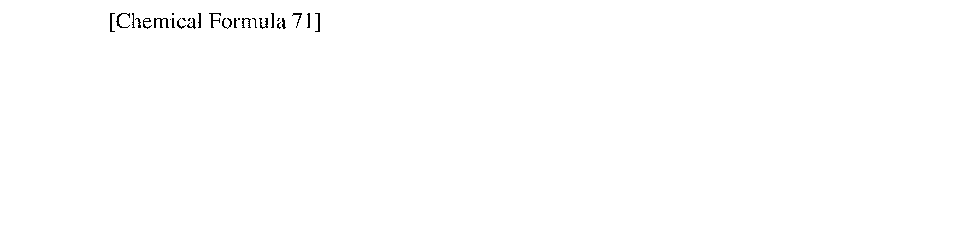

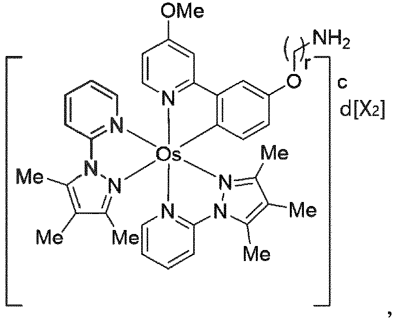

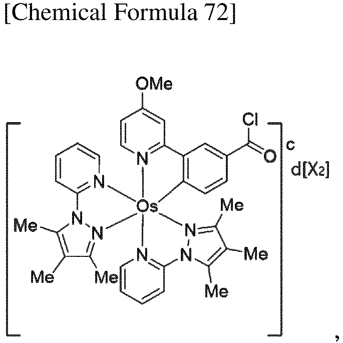

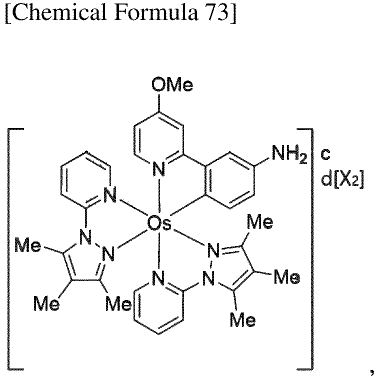

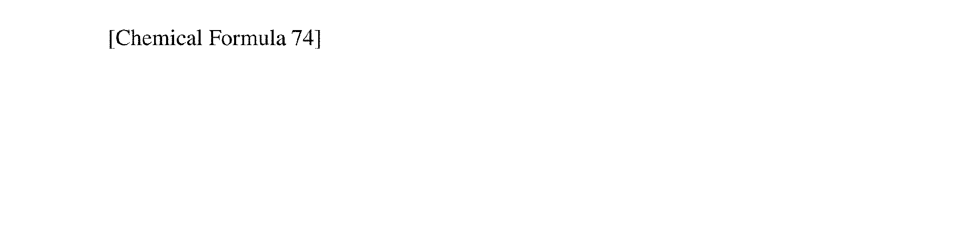

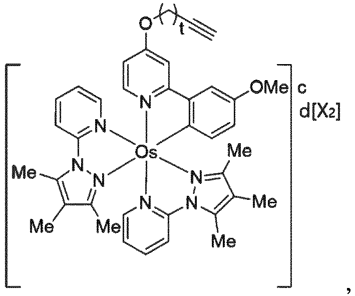

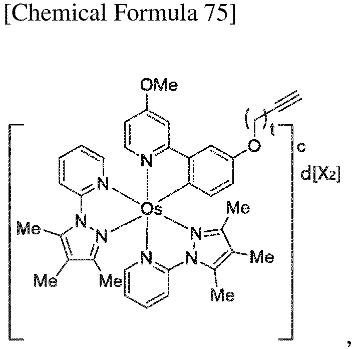

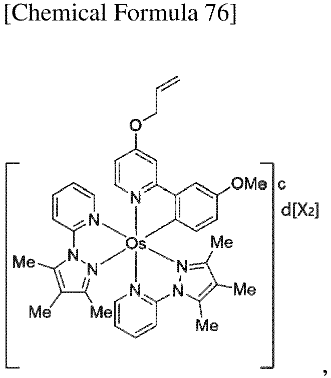

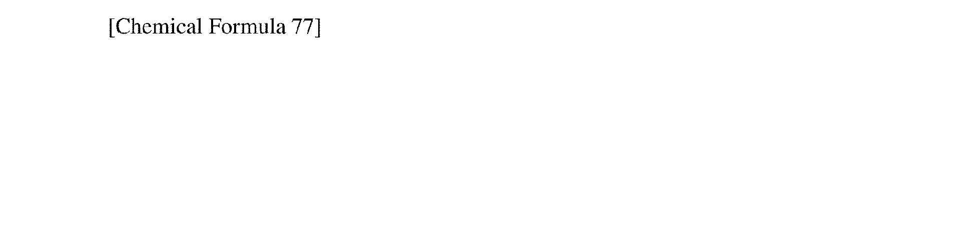

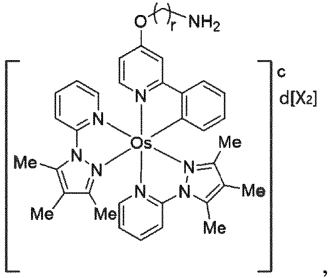

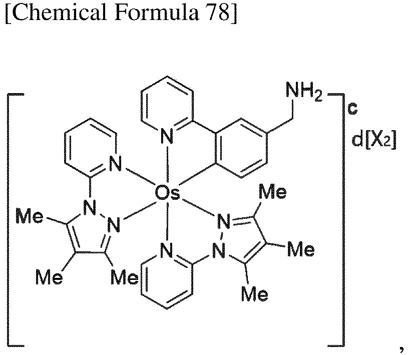

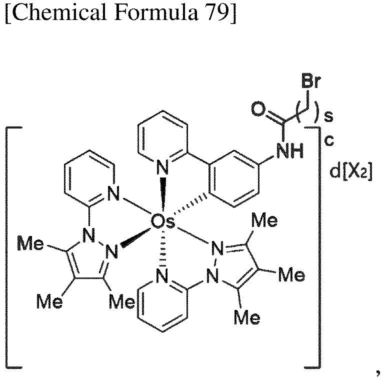

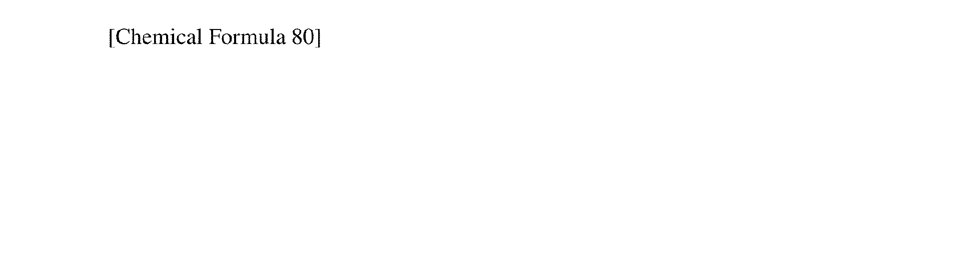

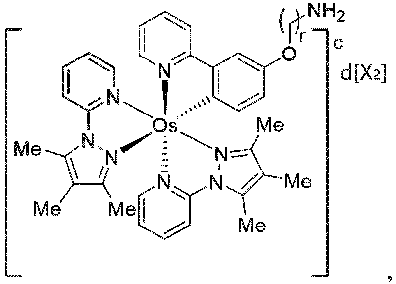

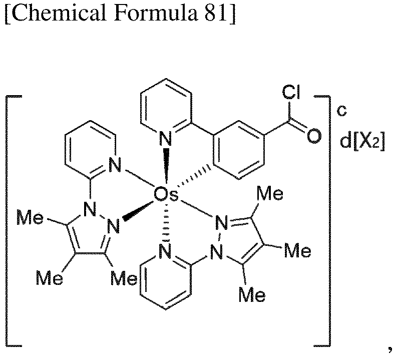

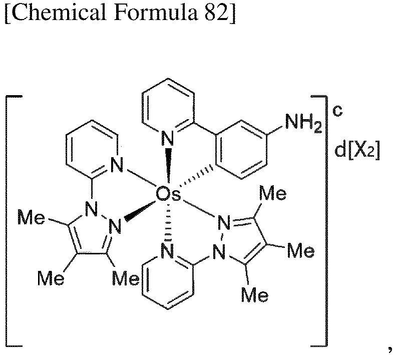

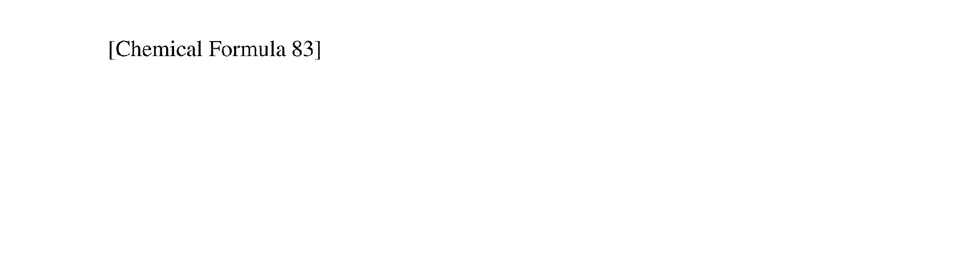

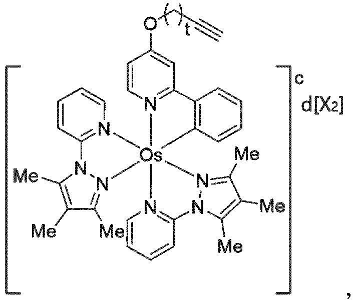

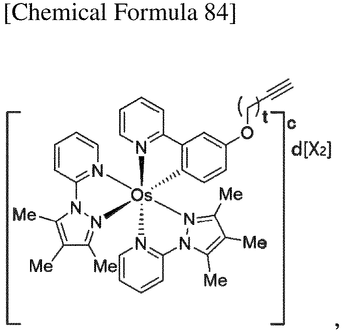

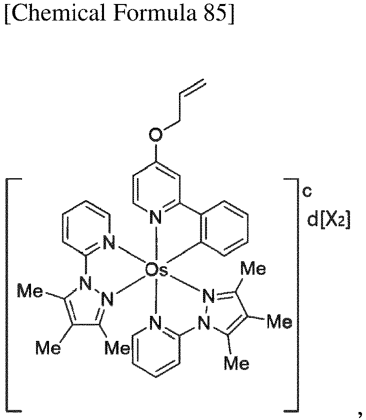

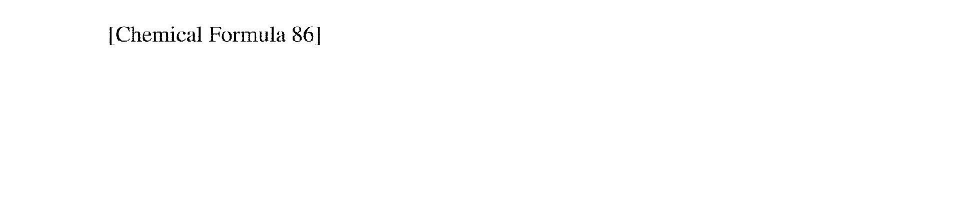

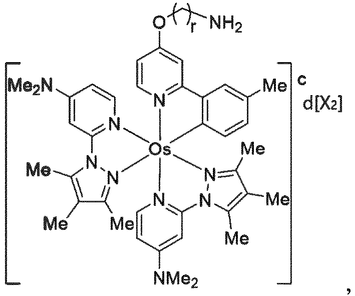

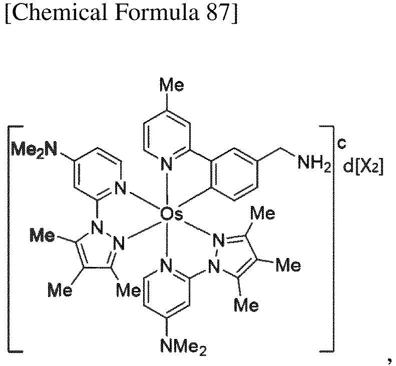

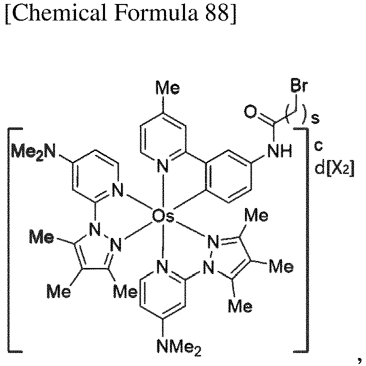

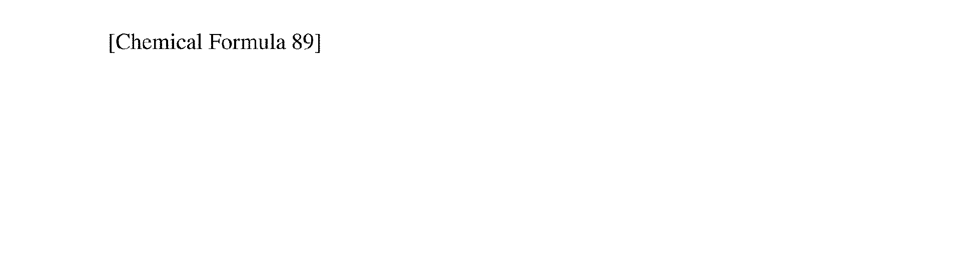

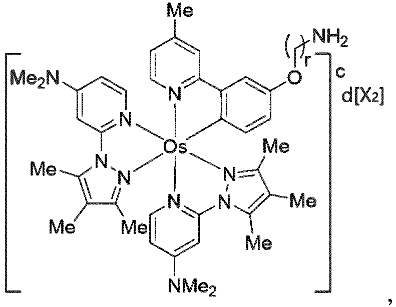

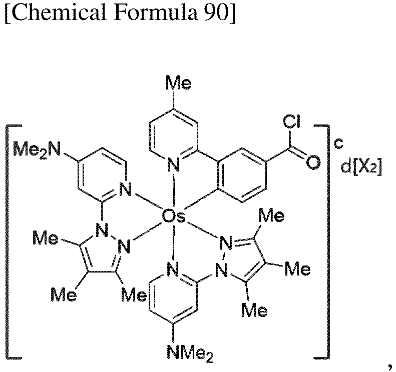

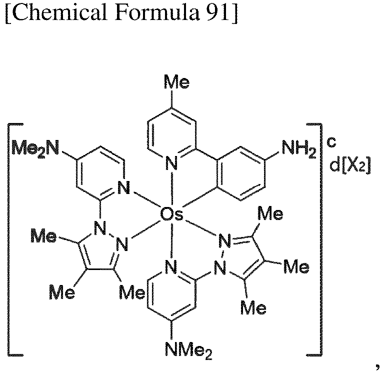

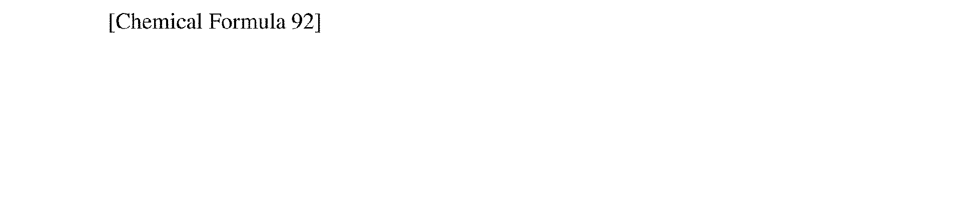

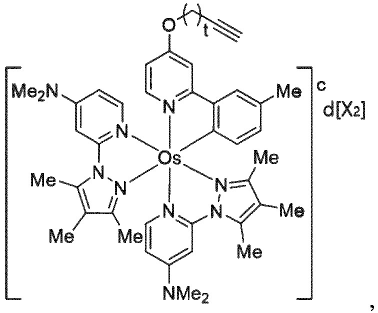

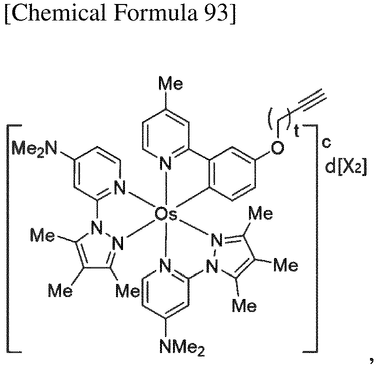

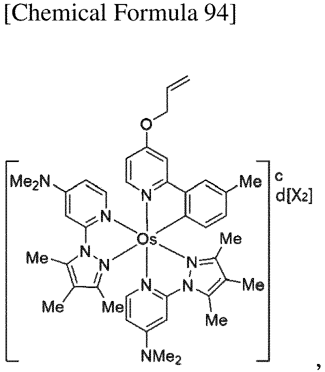

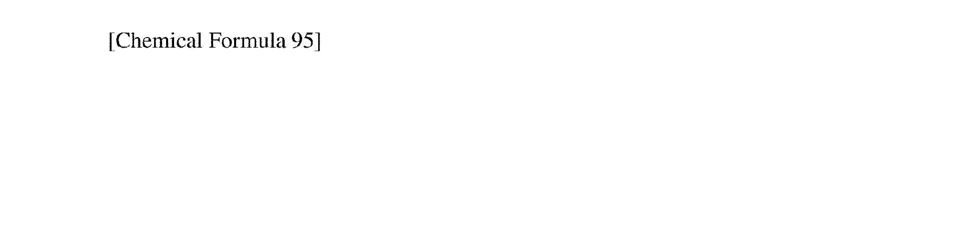

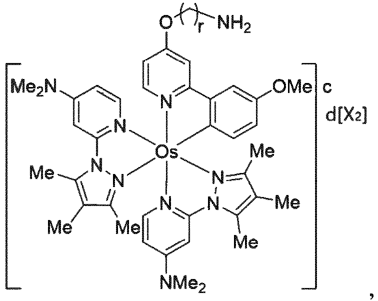

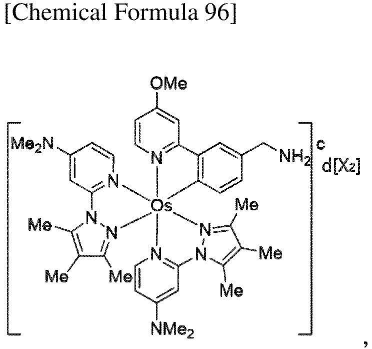

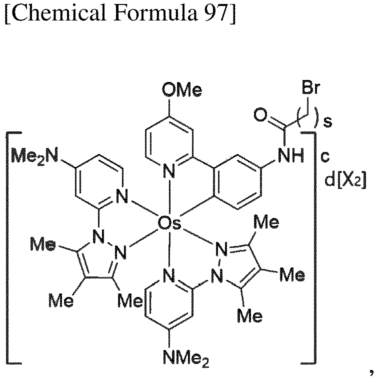

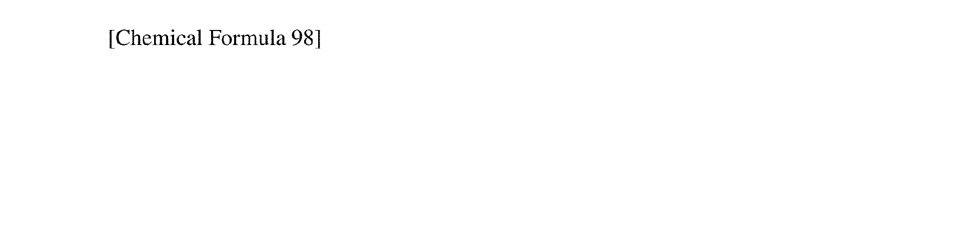

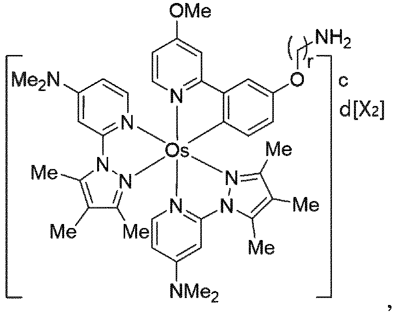

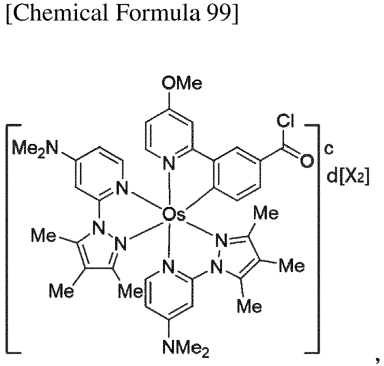

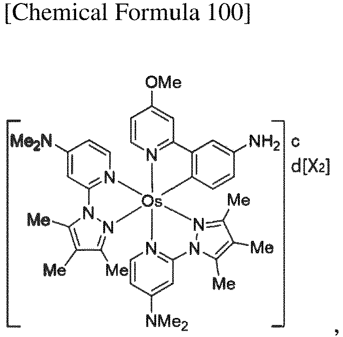

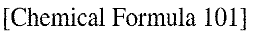

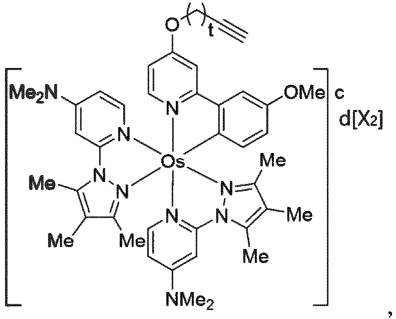

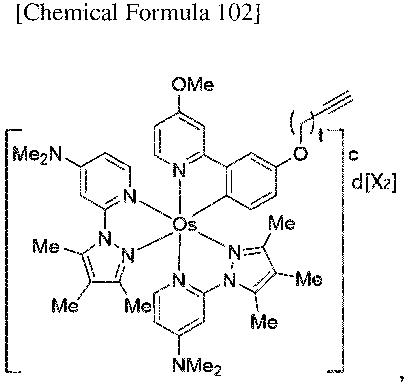

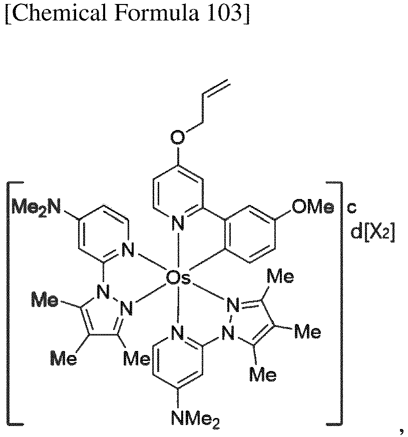

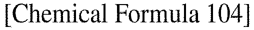

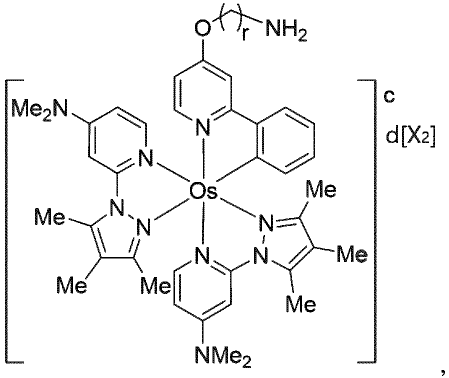

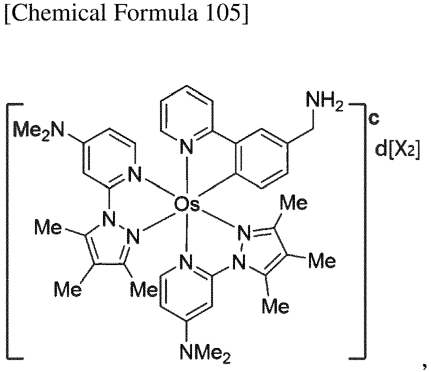

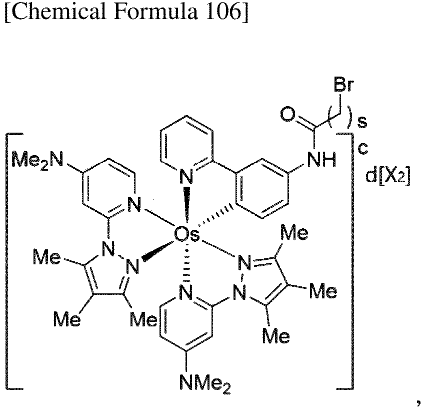

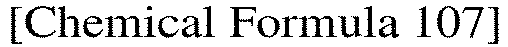

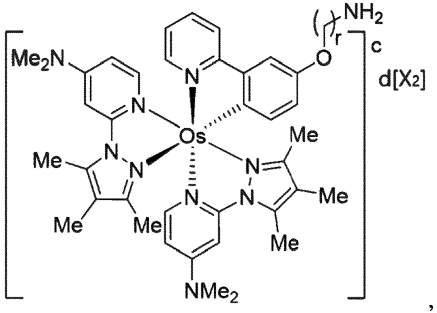

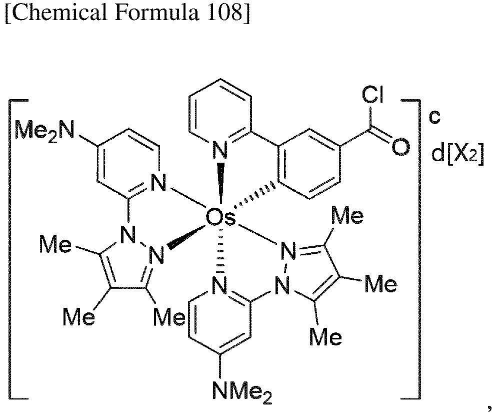

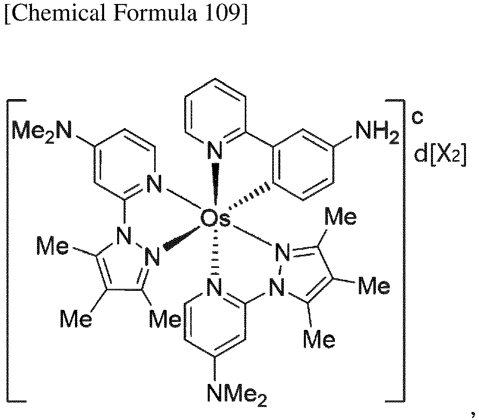

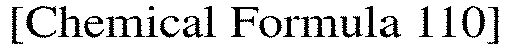

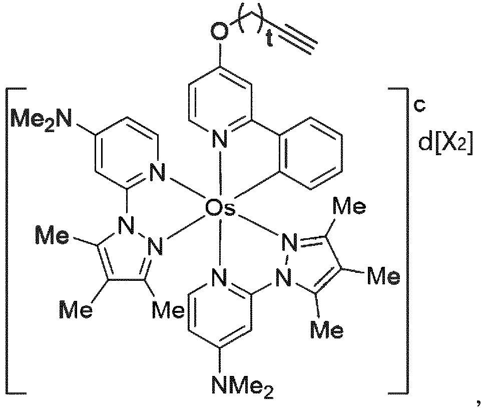

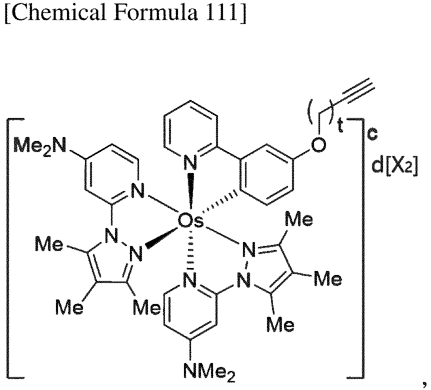

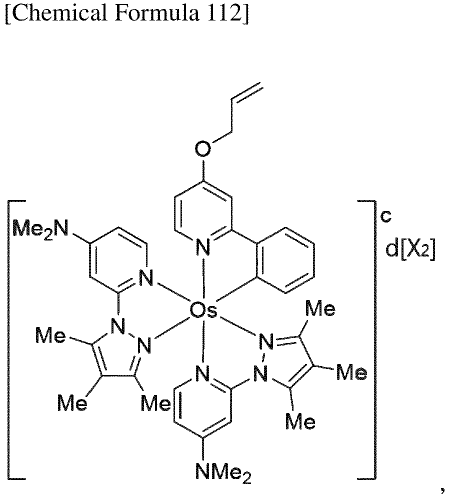

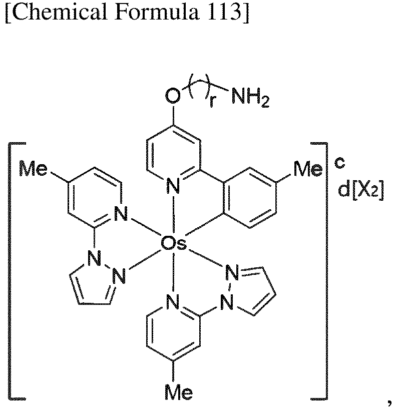

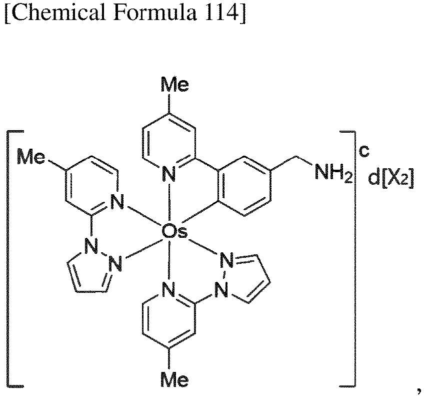

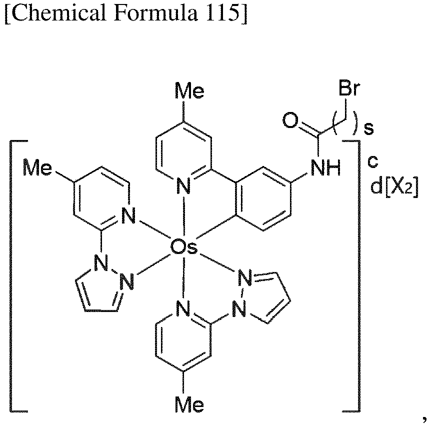

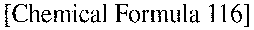

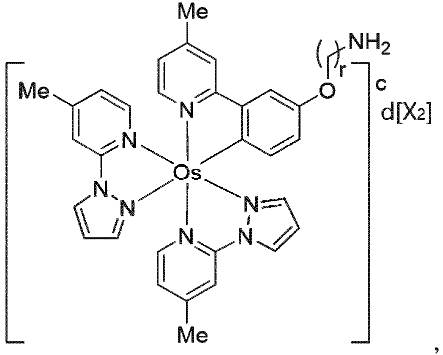

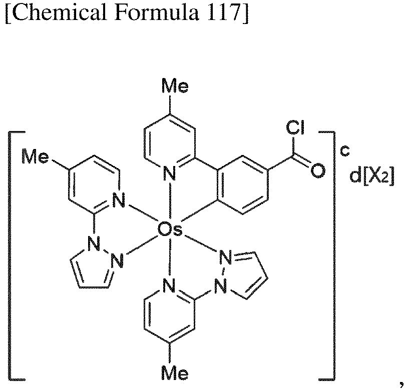

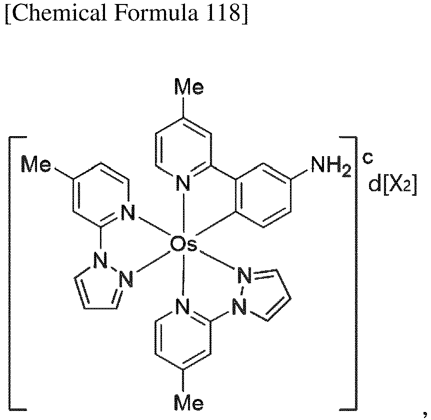

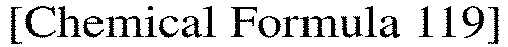

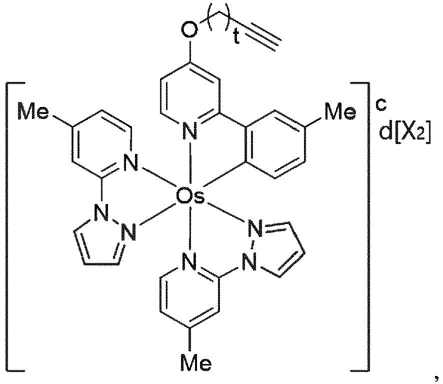

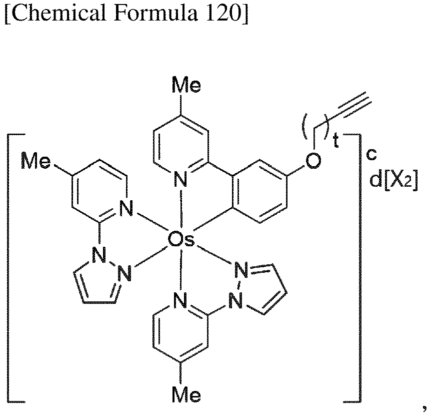

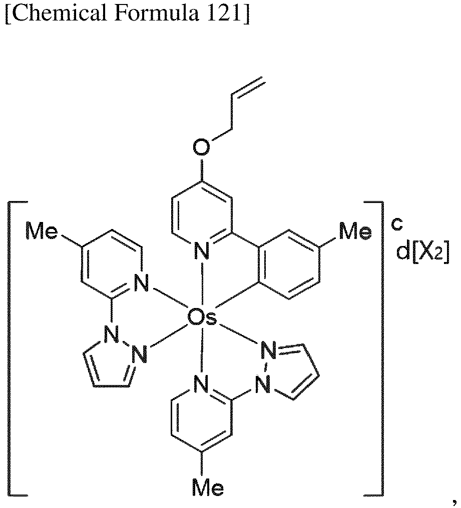

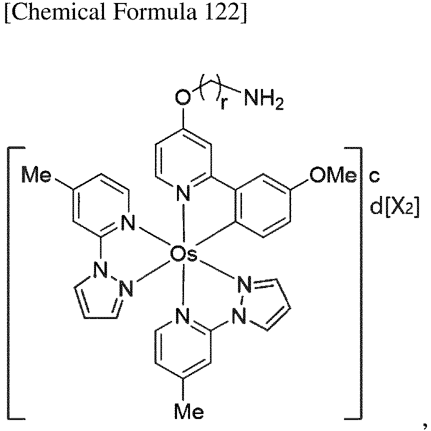

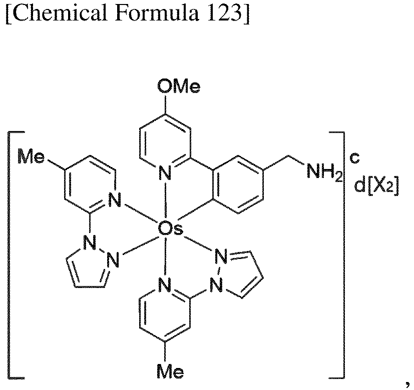

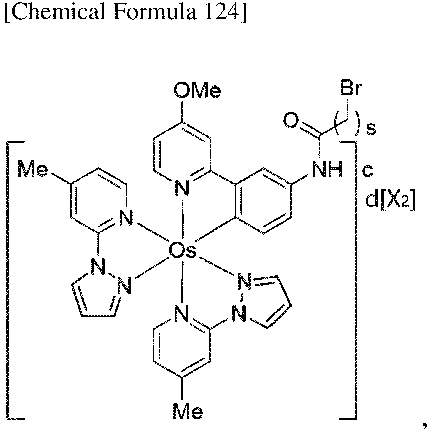

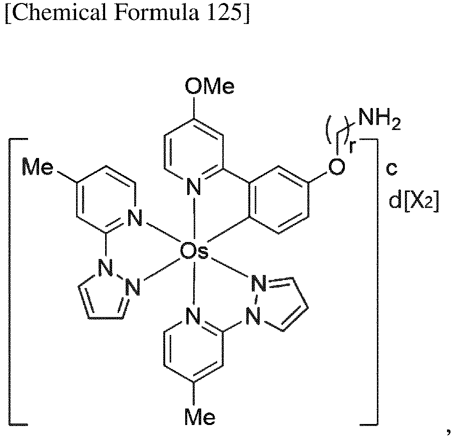

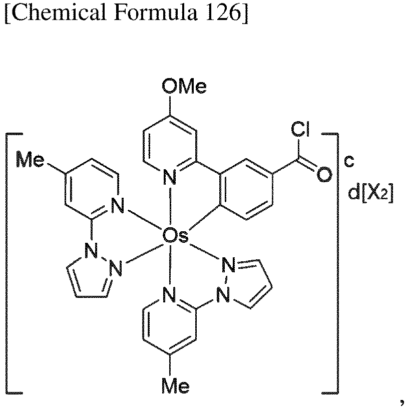

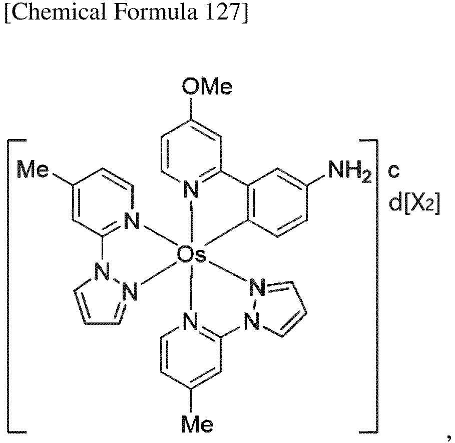

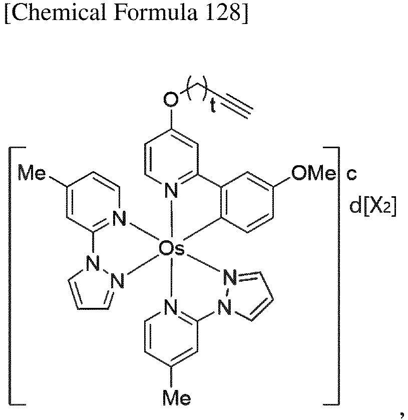

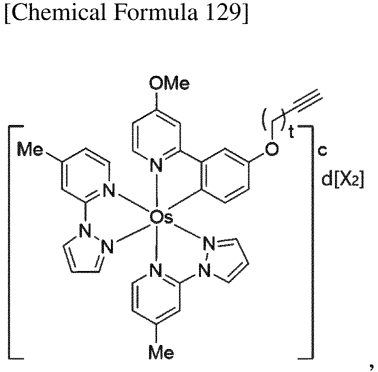

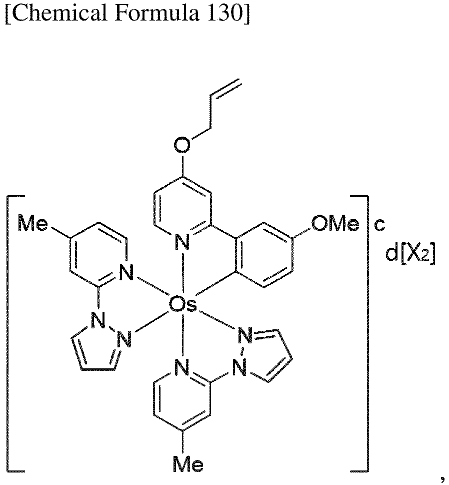

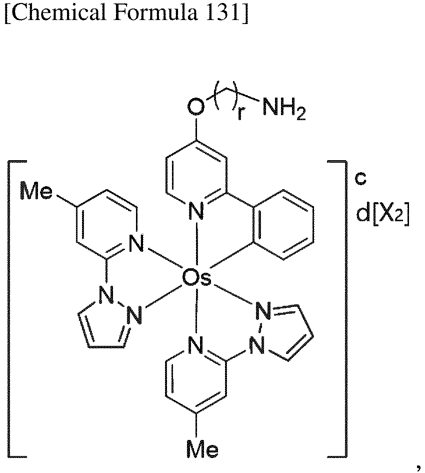

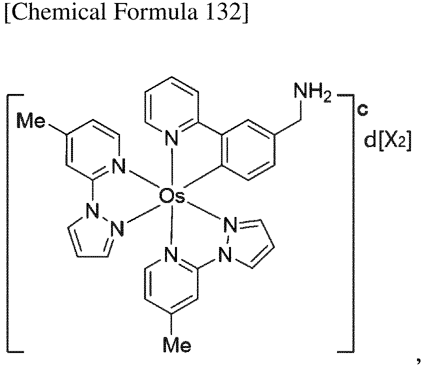

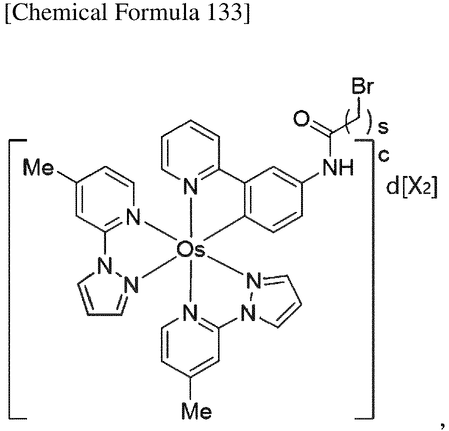

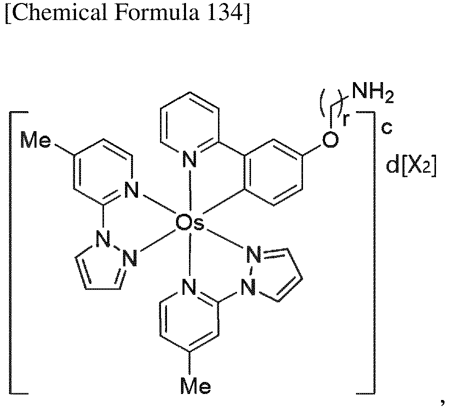

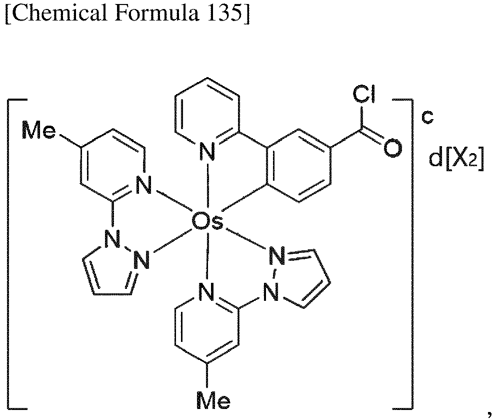

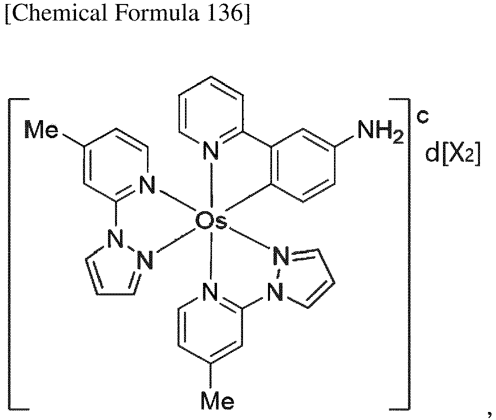

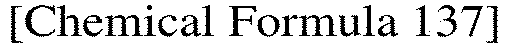

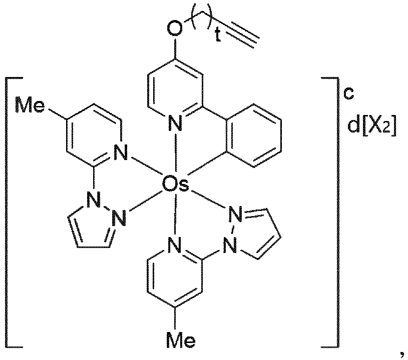

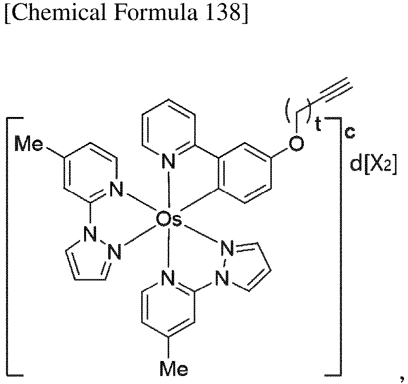

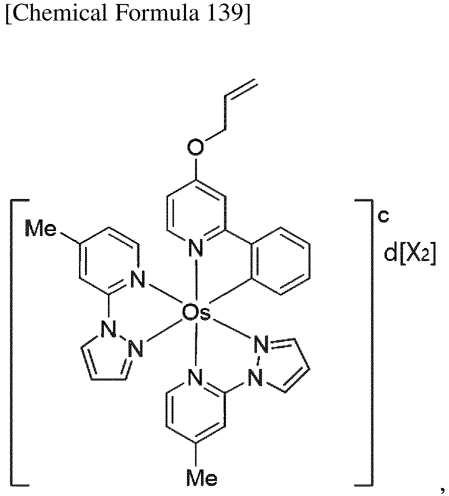

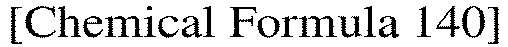

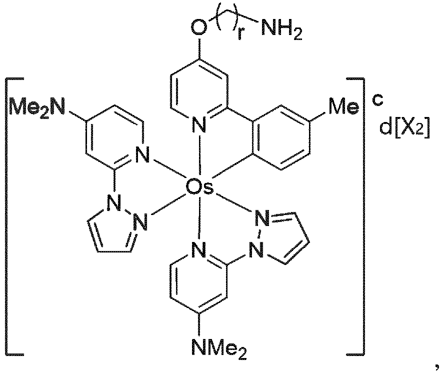

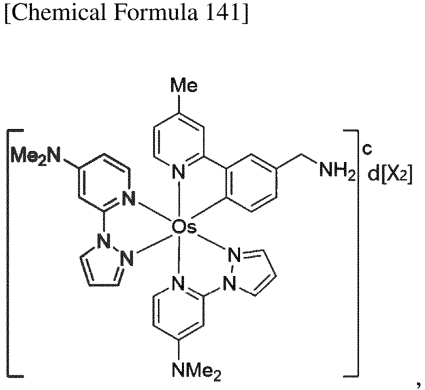

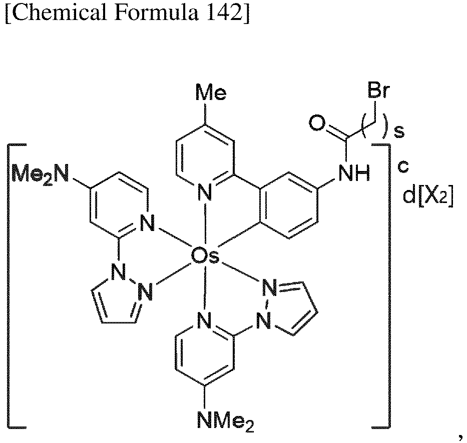

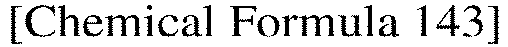

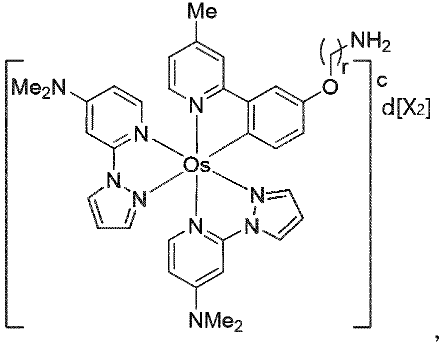

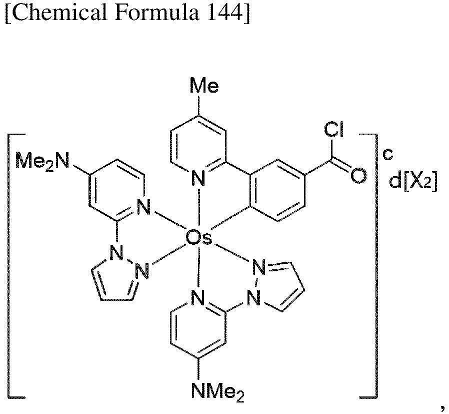

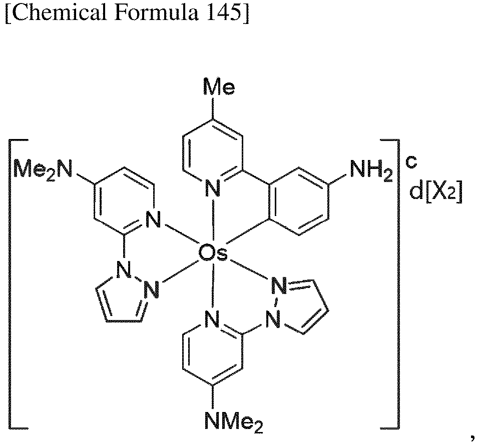

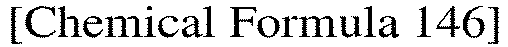

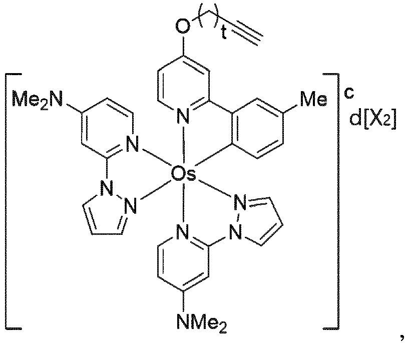

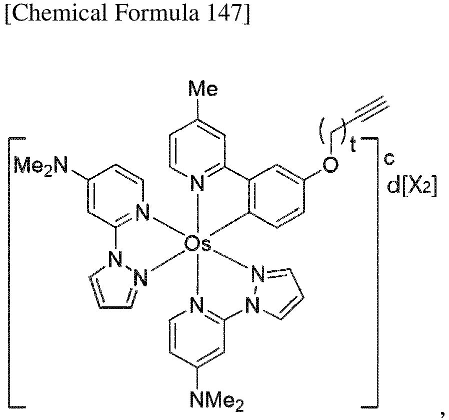

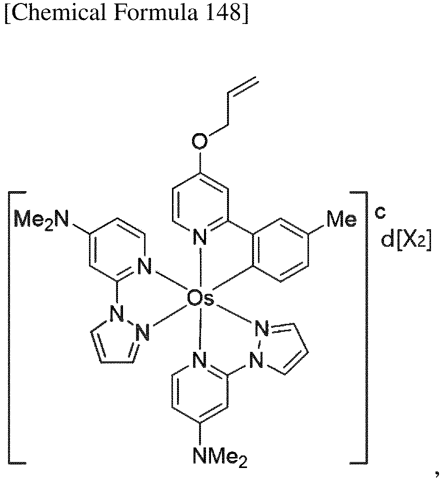

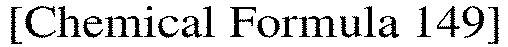

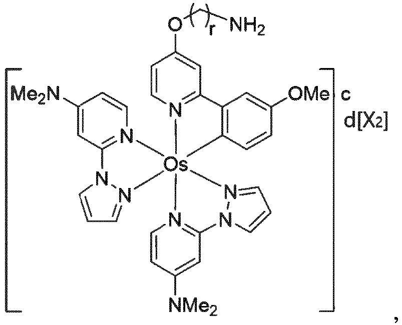

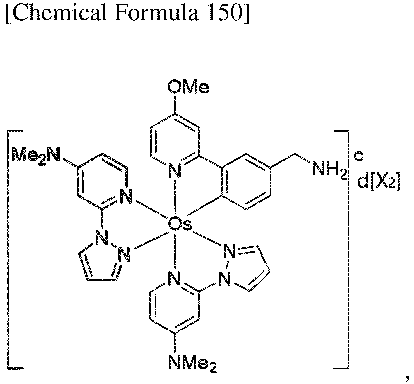

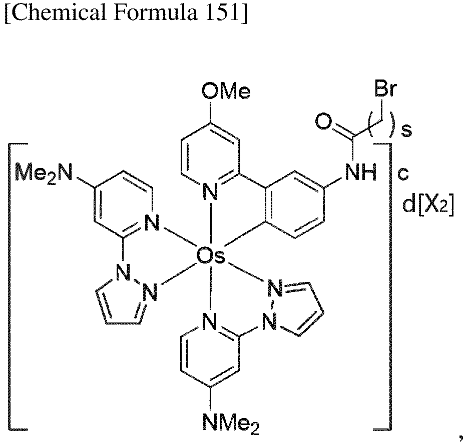

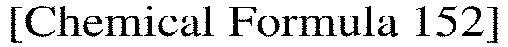

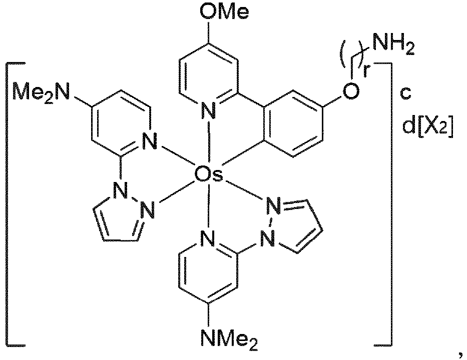

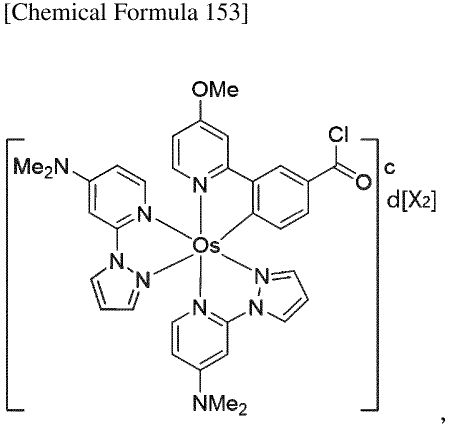

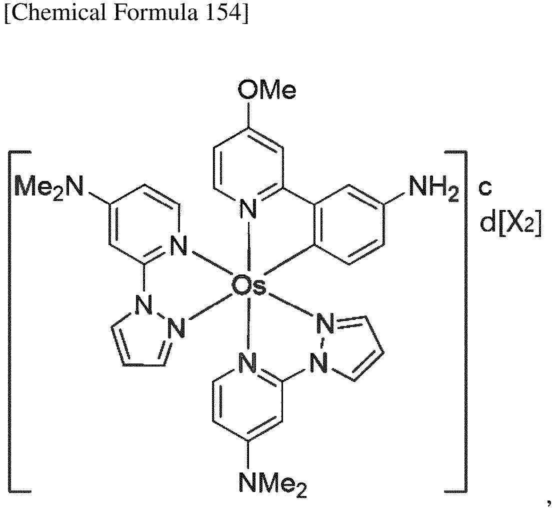

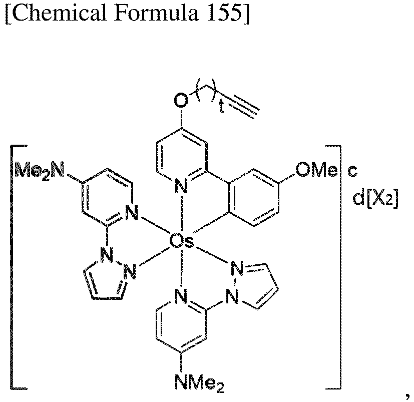

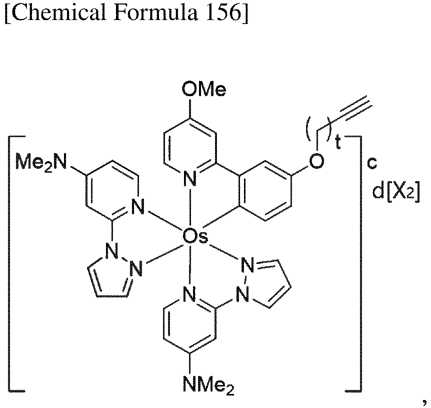

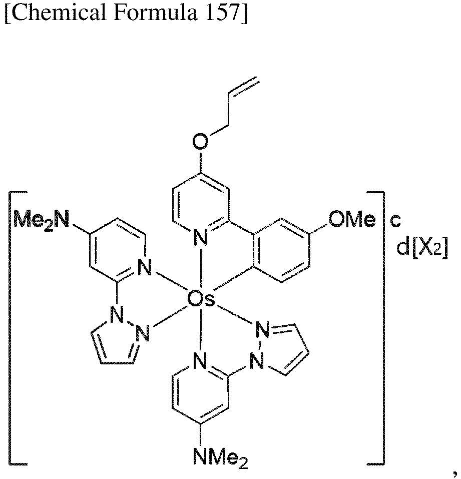

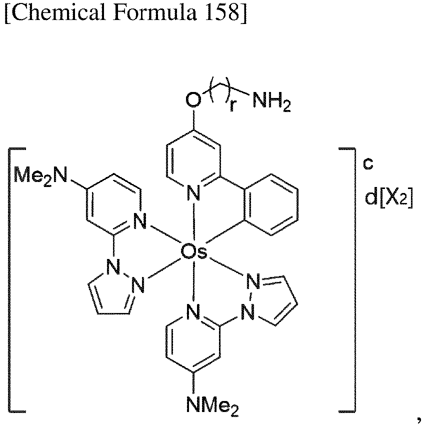

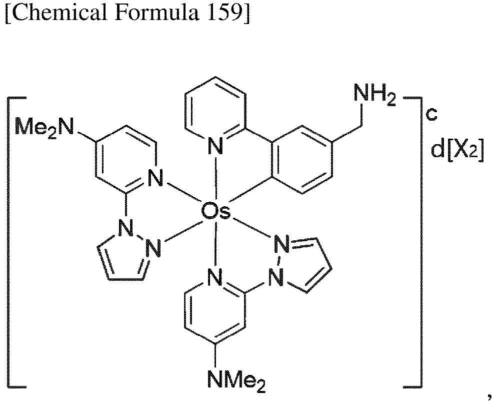

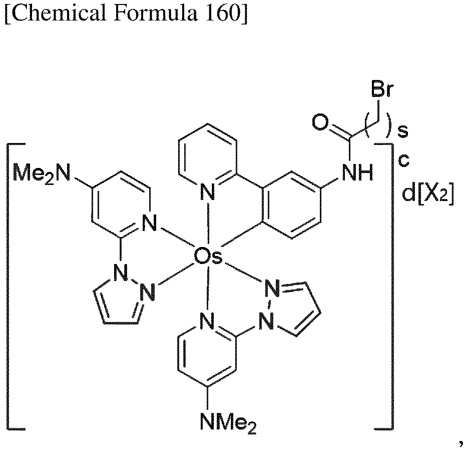

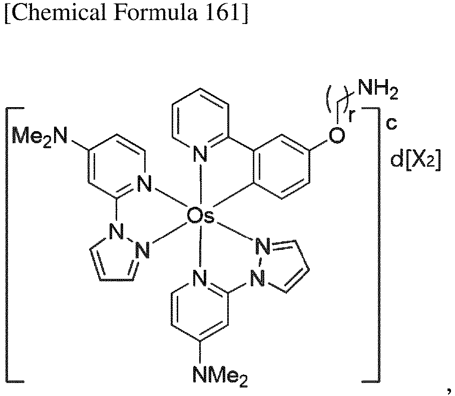

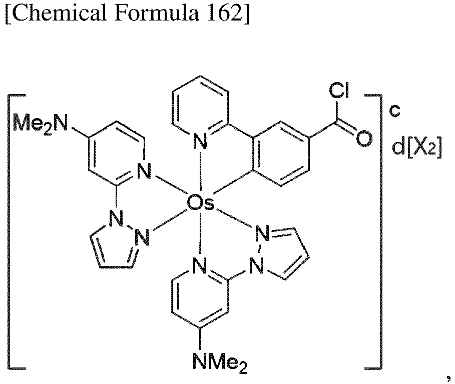

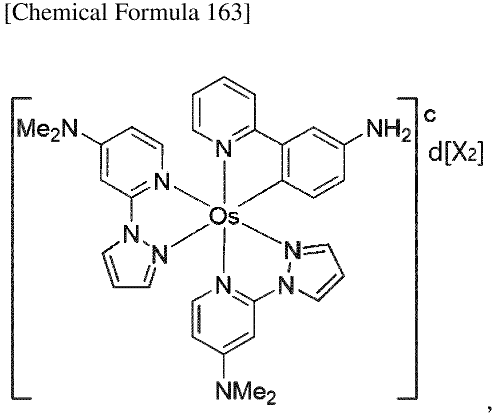

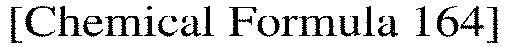

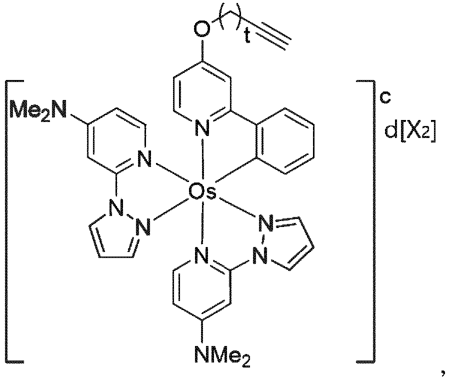

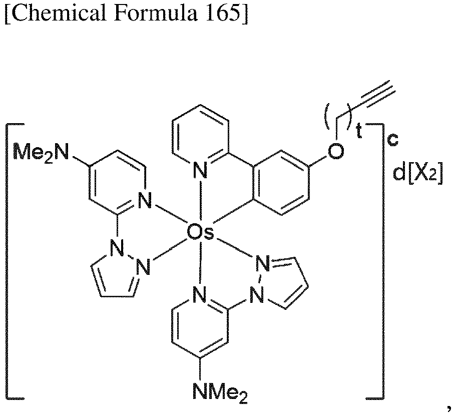

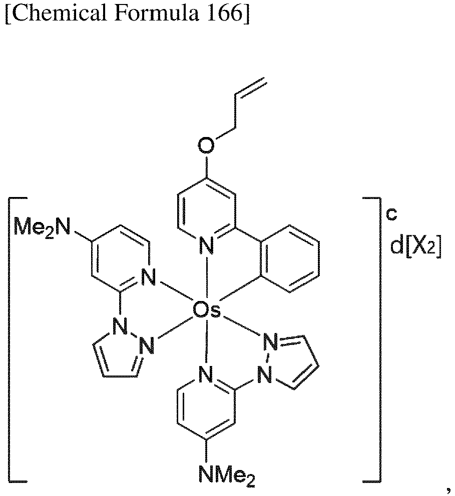

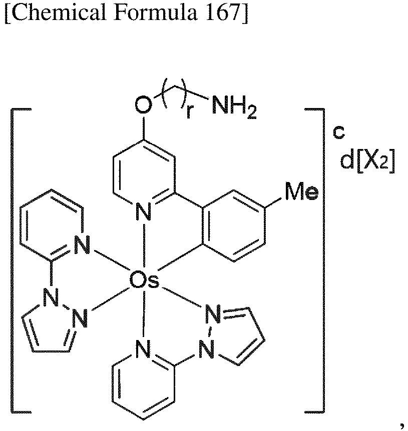

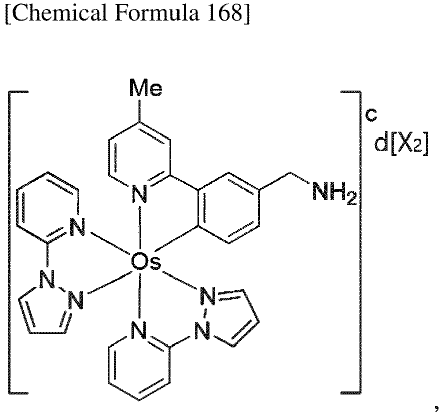

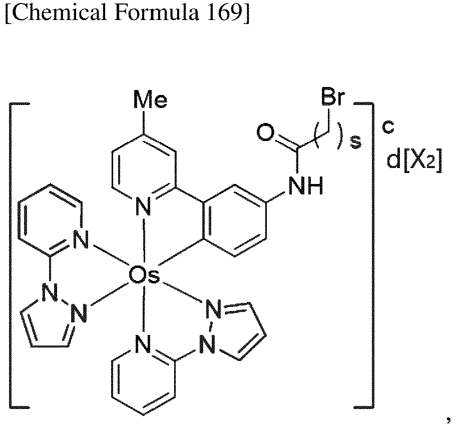

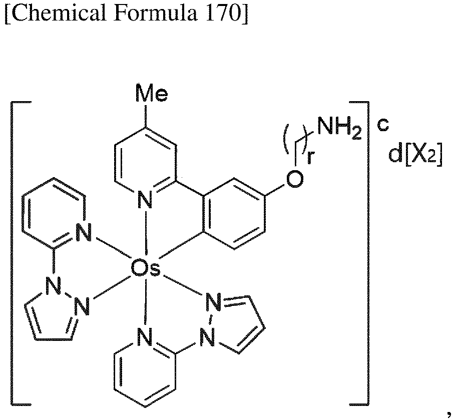

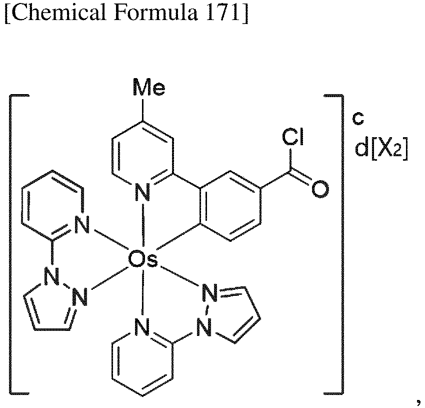

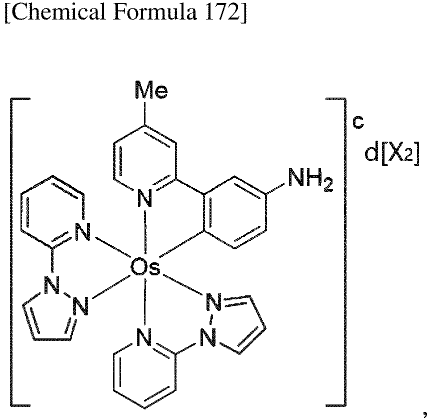

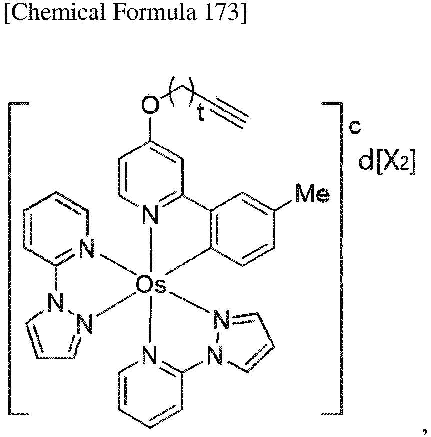

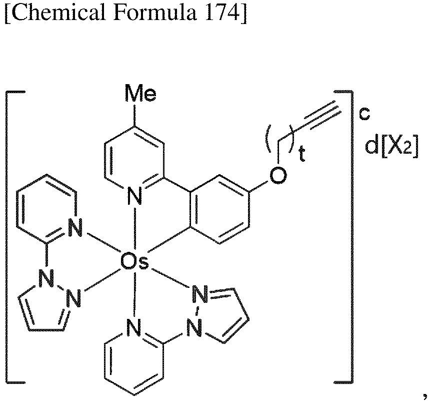

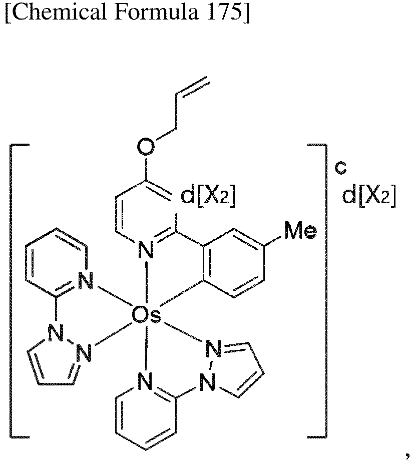

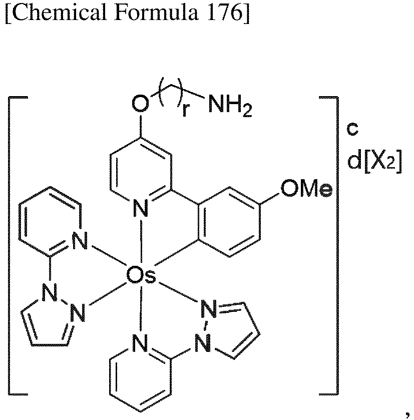

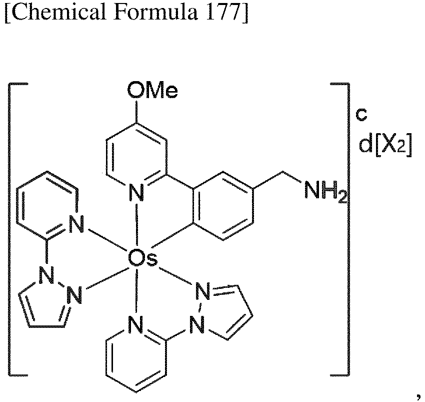

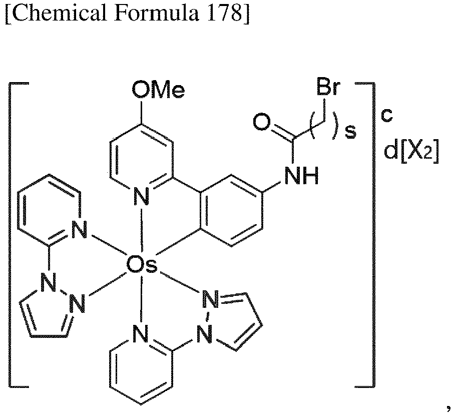

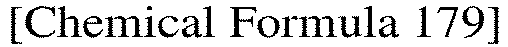

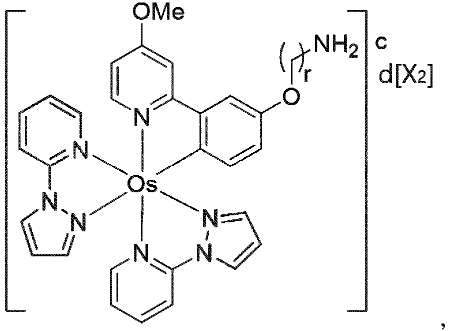

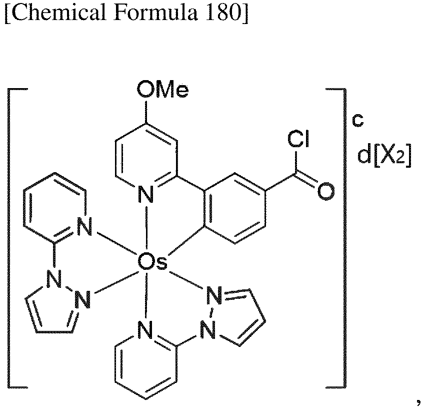

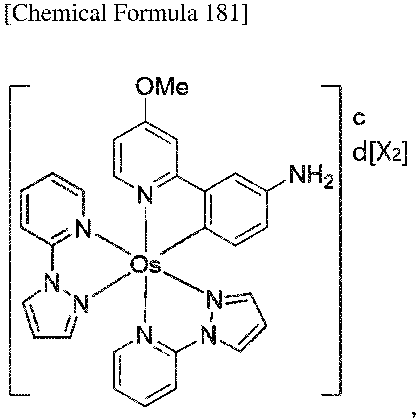

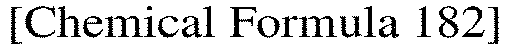

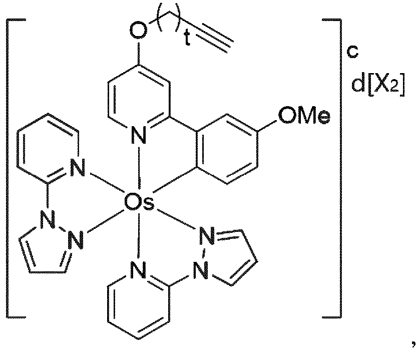

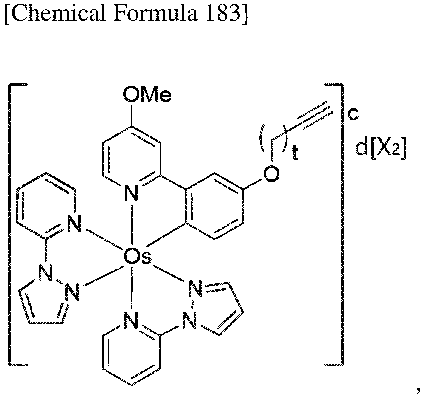

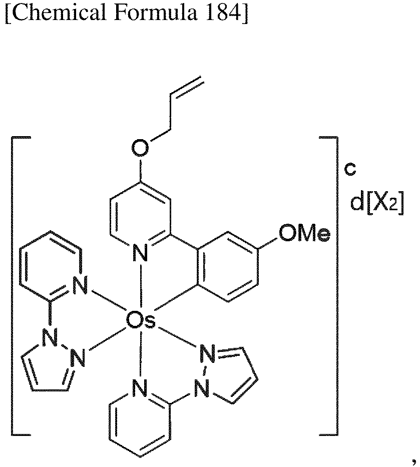

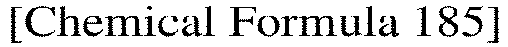

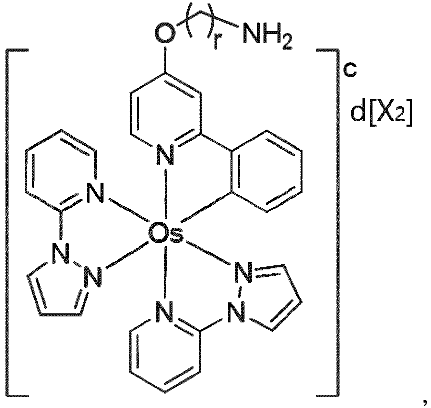

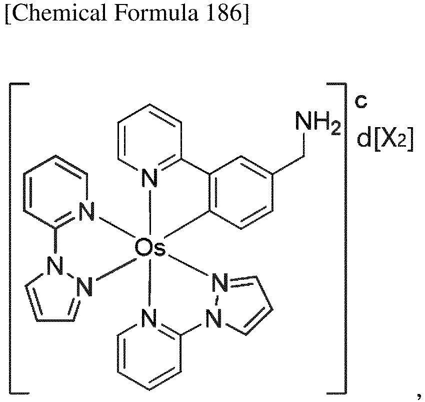

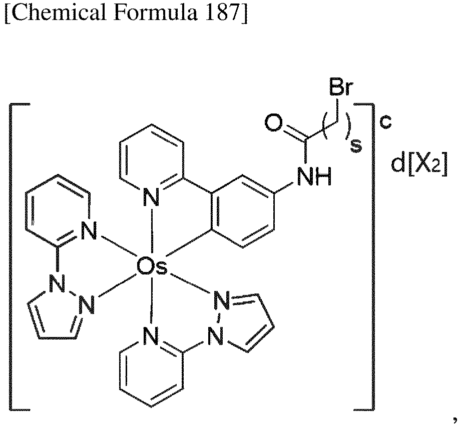

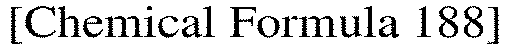

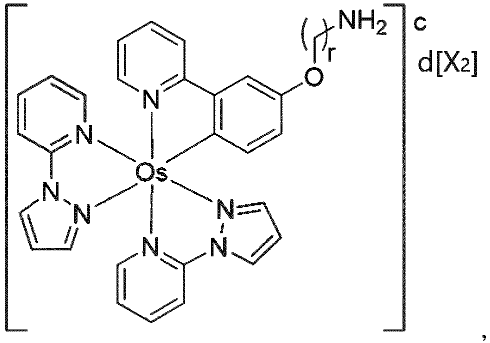

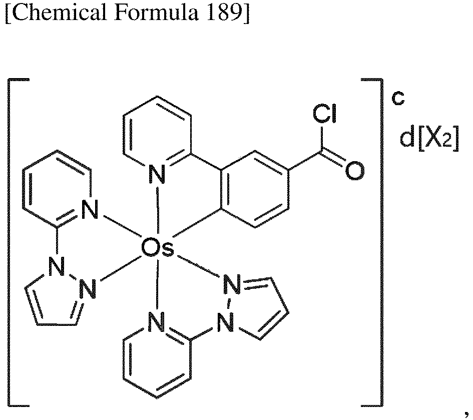

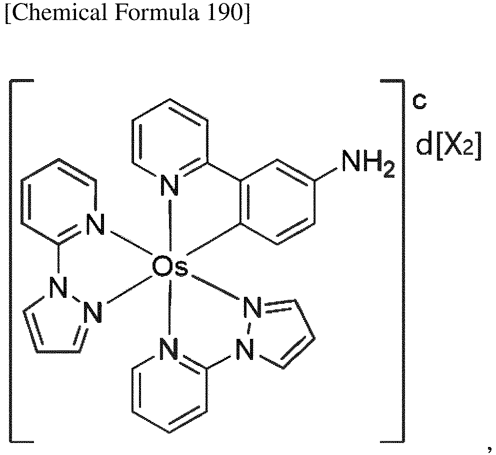

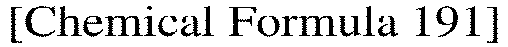

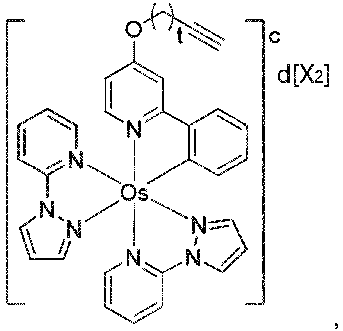

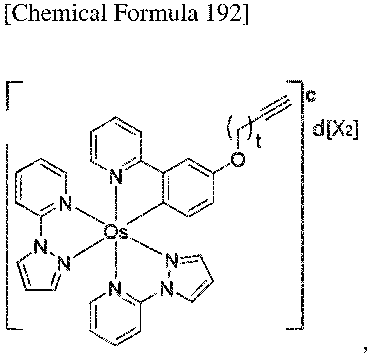

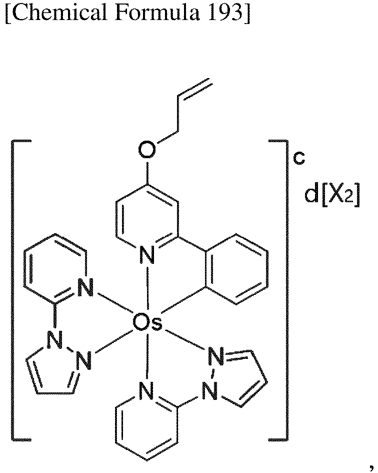

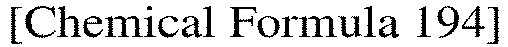

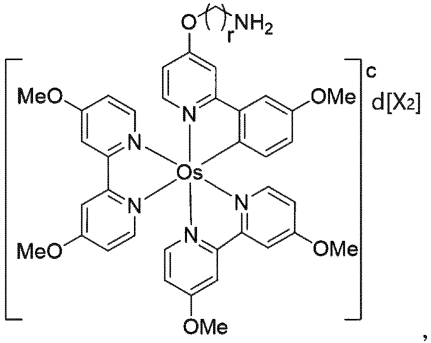

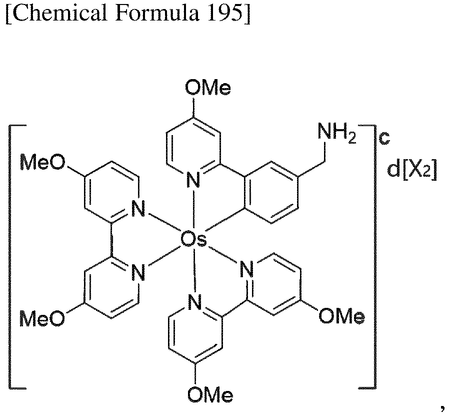

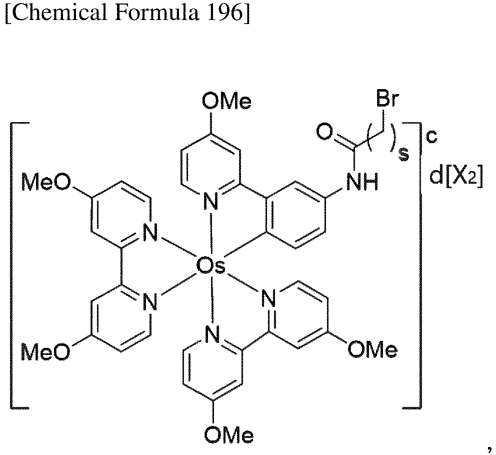

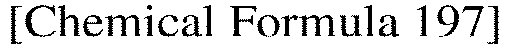

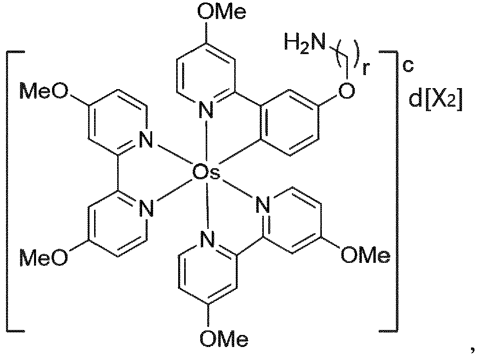

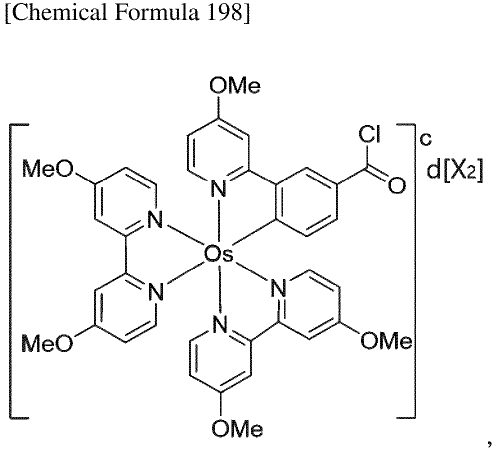

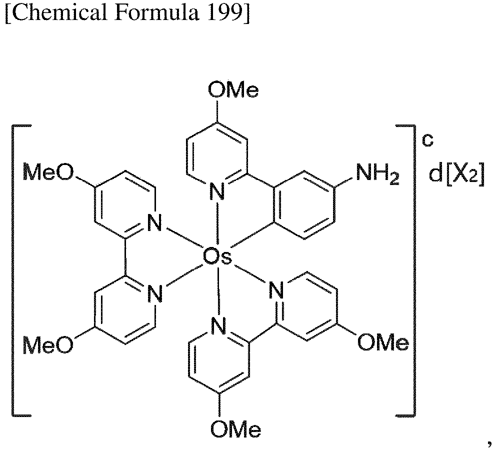

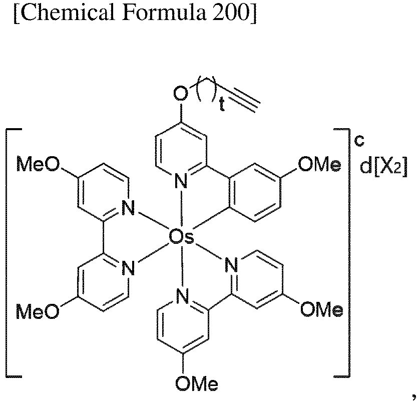

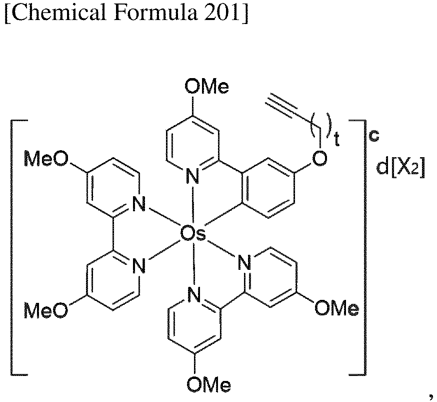

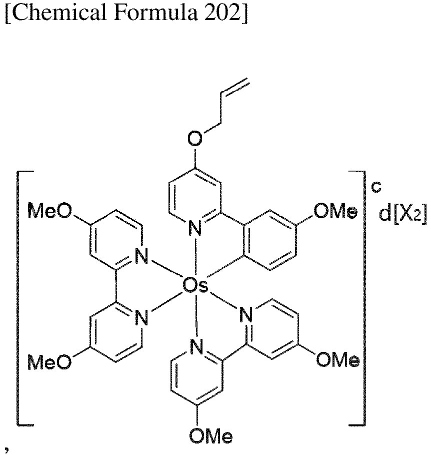

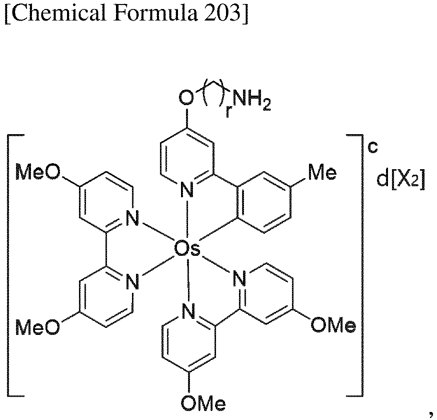

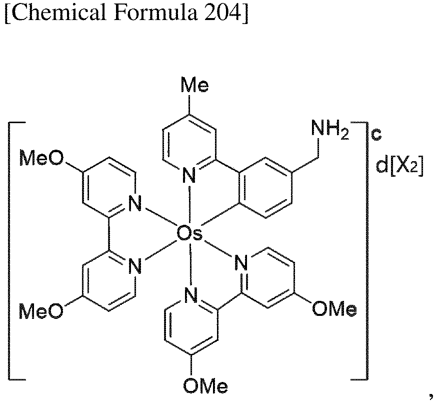

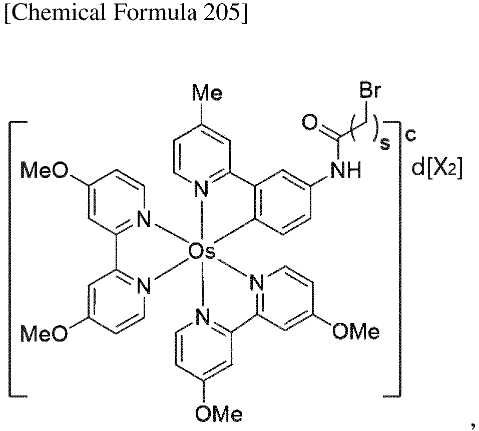

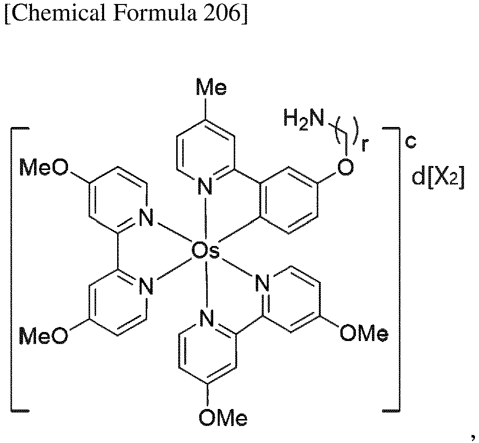

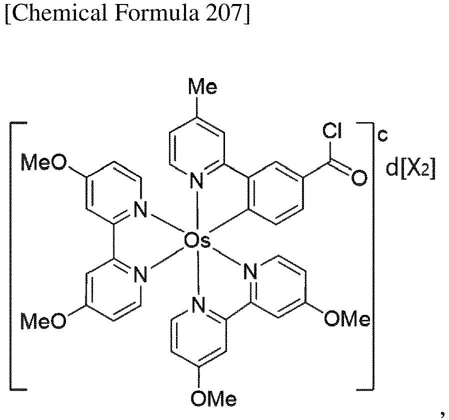

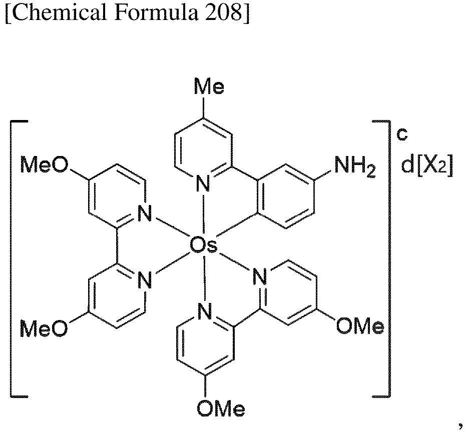

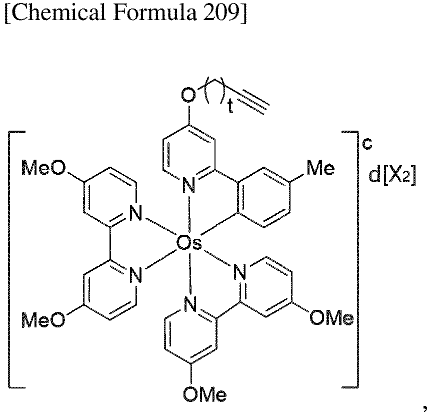

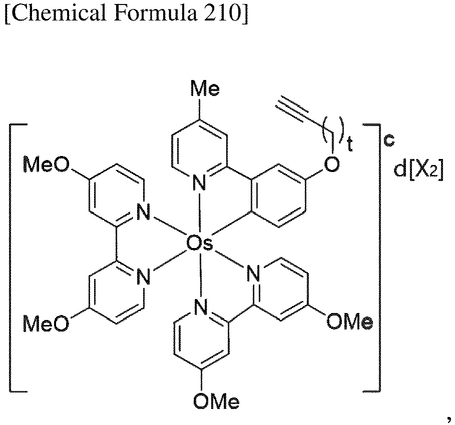

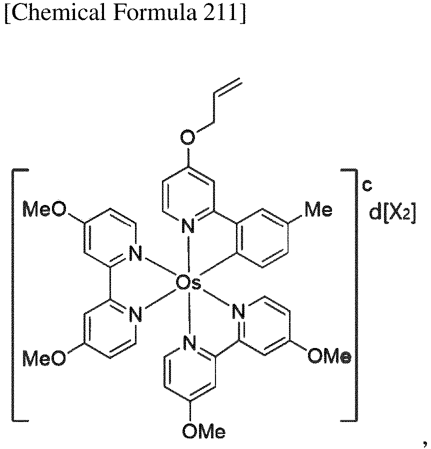

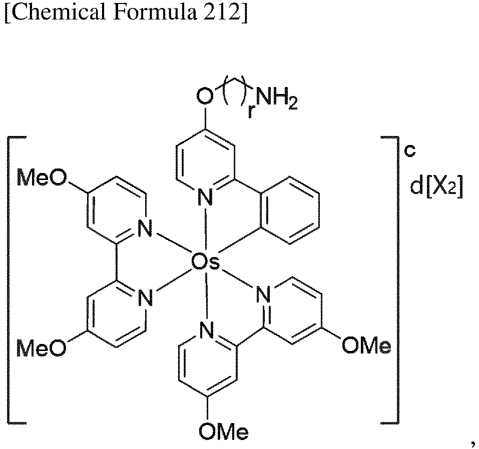

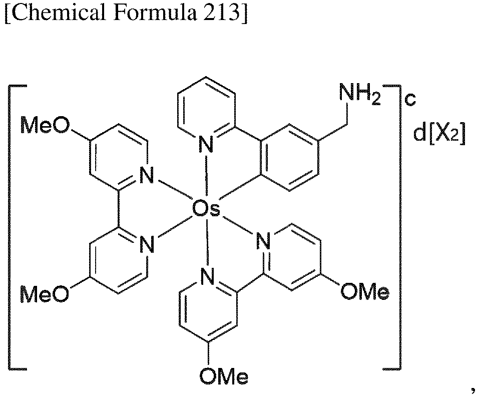

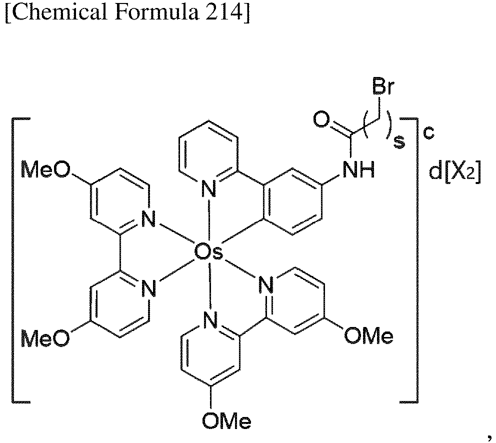

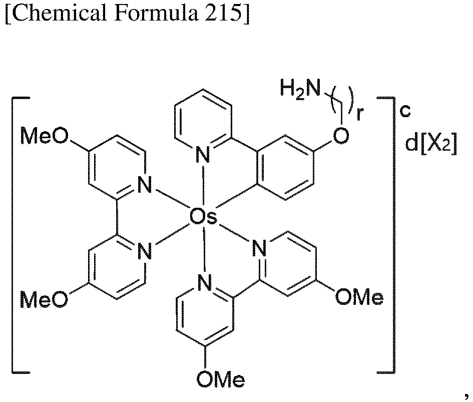

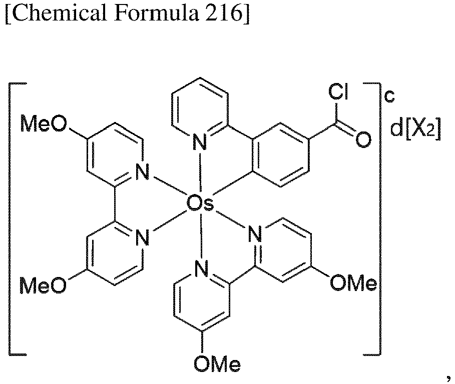

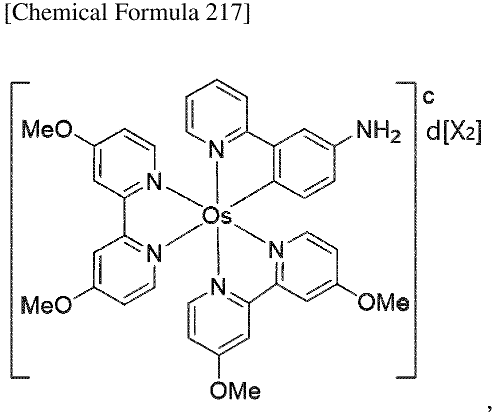

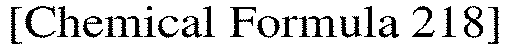

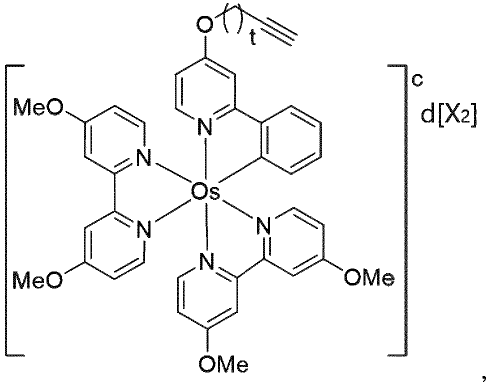

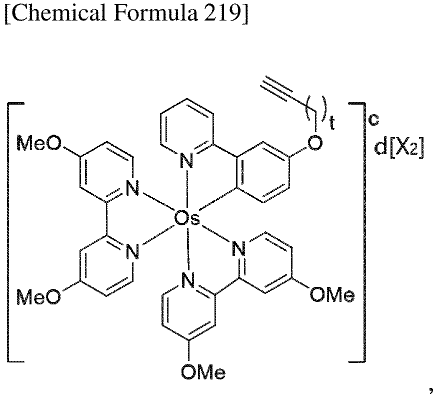

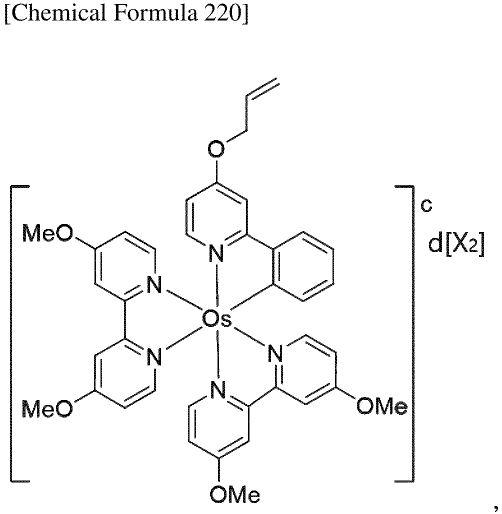

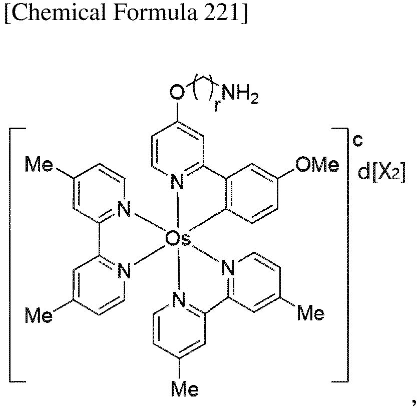

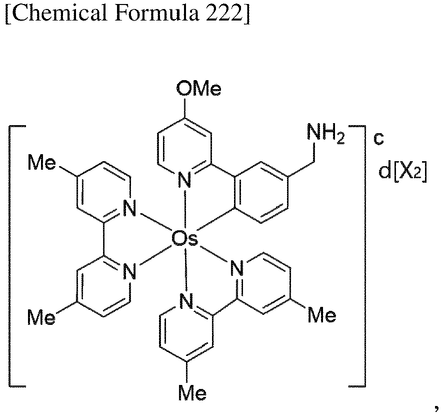

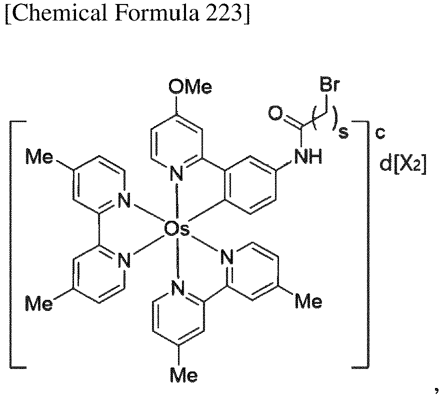

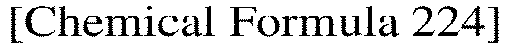

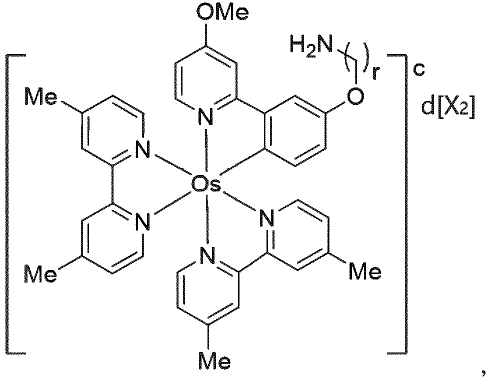

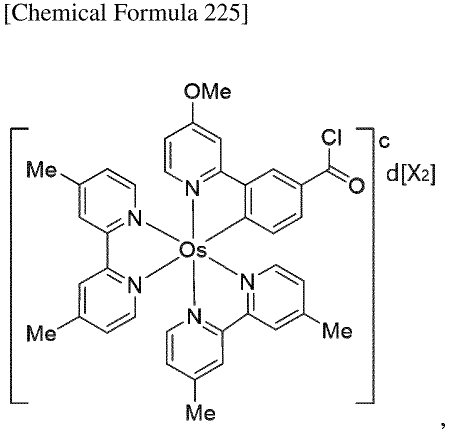

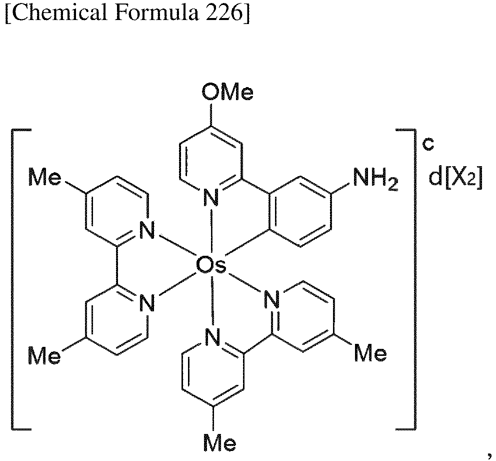

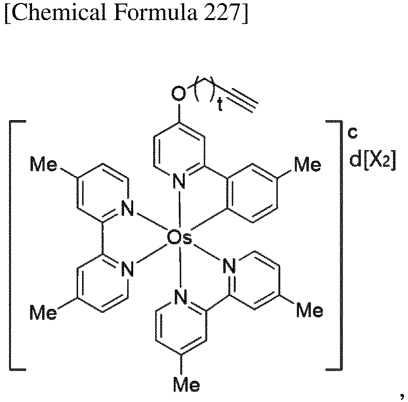

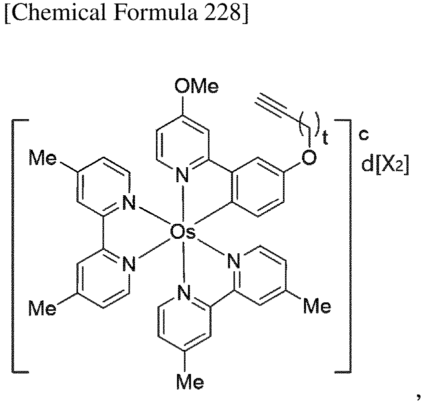

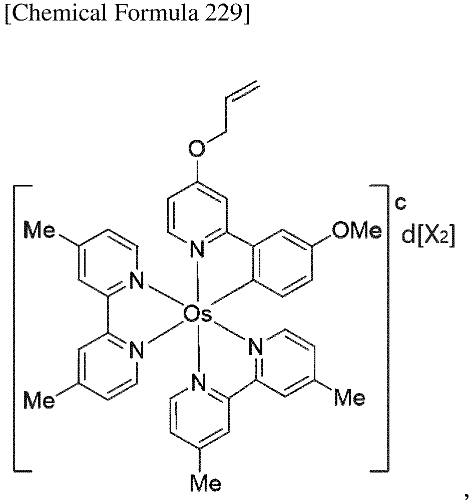

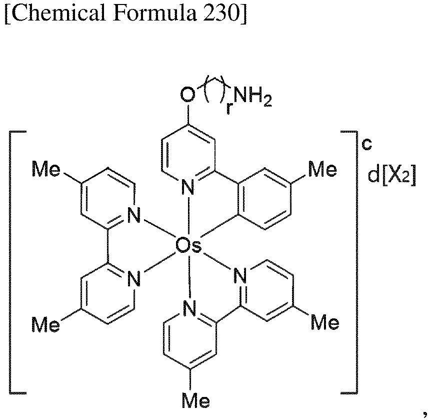

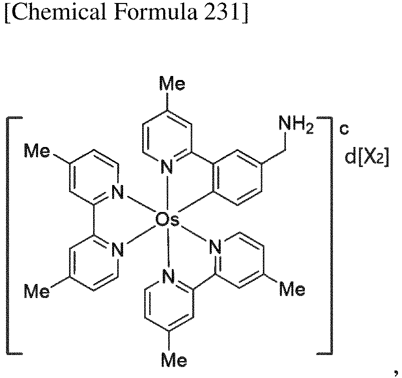

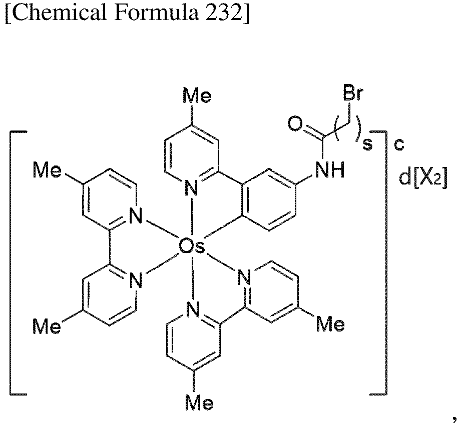

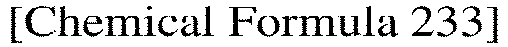

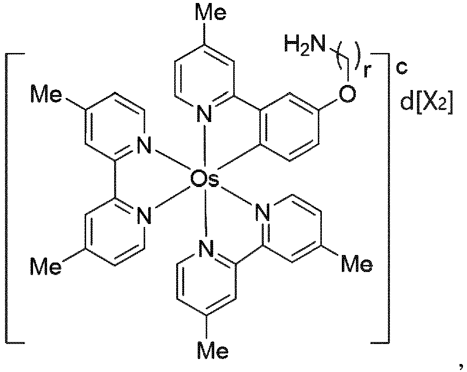

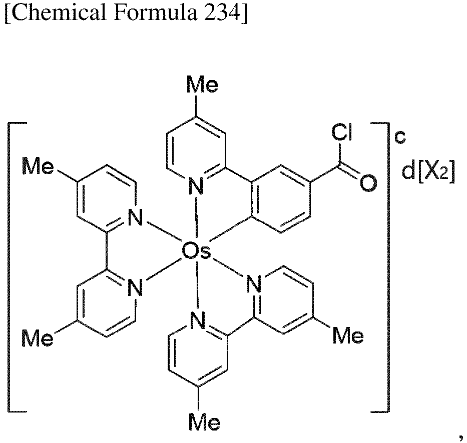

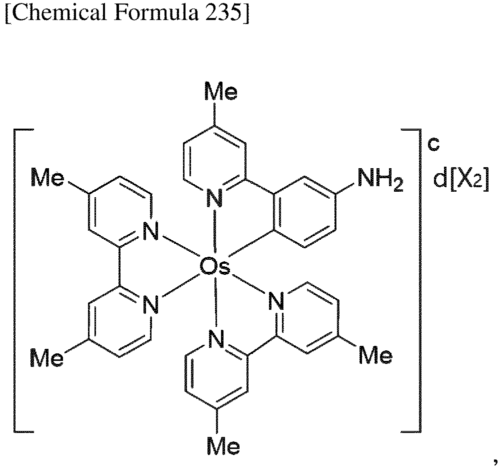

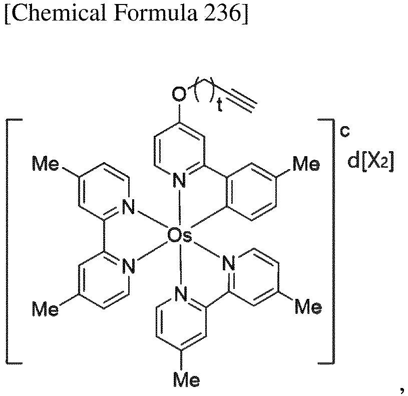

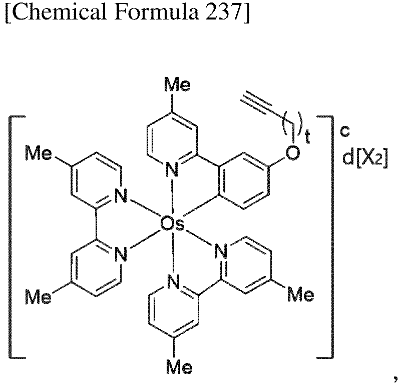

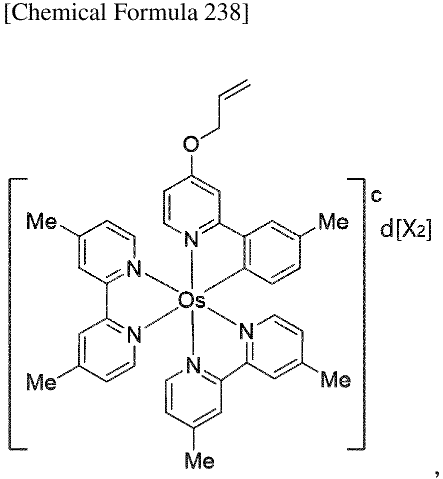

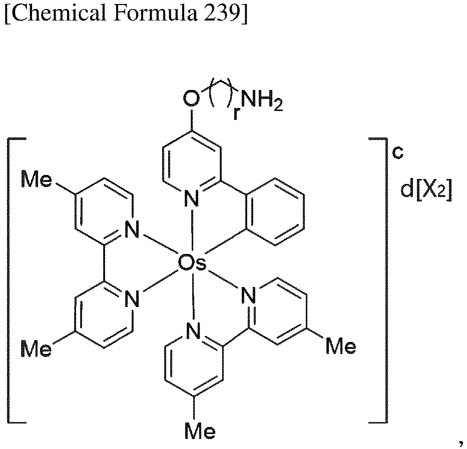

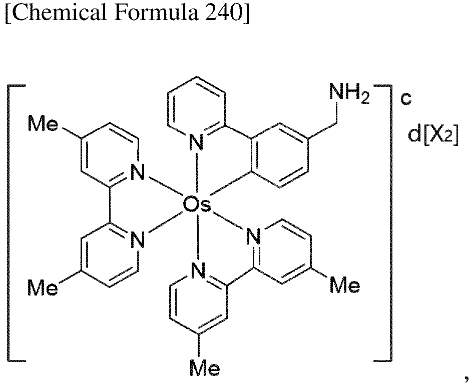

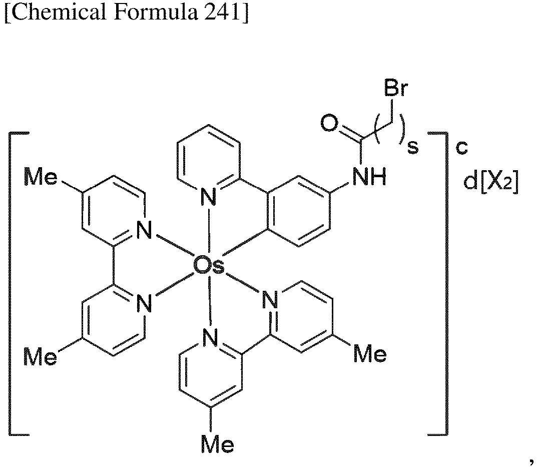

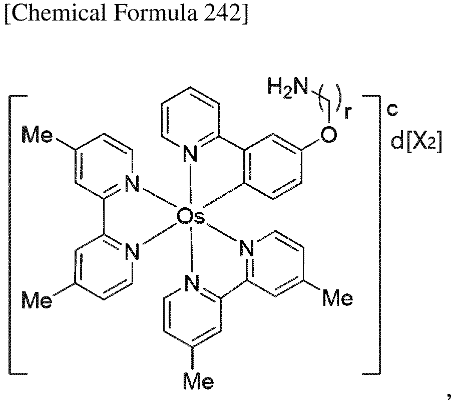

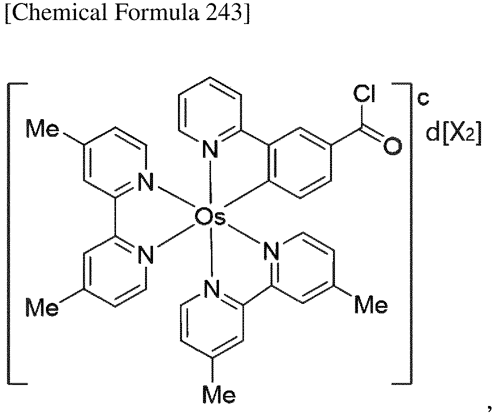

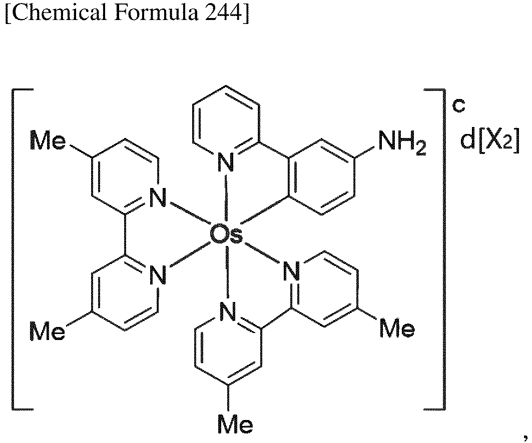

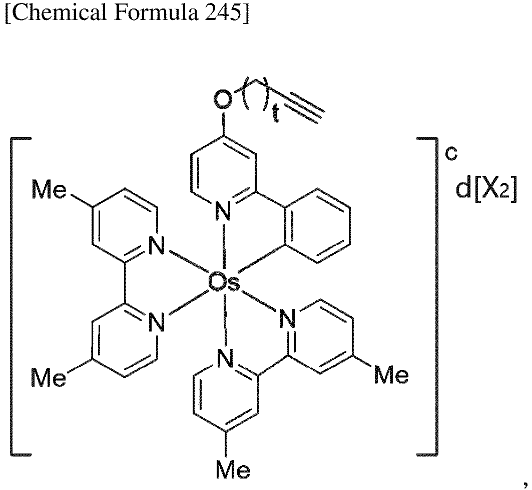

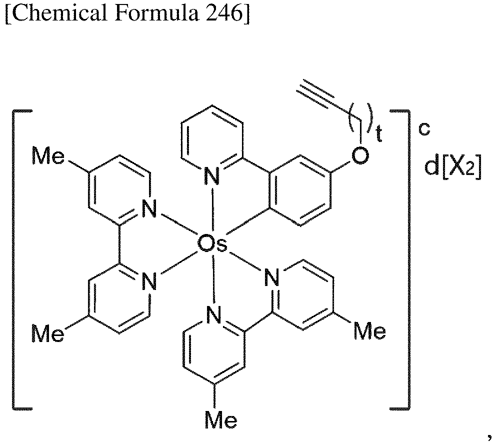

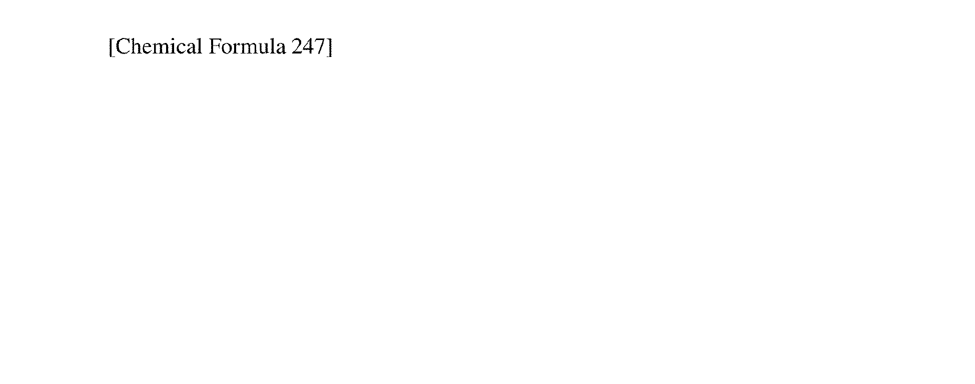

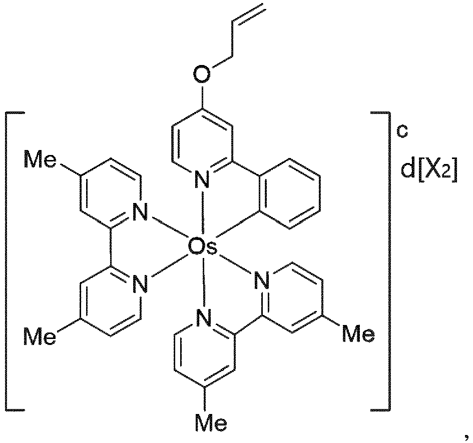

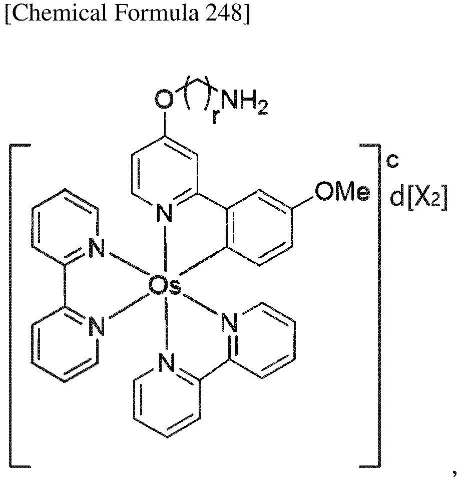

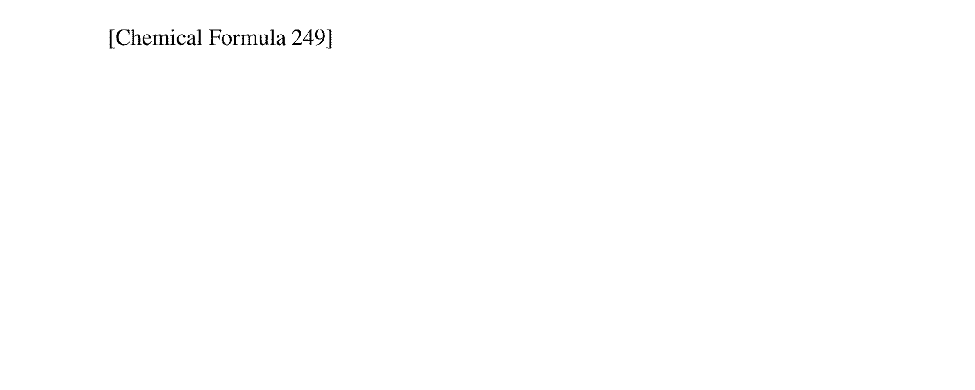

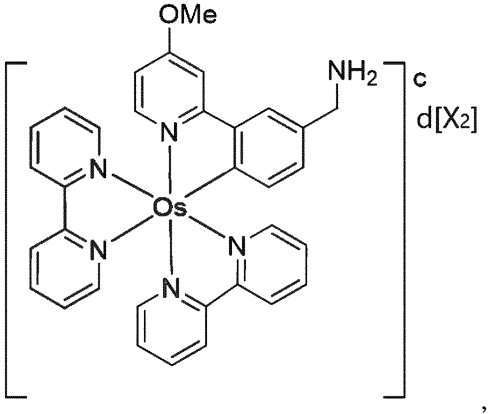

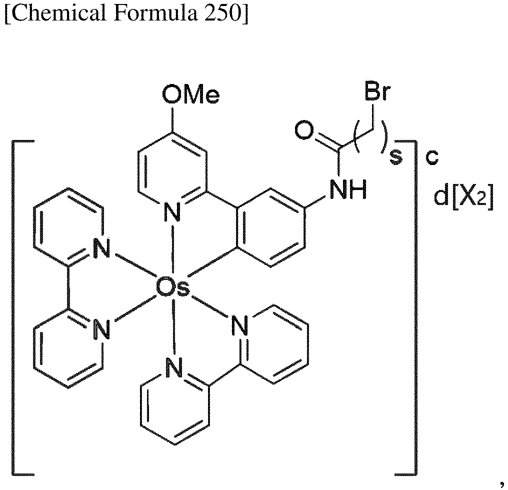

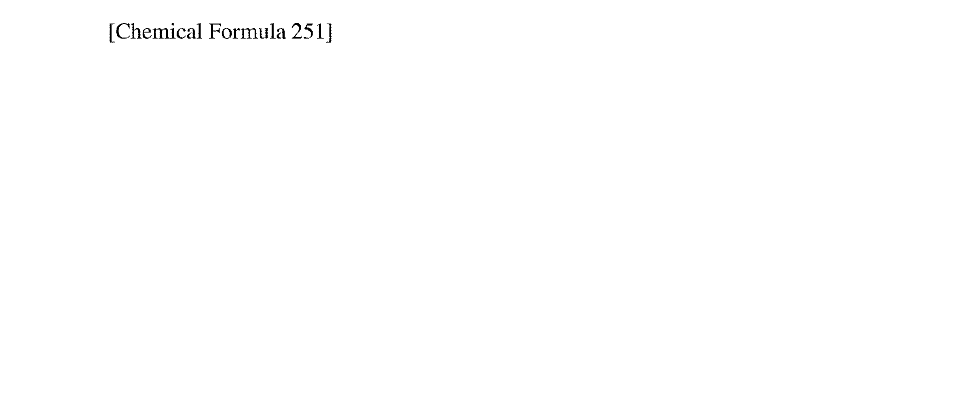

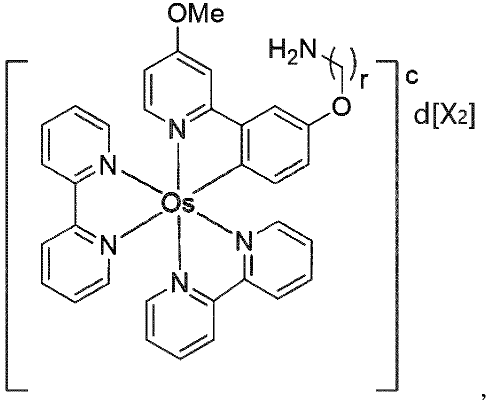

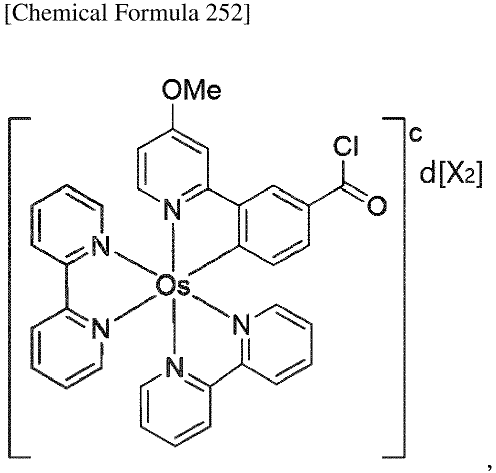

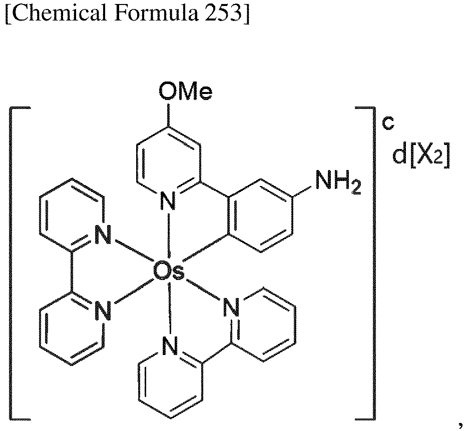

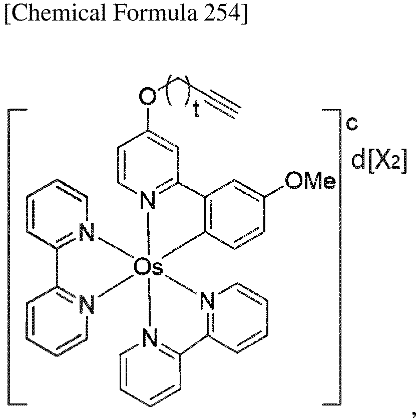

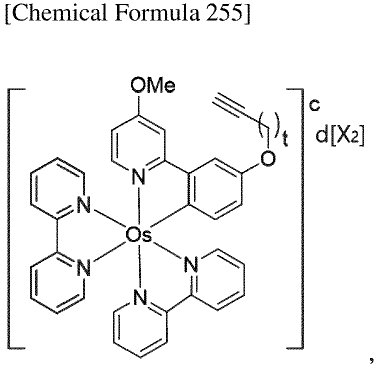

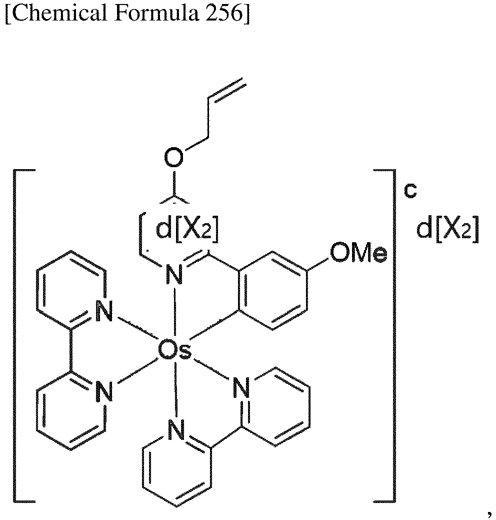

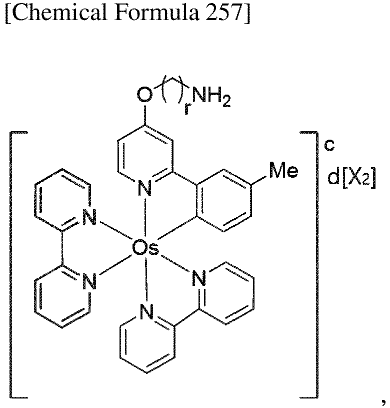

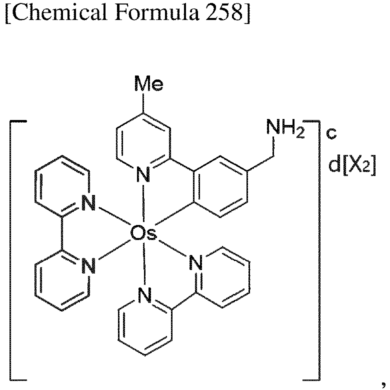

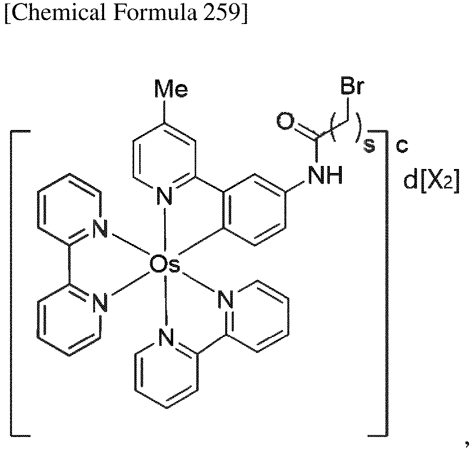

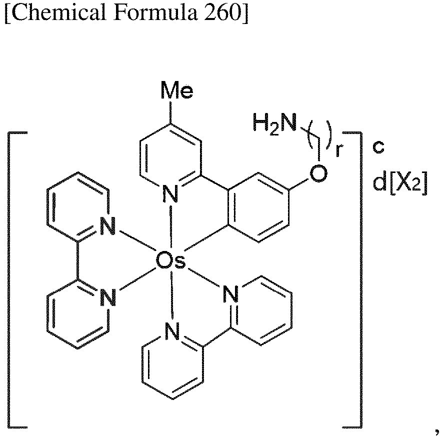

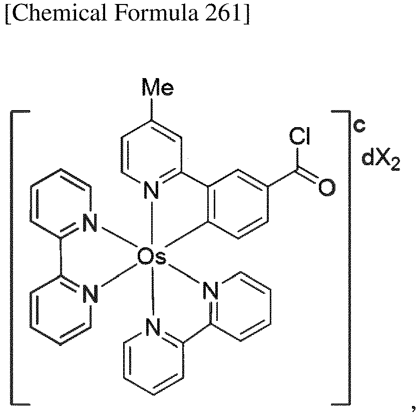

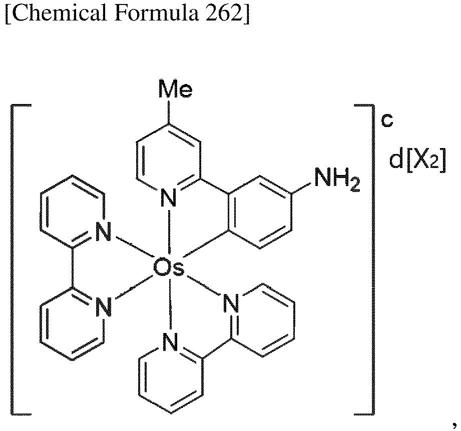

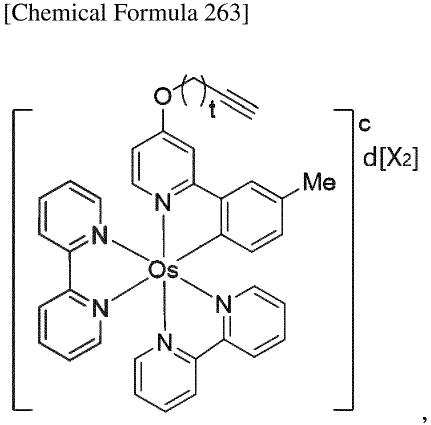

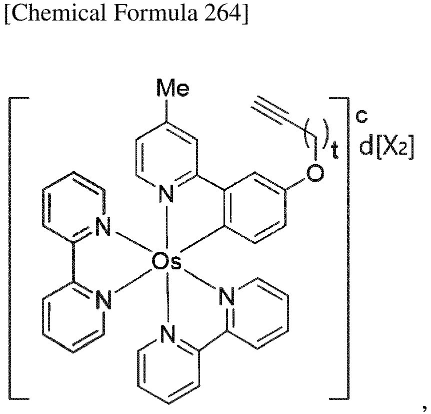

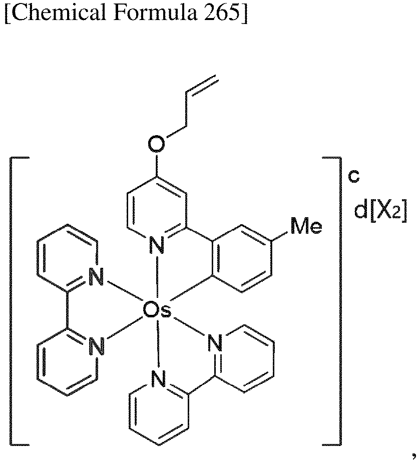

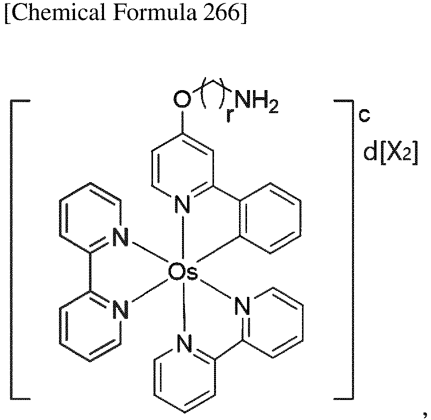

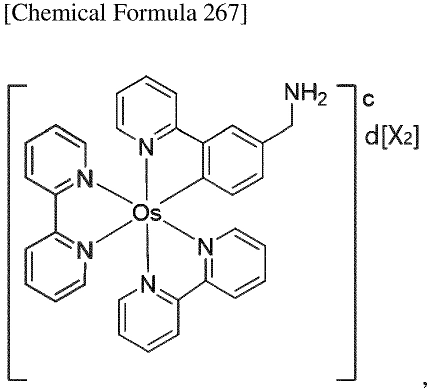

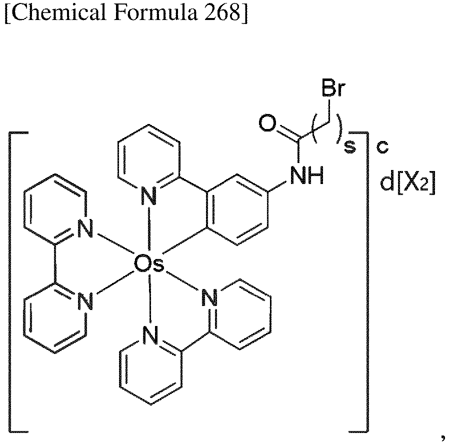

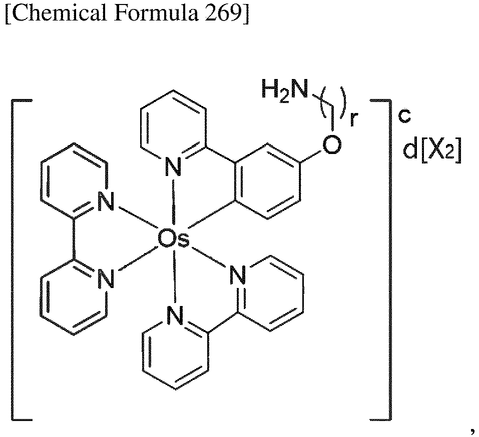

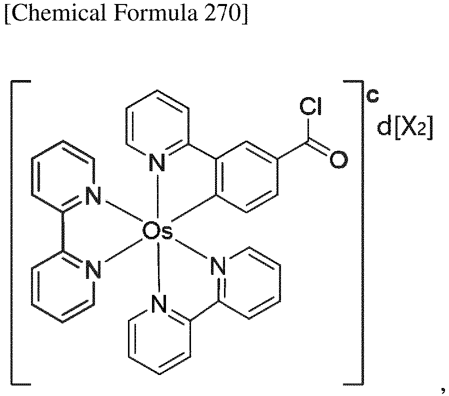

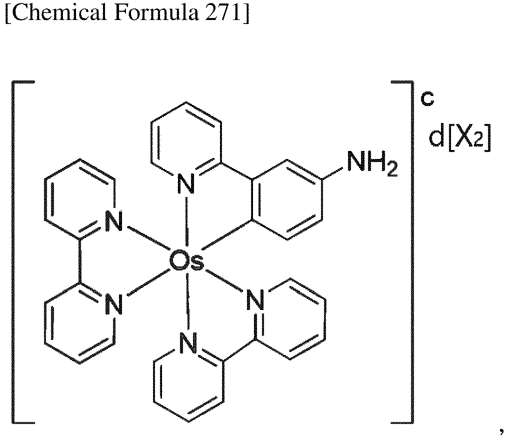

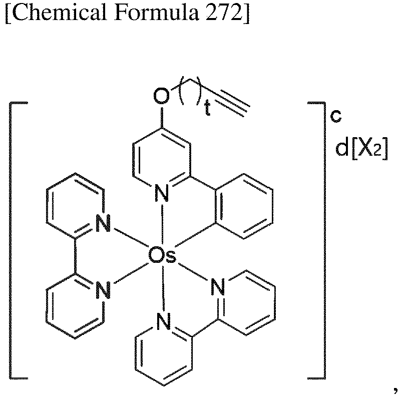

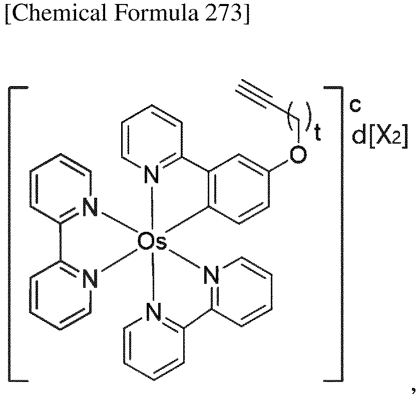

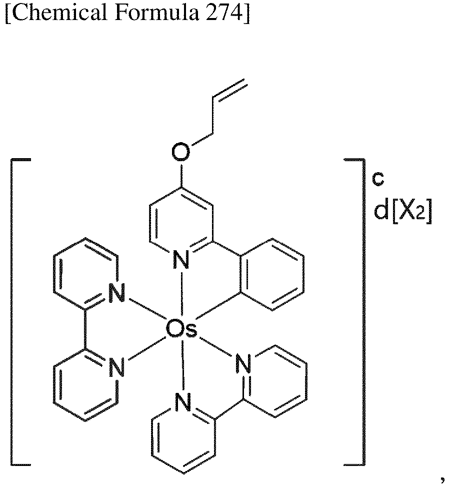

In Chemical Formulae 5 to 274, c and d may each be 1 or 2.

Furthermore, in Chemical Formulae 5 to 274, X₂ may be one counter ion selected from the group consisting of F, Cl, Br, I, and PF₆.

Further, in Chemical Formulae 5 to 274, r may be an integer from 1 to 6.

In addition, in Chemical Formulae 5 to 274, s may be an integer from 1 to 12.

Furthermore, in Chemical Formulae 5 to 274, t may be an integer from 1 to 4.

Further, in one aspect, the present invention is an oxidation-reduction polymer including a combination of the transition metal complex of Chemical Formula 1 or a salt compound thereof and a polymer.

In one aspect, the oxidation-reduction polymer may further include a functional group capable of crosslinking.

As used herein, the "polymer" may refer to a material including a number of repeating units. The polymer may include a homopolymer, a copolymer, and a terpolymer, but is not limited thereto. In addition, the polymers may be classified into a linear polymer, a branched polymer, a crosslinked polymer, and the like according to their structures, but are not limited thereto.

Furthermore, the copolymer may include an alternating copolymer, a random copolymer, a block copolymer, and a graft copolymer, but is not limited thereto.

In one aspect, the polymer constituting the oxidation-reduction polymer may include one or more selected from the group consisting of polyvinylimidazole, polyvinylpyridine, poly(allyl glycidyl ether), poly(dimethylaminoethyl methacrylate), poly(pentafluorophenyl acrylate), poly(pentafluorophenyl methacrylate), a poly(pentafluorophenyl acrylate)-polyacrylamide copolymer, a poly(dimethylaminoethyl methacrylate)-poly(pentafluorophenyl methacrylate) copolymer, and polymers thereof, but is not limited thereto.

In one aspect, the oxidation-reduction polymer may include one in which the polymer is covalently bonded to Adi of Chemical Formula 1.

Further, in one aspect, the polymer may be one of the following Chemical Formulae 275 to 285, but is not limited thereto.

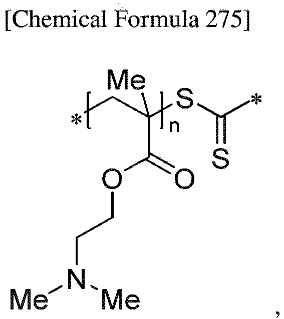

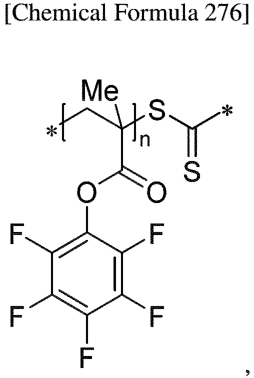

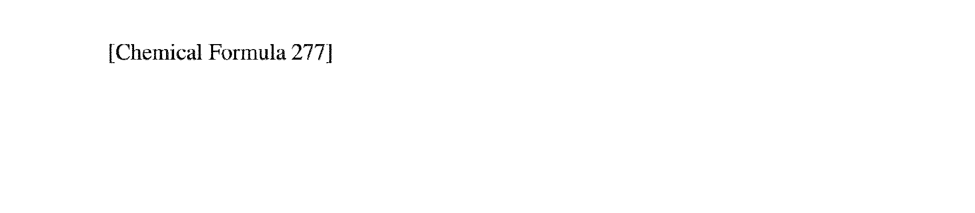

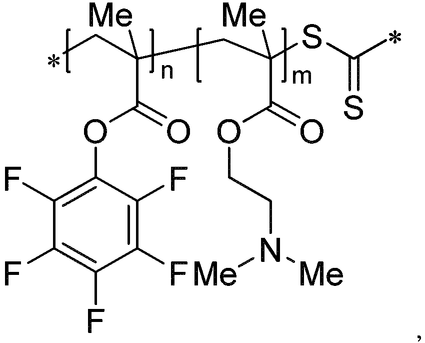

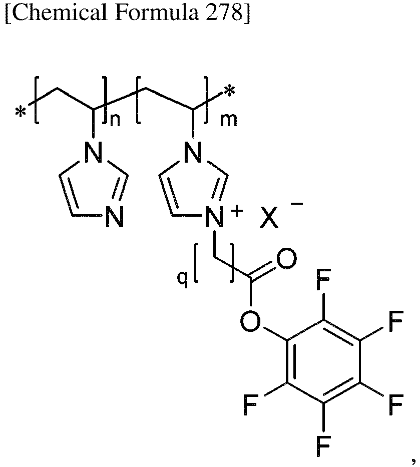

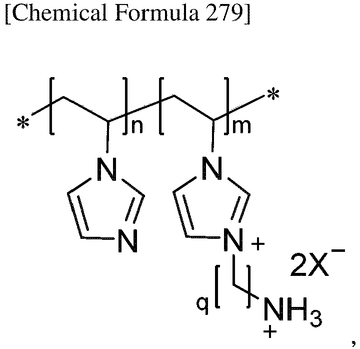

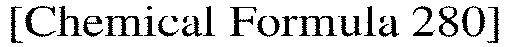

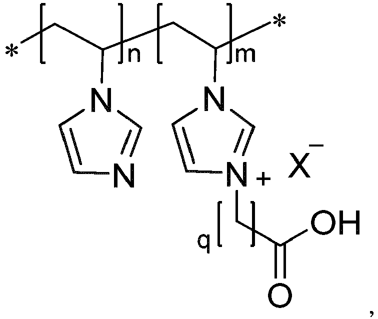

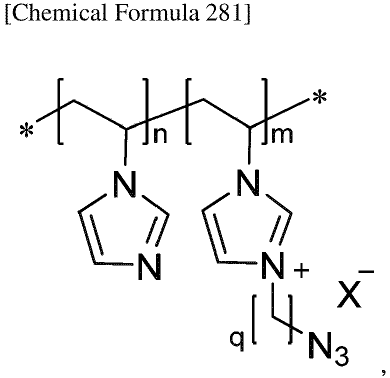

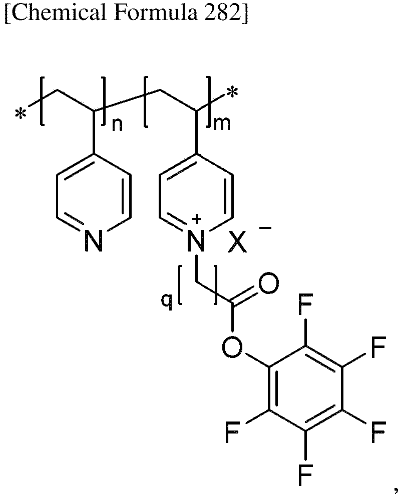

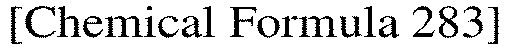

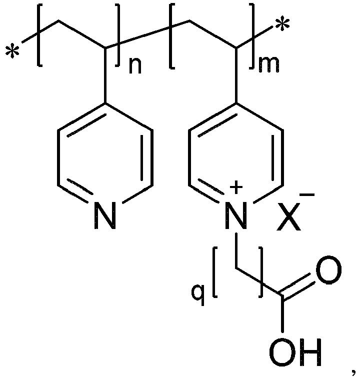

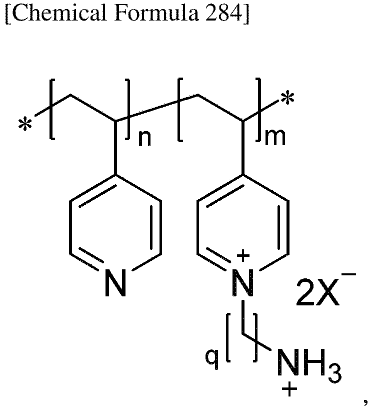

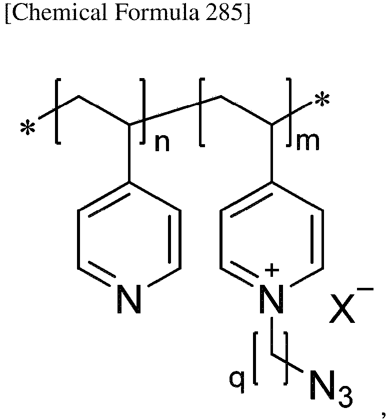

In Chemical Formulae 275 to 285, n and m may each be an integer from 3 to 300.

In addition, in Chemical Formulae 275 to 285, q may be an integer from 1 to 12.

Furthermore, in Chemical Formulae 275 to 285, X may be one counter ion selected from the group consisting of F, Cl, Br, and I.

In one aspect, the oxidation-reduction polymer of the present invention may include one or more compounds of the following Chemical Formulae 286 to 304.

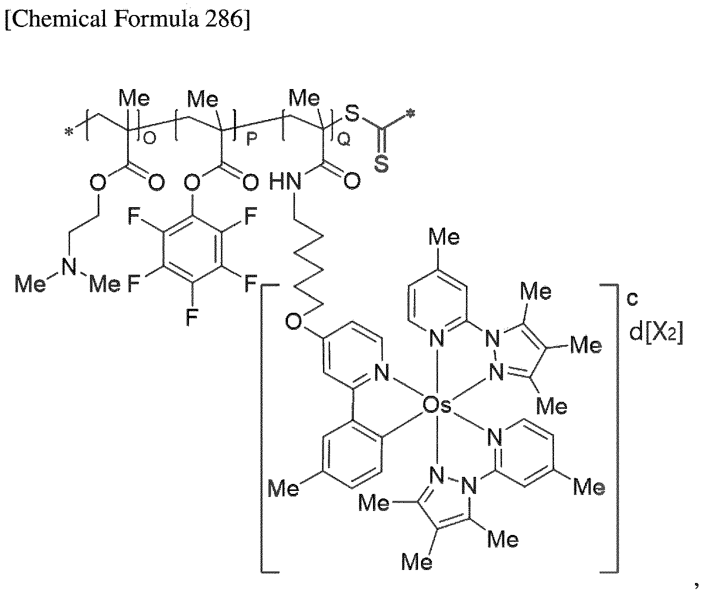

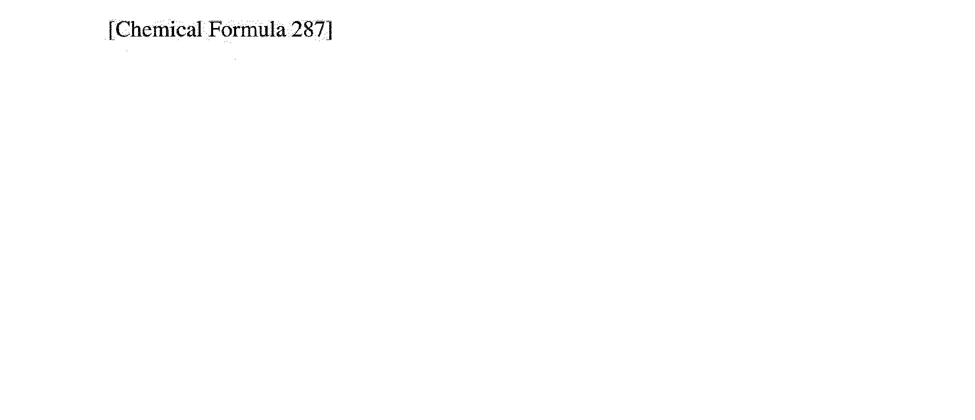

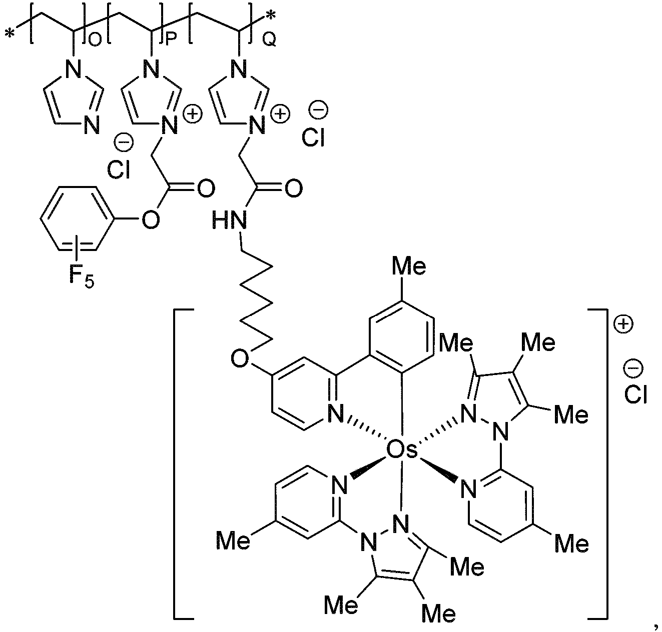

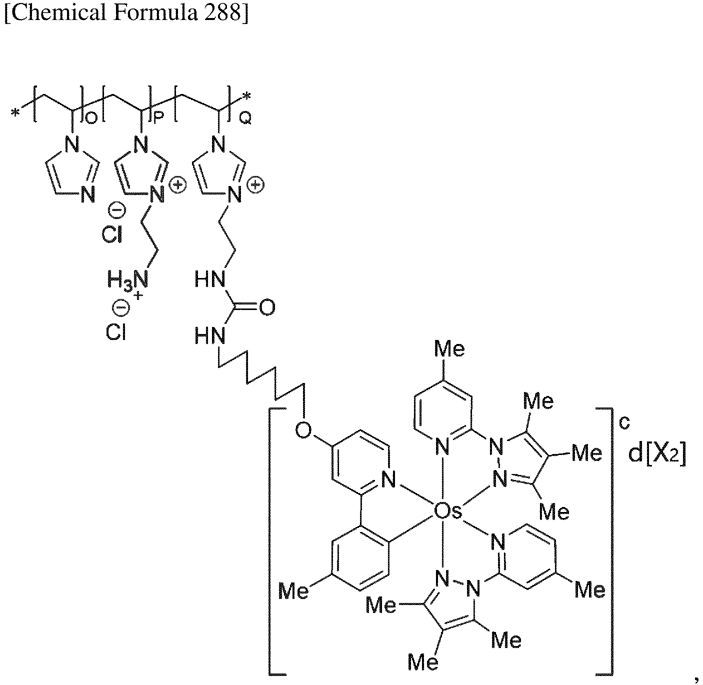

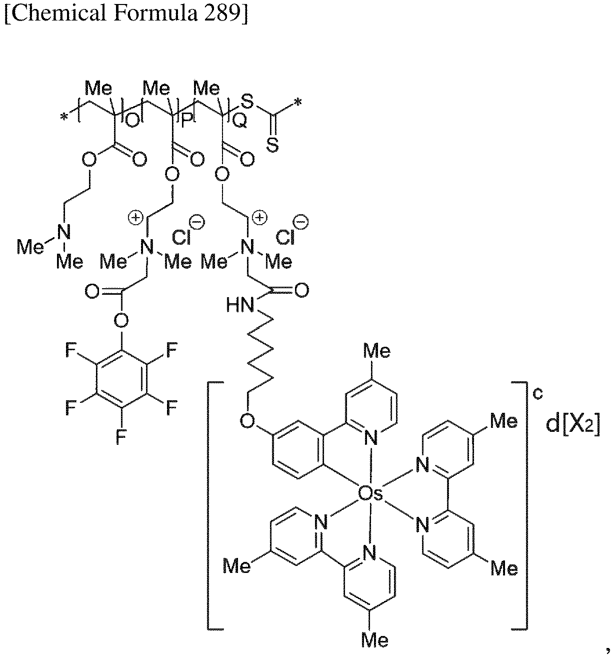

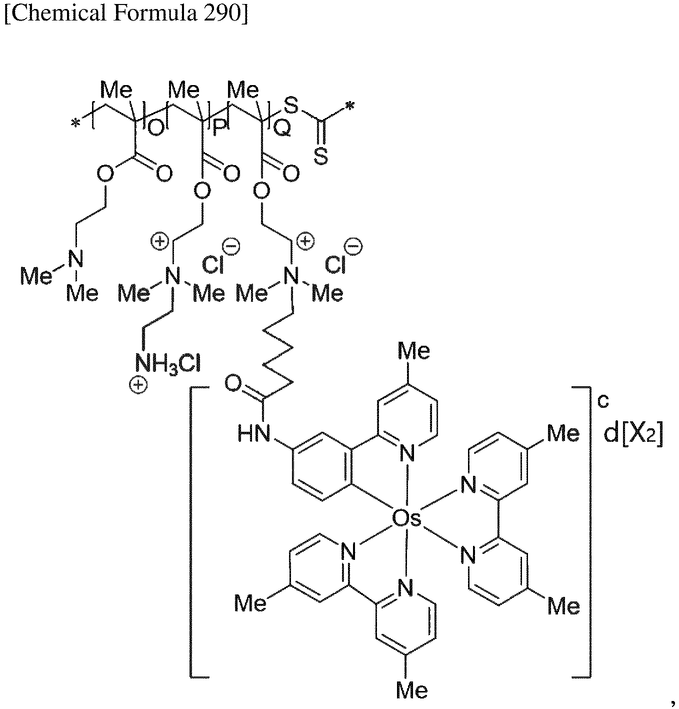

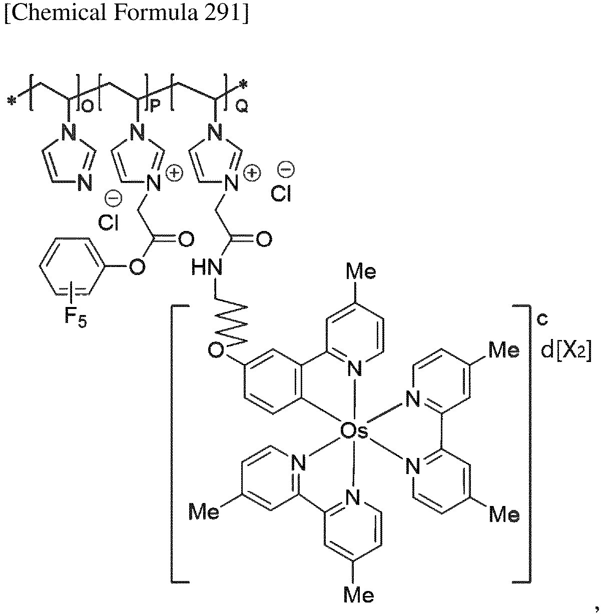

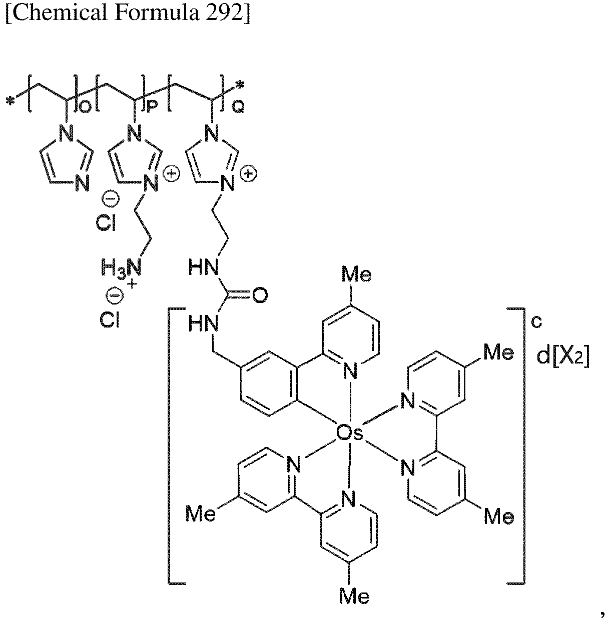

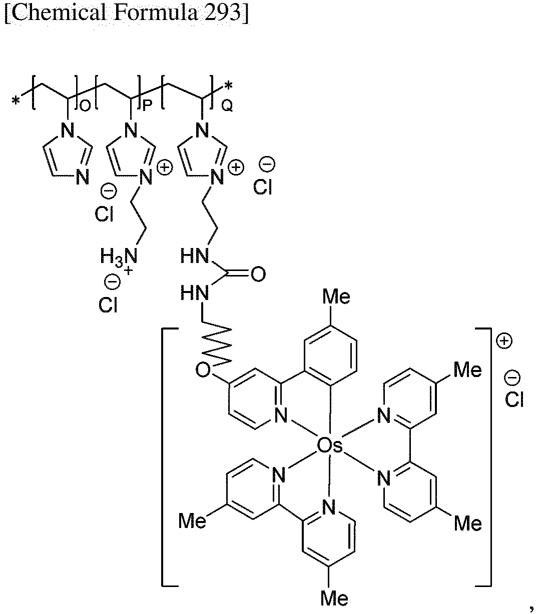

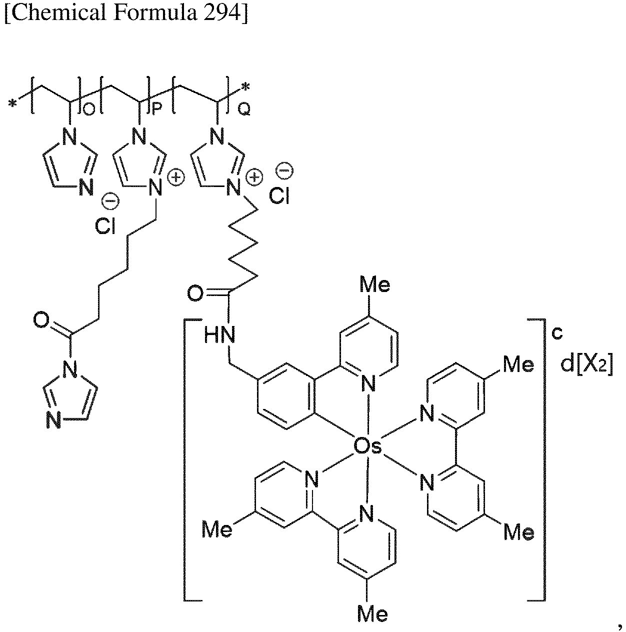

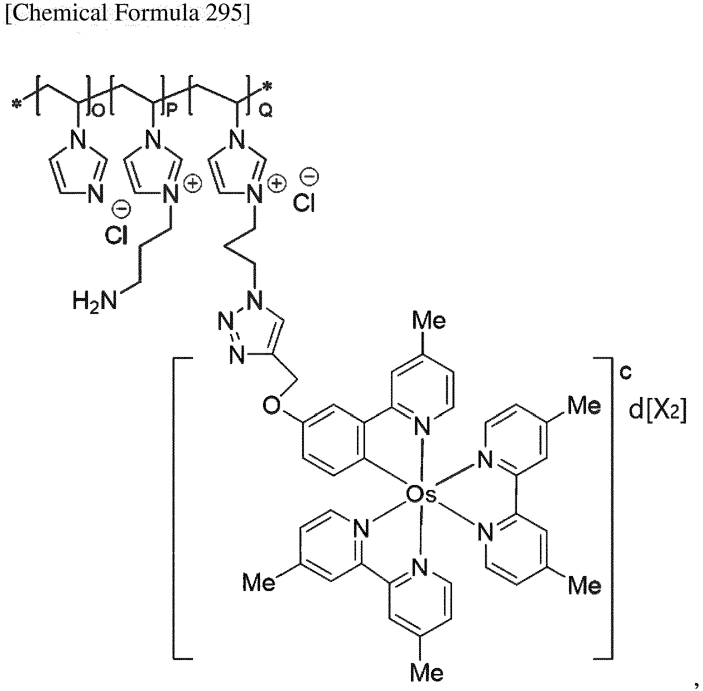

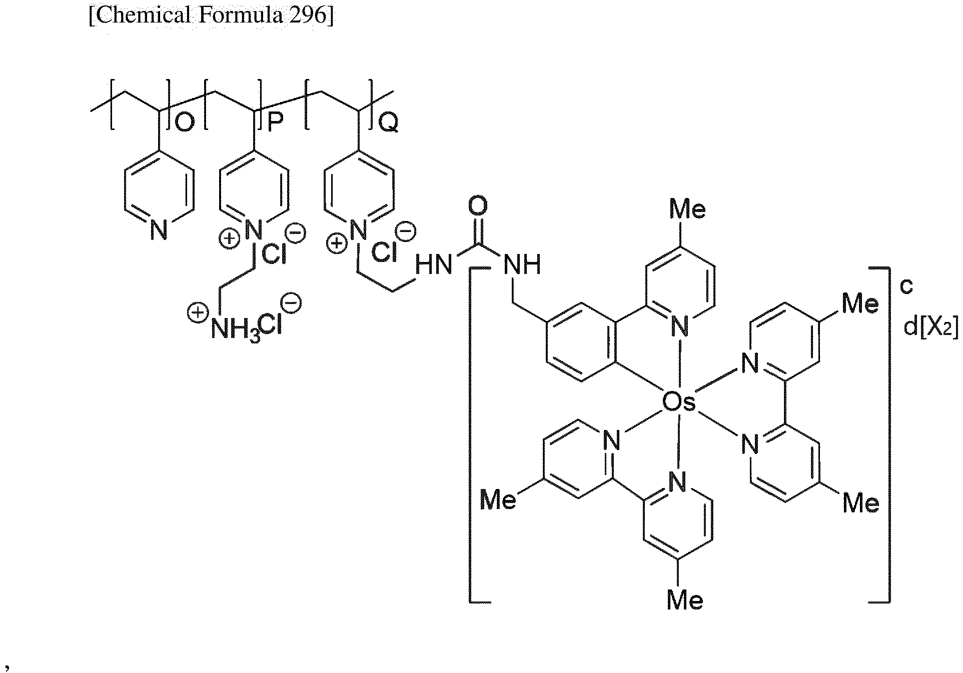

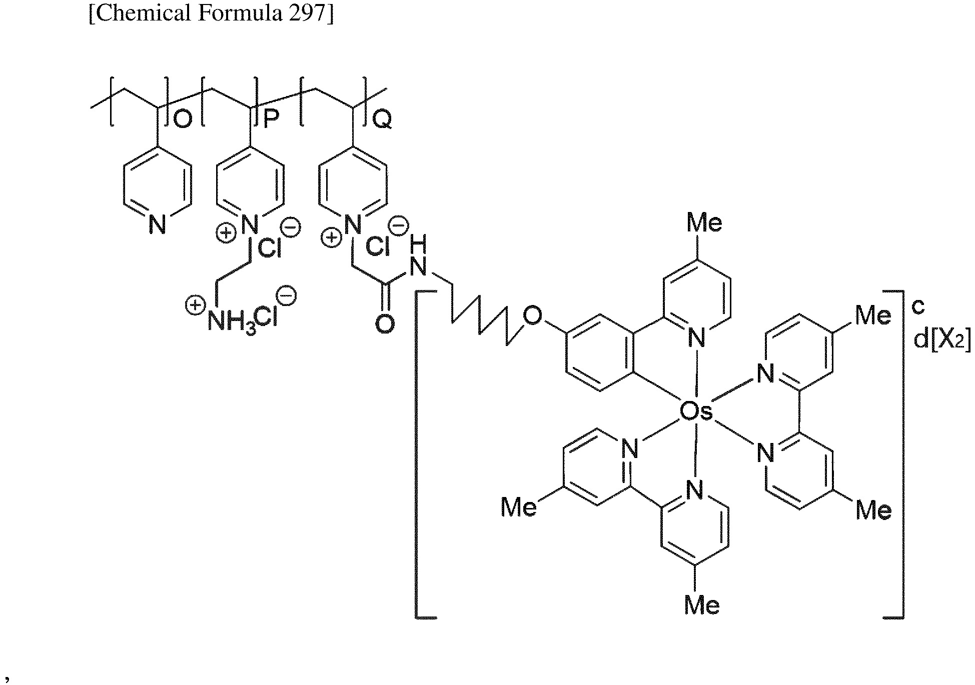

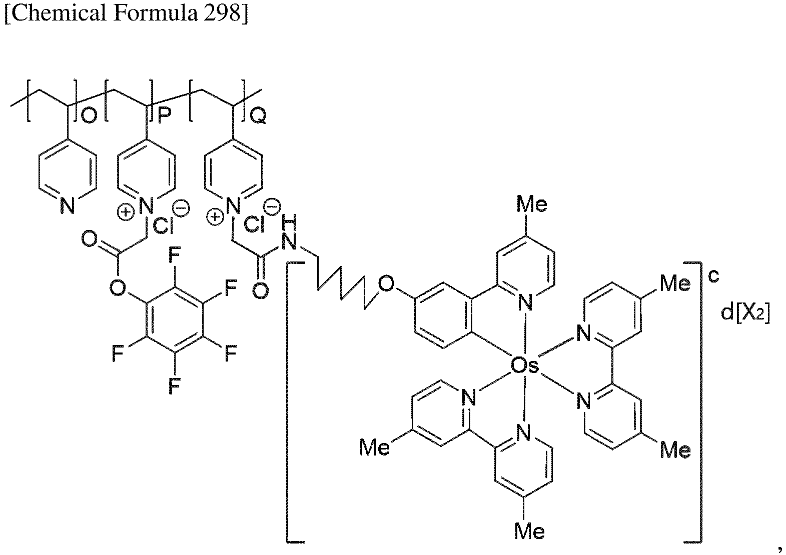

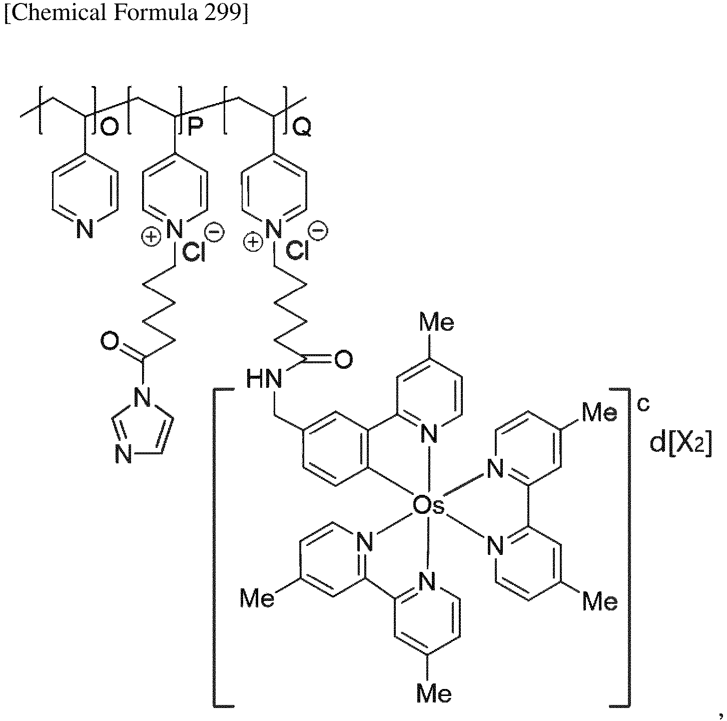

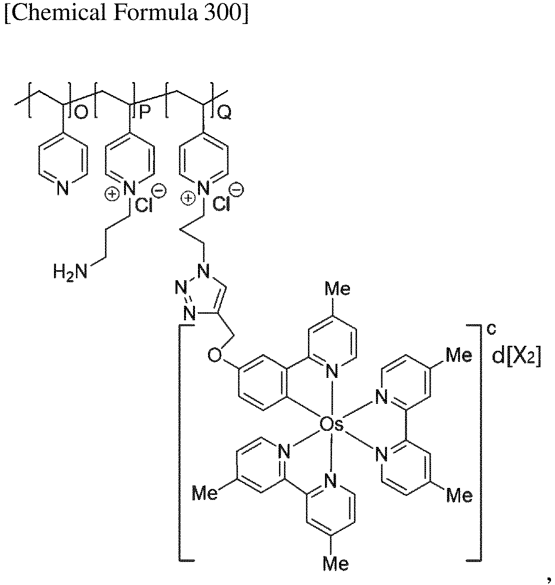

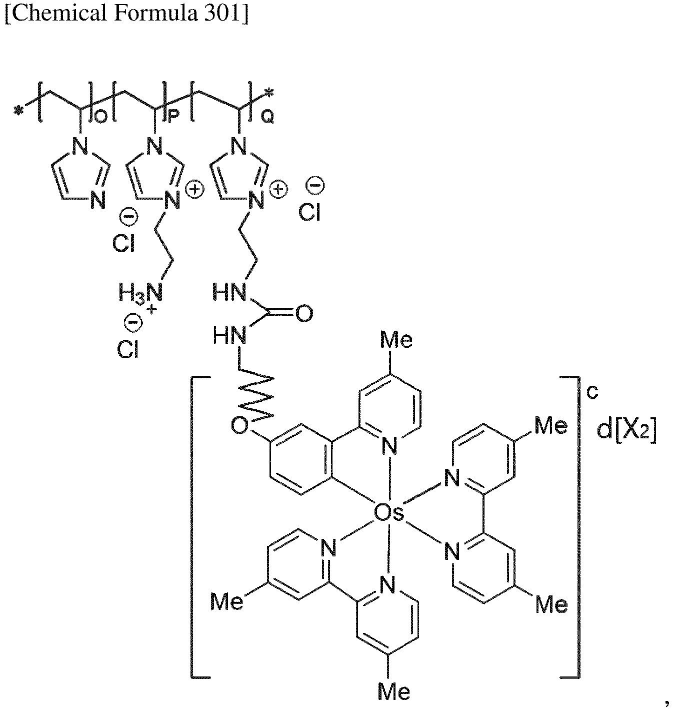

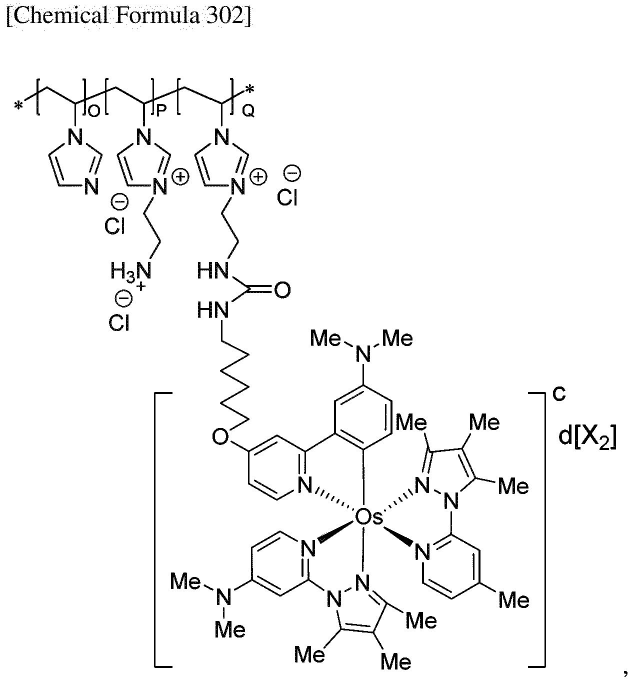

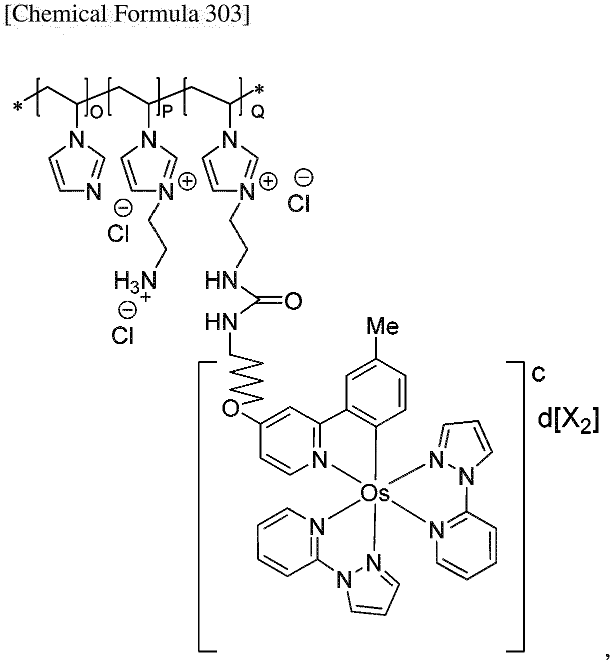

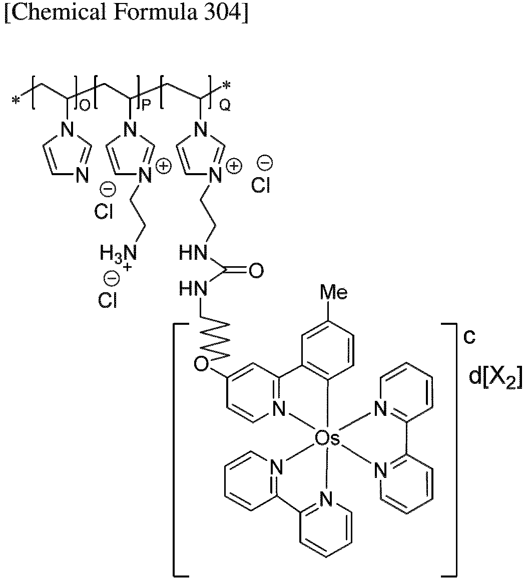

In Chemical Formulae 286 to 304, c and d may each be 1 or 2.

Further, in Chemical Formulae 286 to 304, X may be one counter ion selected from the group consisting of F, Cl, I, and PF₆.

In addition, in Chemical Formulae 286 to 304, O, P, and Q may each be an integer from 1 to 100.

In one aspect, the oxidation-reduction polymer may be for use in an electrochemical biosensor.

In one aspect, the electrochemical biosensor may be an electrochemical biosensor which is capable of being inserted into the body.

Furthermore, in one aspect, the electrochemical biosensor may be for measuring blood glucose.

Further, in one aspect, the electrochemical biosensor may be a continuous blood glucose monitoring sensor.

In addition, in one aspect, the present invention may be a sensing layer for an electrochemical biosensor, including an enzyme capable of oxidizing-reducing a biological sample; and the transition metal complex or the salt compound thereof, or the oxidation-reduction polymer as an electron transfer mediator.

Furthermore, in one aspect, the present invention may be a continuous blood glucose monitoring sensor including the sensing layer for an electrochemical biosensor.

In one aspect, the continuous blood glucose monitoring sensor may include, for example, an electrode, an insulator, a substrate, a sensing layer including the oxidation-reduction polymer and the oxidation-reduction enzyme, a diffusion layer, a protection layer, and the like. The electrode may include two types of electrodes, such as a working electrode and a facing (counter) electrode, and may include three types of electrodes, such as a working electrode, a counter electrode, and a reference electrode.

In one aspect, the biosensor may be an electrochemical biosensor manufactured by applying a reagent composition including the transition metal complex or the salt compound thereof; or the oxidation-reduction polymer and an enzyme capable of oxidizing-reducing a liquid biological sample to a substrate having at least two, preferably two or three, electrodes, and then drying the applied composition.

For example, in the electrochemical biosensor, there is provided a planar electrochemical biosensor, characterized in that a working electrode and a counter electrode are provided on opposite sides of a substrate, a sensing layer including the transition metal complex or the oxidation-reduction polymer of the present invention is stacked on the working electrode, and an insulator, a diffusion layer, and a protection layer are sequentially stacked on both surfaces of the substrate on which the working electrode and the counter electrode are provided.

In one aspect, the substrate may be made of one or more materials selected from the group consisting of polyethylene terephthalate (PET), polycarbonate (PC), and polyimide (PI).

Further, a carbon, gold, platinum, silver or silver/silver chloride electrode may be used as the working electrode.

In addition, in the case of an electrochemical biosensor having two electrodes, since the counter electrode also serves as the reference electrode, a gold, platinum, silver, or silver/silver chloride electrode may be used as the counter electrode, and in the case of an electrochemical biosensor having three electrodes including a reference electrode, a gold, platinum, silver, or silver/silver chloride electrode may be used as the reference electrode, and a carbon electrode may be used as the counter electrode.

As the diffusion layer, Nafion, cellulose acetate, silicone rubber, and the like may be used, and as the protection layer, silicone rubber, polyurethane, a polyurethane-based copolymer, and the like may be used, but the diffusion layer and the protection layer are not limited thereto.

In one aspect, when the electrochemical biosensor has two electrodes, the counter electrode also serves as the reference electrode, so that silver chloride or silver may be used, and when the electrochemical biosensor has three electrodes, silver chloride or silver is used as the reference electrode, and a carbon electrode may be used as the counter electrode.

By varying the type of enzyme included in the reagent composition of the present invention, the composition may also be applied to biosensors for quantifying various materials such as cholesterol, lactate, creatinine, hydrogen peroxide, an alcohol, an amino acid, and glutamate.

In one aspect, the biological sample may include a liquid biological sample.

The liquid biological sample may be, for example, one or more, two or more, three or more, four or more, or five or more selected from the group consisting of tissue fluid, blood, cells, plasma, serum, urine, cyst fluid, and saliva from a human, an animal, or the like.

Furthermore, in one aspect, the enzyme may include one or more oxido-reductases selected from the group consisting of a dehydrogenase, an oxidase, and an esterase; and one or more oxido-reductases selected from the group consisting of a dehydrogenase, an oxidase, and an esterase, and one or more cofactors selected from the group consisting of a flavin adenine dinucleotide (FAD), a nicotinamide adenine dinucleotide (NAD), and a pyrroloquinoline quinone (PQQ).

The oxido-reductase is a general term for enzymes that catalyze oxidation-reduction reactions in a living organism, and refers, in the present invention, to an enzyme that reacts with a target material to be measured, for example, in the case of a biosensor, a target material to be measured and is reduced. The enzyme reduced as described above reacts with an electron transfer mediator, and the target material is quantified by measuring a signal generated in this case, such as a change in electric current. The oxido-reductase that can be used in the present invention may be one or more types selected from the group consisting of various dehydrogenases, oxidases, esterases, and the like, and depending on the oxidation reduction or the material to be detected, an enzyme that uses the target material as a substrate may be selected from the enzymes belonging to the above enzyme group and used.

More specifically, the oxido-reductase may be one or more selected from the group consisting of a glucose dehydrogenase, a glutamate dehydrogenase, a glucose oxidase, a cholesterol oxidase, a cholesterol esterase, a lactate oxidase, an ascorbic acid oxidase, an alcohol oxidase, an alcohol dehydrogenase, a bilirubin oxidase, and the like.

The oxido-reductase may include a cofactor that serves to store the hydrogen removed by the oxido-reductase from the target material to be measured (for example, glucose), and may be, for example, one or more selected from the group consisting of a flavin adenine dinucleotide (FAD), a nicotinamide adenine dinucleotide (NAD), a pyrroloquinoline quinone (PQQ), and the like.

For example, when measuring the concentration of blood glucose, a glucose dehydrogenase (GDH) may be used as the oxido-reductase, and the glucose dehydrogenase may be a flavin adenine dinucleotide-glucose adenine dehydrogenase (FAD-GDH) including the FAD as a cofactor, and/or a nicotinamide adenine dinucleotide-glucose dehydrogenase including the FAD-GDH as a cofactor.

In a specific example, the oxido-reductase that can be used may be one or more selected from the group consisting of a FAD-GDH (for example, EC 1.1.99.10, and the like), a NAD-GDH (for example, EC 1.1.1.47, and the like), a PQQ-GDH (for example, EC 1.1.5.2, and the like), a glutamate dehydrogenase (for example, EC 1.4.1.2, and the like), a glucose oxidase (for example, EC 1.1.3.4, and the like), a cholesterol oxidase (for example, EC 1.1.3.6, and the like), a cholesterol esterase (for example, EC 3.1.1.13, and the like), a lactate oxidase (for example, EC 1.1.3.2, and the like), an ascorbic acid oxidase (for example, EC 1.10.3.3, and the like), an alcohol oxidase (for example, EC 1.1.3.13, and the like), an alcohol dehydrogenase (for example, EC 1.1.1.1, and the like), a bilirubin oxidase (for example, EC 1.3.3.5, and the like), and the like.

In one aspect, the oxido-reductase is a glucose dehydrogenase that can maintain an activity of 70% or more in a 37°C buffer solution for one week.

The sensing layer according to one aspect may contain the oxidation-reduction polymer in an amount of 20 to 700 parts by weight, or for example, 60 to 700 parts by weight, or for example, 30 to 340 parts by weight based on 100 parts by weight of the oxido-reductase. The content of the oxidation-reduction polymer may be appropriately adjusted depending on the activity of the oxido-reductase.

Further, the sensing layer may further include carbon nanotubes to increase the film performance. Specifically, when carbon nanotubes are used with a transition metal complex, particularly, osmium, the electron transfer rate increases, further enhancing the performance of the sensing layer.

In addition, in one aspect, the sensing layer may further include a crosslinker.

In one aspect, the sensing layer may further include one or more additives selected from the group consisting of a surfactant, a water-soluble polymer, a quaternary ammonium salt, a fatty acid, a thickener, and the like to serve as a dispersant when a reagent is dissolved, an adhesive when a reagent is prepared, a stabilizer for long-term storage, and the like.

The surfactant may allow a composition to be dispensed evenly on the electrodes when the composition is dispensed, thereby allowing the composition to be dispensed with a uniform thickness. As the surfactant, one or more selected from the group consisting of Triton X-100, sodium dodecyl sulfate, perfluorooctane sulfonate, sodium stearate, and the like may be used.

The reagent composition may contain the surfactant in an amount of 3 to 25 parts by weight, for example, 10 to 25 parts by weight, based on 100 parts by weight of the oxido-reductase, such that when the reagent is dispensed, the reagent is evenly spread on an electrode and can be dispensed to a uniform thickness. For example, when an oxido-reductase having an activity of 700 U/mg is used, the surfactant may be contained in an amount of 10 to 25 parts by weight based on 100 parts by weight of the oxido-reductase, and when the activity of the oxido-reductase is higher than this value, the content of the surfactant may be adjusted to a lower value.

The water-soluble polymer may serve to aid in stabilizing and dispersing the enzyme, as a polymeric support for the reagent composition. As the water-soluble polymer, it is possible to use one or more selected from the group consisting of polyvinyl pyrrolidone (PVP), polyvinyl alcohol (PVA), polyperfluoro sulfonate, hydroxyethyl cellulose (HEC), hydroxypropyl cellulose (HPC), carboxy methyl cellulose (CMC), cellulose acetate, polyamide, and the like. The reagent composition may contain the water-soluble polymer in an amount of 10 to 70 parts by weight, for example 30 to 70 parts by weight, based on 100 parts by weight of the oxido-reductase, in order to sufficiently and appropriately exhibit a role to aid in stabilizing and dispersing the oxido-reductase. For example, when an oxido-reductase having an activity of 700 U/mg is used, the water-soluble polymer may be contained in an amount of 30 to 70 parts by weight based on 100 parts by weight of the oxido-reductase, and when the activity of the oxido-reductase is higher than this value, the content of the water-soluble polymer may be adjusted to a lower value.

The water-soluble polymer may have a weight average molecular weight of about 2,500 g/mol to about 3,000,000 g/mol, for example, about 5,000 g/mol to about 1,000,000 g/mol, in order to effectively perform the role of aiding in stabilizing and dispersing the support and the enzyme.

The thickener serves to cause the reagent to be firmly attached to the electrode. As the thickener, one or more selected from the group consisting of Natrosol, diethylaminoethyl-dextran hydrochloride (DEAE-dextran hydrochloride), and the like may be used. In one aspect, the electrochemical sensor may contain the thickener in an amount of 10 to 90 parts by weight, for example, 30 to 90 parts by weight, based on 100 parts by weight of the oxido-reductase, such that the oxidation-reduction polymer is firmly attached to the electrode. For example, when an oxido-reductase having an activity of 700 U/mg is used, the thickener may be contained in an amount of 30 to 90 parts by weight based on 100 parts by weight of the oxido-reductase, and when the activity of the oxido-reductase is higher than this value, the content of the thickener may be adjusted to a lower value.

Hereinafter, the present invention will be specifically described through Examples and Experimental Examples. Since these examples are provided only for exemplifying the present invention, the scope of the present invention is not limited by these examples.

### [Examples]

### [Example 1] Preparation of transition metal complex according to the present invention

### [Example 1.1] Synthesis of transition metal complex of Chemical Formula 5 (r = 6)

### 1) Synthesis of 2-(3,4,5-trimethyl-1H-pyrazol-1-yl)-4-methylpyridine

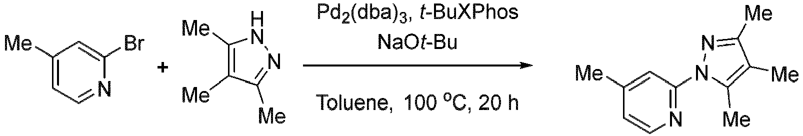

1.3 g (7.6 mmol) of 2-bromo-4-methylpyridine, 1.1 g (9.8 mmol) of 3,4,5-trimethylpyrazole, 0.41 g (0.45 mmol) of Pd₂(dba)₃, 0.38 g (0.9 mmol) of t-BuXPhos, 1.1 g (11.4 mmol) of sodium tertiary butoxide, and 25 mL of anhydrous toluene were put into a 100 mL Schlenk flask dried in an oven, and the resulting mixture was heated to 100°C in an argon gas atmosphere and stirred for 20 hours. After the reaction was completed, the reaction mixture was cooled to room temperature and extracted with water (100 mL) and ethyl acetate (100 mL x 3). The organic layers were collected and concentrated under reduced pressure, and after the solvent was removed, the residue was purified by column chromatography using ethyl acetate and hexane as developing solvents (hexane : ethyl acetate = 9:1). Finally, 2-(3,4,5-trimethyl-1H-pyrazol-1-yl)-4-methylpyridine was obtained as a white solid (1.4 g, 85%).

### 2) Synthesis of Os(3,4,5-TriMe-pz-4-Me-py)₂Cl₂

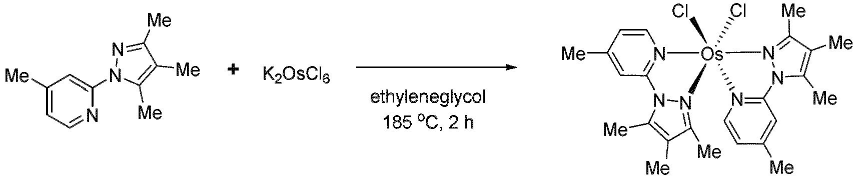

0.6 g (1.3 mmol) of potassium hexachloroosmate (IV) and 0.5 g (2.5 mmol) of 2-(3,4,5-trimethyl-1*H*-pyrazol-1-yl)-4-methylpyridine prepared in 1.1 above were put into a 50 mL Schlenk flask, and the resulting mixture was dissolved in 30 mL of ethylene glycol in an argon gas atmosphere, and then degassed with argon for 15 minutes. The reaction mixture was heated to 185°C and stirred for 2 hour. After the reaction was completed, the reaction mixture was cooled to room temperature, and the resulting red precipitate was removed by filtration under reduced pressure. The filtrate was added dropwise to a 1.0 M aqueous solution (100 mL) of sodium dithionite to obtain a precipitate of a reduced osmium composite. The resulting solid was filtered under reduced pressure, washed several times with water and acetonitrile, and then dried in a vacuum oven to obtain an osmium composite as a green final compound ((0.7 g, 89%) HRMS (¹⁹²Os): m/z [M₀]⁺ = 636.1200([M₀]⁺ required 636.1205)).

### 3) Synthesis of Chemical Formula 5 (r = 6)

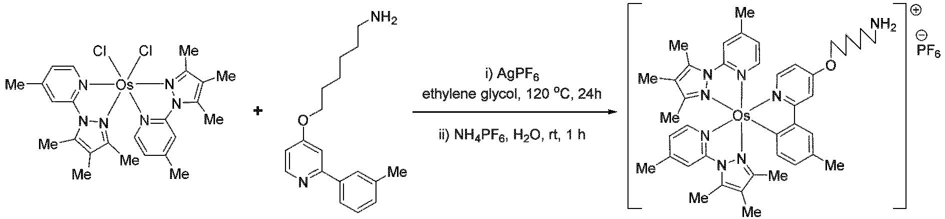

1.0 g (1.5 mmol) of Os(3,4,5-TriMe-pz-4-Me-py)₂Cl₂ prepared in 1.1-2 above, 0.8 g (2.9 mmol) of a phenylpyridine derivative AP1, and 0.6 g (2.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 120°C and stirred for 24 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark green final compound ((1.0 g, 68%) HRMS (¹⁹²Os): m/z [(M₀+H)-PF₆]³⁺ 292.8007 required 292.8006)).

The overall method for preparing the compound of Chemical Formula 56 (r = 6) is as described in Reaction Scheme 1 below.

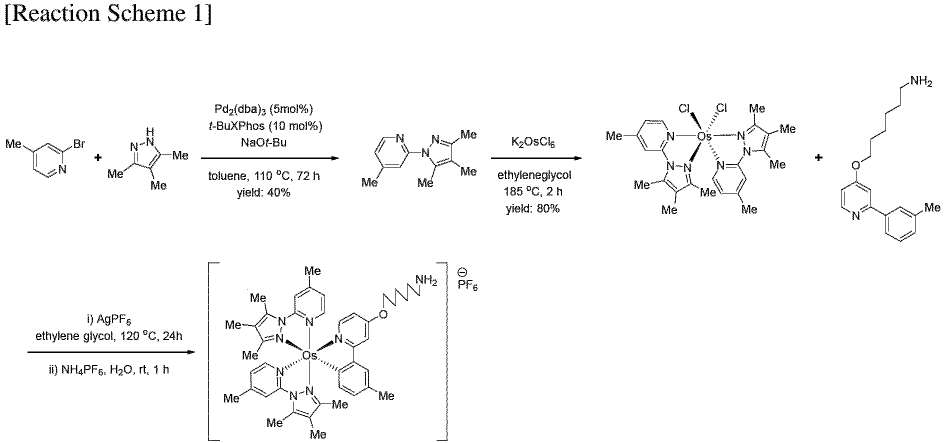

### [Example 1.2] Synthesis of transition metal complex of Chemical Formula 56 (r = 6)

### 1) Synthesis of 2-(3,4,5-trimethyl-1H-pyrazol-1-yl)-4-methoxypyridine

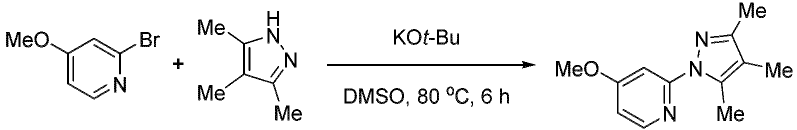

A 50 mL two-neck round-bottom flask was equipped with a reflux condenser and a gas inlet, and 0.6 g (6 mmol) of 3,4,5-trimethylpyrazole and 0.7 g (6 mmol) of potassium tertiary butyoxide were put into the flask and dissolved in 8 mL of anhydrous dimethyl sulfoxide in an argon gas atmosphere. 1.0 g (5.0 mmol) of 2-bromo-4-methoxypyridine was added to the reaction mixture, and the resulting mixture was heated to 80°C in an argon gas atmosphere and stirred for 6 hours. After the reaction was completed, the reaction mixture was cooled to room temperature and extracted with water (50 mL) and ethyl acetate (50 mL x 3). The organic layers were collected and concentrated under reduced pressure, and after the solvent was removed, the residue was purified by column chromatography using ethyl acetate and hexane as developing solvents. (hexane : ethyl acetate = 3:1) Finally, 4-methyl-2-(3-methyl-1H-pyrazol-1-yl)pyridine was obtained as a clear solid (0.4 g, 37%).

### 2) Synthesis of Os(3,4,5-TriMe-pz-4-MeO-py)₂Cl₂

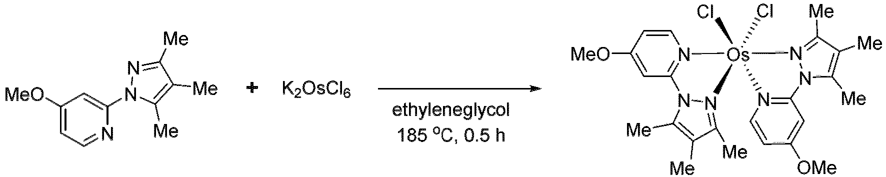

A magnetic bar, 0.5 g (1.0 mmol) of potassium hexachloroosmate (IV), and
0.4 g (2.0 mmol) of 2-(3,4,5-trimethyl-1H-pyrazol-1-yl)-4-methoxypyridine prepared in 1.2-1 above were put into a 50 ml Schlenk flask, and the resulting mixture was dissolved in 15 mL of ethylene glycol in an argon gas atmosphere, and then degassed with argon for 15 minutes. The reaction mixture was heated to 180°C and stirred for 30 minutes. After the reaction was completed, the reaction mixture was cooled to room temperature, and the resulting red precipitate was removed by filtration under reduced pressure. The filtrate was added dropwise to a 1.0 M aqueous solution (100 mL) of sodium dithionite to obtain a precipitate of a reduced osmium composite. The resulting solid was filtered under reduced pressure, washed several times with water and acetonitrile, and then dried in a vacuum oven to obtain an osmium composite as a brown final compound (0.4 g, 64%) HRMS (¹⁹²Os): m/z [M₀]⁺ = 696.1403 ([M⁺] required 696.1411)

### 3) Synthesis of Chemical Formula 56 (r = 6)

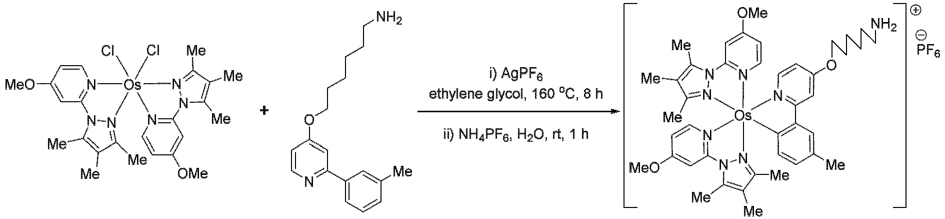

0.20 g (0.3 mmol) of Os(3,4,5-TriMe-pz-4-MeO-py)₂Cl₂ prepared in 1.2-2 above, 0.25 g (0.9 mmol) of a phenylpyridine derivative AP1, and 0.22 g (0.9 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark green final compound (0.2 g, 63%) HRMS (¹⁹²Os): m/z [(M₀+H)-PF₆]³⁺ = 303.4642 ([(M₀+H)-PF₆]³⁺ = required 303.4639).

The overall method for preparing the compound of Chemical Formula 56 (r = 6) is as described in Reaction Scheme 2 below.

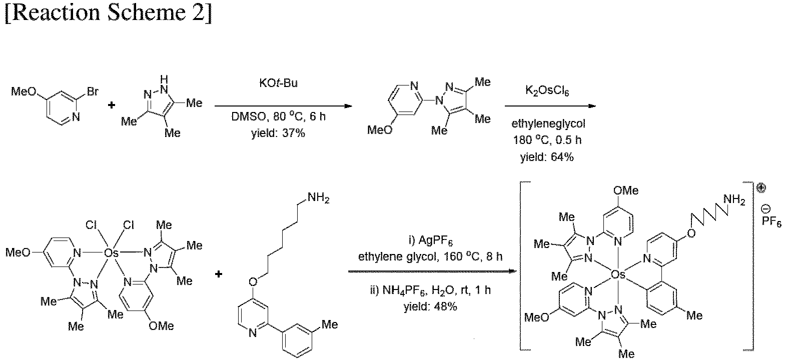

### [Example 1.3] Synthesis of transition metal complex of Chemical Formula 86 (r = 6)

### 1) Synthesis of N,N-dimethyl-2-(3,4,5-trimethyl-1H-pyrazol-1-yl)pyridin-4-amine

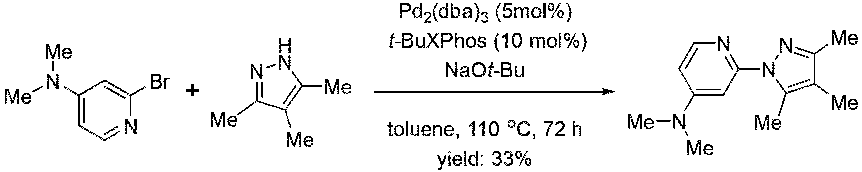

A 500 mL three-neck round-bottom flask was equipped with a reflux condenser, a gas inlet, and a thermometer, 1.4 g (7.0 mmol) of 4-dimethylamino-2-bromopyridine, 1.0 g (9.0 mmol) of 3,4,5-trimethylpyrazole, 0.4 g (0.4 mmol) of Pd₂(dba)₃, 0.4 g (0.8 mmol) of t-BuXPhos, 1.0 g (10.5 mmol) of sodium tertiary butoxide, and 200 mL of anhydrous toluene were put into the flask, and the resulting mixture was heated to 110°C in an atmosphere and stirred for 72 hours. After the reaction was completed, the reaction mixture was cooled to room temperature and extracted with water (150 mL) and ethyl acetate (150 mL x 3). The organic layers were collected and concentrated under reduced pressure, and after the solvent was removed, the residue was purified by column chromatography using ethyl acetate and hexane as developing solvents. (Hexane : ethyl acetate = 2:1) Finally, N,N-dimethyl-2-(3,4,5-trimethyl-1H-pyrazol-1-yl)-pyridin-4-amine was obtained as a white solid (0.6 g, 33%).

### 2) Synthesis of Os(3,4,5-TriMe-pz-4-DiAM-py)₂Cl₂

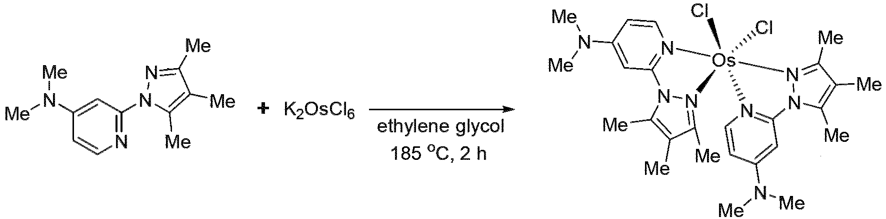

0.6 g (1.2 mmol) of potassium hexachloroosmate (IV) and 0.5 g (2.4 mmol) of *N,N-*dimethyl-2-(4-methyl-1H-pyrazol-1-yl)pyridin-4-amine prepared in 1.3-1 above were put into 50 mL Schlenk flask, and the resulting mixture was dissolved in 15 mL of ethylene glycol in an argon gas atmosphere, and then degassed with argon for 15 minutes. The reaction mixture was heated to 185°C and stirred for 2 hour. After the reaction was completed, the reaction mixture was cooled to room temperature, and the resulting red precipitate was removed by filtration under reduced pressure. The filtrate was added dropwise to a 1.0 M aqueous solution (100 mL) of sodium dithionite to obtain a precipitate of a reduced osmium composite. The resulting solid was filtered under reduced pressure, washed several times with water and acetonitrile, and then dried in a vacuum oven to obtain an osmium composite as a reddish-black final compound. (0.5 g, 75%) HRMS (¹⁹²Os): m/z [M₀]⁺ = 722.2027 ([M₀]⁺ = required 722.2043)

### 3) Synthesis of Chemical Formula 86 (r = 6)

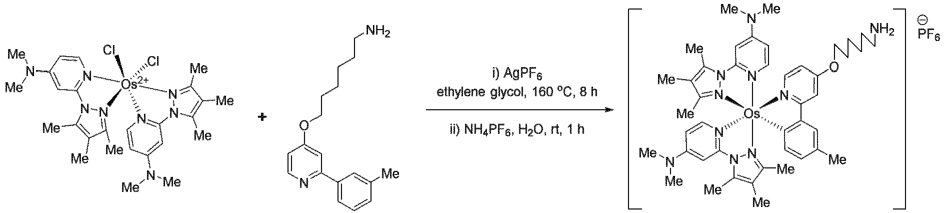

0.21 g (0.3 mmol) of Os(3,4,5-TriMe-pz-4-DiAM-py)₂Cl₂ prepared in 1.3-2 above, 0.26 g (0.9 mmol) of a phenylpyridine derivative AP1, and 0.23 g (0.9 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark green final compound (0.15 g, 48%) HRMS (¹⁹²Os): m/z [(M₀+H)-PF₆]³⁺ = 312.1513 ([(M₀+H)-PF₆]³⁺ = required 312.1516).

The overall method for preparing the compound of Chemical Formula 86 (r = 6) is as described in Reaction Scheme 3 below.

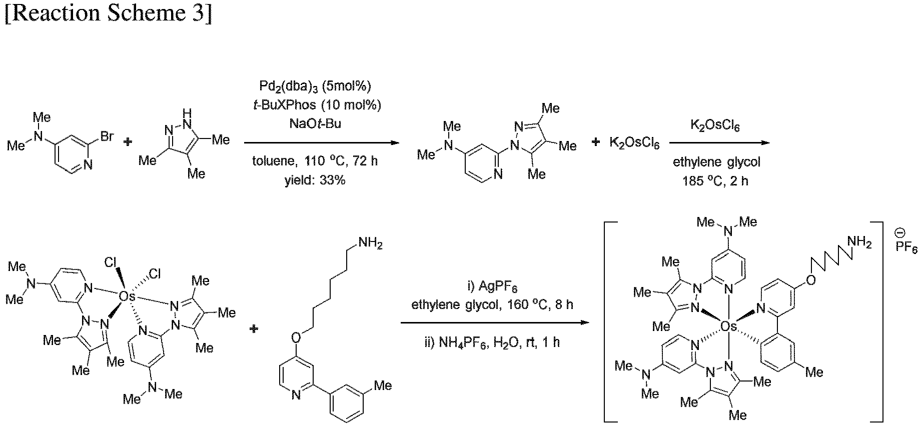

### [Example 1.4] Synthesis of transition metal complex of Chemical Formula 168

### 1) Synthesis of 2-(1H-pyrazol-1-yl)pyridine

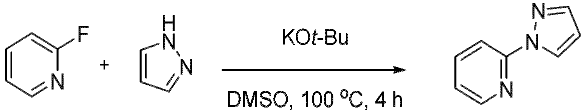

A 250 mL two-neck round-bottom flask was equipped with a reflux condenser and a gas inlet, and 4.7 g (69 mmol) of pyrazole and 9.3 g (83 mmol) of potassium tertiary butyoxide were put into the flask and dissolved in 40 mL of anhydrous dimethyl sulfoxide in an argon gas atmosphere. 8.0 g (83 mmol) of 2-fluoropyridine was added to the reaction mixture, and the resulting mixture was heated to 100°C in an argon gas atmosphere and stirred for 4 hours. After the reaction was completed, the reaction mixture was cooled to room temperature and extracted with water (100 mL) and ethyl acetate (100 mL x 3). The organic layers were collected, dried over magnesium sulfate, and concentrated under reduced pressure to obtain a clear, colorless solid. (7.2 g, 72%)

### 2) Synthesis of Os(pzpy)₂Cl₂ [Chemical Formula 3]

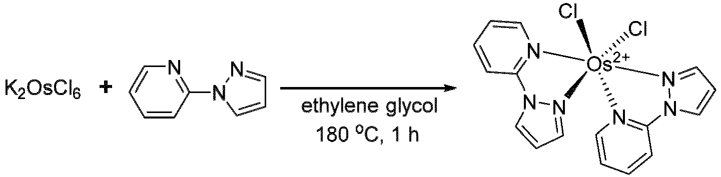

5.0 g (10 mmol) of potassium hexachloroosmate (IV) and 2.9 g (20 mmol) of 2-(1N-pyrazol-1-yl)pyridine prepared in 1.4-1 above were put into a 500 mL Schlenk flask, and the resulting mixture was dissolved in 200 mL of ethylene glycol in an argon gas atmosphere, and then degassed with argon for 15 minutes. The reaction mixture was heated to 180°C and stirred for 1 hour. After the reaction was completed, the reaction mixture was cooled to room temperature, and the resulting red precipitate was removed by filtration under reduced pressure. The filtrate was added dropwise to a 1.0 M aqueous solution (250 mL) of sodium dithionite to obtain a precipitate of a reduced osmium composite. The resulting solid was filtered under reduced pressure, washed several times with water, and then dried in a vacuum oven at 40°C to obtain an osmium composite as a green final compound. (4.0 g, 75%) HRMS (¹⁹²Os): m/z [M₀]⁺ = 552.0240 ([M₀]⁺ = required 552.0261)

### 3) Synthesis of Chemical Formula 168

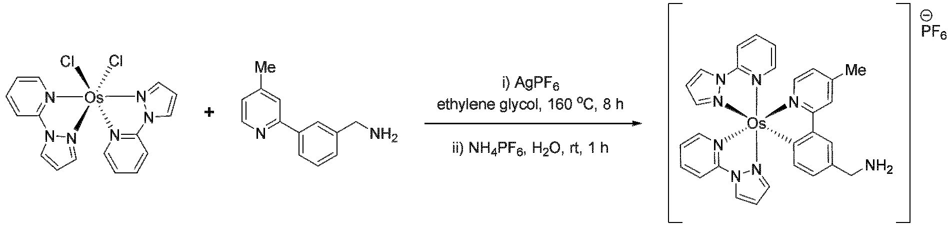

0.20 g (0.4 mmol) of Os(pzpy)₂Cl₂ prepared in 1.4-2 above, 0.24 g (1.2 mmol) of 3-(4-methylpyridin-2-yl)phenylmethanamine, and 0.30 g (1.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.24 g, 73%) HRMS (¹⁹²Os): m/z [(M₀+H)-PF₆]³⁺ = 226.7348 ([(M₀+H)-PF₆]³⁺ = required 226.7345)

The overall method for preparing the compound of Chemical Formula 168 is as described in Reaction Scheme 4 below.

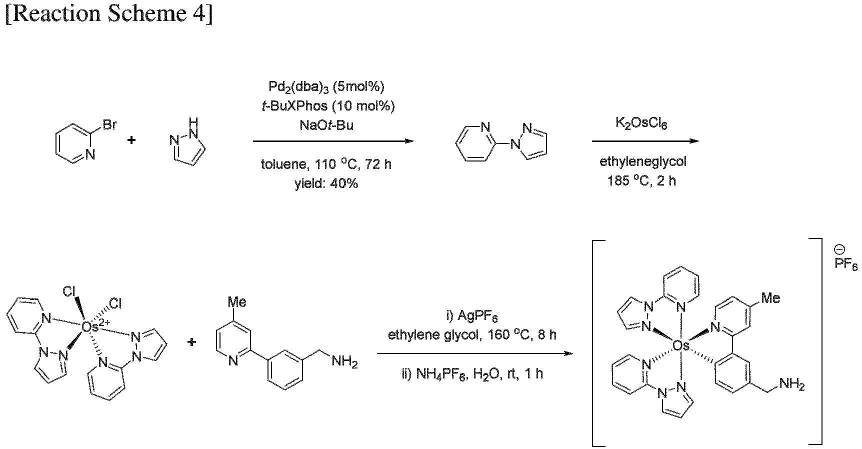

### [Example 1.5] Synthesis of transition metal complex of Chemical Formula 169 (s = 6)

### 1) Synthesis of Chemical Formula 169 (s = 6)

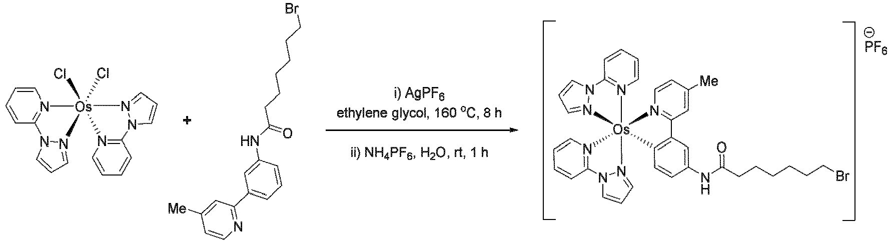

0.20 g (0.4 mmol) of Os(pzpy)₂Cl₂ prepared in 1.4-2 above, 0.45 g (1.2 mmol) of a phenylpyridine derivative AP2, and 0.30 g (1.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.2 g, 63%) HRMS (¹⁹²Os): m/z [(M₀+H)-PF₆]³⁺ = 285.3963 ([(M₀+H)-PF₆]³⁺ = required 285.3957)

The overall method for preparing the compound of Chemical Formula 169 (s = 6) is as described in Reaction Scheme 5 below.

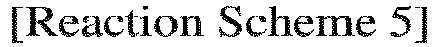

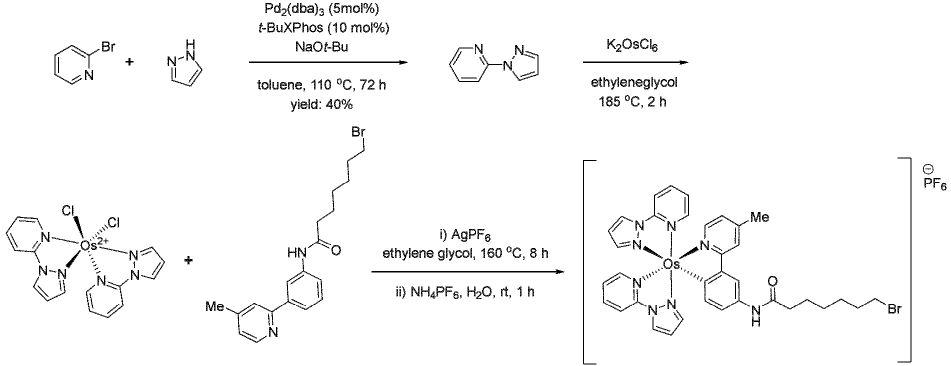

### [Example 1.6] Synthesis of transition metal complex of Chemical Formula 167 (r = 6)

### 1) Synthesis of [Chemical Formula 167] (r = 6)

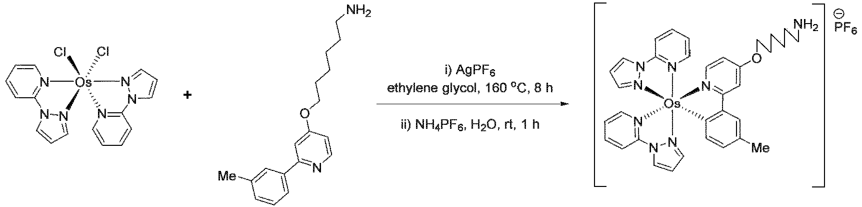

0.20 g (0.4 mmol) of Os(pzpy)₂Cl₂ prepared in 1.4-2 above, 0.34 g (1.2 mmol) of a phenylpyridine derivative AP1, and 0.30 g (1.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.22 g, 61%) HRMS (¹⁹²Os): m/z [(M₀+H)-PF₆]³⁺ = 255.4260 ([(M₀+H)-PF₆]³⁺ = required 255.4256)

The overall method for preparing the compound of Chemical Formula 167 (r = 6) is as described in Reaction Scheme 6 below.

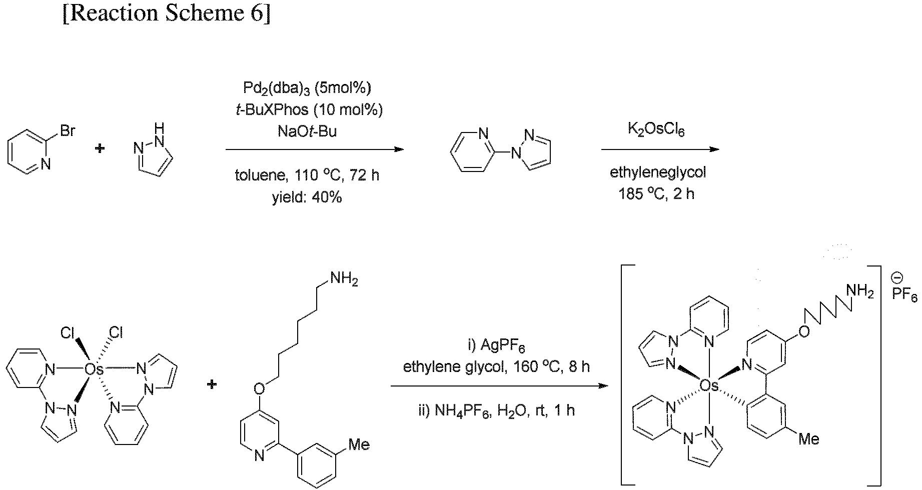

### [Example 1.7] Synthesis of transition metal complex of Chemical Formula 172

### 1) Synthesis of [Chemical Formula 172]

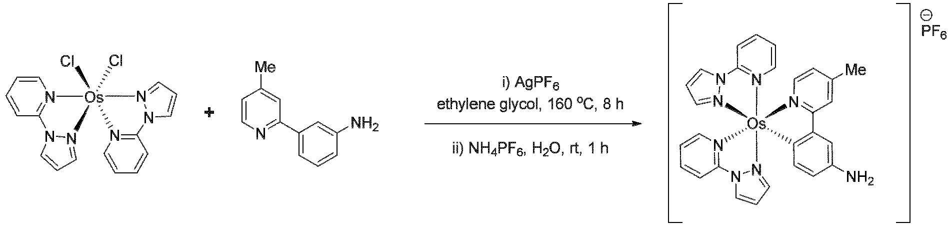

0.20 g (0.4 mmol) of Os(pzpy)₂Cl₂ prepared in 1.4-2 above, (1.2 mmol) of 3-(4-methylpyridin-2-yl)aniline, and 0.30 g (1.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.21 g, 65%) HRMS (¹⁹²Os): m/z [(M₀+H)-PF₆]³⁺ = 222.0632 ([(M₀+H)-PF₆]³⁺ = required 222.0626)

The overall method for preparing the compound of Chemical Formula 172 is as described in Reaction Scheme 7 below.

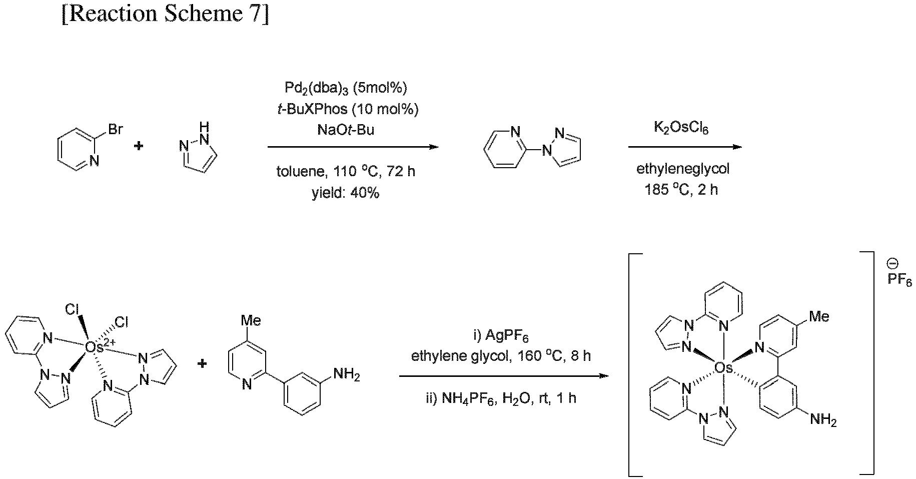

### [Example 1.8] Synthesis of transition metal complex of Chemical Formula 186

### 1) Synthesis of [Chemical Formula 186]

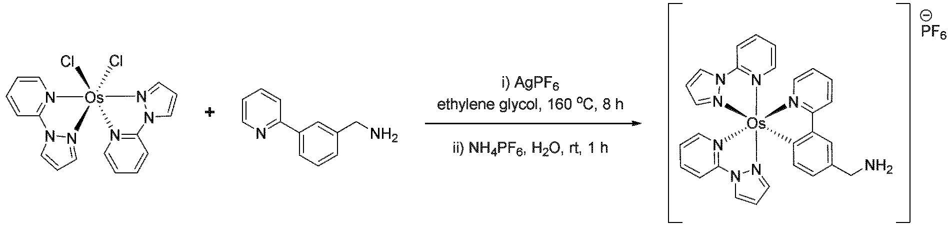

0.20 g (0.4 mmol) of Os(pzpy)₂Cl₂ prepared in 1.4-2 above, 0.22 g (1.2 mmol) of 3-(pyridin-2-yl)phenylmethanamine, and 0.30 g (1.2 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.23 g, 72%) HRMS (¹⁹²Os): m/z [(M₀+H)-PF₆]³⁺ = 222.0635 ([(M₀+H)-PF₆]³⁺ = required 222.0626)

The overall method for preparing the compound of Chemical Formula 186 is as described in Reaction Scheme 8 below.

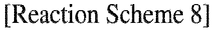

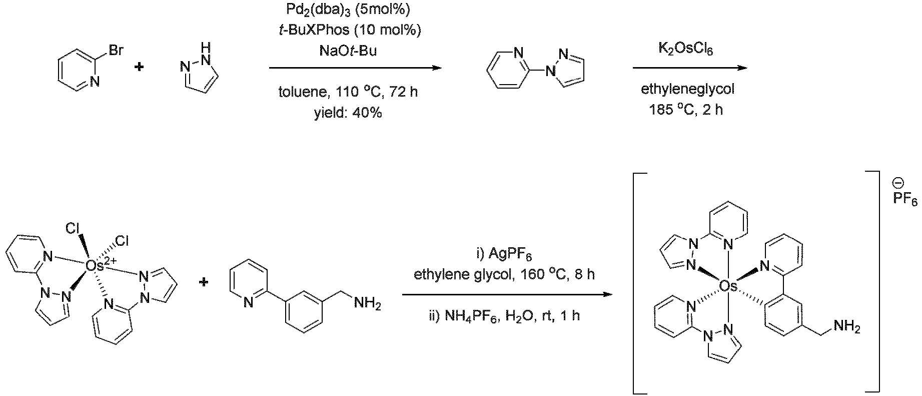

### [Example 1.9] Synthesis of transition metal complex of Chemical Formula 188

### 1) Synthesis of [Chemical Formula 188] (r = 6)

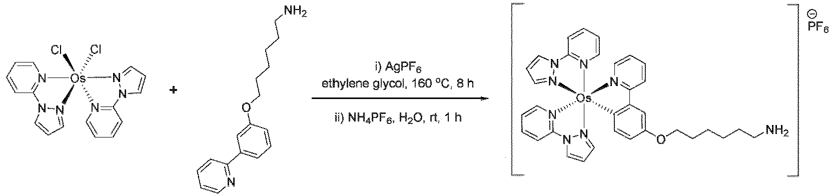

0.20 g (0.4 mmol) of Os(pzpy)₂Cl₂ prepared in 1.4-2 above, 0.32 g (1.2 mmol) of a phenylpyridine derivative AP3, and 0.30 g (1.2 mmol) of silver hexafluorophosphate were put into a 100 mL Schlenk flask, and the resulting mixture was dissolved in 50 mL of ethylene glycol in an argon gas atmosphere, and then degassed with argon for 15 minutes. The reaction mixture was heated to 160°C and stirred for 8 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a green final compound. (0.24 g, 69%) HRMS (¹⁹²Os): m/z [(M₀+H)-PF₆]³⁺ = 250.7540 ([(M₀+H)-PF₆]³⁺ = required 250.7537)

The overall method for preparing the compound of Chemical Formula 188 (r = 6) is as described in Reaction Scheme 9 below.

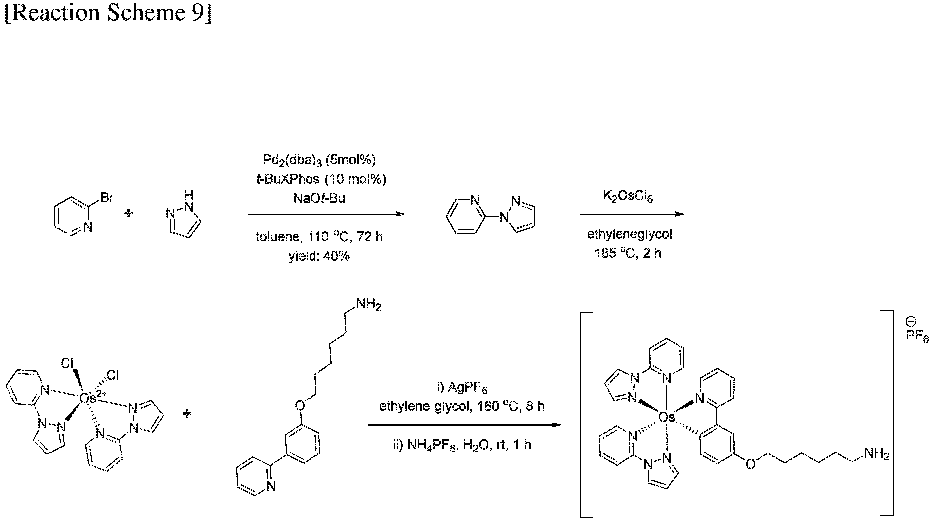

### [Example 1.10] Synthesis of transition metal complex of Chemical Formula 203 (r = 6)

### 1) Synthesis of Os(4,4-Dimethoxy-2,2-dipyridyl)₂Cl₂

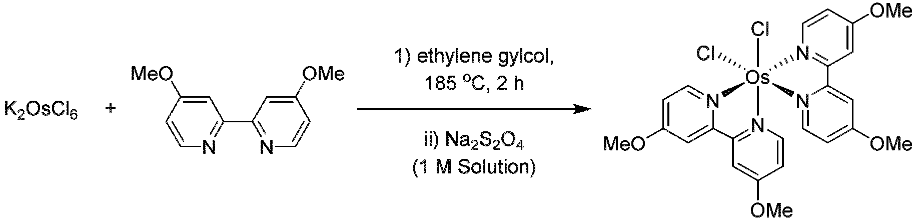

A magnetic bar, 2.0 g (4.2 mmol) of potassium hexachloroosmate (IV), and 1.8 g (8.3 mmol) of 4,4-dimethoxy-2,2'-dipyridine were put into a 100 mL Schlenk flask, and the resulting mixture was dissolved in 50 mL of ethylene glycol in an argon gas atmosphere, and then degassed with argon for 15 minutes. The reaction mixture was heated to 185°C and stirred for 2 hours. After the reaction was completed, the reaction mixture was cooled to room temperature, and the resulting red precipitate was removed by filtration under reduced pressure. The filtrate was added dropwise to a 1.0 M aqueous solution (200 mL) of sodium dithionite to obtain a precipitate of a reduced osmium composite. The resulting solid was filtered under reduced pressure, washed several times with water and acetonitrile, and then dried in a vacuum oven to obtain an osmium composite as a reddish-brown final compound (2.2 g, 76%) HRMS (¹⁹²Os): m/z [M₀]⁺ = 694.0789 ([M₀]⁺ = required 694.0783).

### 2) Synthesis of Chemical Formula 203 (r = 6)

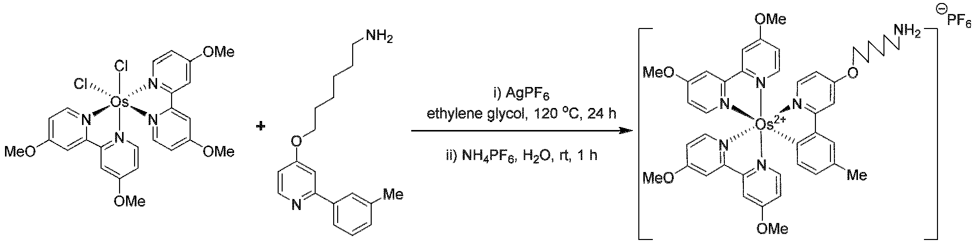

0.3 g (0.4 mmol) of Os(4,4-dimethoxy-2,2-dipyridyl)₂Cl₂ prepared in 1.10-1 above, 0.22 g (0.8 mmol) of a phenylpyridine derivative API, and 0.15 g (0.6 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 120°C and stirred for 24 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.2 g, 47%) HRMS (¹⁹²Os): m/z [(M₀+H)-PF₆]²⁺ = 454.1645 ([(M₀+H)-PF₆]²⁺ = required 454.1650).

The overall method for preparing the compound of Chemical Formula 203 is as described in Reaction Scheme 10 below.

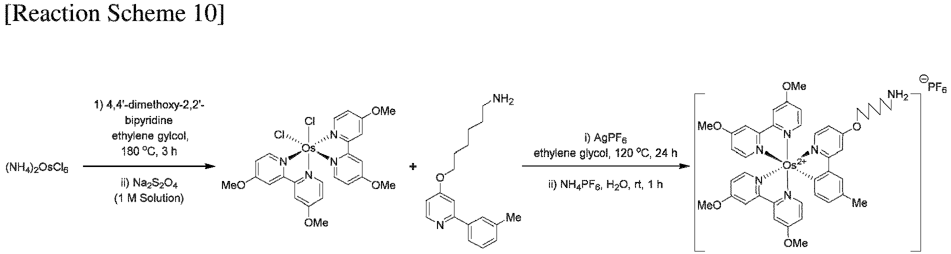

### [Example 1.11] Synthesis of transition metal complex of Chemical Formula 230 (r = 6)

### 1) Synthesis of Os(4,4-Dimethyl-2,2-dipyridyl)₂Cl₂

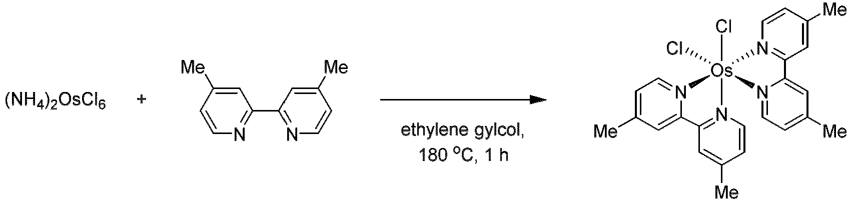

A magnetic bar, 4.0 g (9.1 mmol) of ammonium hexachloroosmate (IV), and 3.4 g (18.2 mmol) of 4,4-dimethyl-2,2'-dipyridine were put into a 250 mL round Schlenk flask, and the resulting mixture was dissolved in 150 mL of ethylene glycol in an argon gas atmosphere, and then degassed with argon for 15 minutes. The reaction mixture was heated to 185°C and stirred for 1 hour. After the reaction was completed, the reaction mixture was cooled to room temperature, and the resulting red precipitate was removed by filtration under reduced pressure. The filtrate was added dropwise to a 1.0 M aqueous solution (200 mL) of sodium dithionite to obtain a precipitate of a reduced osmium composite. The resulting solid was filtered under reduced pressure, washed several times with water and acetonitrile, and then dried in a vacuum oven to obtain an osmium composite as a blackish violet final compound. (5.1 g, 87%) HRMS (¹⁹²Os): m/z 630.0990 ([M⁺] required 630.0993).

### 2) Synthesis of Chemical Formula 230 (r = 6)

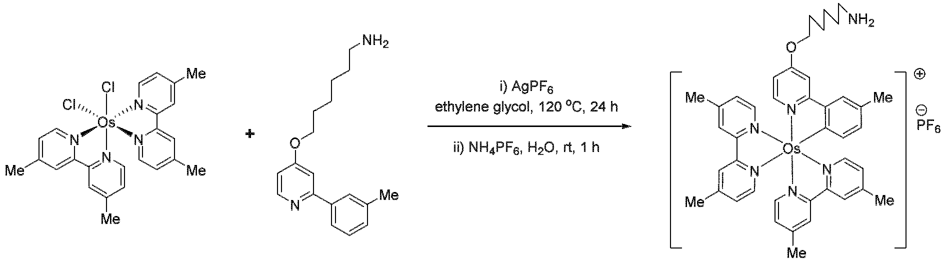

0.25 g (0.4 mmol) of Os(4,4-dimethyl-2,2-dipyridyl)₂Cl₂ prepared in 1.11-1 above, 0.22 g (0.8 mmol) of a phenylpyridine derivative AP1, and 0.15 g (0.6 mmol) of silver hexafluorophosphate were put into a 100 mL Schlenk flask, and the resulting mixture was dissolved in 50 mL of ethylene glycol in an argon gas atmosphere, and then degassed with argon for 15 minutes. The reaction mixture was heated to 120°C and stirred for 24 hours. After the reaction was completed, the reaction mixture was cooled to room temperature, and the resulting precipitate was removed by filtration under reduced pressure. The filtrate was added dropwise to a saturated aqueous solution (500 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.3 g, 88%) HRMS (¹⁹²Os): m/z [[(M₀+H)-PF₆]]²⁺ = 422.1747 ([[(M₀+H)-PF₆]²⁺ = required 422.1750).

The overall method for preparing the compound of Chemical Formula 230 is as described in Reaction Scheme 11 below.

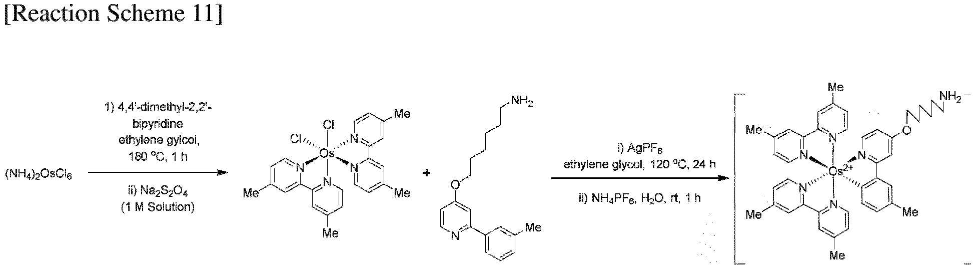

### [Example 1.12] Synthesis of transition metal complex of Chemical Formula 235

### 1) Synthesis of Chemical Formula 235

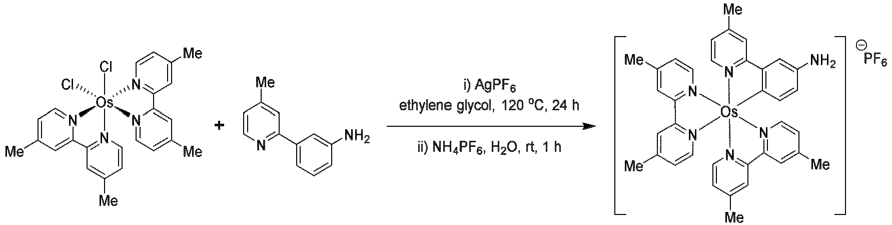

0.25 g (0.4 mmol) of Os(4,4-dimethyl-2,2-dipyridyl)₂Cl₂ prepared in 1.11-1 above, 0.15 g (0.8 mmol) of 3-(4-methylpyridin-2-yl)aniline, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 120°C and stirred for 24 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.2 g, 57%) HRMS (¹⁹²Os): m/z [M₀-PF₆]²⁺ = 364.6185 ([M₀-PF₆]²⁺ = required 364.6191).

The overall method for preparing the compound of Chemical Formula 235 is as described in Reaction Scheme 12 below.

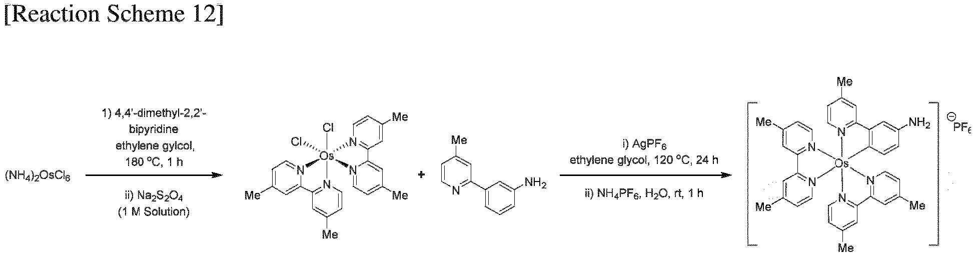

### [Example 1.13] Synthesis of transition metal complex of Chemical Formula 240

### 1) Synthesis of Chemical Formula 240

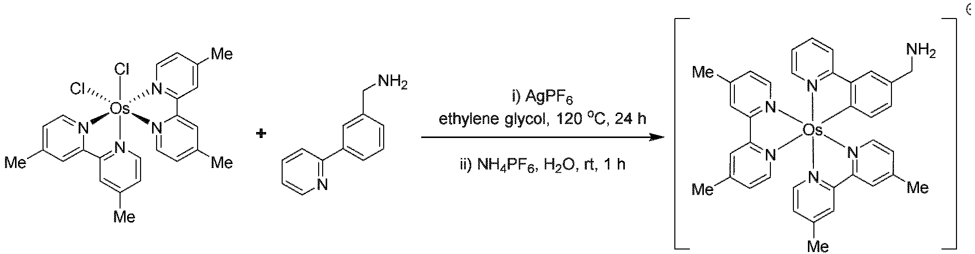

0.25 g (0.4 mmol) of Os(4,4-dimethyl-2,2-dipyridyl)₂Cl₂ prepared in 1.11-1 above, 0.15 g (0.8 mmol) of 3-(pyridin-2-yl)phenylmethanamine, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 120°C and stirred for 24 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.1 g, 28%) HRMS (¹⁹²Os): m/z [(M₀+H)-PF₆]²⁺ = 344.0990 ([(M₀+H)-PF₆]²⁺ = required 344.1001).

The overall method for preparing the compound of Chemical Formula 240 is as described in Reaction Scheme 13 below.

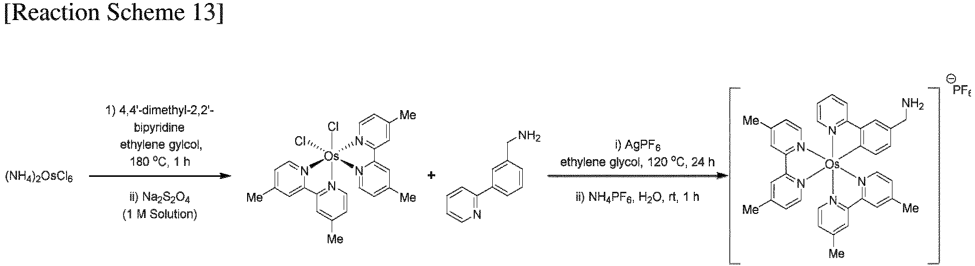

### [Example 1.14] Synthesis of transition metal complex of Chemical Formula 257 (r = 6)

### 1) Synthesis of Os(2,2-dipyridyl)₂Cl₂

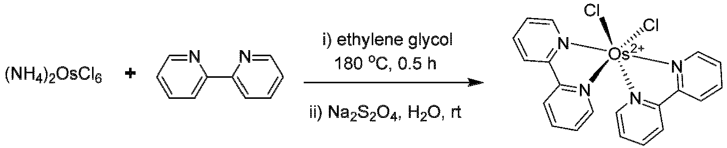

A magnetic bar was put into a 500 ml three-neck round bottom flask, and the flask is equipped with a thermometer, a reflux condenser, and a rubber septum. 5.0 g (11.4 mmol) of ammonium hexachloro-osmate (IV)(NH₄)₂OsCl₆ and 3.5 g (22.8 mmol) of 2,2'-dipyridine were added to the flask, and the resulting mixture was dissolved in 200 mL of ethylene glycol in an argon gas atmosphere, and then was degassed with argon for 15 minutes. The reaction mixture was heated to 180°C and stirred for 20 minutes. After the reaction was completed, the reaction mixture was cooled to room temperature, and the resulting red precipitate was removed by filtration under reduced pressure. The filtrate was added dropwise to a 1.0 M aqueous solution (250 mL) of sodium dithionite to obtain a precipitate of a reduced osmium composite. The resulting solid was filtered under reduced pressure, washed several times with water and acetonitrile, and then dried in a vacuum oven to obtain an osmium composite as a reddish-black final compound (5.7 g, 87%) HRMS (¹⁹²Os): m/z [M₀]⁺=574.0356 ([M₀]⁺ = required 574.0360).

### 2) Synthesis of Chemical Formula 257 (r = 6)

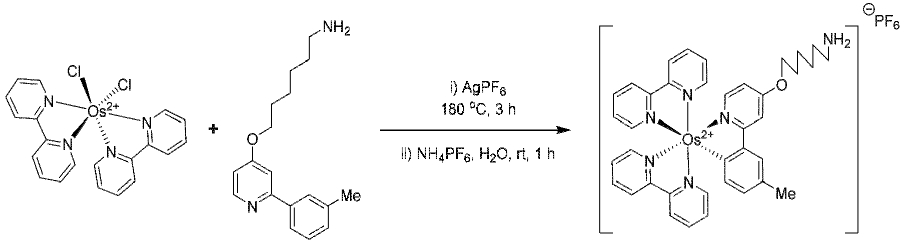

0.3 g (0.5 mmol) of Os(2,2-dipyridyl)₂Cl₂ prepared in 1.14-1 above, 0.22 g (0.8 mmol) of a phenylpyridine derivative AP1, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 180°C and stirred for 3 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.3 g, 51%) HRMS (¹⁹²Os): m/z [(M₀+H)-PF₆]²⁺ = 394.1434 ([(M₀+H)-PF₆]²⁺ = required 394.1430).

The overall method for preparing the compound of Chemical Formula 257 is as described in Reaction Scheme 14 below.

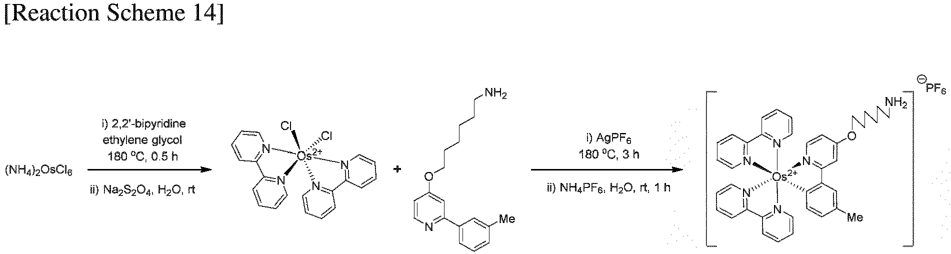

### [Example 1.15] Synthesis of transition metal complex of Chemical Formula 266 (r = 6)

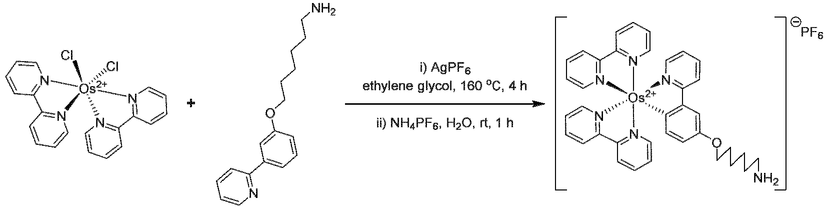

0.3 g (0.5 mmol) of Os(2,2-dipyridyl)₂Cl₂ prepared in 1.14-1 above, 0.22 g (0.8 mmol) of a phenylpyridine derivative AP3, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 180°C and stirred for 4 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.3 g, 52%) HRMS (¹⁹²Os): m/z [(M₀+H)-PF₆]²⁺ = 387.1355 ([(M₀+H)-PF₆]²⁺- required 387.1362).

The overall method for preparing the compound of Chemical Formula 266 is as described in Reaction Scheme 15 below.

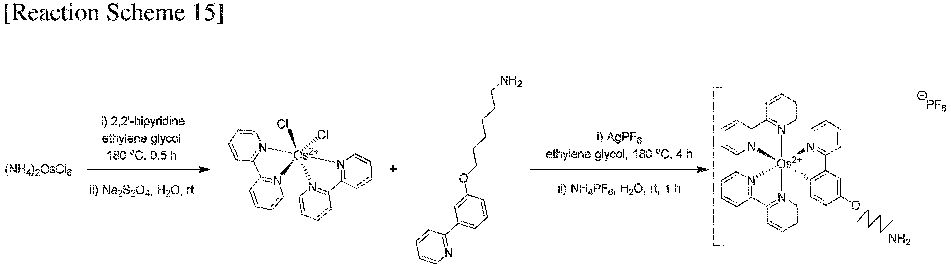

### [Example 1.16] Synthesis of transition metal complex of Chemical Formula 267

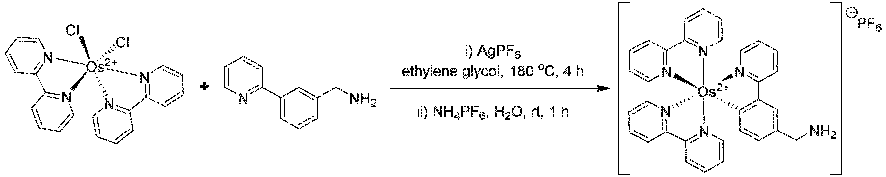

0.3 g (0.5 mmol) of Os(2,2-dipyridyl)₂Cl₂ prepared in 1.14-1 above, 0.15 g (0.8 mmol) of 3-(pyridin-2-yl)phenylmethanamine, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 180°C and stirred for 4 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.2 g, 55%) HRMS (¹⁹²Os): m/z [(M₀+H)-PF₆]²⁺= 344.0990 ([(M₀+H)-PF₆]²⁺ = required 344.1001).

The overall method for preparing the compound of Chemical Formula 267 is as described in Reaction Scheme 16 below.

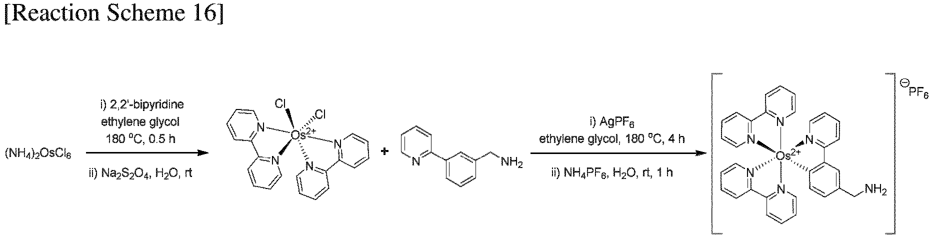

### [Example 1.17] Synthesis of transition metal complex of Chemical Formula 271

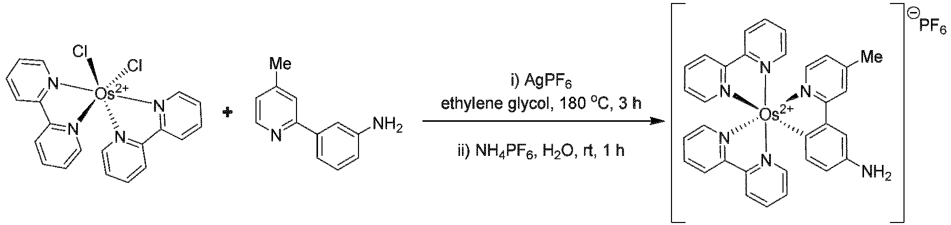

0.3 g (0.5 mmol) of Os(2,2-dipyridyl)₂Cl₂ prepared in 1.14-1 above, 0.15 g (0.8 mmol) of 3-(4-methylpyridin-2-yl)aniline, and 0.6 g (2.3 mmol) of silver hexafluorophosphate were put into a 250 mL Schlenk flask, and the resulting mixture was dissolved in 100 mL of ethylene glycol in an argon gas atmosphere, and then degassed with argon for 15 minutes. The reaction mixture was heated to 180°C and stirred for 3 hours. After the reaction was completed, the reaction mixture was cooled to room temperature, and the resulting precipitate was removed by filtration under reduced pressure. The filtrate was added dropwise to a saturated aqueous solution (300 mL) of ammonium hexafluorophosphate to obtain a precipitate of an ion-exchanged osmium composite. The resulting solid was filtered under reduced pressure, washed several times with water, and then dried in a vacuum oven to obtain an osmium composite as a dark violet final compound (0.2 g, 55%) HRMS (¹⁹²Os): m/z [(M₀-PF₆]²⁺= 343.5960 ([(M₀-PF₆]²⁺= required 343.5951).

The overall method for preparing the compound of Chemical Formula 271 is as described in Reaction Scheme 17 below.

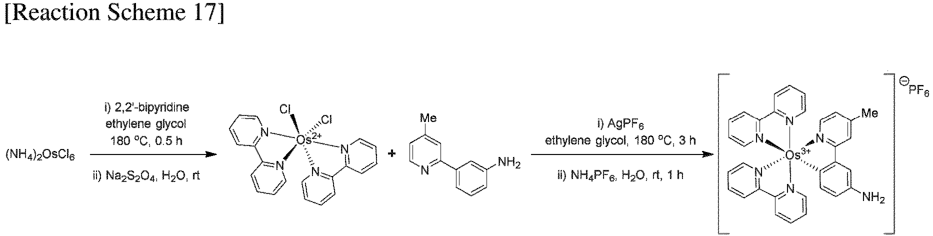

### [Example 2] Synthesis of oxidation-reduction polymer according to the present invention

### 1) Synthesis of perfluorophenyl 6-bromohexanoate

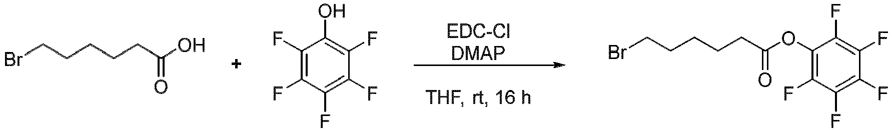

3.4 g (17 mmol) of 6-bromohexanoic acid and 2.9 g (16 mmol) of pentafluorophenol were put into a 250 mL round-bottom flask, and the resulting mixture was dissolved in 120 mL of tetrahydrofuran. Thereafter, 3.3 g (17 mmol) of 1-ethyl-3-(3-dimethylaminopropyl)carbodiimide hydrochloride and 0.39 g (3.1 mmol) of 4-(dimethylamino)pyridine were added thereto, and the resulting mixture was stirred at room temperature for 16 hours. After the reaction was completed, the reaction mixture was extracted with water (200 mL) and ethyl acetate (200 mL × 3). The organic layers were collected, dried over magnesium sulfate, and concentrated under reduced pressure to remove the solvent, and then the residue was purified by column chromatography using ethyl acetate and hexane as developing solvents (hexane : ethyl acetate = 24:1). Finally, perfluorophenyl 6-bromohexanoate was obtained as a colorless oil (3.9 g, 72%).

### 2) Synthesis of perfluorophenyl 2-bromoacetate

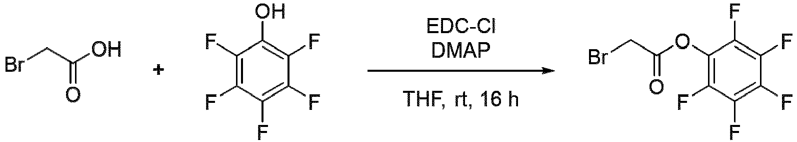

4 g (29 mmol) of bromoacetic acid and 4.8 g (26 mmol) of pentafluorophenol were put into a 250 mL round-bottom flask, and the resulting mixture was dissolved in 150 mL of tetrahydrofuran. Thereafter, 5.5 g (29 mmol) of 1-ethyl-3-(3-dimethylaminopropyl)carbodiimide hydrochloride and 0.64 g (5.2 mmol) of 4-(dimethylamino)pyridine were added thereto, and the resulting mixture was stirred at room temperature for 16 hours. After the reaction was completed, the reaction mixture was extracted with water (200 mL) and ethyl acetate (200 mL x 3). The organic layers were collected, dried over magnesium sulfate, and concentrated under reduced pressure to remove the solvent, and then the residue was purified by column chromatography using ethyl acetate and hexane as developing solvents (hexane : ethyl acetate = 24:1). Finally, perfluorophenyl 2-bromoacetate was obtained as a colorless oil (1.4 g, 18%).

### 3) Synthesis of perfluorophenyl methacrylate

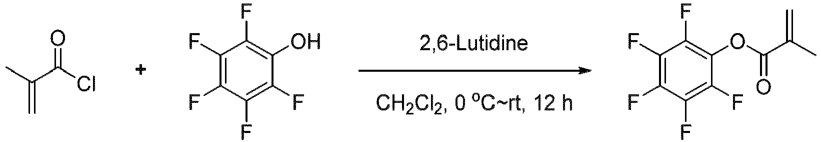

21 g (114 mmol) of pentafluorophenol and 13 g (120 mmol) of 2,6-lutidine were put into a 250 mL round-bottom flask, and the resulting mixture was dissolved in 150 mL of dichloromethane. 12 mL (125 mmol) of methacryloyl chloride was slowly added dropwise to the reaction mixture at 0°C, and the resulting mixture was stirred at room temperature for 12 hours. After the reaction was completed, the reaction mixture was filtered under reduced pressure to remove the resulting impurity (2,6-lutidine hydrochloride), and the remaining solvent was extracted with water (200 mL) and dichloromethane (200 mL × 3). The organic layers were collected, dried over magnesium sulfate, and concentrated under reduced pressure to remove the solvent, and then a colorless oil (16 g, 56%) was obtained at 60°C using a vacuum distillation apparatus.

### 4) Synthesis of polyvinylimidazole-co-polyimidazolium pentafluorophenol (Chemical Formula [278] (q = 6))

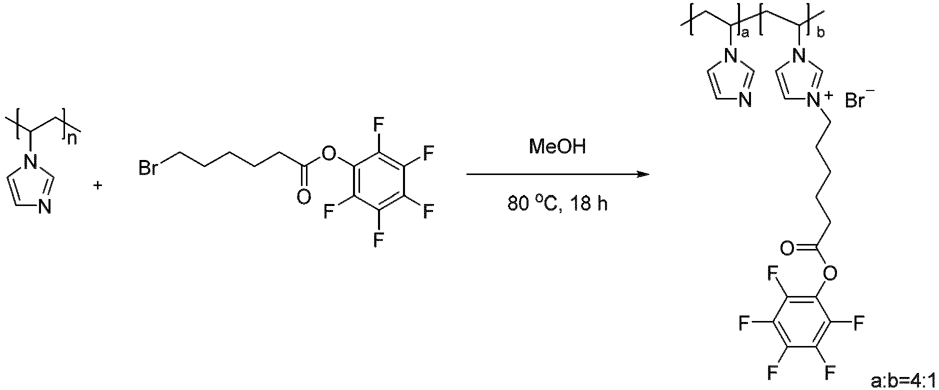

0.1 g (1.1 mmol) of polyvinylimidazole (Mn = 10,000 g/mol) was completely dissolved in 3 mL of methanol in a 70 mL culture tube. 0.11 g (0.32 mmol) of perfluorophenyl 6-bromohexanoate prepared in Example 2-1 above was added to the reaction mixture, and the resulting mixture was heated to 80°C and stirred for 18 hours. After the reaction was completed, the reaction mixture was cooled to room temperature and added dropwise to diethyl ether to obtain a polymer precipitate. The resulting solid was filtered under reduced pressure, washed several times with diethyl ether, and then dried in a vacuum oven for 24 hours to finally obtain a yellow solid (0.15 g, 71%).

### 5) Synthesis of polyvinylimidazole-co-polyimidazolium pentafluorophenol (Chemical Formula [278] (q = 1))

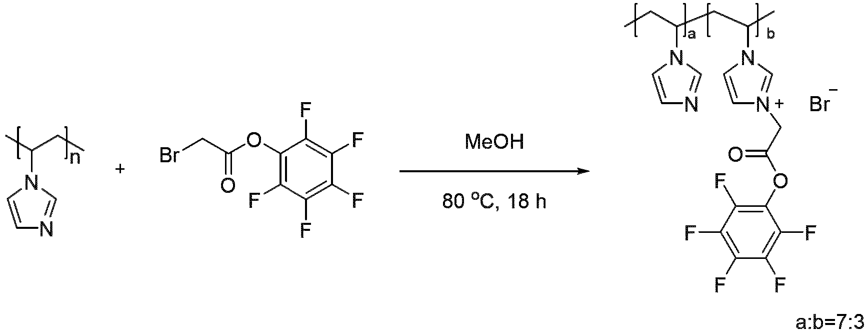

1.3 g (14 mmol) of polyvinylimidazole (Mn = 10,000 g/mol) was completely dissolved in 15 mL of methanol in a 70 mL culture tube. 1.2 g (4.1 mmol) of perfluorophenyl 2-bromoacetate prepared in Example 1.2 above was added to the reaction mixture, and the resulting mixture was heated to 80°C and stirred for 18 hours. After the reaction was completed, the reaction mixture was cooled to room temperature and added dropwise to diethyl ether to obtain a polymer precipitate. The resulting solid was filtered under reduced pressure, washed several times with diethyl ether, and then dried in a vacuum oven for 24 hours to finally obtain a white solid (1.4 g, 55%).

### 6) Synthesis of polydimethylaminoethyl methacrylate (Chemical Formula [275])

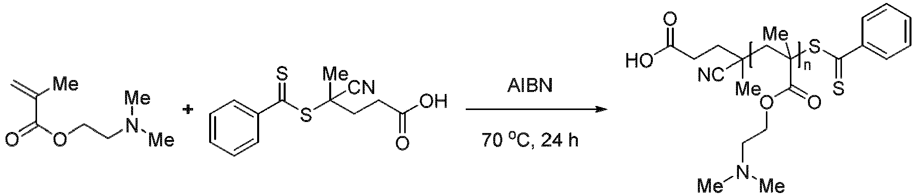

2 mg (0.012 mmol) of azobisisobutyronitrile and 34 mg (0.12 mmol) of 4-cyano-4-(phenylcarbonothioylthio)pentanoic acid were put into a 100 mL Schlenk flask, and the resulting mixture was dissolved in 10 mL (59 mmol) of 2-(dimethylamino)ethyl methacrylate in an argon gas atmosphere, and then degassed with argon for 15 minutes. The reaction mixture was heated to 70°C and stirred for 24 hours. After the reaction was completed, the reaction mixture was cooled to room temperature, diluted with dichloromethane, and added dropwise to hexane to obtain a polymer precipitate. The resulting solid was filtered under reduced pressure, washed several times with hexane and diethyl ether, and then dried in a vacuum oven for 24 hours to finally obtain a pink solid (5.4 g, 58%).

### 7) Synthesis of polypentafluorophenyl (Chemical Formula [276])

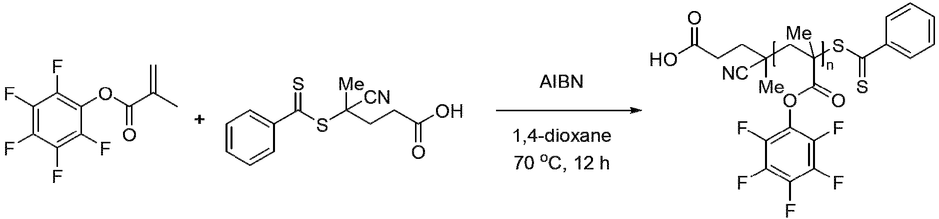

20 mg (0.12 mmol) of azobisisobutyronitrile and 0.17 g (0.62 mmol) of 4-cyano-4-(phenylcarbonothioylthio)pentanoic acid were put into a 100 mL Schlenk flask, and the resulting mixture was dissolved in 25 mL of 1,4-dioxane in an argon gas atmosphere. Thereafter, 16 g (62 mmol) of perfluorophenyl methacrylate was added thereto, and the resulting mixture was degassed with argon for 15 minutes. The reaction mixture was heated to 70°C and stirred for 12 hours. After the reaction was completed, the reaction mixture was cooled to room temperature and added dropwise to hexane to obtain a polymer precipitate. The resulting solid was filtered under reduced pressure, washed several times with hexane and diethyl ether, and then dried in a vacuum oven for 24 hours to finally obtain a pink solid (10 g, 66%).

### 8) Synthesis of polypentafluorophenyl-co-polydimethylaminoethyl methacrylate diblock copolymer (Chemical Formula [277])

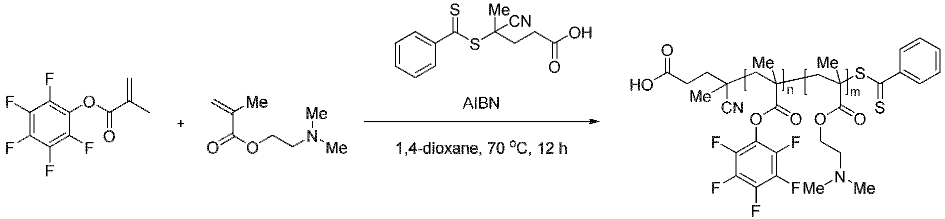

4 mg (0.022 mmol) of azobisisobutyronitrile and 31 mg (0.11 mmol) of 4-cyano-4-(phenylcarbonothioylthio)pentanoic acid were put into a 100 mL Schlenk flask, and the resulting mixture was dissolved in 3 mL of 1,4-dioxane in an argon gas atmosphere. Thereafter, 2 g (7.9 mmol) of perfluorophenyl methacrylate and 0.53 g (3.4 mmol) of 2-(dimethylamino)ethyl methacrylate were added thereto, and the resulting mixture was degassed with argon for 15 minutes. The reaction mixture was heated to 70°C and stirred for 12 hours. After the reaction was completed, the reaction mixture was cooled to room temperature and added dropwise to hexane to obtain a polymer precipitate. The resulting solid was filtered under reduced pressure, washed several times with hexane and diethyl ether, and then dried in a vacuum oven for 24 hours to finally obtain a pink solid (1.6 g, 64%).

### 9) Synthesis of polyvinylimidazole-co-polyimidazolium ethylamine (Chemical Formula [279] (q = 2))

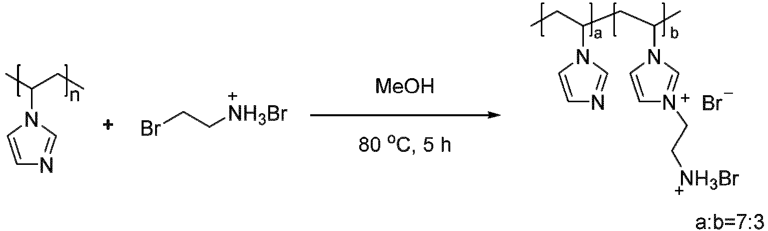

A 250 mL two-neck round-bottom flask was equipped with a reflux condenser and a gas inlet, and 2.1 g (21.2 mmol) of polyvinylimidazole (Mn = 10,000 g/mol) was completely dissolved in 100 mL of methanol. 1.3 g (6.4 mmol) of 2-bromoethylamine bromate was added to the solution, and the resulting mixture was heated to 80°C and stirred for 5 hours. After the reaction was completed, the reaction mixture was cooled to room temperature and added dropwise to diethyl ether to obtain a polymer precipitate. The resulting solid was filtered under reduced pressure, washed several times with diethyl ether, and then dried in a vacuum oven for 24 hours to finally obtain a white solid (2.5 g, 76%).

### 10) Synthesis of polyvinylimidazole-co-polyimidazolium propylamine (Chemical Formula [279] (q = 3))

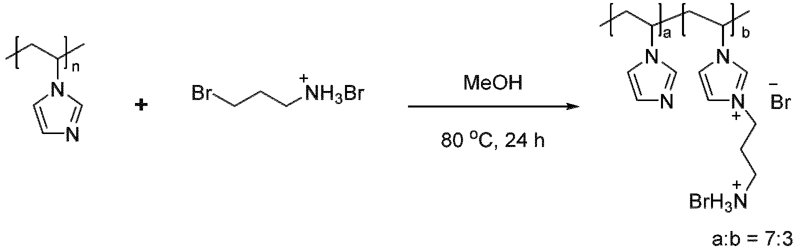

A 250 mL two-neck round-bottom flask was equipped with a reflux condenser and a gas inlet, and 2.0 g (21.2 mmol) of polyvinylimidazole (Mn = 10,000 g/mol) was completely dissolved in 100 mL of methanol. 1.5 g (7.0 mmol) of 3-bromopropylamine bromate was added to the solution, and the resulting mixture was heated to 80°C and stirred for 5 hours. After the reaction was completed, the reaction mixture was cooled to room temperature and added dropwise to diethyl ether to obtain a polymer precipitate. The resulting solid was filtered under reduced pressure, washed several times with diethyl ether, and then dried in a vacuum oven for 24 hours to finally obtain a white solid (1.8 g, 61%).

### [Example 3] Synthesis of oxidation-reduction polymer including transition metal complex according to the present invention

### [Example 3.1] Synthesis of oxidation-reduction polymer of Chemical Formula 286

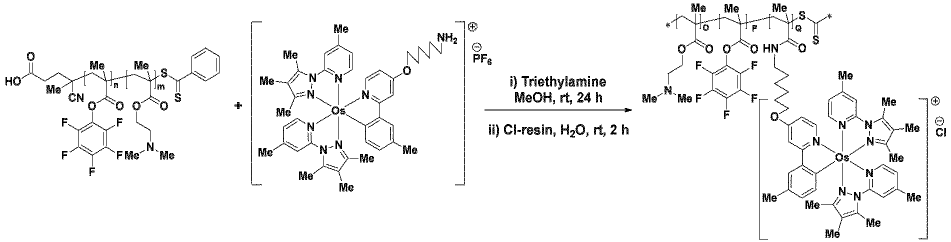

In a 100 mL round-bottom flask, 0.5 g (0.2 mmol) of the polymer of [Chemical Formula 277] and 0.2 g (0.21 mmol) of the osmium complex of [Chemical Formula 56] were completely dissolved in 50 mL of methanol. 56 µL (0.4 mmol) of triethylamine was added to the solution, and the resulting mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.4 g of a dark green oxidation-reduction polymer of [Chemical Formula 286] was obtained (0.4 g, 61%).

### [Example 3.2] Synthesis of oxidation-reduction polymer of Chemical Formula 288

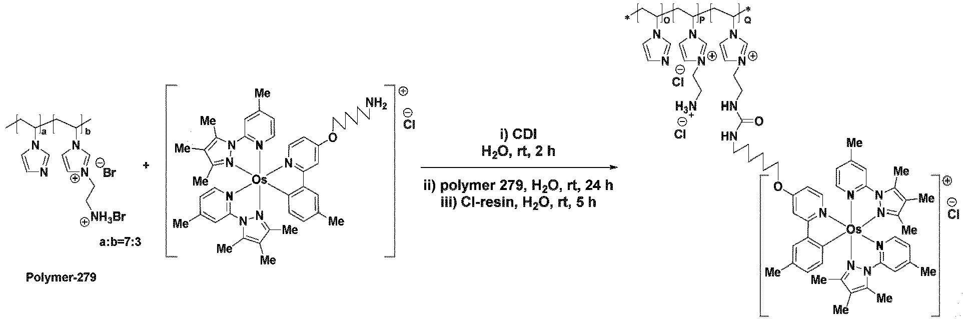

In a 5 mL conical vial, 0.1 g (0.11 mmol) of the osmium complex of [Chemical Formula 56] was completely dissolved in 3 mL of water. 20 mg (0.13 mmol) of carbonyldimidazole (CDI) was added to the solution, and the resulting mixture was stirred at room temperature for 2 hours. After it was confirmed by ESI-MS that an intermediate had been formed, this solution was added to a 50 mL round-bottom flask containing 0.1 g (0.20 mmol per amine) of the polymer of [Chemical Formula 279] that had been completely dissolved in 5 mL of water in advance. The reaction mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.18 g of a dark green oxidation-reduction polymer of [Chemical Formula 288] was obtained (0.18 g, 90%).

### [Example 3.3] Synthesis of oxidation-reduction polymer of Chemical Formula 291

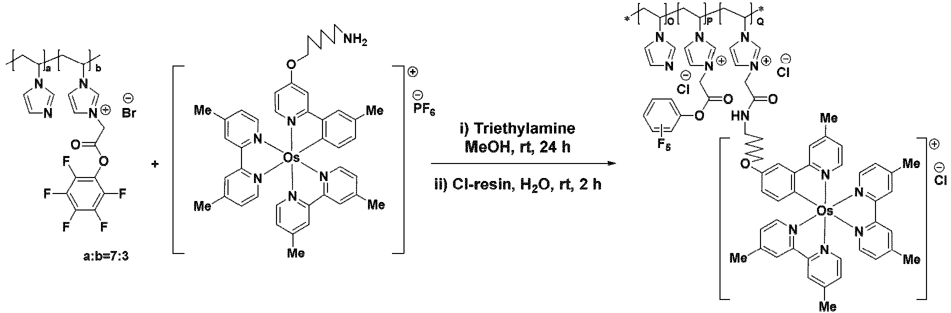

In a 100 mL round-bottom flask, 0.3 g (0.42 mmol per pentafluorophenyl) of the polymer of [Chemical Formula 278] and 0.2 g (0.22 mmol) of the osmium complex of [Chemical Formula 230] were completely dissolved in 60 mL of methanol. 56 µL (0.42 mmol) of triethylamine was added to the solution, and the resulting mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.3 g of a dark violet oxidation-reduction polymer of [Chemical Formula 291] was obtained (0.3 g, 63%).

### [Example 3.4] Synthesis of oxidation-reduction polymer of Chemical Formula 301

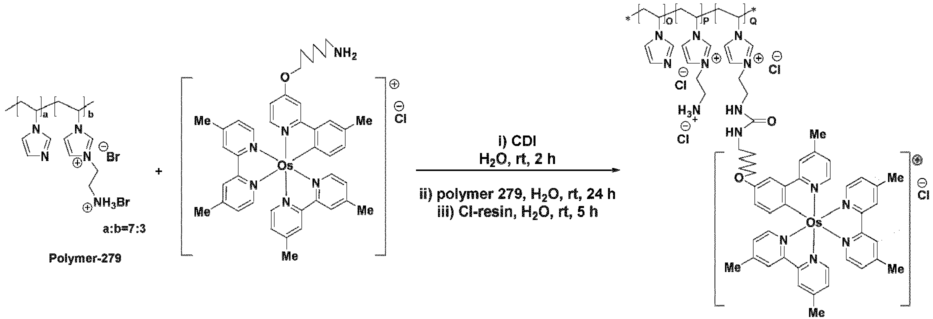

In a 5 mL conical vial, 0.1 g (0.11 mmol) of the osmium complex of [Chemical Formula 230] was completely dissolved in 2 mL of water. 20 mg (0.13 mmol) of carbonyldimidazole (CDI) was added to the solution, and the resulting mixture was stirred at room temperature for 2 hours. After it was confirmed by ESI-MS that an intermediate had been formed, this solution was added to a 50 mL round-bottom flask containing 0.1 g (0.2 mmol per amine) of the polymer of [Chemical Formula 279] that had been completely dissolved in 5 mL of water in advance. The reaction mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.15 g of a violet oxidation-reduction polymer of [Chemical Formula 301] was obtained (0.15 g, 75%).

### [Example 3.5] Synthesis of oxidation-reduction polymer of Chemical Formula 302

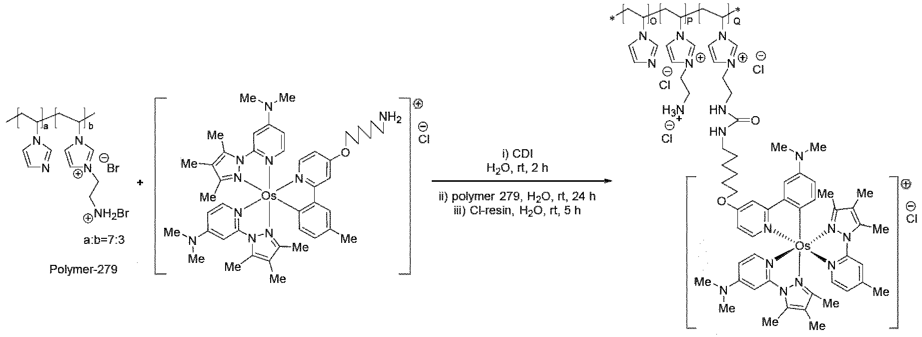

In a 5 mL conical vial, 0.1 g (0.10 mmol) of the osmium complex of [Chemical Formula 86] was completely dissolved in 2 mL of water. 20 mg (0.13 mmol) of carbonyldimidazole (CDI) was added to the solution, and the resulting mixture was stirred at room temperature for 2 hours. After it was confirmed by ESI-MS that an intermediate had been formed, this solution was added to a 50 mL round-bottom flask containing 0.1 g (0.2 mmol per amine) of the polymer of [Chemical Formula 279] that had been completely dissolved in 5 mL of water in advance. The reaction mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.10 g of a violet oxidation-reduction polymer of [Chemical Formula 302] was obtained (0.1 g, 50%).

### [Example 3.6] Synthesis of oxidation-reduction polymer of Chemical Formula 303

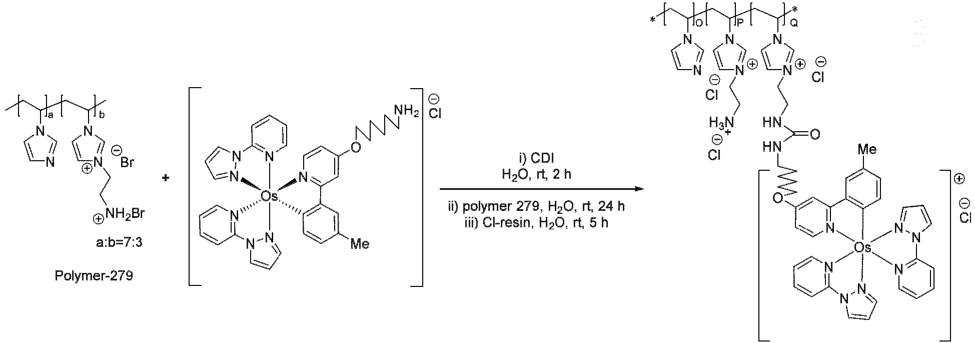

In a 5 mL conical vial, 0.2 g (0.25 mmol) of the osmium complex of [Chemical Formula 167] was completely dissolved in 4 mL of water. 48 mg (0.30 mmol) of carbonyldimidazole (CDI) was added to the solution, and the resulting mixture was stirred at room temperature for 2 hours. After it was confirmed by ESI-MS that an intermediate had been formed, this solution was added to a 50 mL round-bottom flask containing 0.2 g (0.2 mmol per amine) of the polymer of [Chemical Formula 279] that had been completely dissolved in 10 mL of water in advance. The reaction mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.25 g of a violet oxidation-reduction polymer of [Chemical Formula 303] was obtained (0.25 g, 64%).

### [Example 3.7] Synthesis of oxidation-reduction polymer of Chemical Formula 304

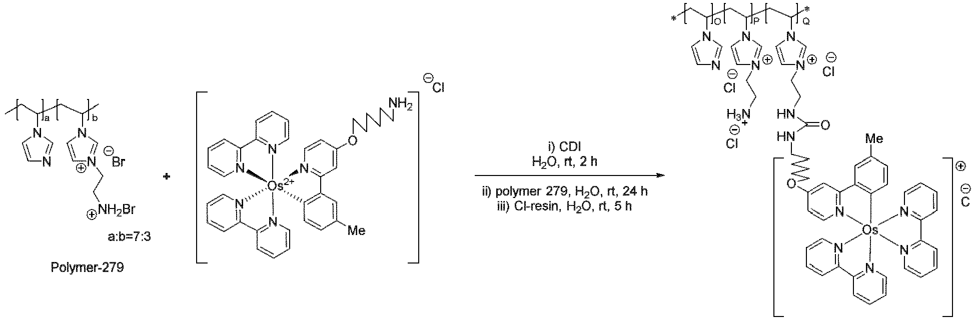

In a 5 mL conical vial, 0.25 g (0.30 mmol) of the osmium complex of [Chemical Formula 257] was completely dissolved in 4 mL of water. 60 mg (0.36 mmol) of carbonyldimidazole (CDI) was added to the solution, and the resulting mixture was stirred at room temperature for 2 hours. After it was confirmed by ESI-MS that an intermediate had been formed, this solution was added to a 50 mL round-bottom flask containing 0.25 g (0.2 mmol per amine) of the polymer of [Chemical Formula 279] that had been completely dissolved in 10 mL of water in advance. The reaction mixture was stirred at room temperature for 24 hours. After the reaction was completed, the reaction mixture was added dropwise to diethyl ether to obtain a polymer precipitate. After the resulting solid was again dissolved in water, the resulting solution was stirred with a Cl resin for 2 hours for ion exchange. The resin mixture solution is filtered under reduced pressure to remove the resin, and the solution is freeze-dried. Finally, 0.30 g of a violet oxidation-reduction polymer of
[Chemical Formula 304] was obtained (0.3 g, 60%).

### [Experimental Examples]

[Experimental Example 1] Confirmation of electrochemical characteristics of transition metal complex and oxidation-reduction polymer according to the present invention using cyclic voltammetry
In order to confirm the performance of the transition metal complex of the present invention and the oxidation-reduction polymer including the same as an electron transfer mediator, the electrochemical characteristics were measured using cyclic voltammetry according to the following experimental method.

### Experimental Method

1. 20 mg of each of the compounds of Chemical Formula 5, 86, 169, 167, 230, 240, 257, and 267 according to the present invention and the compound of Chemical Formula 305 (transition metal complex-PF₆ type) as a comparison group was dissolved in 2 mL of a 0.1 M tetrabutylammonium perchlorate dimethylsulfoxide solution.
   Further, 20 mg of each of the compounds (oxidation-reduction polymers) of Chemical Formulae 286, 288, 301, 302, 303, and 304 according to the present invention was dissolved in 2 mL of a deionized aqueous solution.
   As a comparison group, 20 mg of the compound of the following Chemical Formula 306 was dissolved in 2 mL of a deionized water solution. 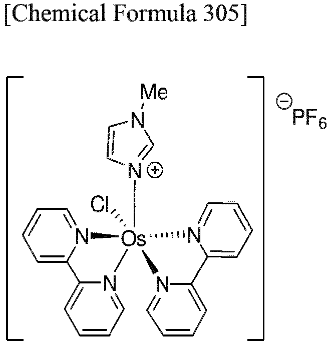 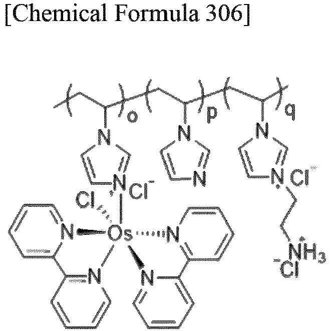
2. The solution was degassed with argon for 5 to 10 minutes in order to remove oxygen in the solution.
3. A working electrode, a reference electrode, and a counter electrode were connected to the solution from which oxygen had been removed, and the change in electrical signal according to the change in voltage was measured in an argon gas atmosphere. The experiment was carried out under the following conditions.

### Experimental materials/conditions

Working electrode: glass carbon electrode (dia: 3.0 mm)
Reference electrode: Ag/AgCl electrode
Counter electrode: Platinum rod
Test parameters
   - Equipment: EmStat (PalmSens Co.)
   - Technique: cyclic voltammetry
   - Potential range: -1.0 to 1.0 V
   - Scan rate: 10 mV/s

### Experimental results

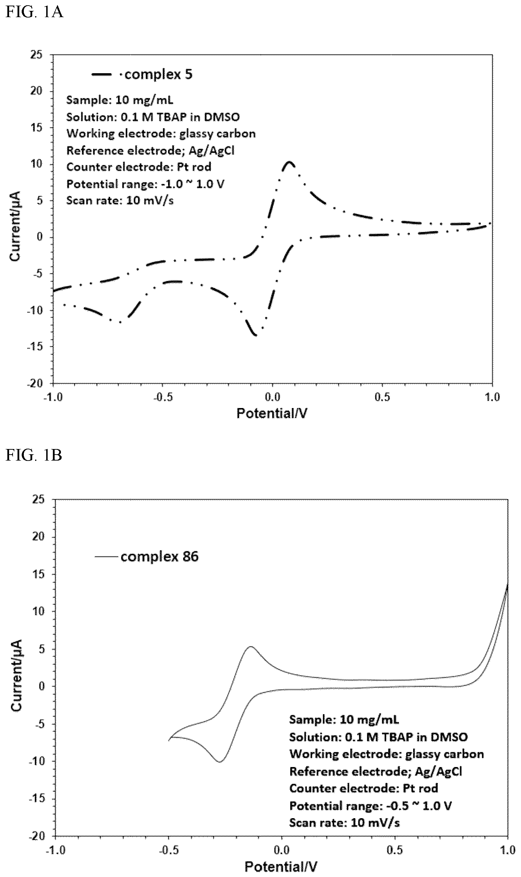

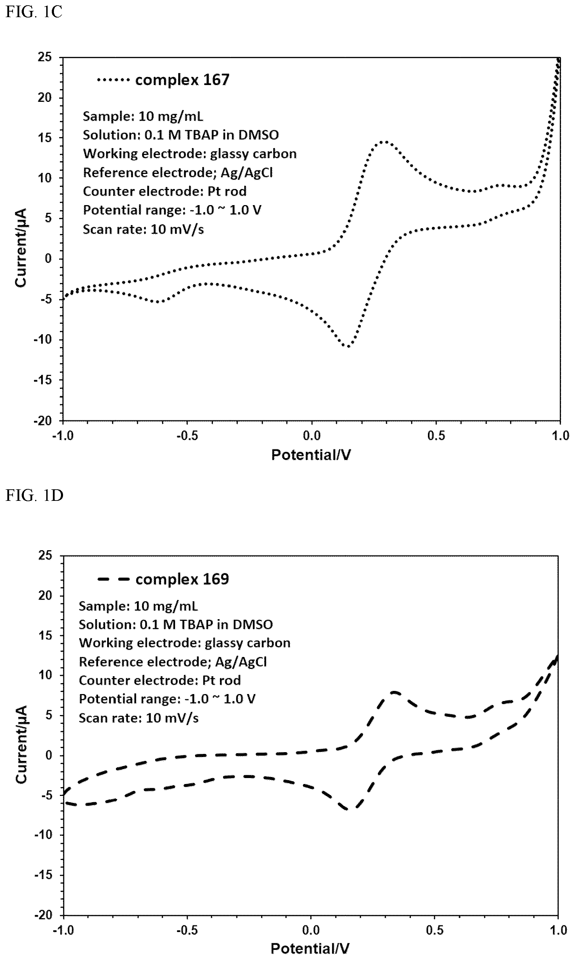

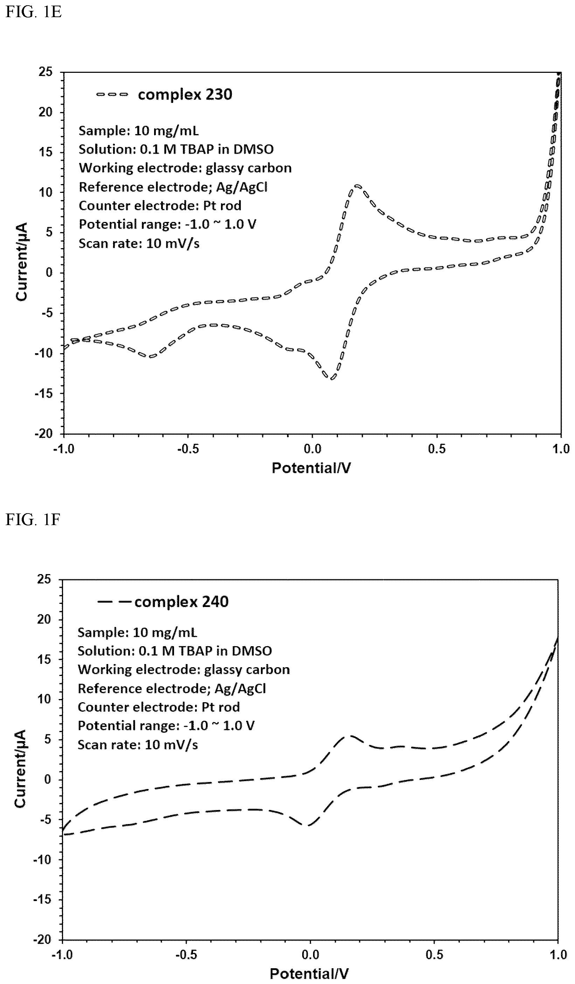

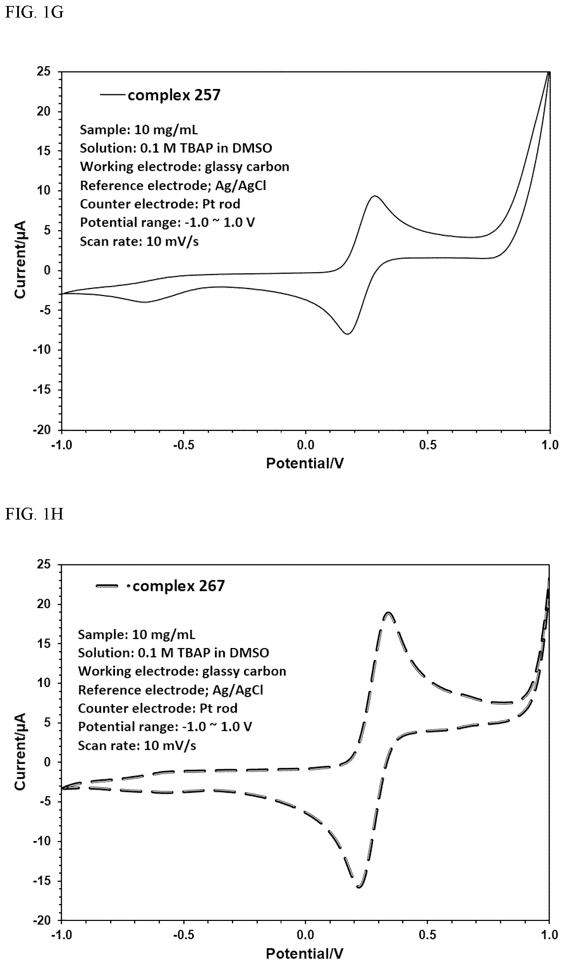

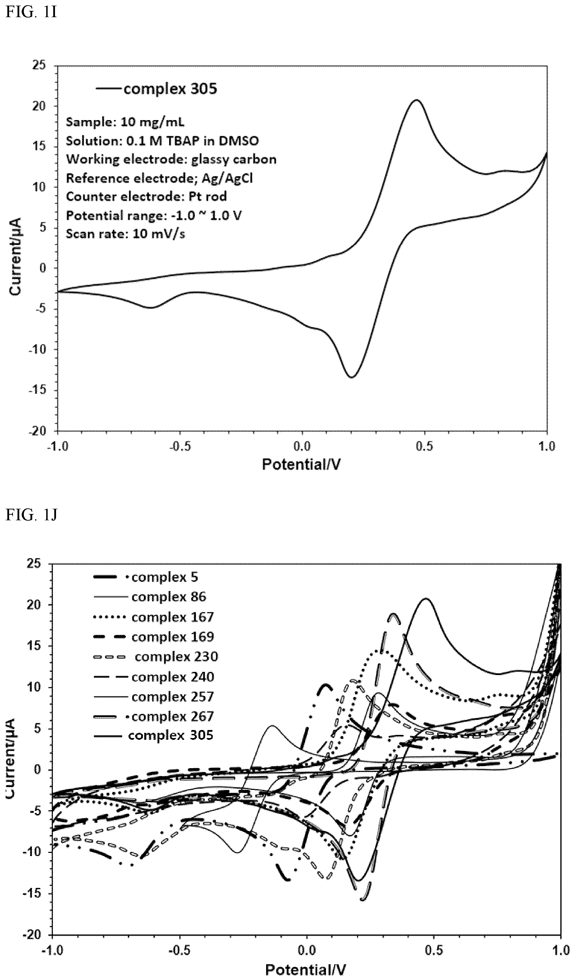

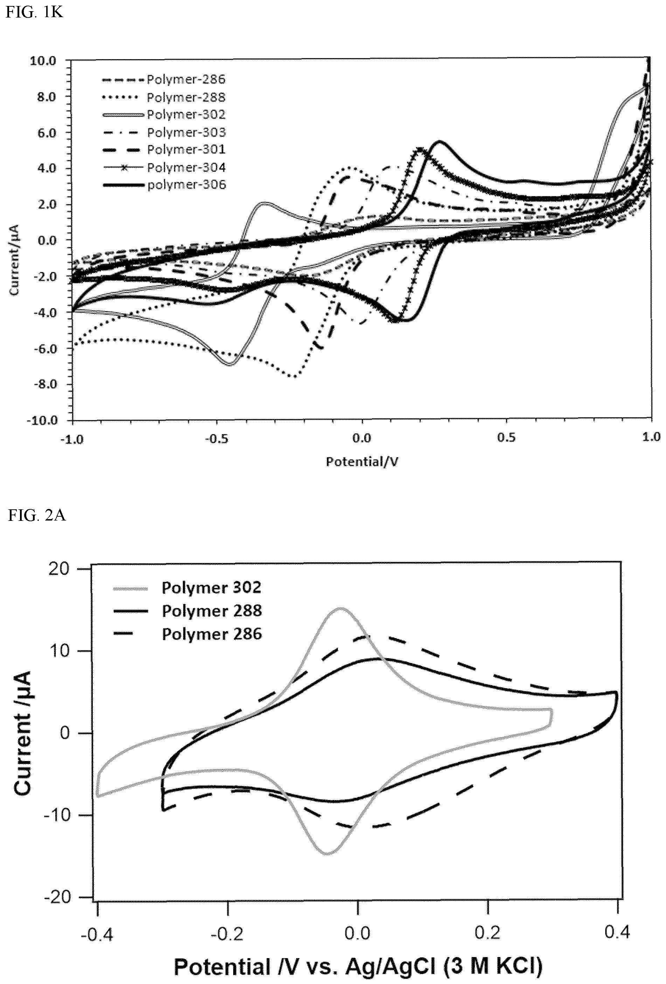

As a result of the experiment, as can be confirmed from Table 1 and FIGS. 1A to 1K below, it was confirmed that the transition metal complex according to the present invention exhibits various potential values according to the type of ligand.

In addition, it was confirmed that when a polymer was introduced into the transition metal complex of the present invention, the potential value was rarely affected by the synthesis of the oxidation-reduction polymer.

Furthermore, it was confirmed that the compounds of Chemical Formulae 286, 288, 301, 302, 303, and 304 exhibited lower potential values than the compound of Chemical Formula 306, which is a conventionally known control, and thus were able to serve as oxidation-reduction mediators with a high efficiency.

**[Table 1]**

| Transition metal complex and oxidation-reduction polymer | *E*_{pc} (V) | *E*ₚₐ (V) |
|---|---|---|
| [Chemical Formula 5] [PF₆ form] | 0.07 | -0.07 |
| [Chemical Formula 86] [PF₆ form] | -0.14 | -0.27 |
| [Chemical Formula 167] [PF₆ form] | 0.29 | 0.14 |
| [Chemical Formula 169] [PF₆ form] | 0.34 | 0.16 |
| [Chemical Formula 230] [PF₆ form] | 0.18 | 0.07 |
| [Chemical Formula 240] [PF₆ form] | 0.16 | -0.02 |
| [Chemical Formula 257] [PF₆ form] | 0.28 | 0.17 |
| [Chemical Formula 267] [PF₆ form] | 0.34 | 0.22 |
| [Chemical Formula 305] [PF₆ form] | 0.47 | 0.20 |
| [Chemical Formula 286] | 0.06 | -0.19 |
| [Chemical Formula 288] | -0.04 | -0.24 |
| [Chemical Formula 301] | -0.04 | -0.14 |
| [Chemical Formula 302] | -0.34 | -0.46 |
| [Chemical Formula 303] | 0.12 | -0.01 |
| [Chemical Formula 304] | 0.21 | 0.10 |
| [Chemical Formula 306] | 0.27 | 0.15 |

### Experimental Example 2: Manufacture of electrochemical sensor for measuring continuous blood glucose including the oxidation-reduction polymer according to the present invention

In order to manufacture an electrochemical sensor (electrochemical biosensor) including an electron transfer mediator of the oxidation-reduction polymer according to the present invention, a sensor was manufactured by the following method.

### Experimental Method

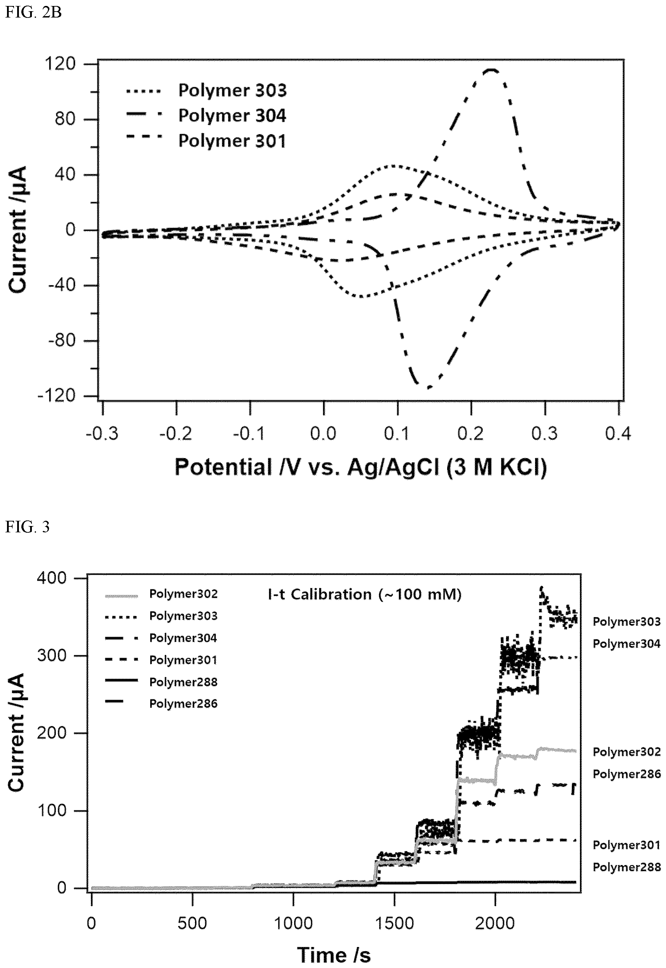

1. The compounds of Chemical Formulae 286, 288, 301, 302, 303, and 304 according to the present invention were each dissolved in an aqueous solution together with an oxidoreductase (glucose dehydrogenase), carbon nanotubes (CNTs), a non-ionic surfactant (Triton-X), and a cross-linking material (polyethylene glycol diglycidylether), and each solution was prepared using stirring and ultrasonic dispersion methods.
2. To manufacture a continuous blood glucose electrochemical sensor, each solution prepared was dispensed and coated on a carbon paste-printed electrode, and then cured through a crosslinking reaction at room temperature for 24 hours. After curing, the manufactured sensor was washed using distilled water.
3. Cyclic voltammetry was used as a method for confirming the electron transfer performance of the manufactured electrode.
4. The results of this experiment are shown in Table 2 and FIG. 2 below.

### Experimental materials/conditions

Working electrode: the electrode manufactured above
Reference electrode: Ag/AgCl electrode
Counter electrode: Platinum wire
Electrolyte: Phosphate buffer with NaCl solution
Test parameters
   - Equipment: EmStat (PalmSens Co.)
   - Technique: cyclic voltammetry
   - Potential range: -0.3 to 0.4V
   - Scan rate: 10 mV/s

**[Table 2]**

| Electrochemical sensor for measuring continuous blood glucose including oxidation-reduction polymer | *E*⁰ (mV) | *E*_{c}-*E*ₐ (mV) | *I*ₚₐ (µA) |
|---|---|---|---|
| [Chemical Formula 286] | 12.8 | 26.3 | 12.3 |
| [Chemical Formula 288] | -2.0 | 60.0 | 8.8 |
| [Chemical Formula 301] | 60.6 | 75.8 | 25.8 |
| [Chemical Formula 302] | -35.2 | 22.3 | 15.0 |
| [Chemical Formula 303] | 75.9 | 52.2 | 46.6 |
| [Chemical Formula 304] | 182.7 | 82.0 | 112.9 |

As shown in Table 2 and FIG. 2, the potential (*E*⁰) of the electrode to which the oxidation-reduction polymer of the present invention was applied was similar to that of Experimental Example 1, and it was also confirmed through this experiment that various potentials can be applied to the electrochemical sensor for measuring continuous blood glucose.

### Experimental Example 3: Comparison of sensitivity of electrochemical sensors for measuring continuous blood glucose to changes in glucose concentration

1. The electrodes manufactured in Experimental Example 2 using the compounds of Chemical Formulae 286, 288, 301, 302, 303, and 304 according to the present invention were compared by performing chronoamperometry in 0 to 100 mM glucose solutions.
2. The voltage applied when performing the chronoamperometry was 0.1 V.
3. The glucose concentrations were 0.01, 0.05, 0.1, 0.5, 1, 5, 10, 50, and 100 mM, and high-concentration glucose solutions were injected into a saline solution containing a phosphate buffer at intervals of 200 seconds to reach each concentration. Each experiment was performed for 50 minutes.
4. The results of this experiment are shown in FIG. 3 below.

### Experimental materials/conditions

Working electrode: the electrode manufactured above
Reference electrode: Ag/AgCl electrode
Counter electrode: Platinum wire
Electrolyte: Phosphate buffer with NaCl solution
Test parameters
   - Equipment: EmStat (PalmSens Co.)
   - Technique: chronoamperometry
   - Potential range: 0.10 V

As shown in FIG. 3, among the electrodes to which the oxidation-reduction polymers of the present invention were applied, particularly, the electrodes to which Compounds 286, 302, 303, and 304 were applied all showed linearity with respect to low concentrations of glucose, and the maximum enzyme activity (Vₘₐₓ) appears to be greater as the current is larger.

### Experimental Example 4: Comparison of glucose sensitivity according to increase in voltage of electrochemical sensor for measuring continuous blood glucose

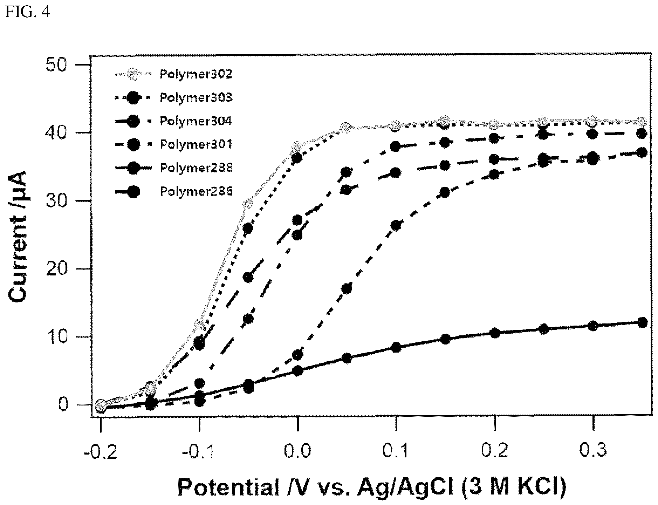

1. The electrodes manufactured in the above Experimental Example 2 using the compounds of Chemical Formulae 286, 288, 301, 302, 303, and 304 according to the present invention were compared by performing a multi-potential step method in a 400 mM glucose solution.
2. When performing the multi-potential step, the current was observed by maintaining the voltage at a voltage of 0.05 V intervals between -0.2 V to 0.35 V for 300 seconds.
3. The results of this experiment are shown in FIG. 4 below.

### Experimental materials/conditions

Working electrode: the electrode manufactured above
Reference electrode: Ag/AgCl electrode
Counter electrode: Platinum wire
Electrolyte: Phosphate buffer with NaCl solution
Test parameters
   - Equipment: EmStat (PalmSens Co.)
   - Technique: multi-potential step
   - Potential range: -0.2 to 0.35 V

As shown in FIG. 4, among the electrodes to which the oxidation-reduction polymer according to the present invention was applied, particularly, the electrodes to which Compounds 286, 302, 303, and 304 were applied showed a limiting current at a voltage lower than the applied voltage. In particular, the electrodes to which Compounds 302 and 303 were applied showed a limiting current at 0 V, confirming that the electrodes showed sensitivity to glucose even at a very low voltage.

## Claims

1. A transition metal complex, which is a compound represented by the following Chemical Formula 1, or a salt compound thereof:
[Chemical Formula 1] [M(L)ₐ(X₁)_{b}]^{c} d(X₂)
wherein,
M is a transition metal selected from the group consisting of Fe, Ru, and Os,
L is a bidentate ligand including pyridine; and one structure selected from the group consisting of pyrazole, triazole, tetrazole, oxadiazole and thiadiazole;
the pyridine is
an unsubstituted pyridine, or a pyridine substituted with one to four selected from the group consisting of a C₁₋₄ alkyl group, a C₁₋₄ alkoxy group, a -(CH₂)-O-C₁₋₄ alkyl group, a - (CH₂CH₂)-O-C₁₋₄ alkyl group, and C₁₋₄ alkylamino group,
one selected from the group consisting of the pyrazole, triazole, tetrazole, and oxadiazole is each independently unsubstituted or substituted with one to three selected from the group consisting of a C₁₋₄ alkyl group, a C₁₋₄ alkoxy group, a -(CH₂)-O-C₁₋₄ alkyl group, a -(CH₂CH₂)-O-C₁₋₄ alkyl group, 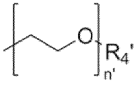 , and a C₁₋₄ alkylamino group,
R'4 is hydrogen or a substituted or unsubstituted C₁₋₄ alkyl,
n' is an integer selected from 1 to 4,
a is 2 or 3,
the C₁₋₄ alkyl group, C₁₋₄ alkoxy group, -(CH₂)-O-C₁₋₄ alkyl group, a -(CH₂CH₂)-O-C₁₋₄ alkyl group, and a C₁₋₄ alkylamino group are each substituted or unsubstituted,
the substituted C₁₋₄ alkyl group, C₁₋₄ alkoxy group, -(CH₂)-O-C₁₋₄ alkyl group, - (CH₂CH₂)-O-C₁₋₄ alkyl group, or C₁₋₄ alkylamino group is one in which a substituted hydrogen atom is substituted with a halogen atom of F, Cl, Br, or I, a cyano group, a hydroxyl group, a thiol group, a nitro group, an amino group, an imino group, an azido group, an amidino group, a hydrazino group, a hydrazono group, an oxo group, a carbonyl group, a carbamyl group, an ester group, an ether group, a carboxyl group or a salt thereof, a sulfonic acid group or a salt thereof, or phosphoric acid or a salt thereof,
b is 0, 1, or 2,
c is an integer selected from 1 to 3,
X₁ is a compound of the following Chemical Formula 2 or 3, 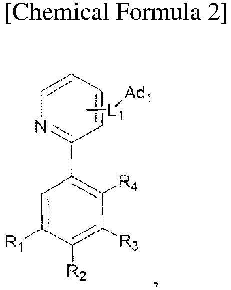 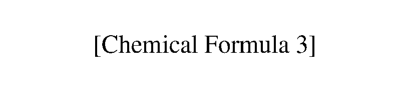 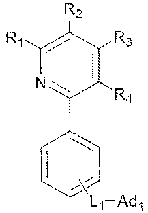
X₂ is one counter ion selected from the group consisting of F, Cl, Br, I, and PF₆,
d is 0, 1, 2, or 3,
in Chemical Formulae 2 and 3,
L₁ is selected from the group consisting a substituted or unsubstituted C₁₋₁₂ alkylene group, a substituted or unsubstituted C₁₋₁₅ alkoxy group, and a substituted or unsubstituted C₂₋₁₀ ethylene glycol group;
Ad₁ is selected from the group consisting of a primary amine group, a secondary amine group, an ammonium group, a halogen group, an epoxy group, an azide group, an alkenyl group, an alkynyl group, a thiol group, and an alcohol group,
R₁ and R₄ are each independently hydrogen, deuterium, a methyl group, an ethyl group, or a methoxy group, and
R₂ and R₃ are each independently hydrogen, a C₁₋₄ alkyl group, a C₁₋₄ alkoxy group, or a C₁₋₄ alkylamino group.

2. The transition metal complex or the salt compound thereof of claim 1, wherein the transition metal complex is a compound of the following Chemical Formula 4: 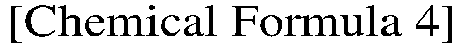 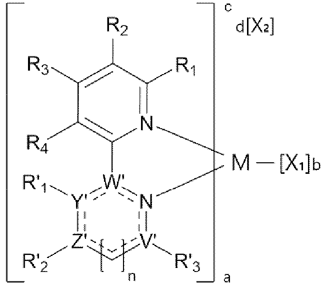 wherein,
R'₁, R'₂, and R'₃ are each independently hydrogen, deuterium, a methyl group, or an ethyl group,
at least one of W', Y', Z', and V' is nitrogen (N),
W' is nitrogen (N) or carbon (C),
Y', Z', and V' are each independently nitrogen (N), sulfur (S), oxygen (O), or carbon (C),
n is 0, and
M, a, b, c, d, X₁, and X₂ are the same as those defined in claim 1.

3. The transition metal complex or the salt compound thereof of claim 2, wherein when W' is carbon (C), Y' is sulfur (S), oxygen (O), or carbon (C).

4. The transition metal complex or the salt compound thereof of claim 2, wherein the compound of Chemical Formula 4 is a compound selected from the group consisting of compounds of the following Chemical Formulae 5 to 274: 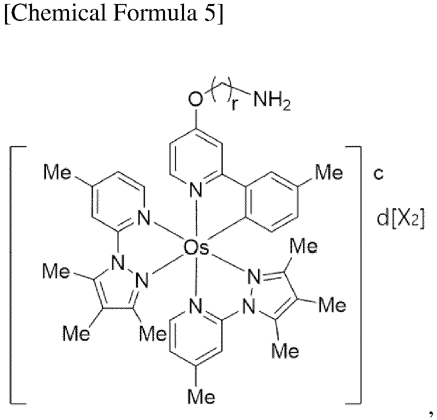 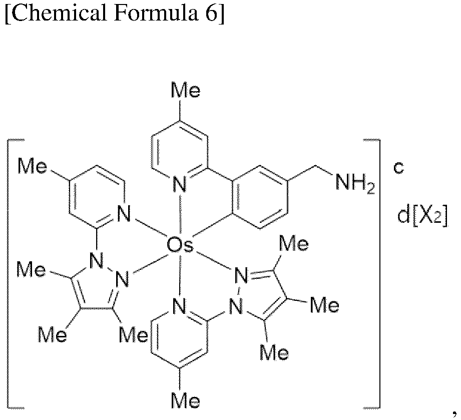 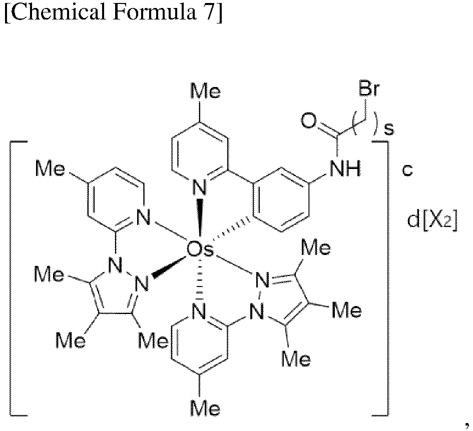 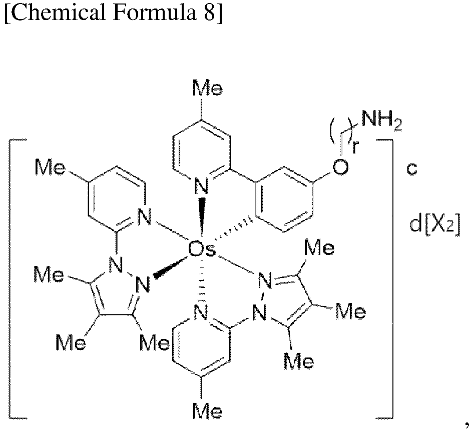 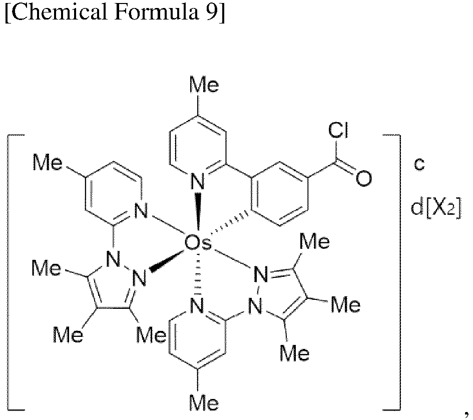 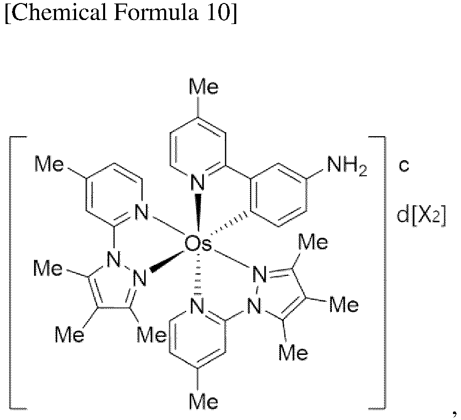 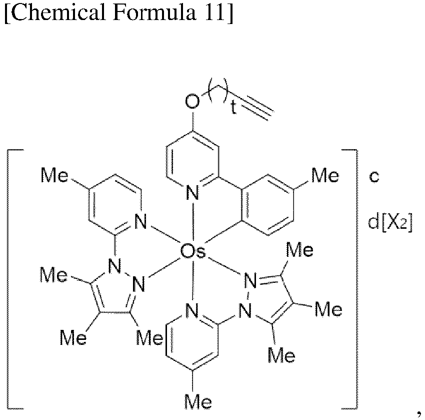 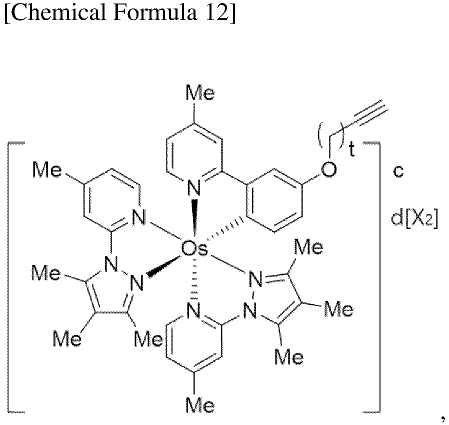 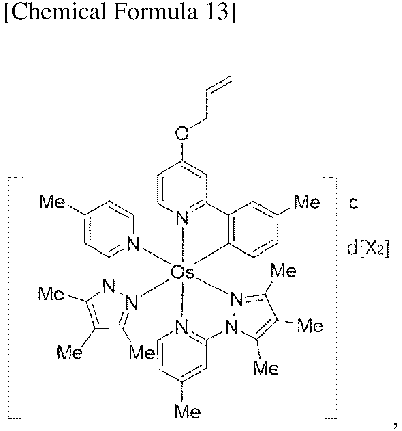 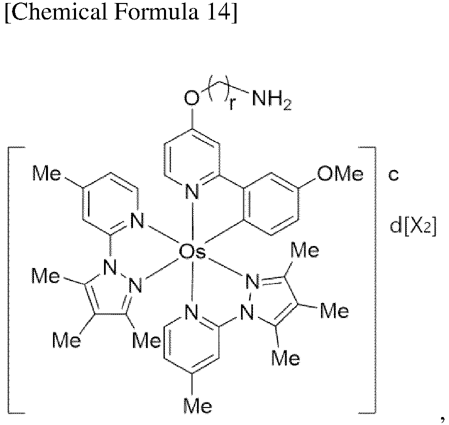 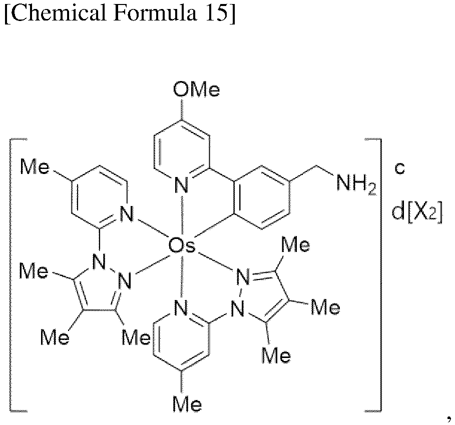 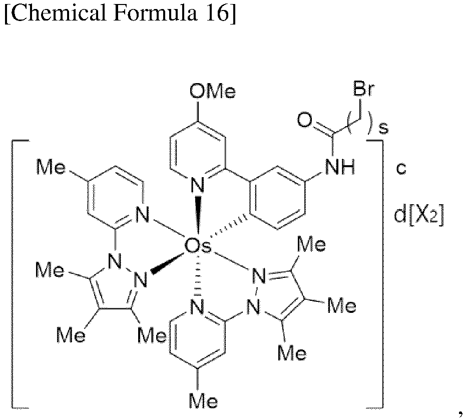 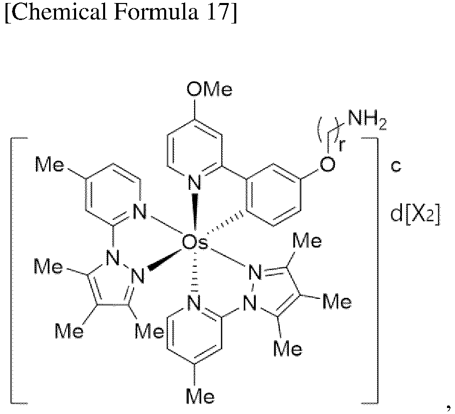 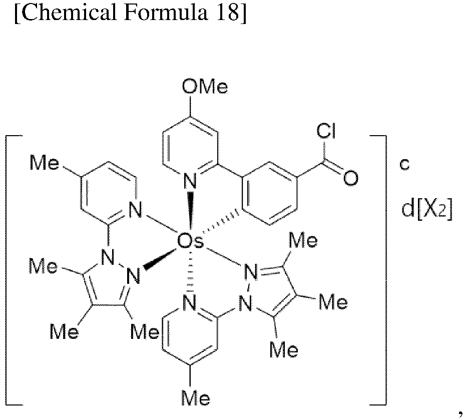 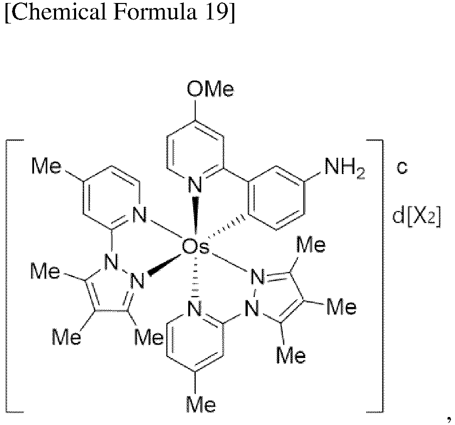 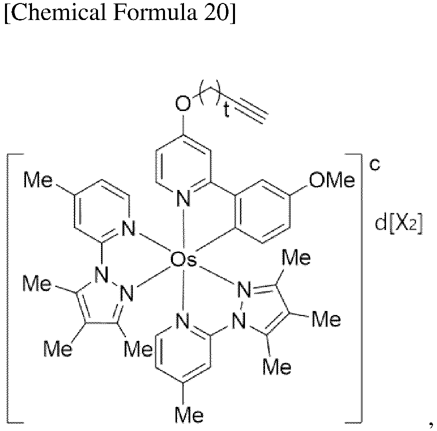 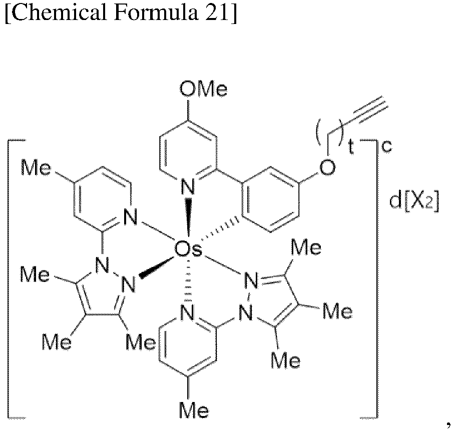 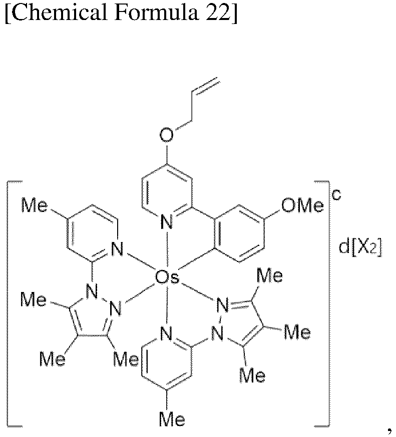 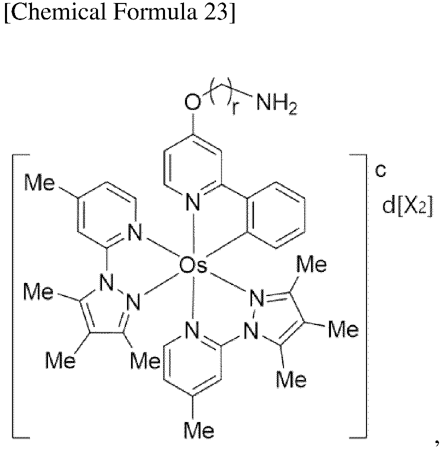 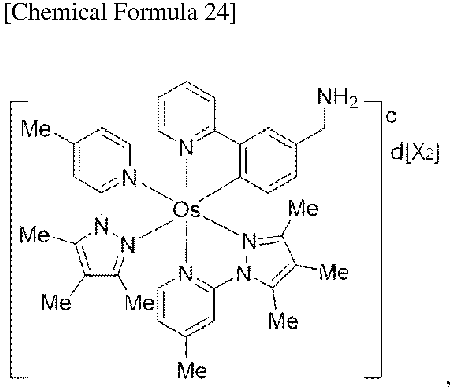 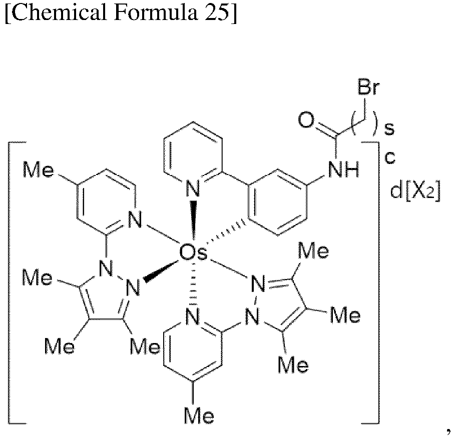 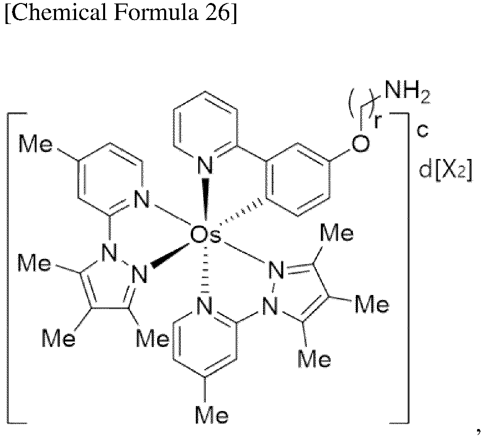 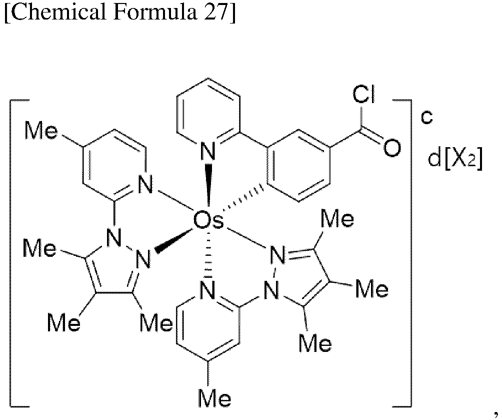 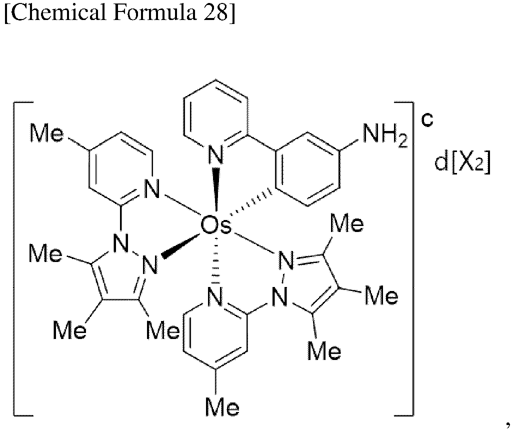 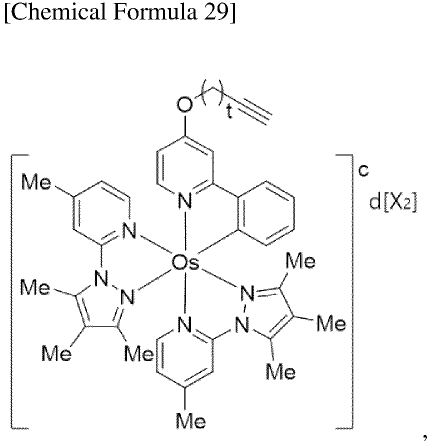 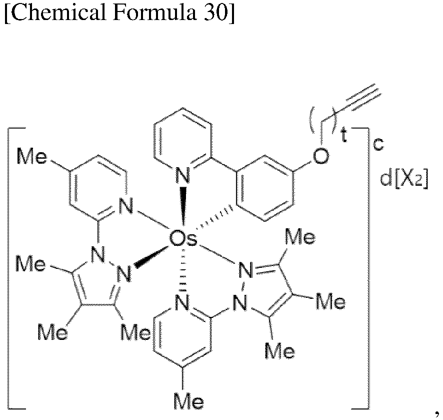 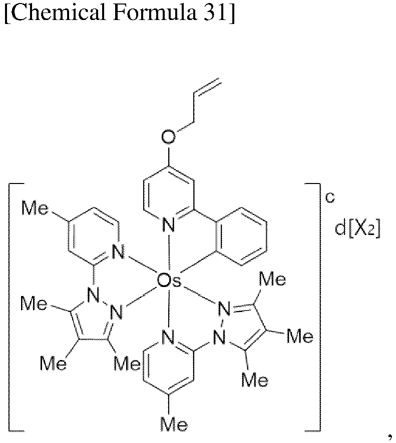 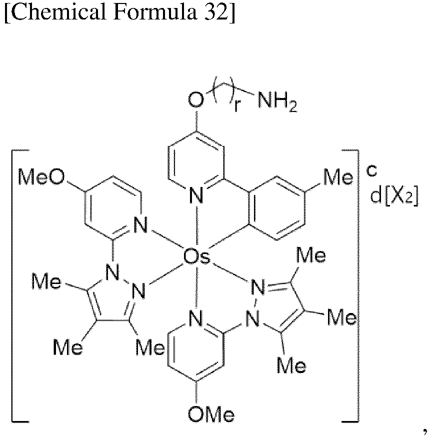 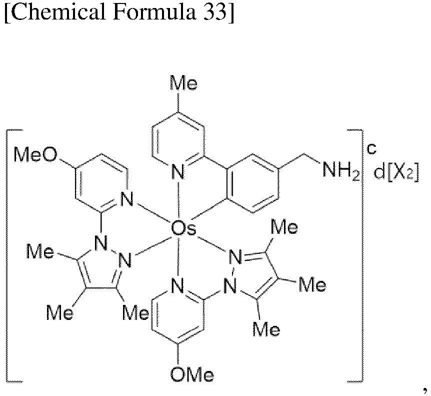 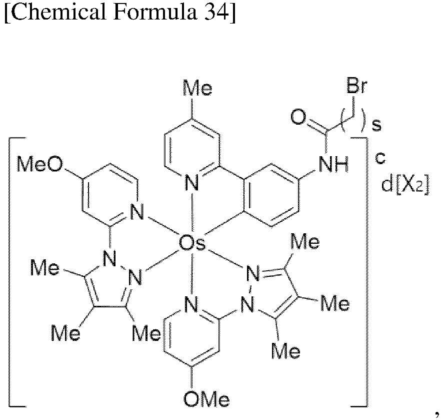 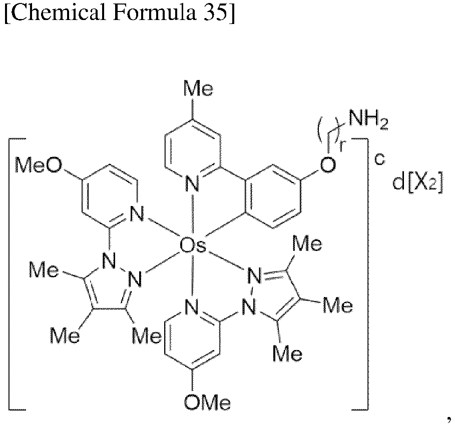 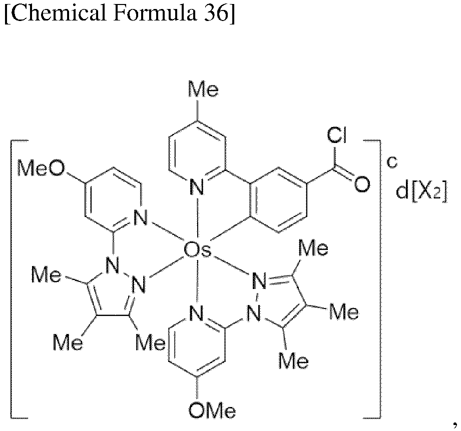 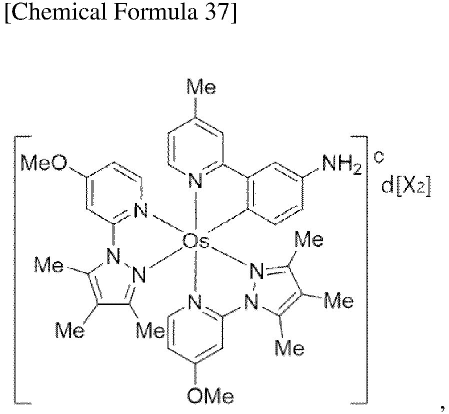 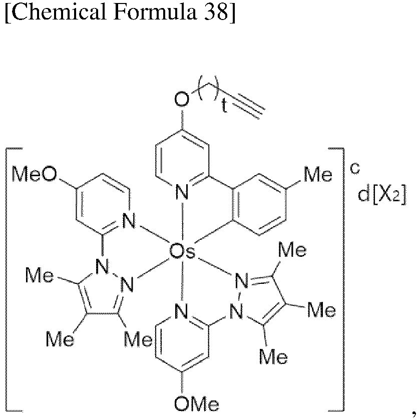 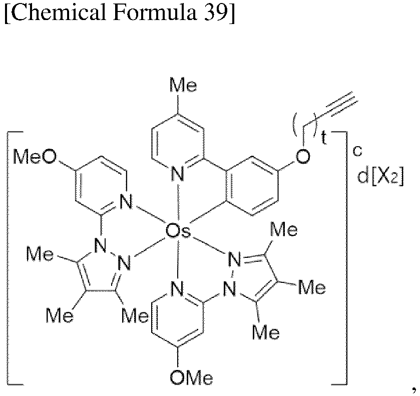 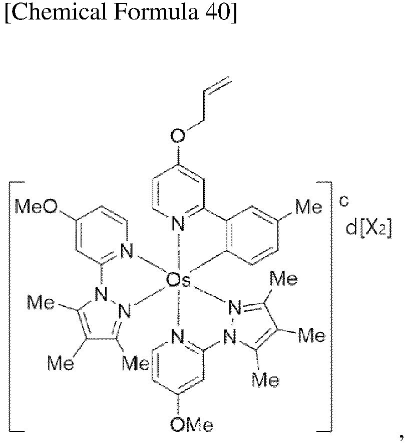 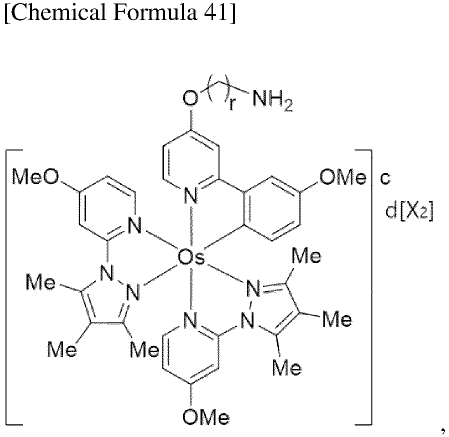 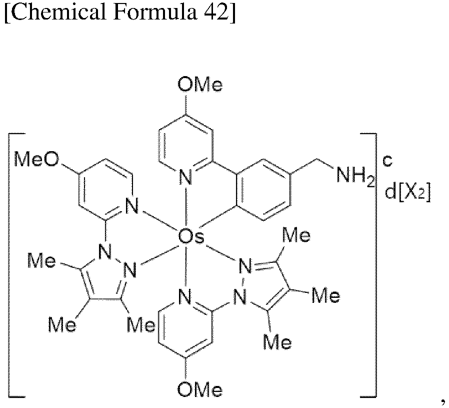 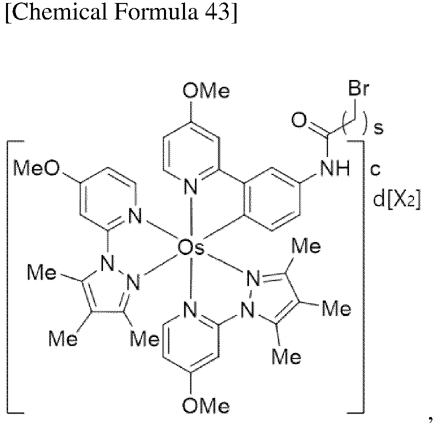 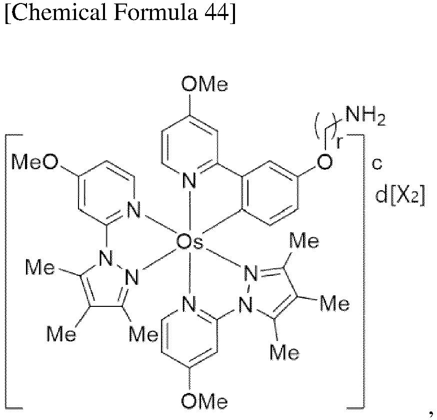 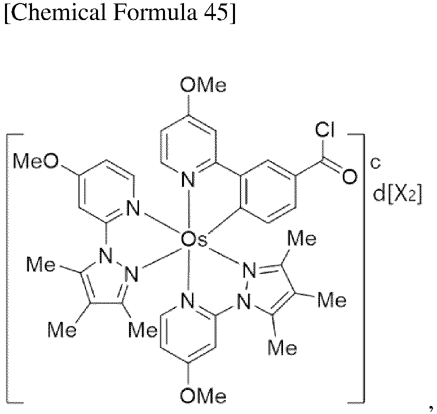 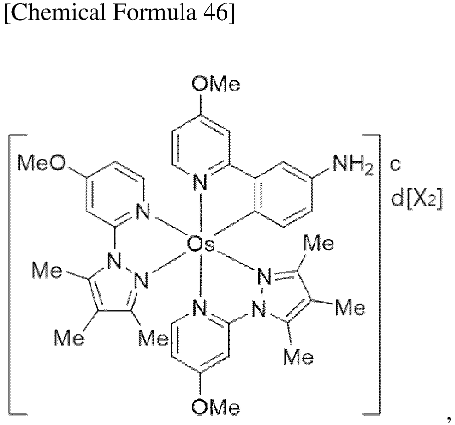 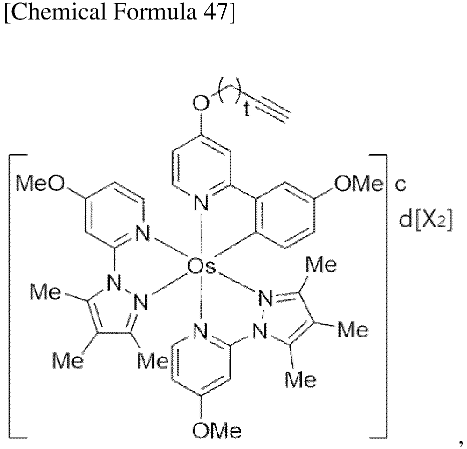 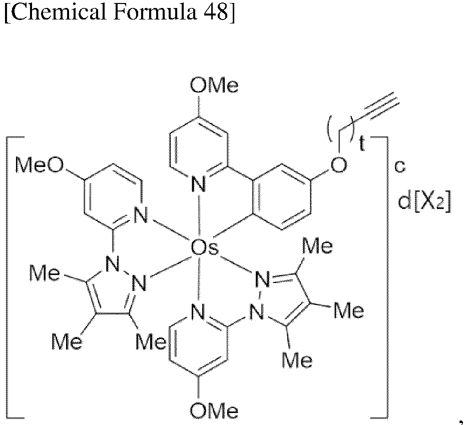 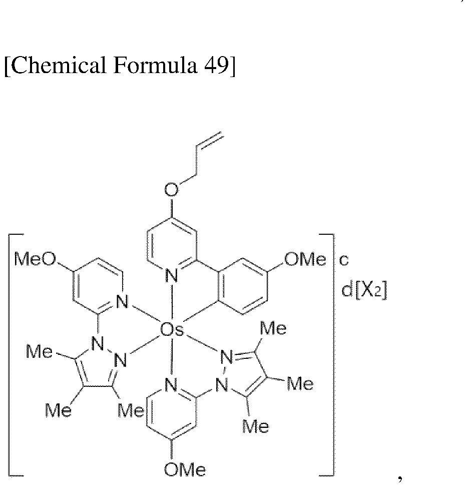 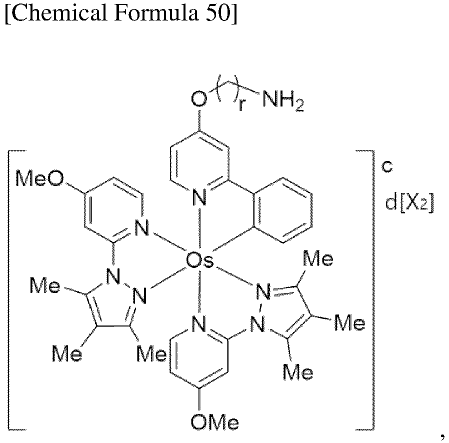 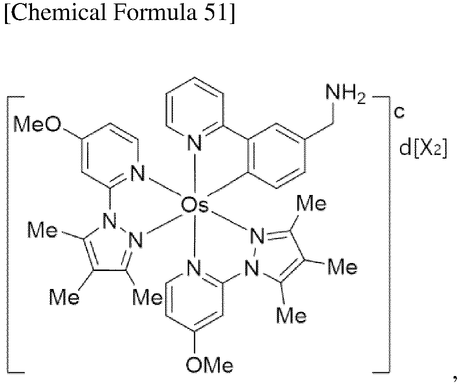 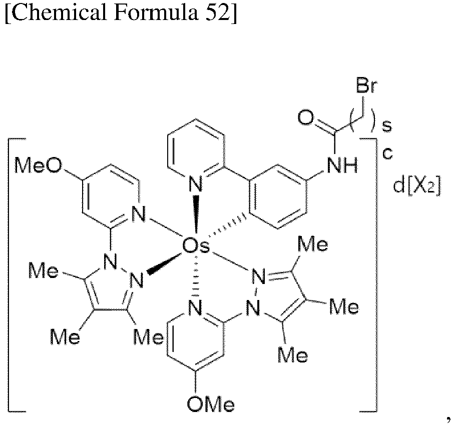 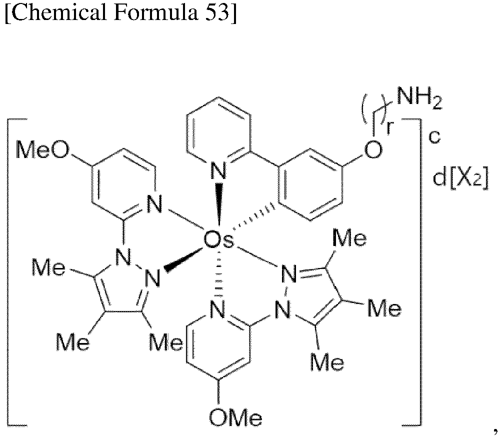 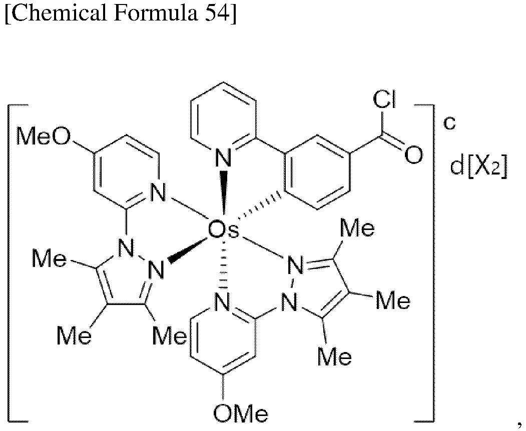 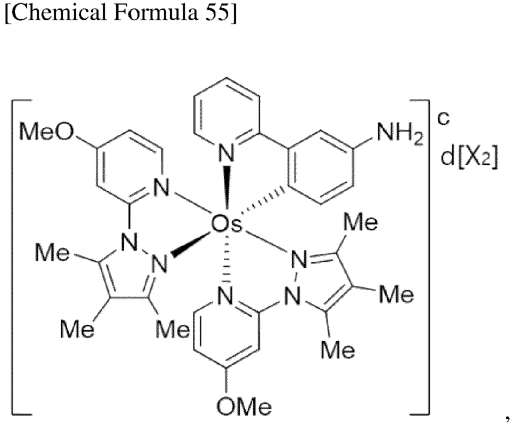 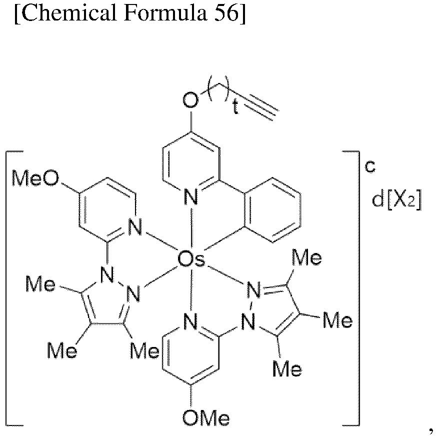 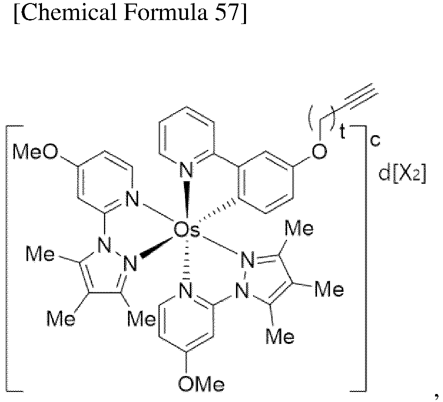 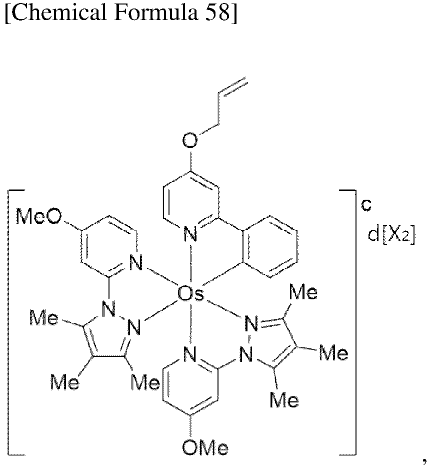 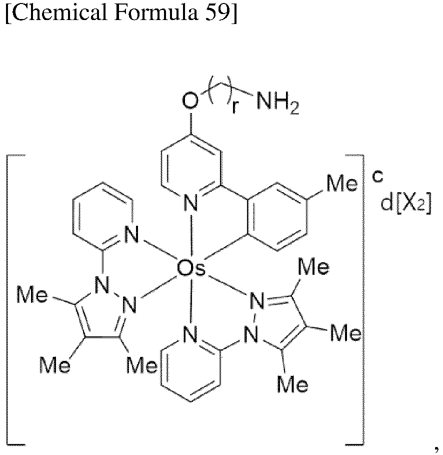 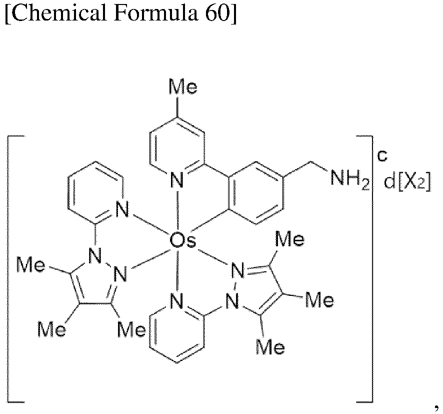 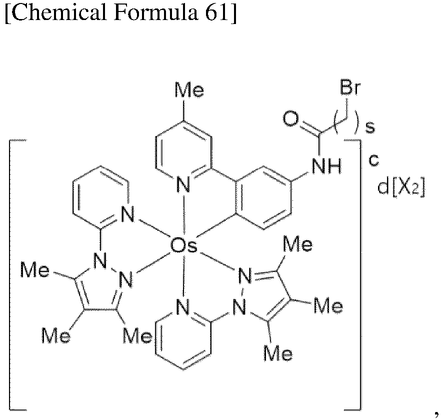 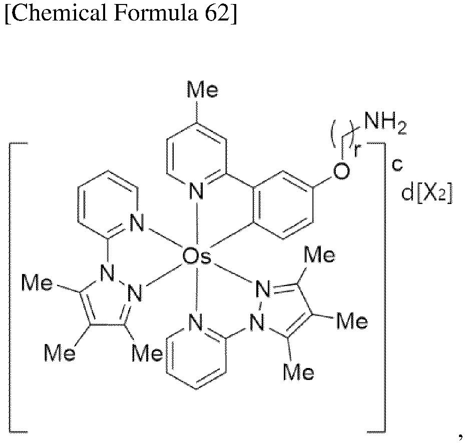 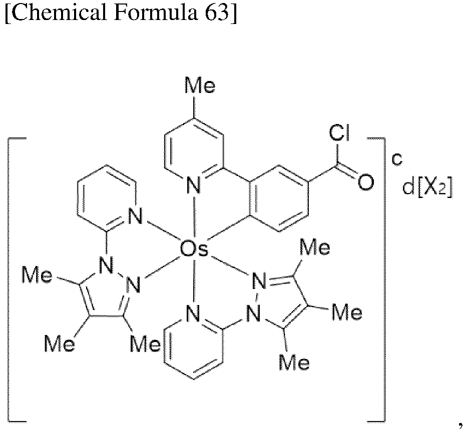 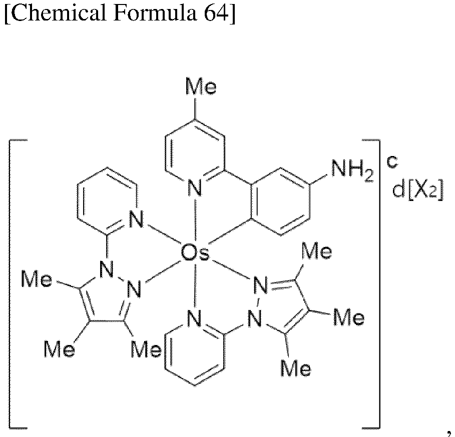 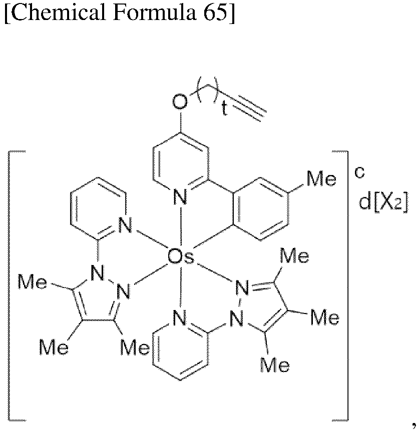 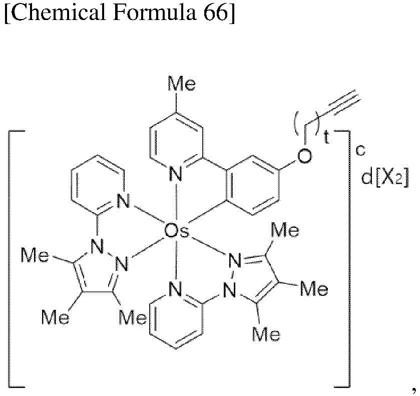 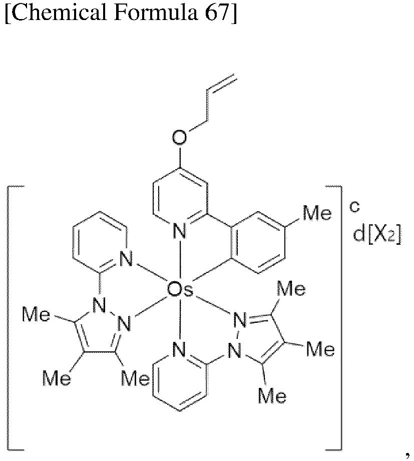 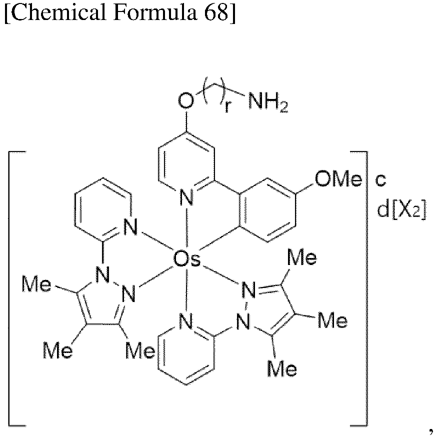 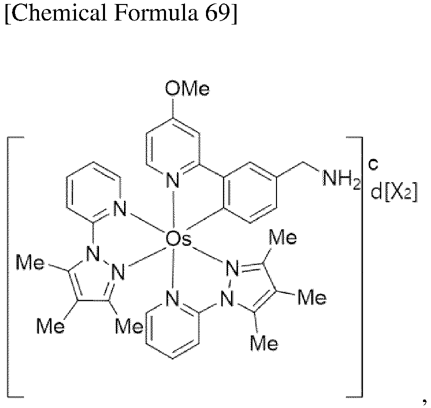 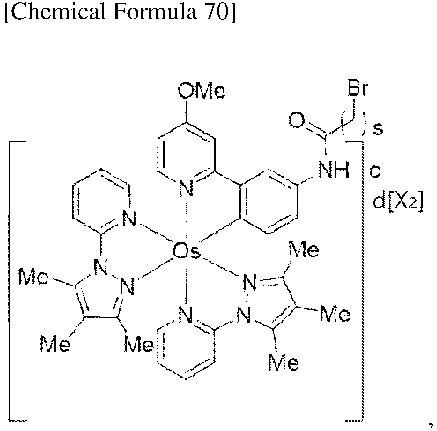 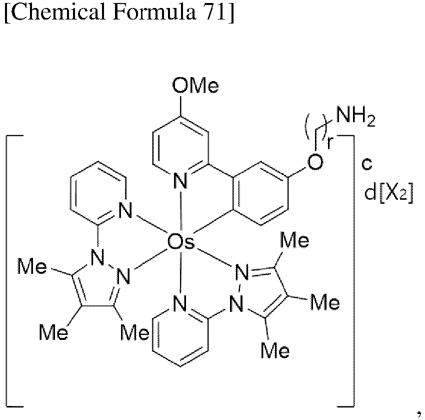 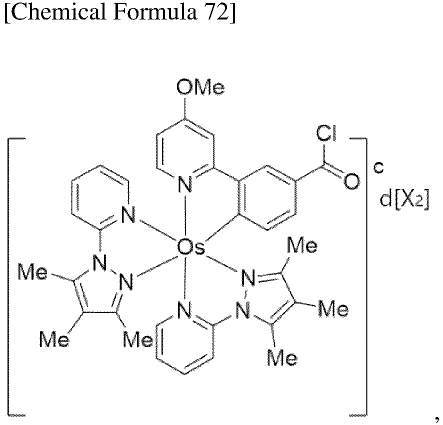 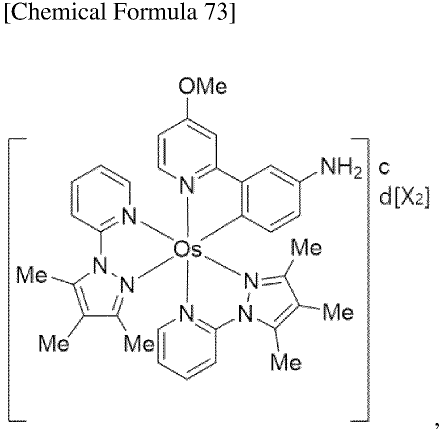 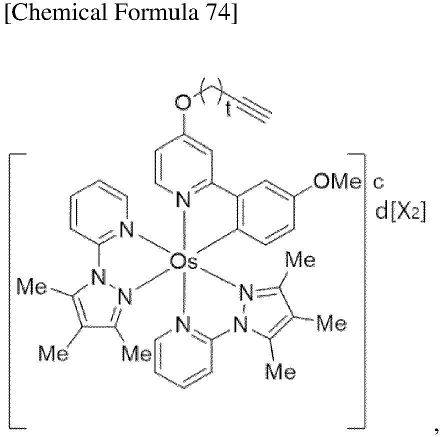 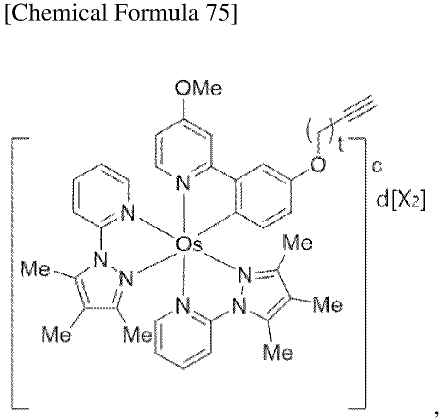 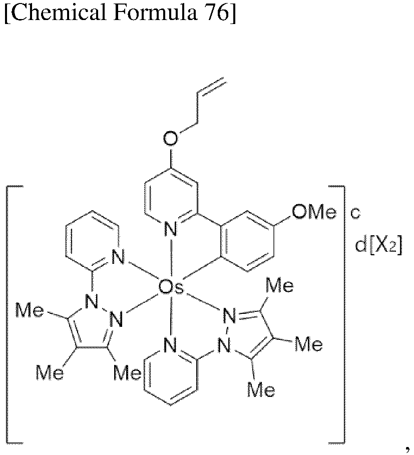 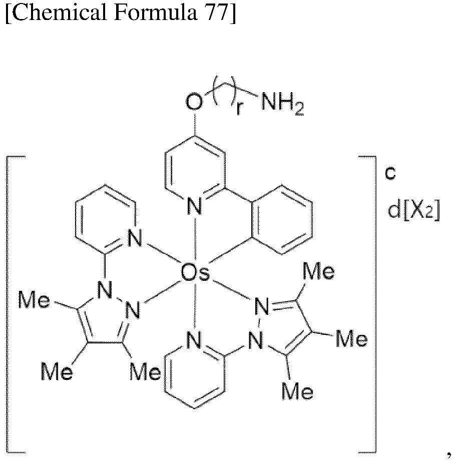 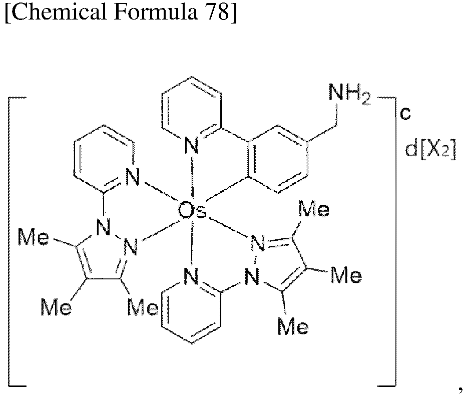 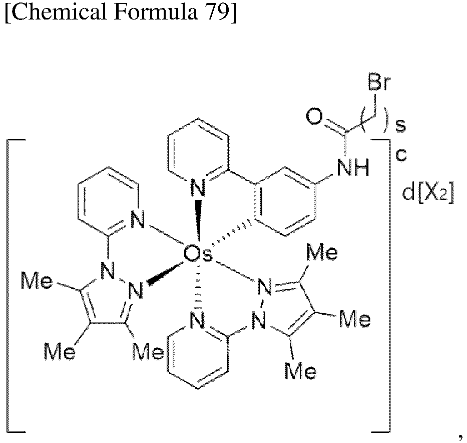 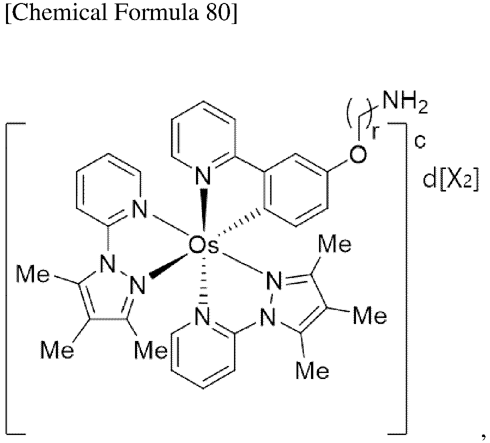 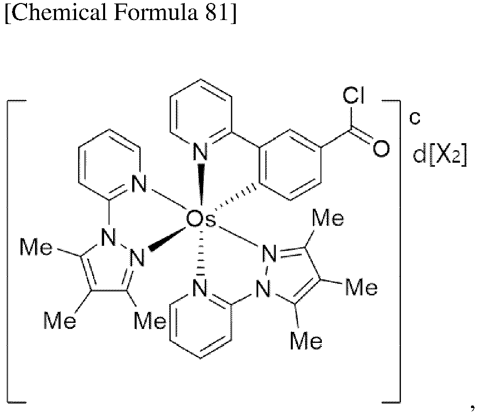 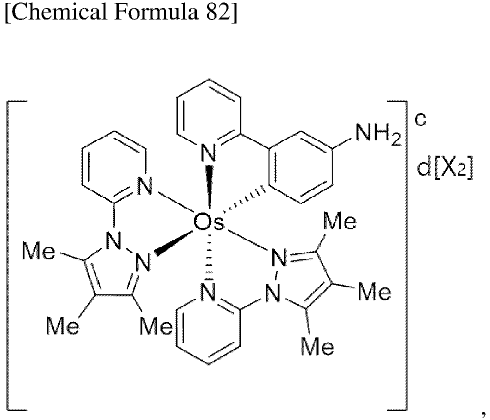 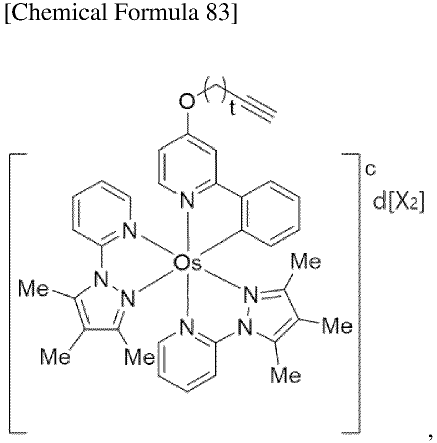 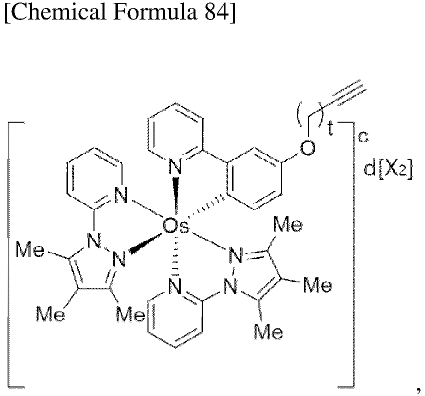 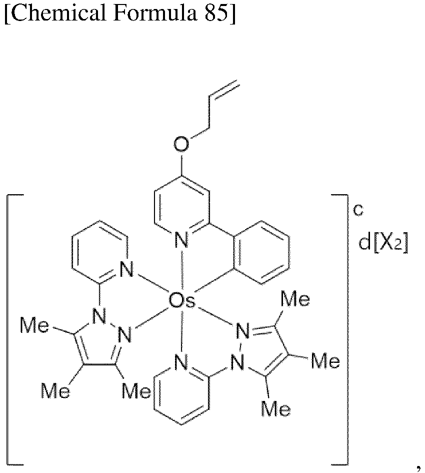 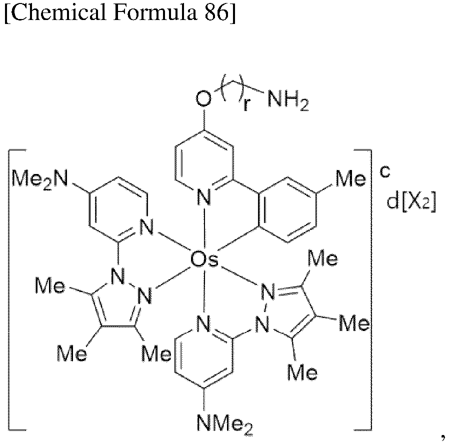 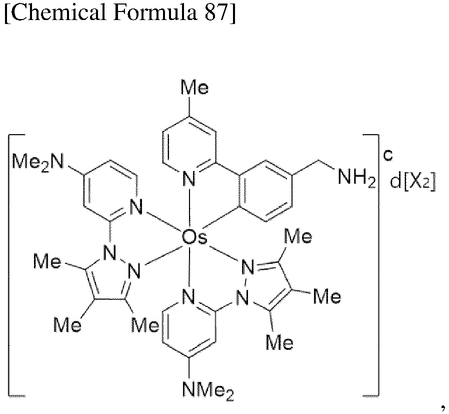 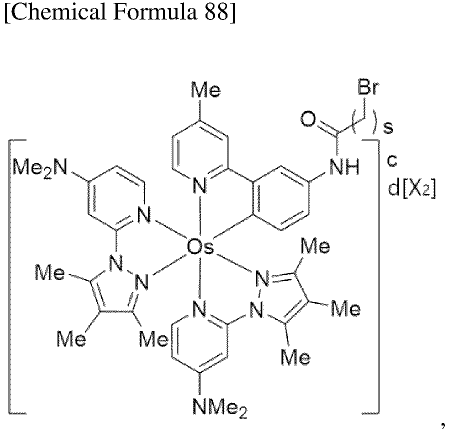 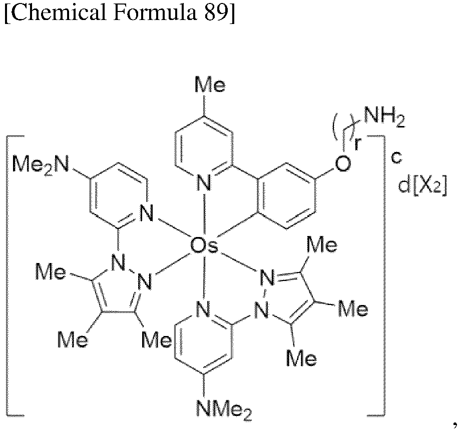 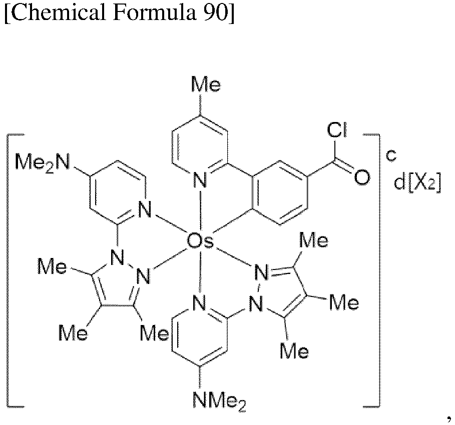 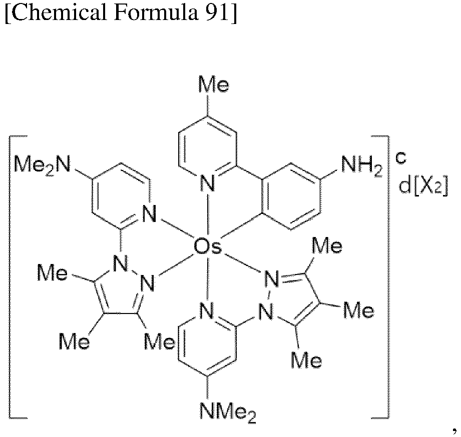 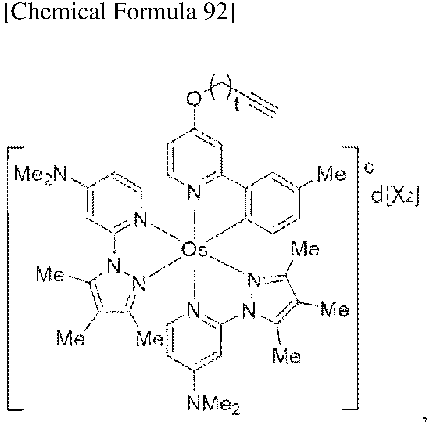 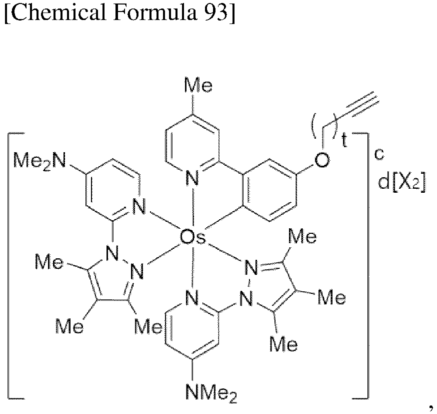 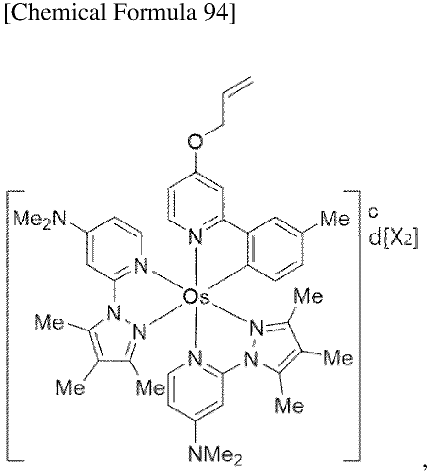 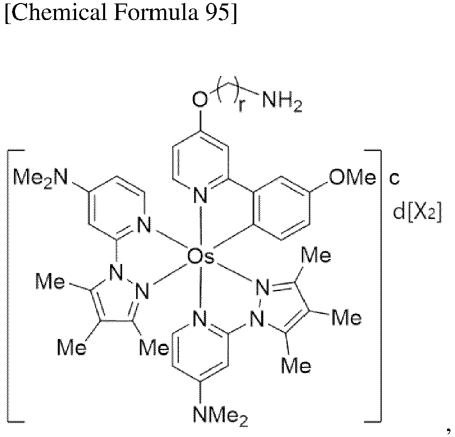 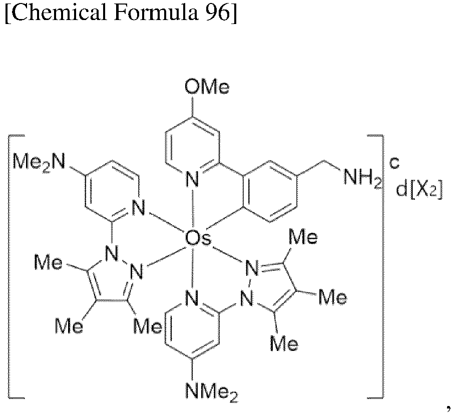 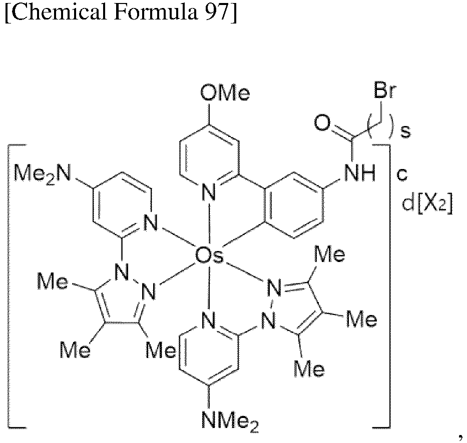 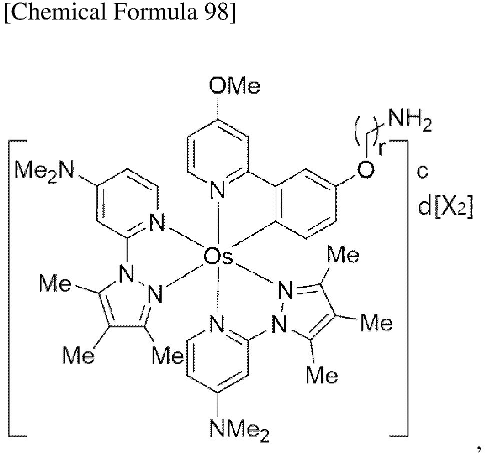 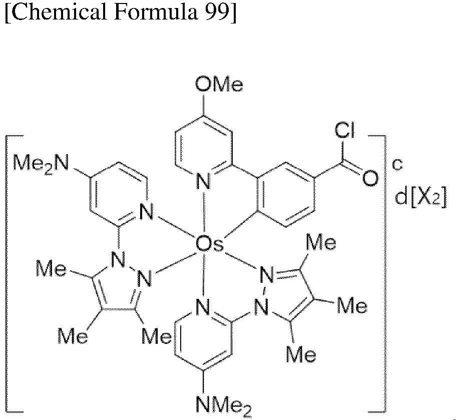 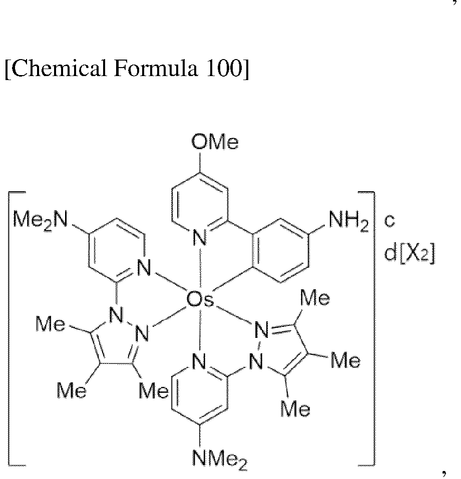 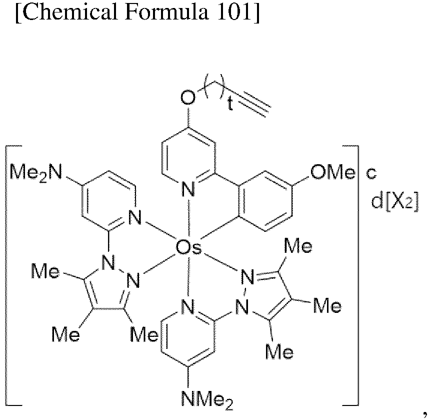 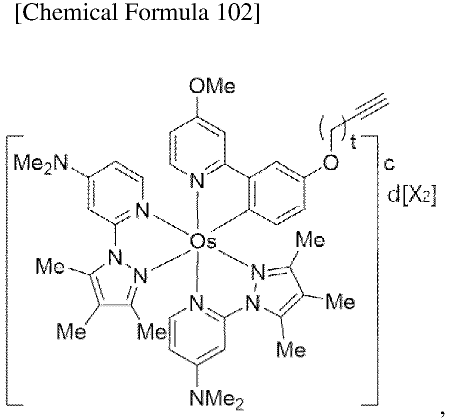 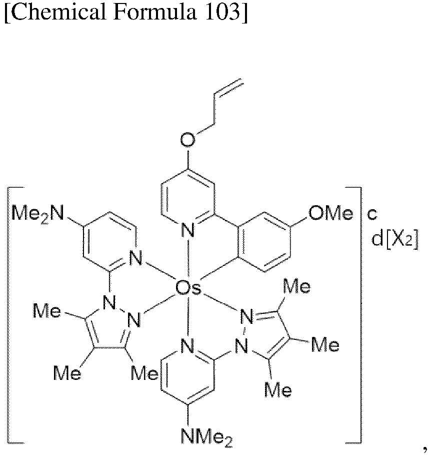 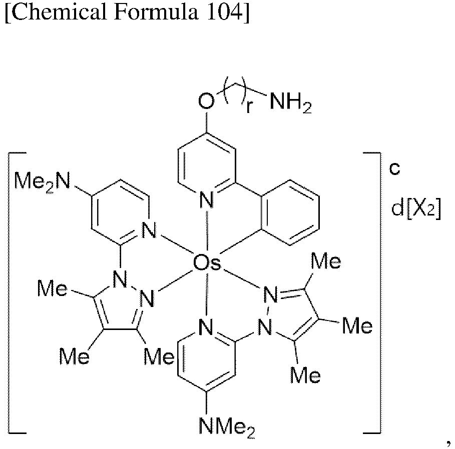 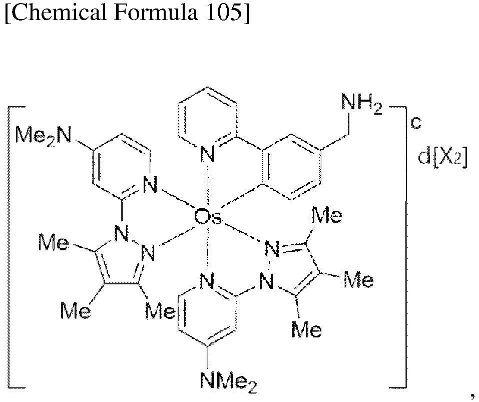 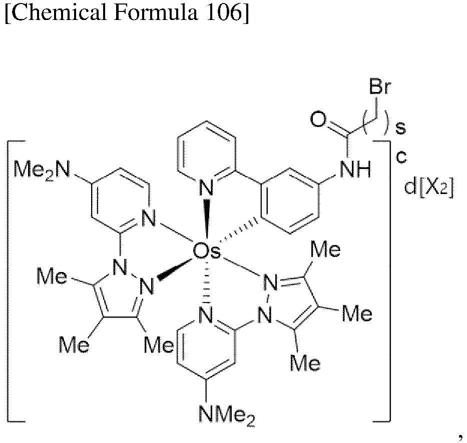 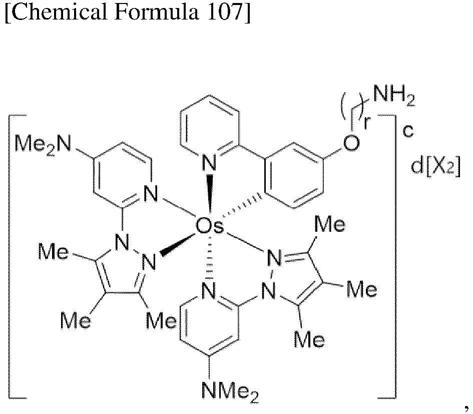 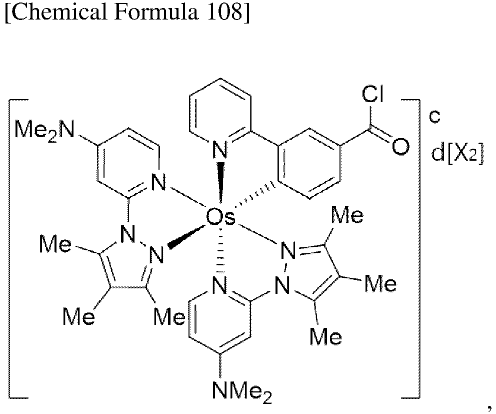 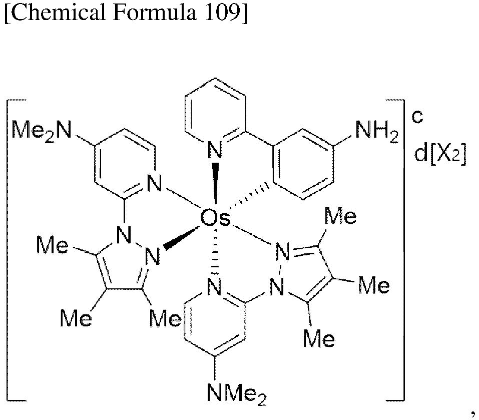 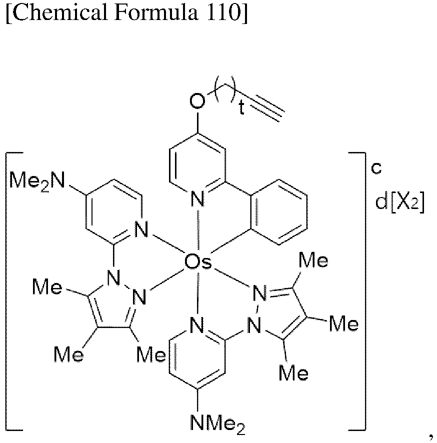 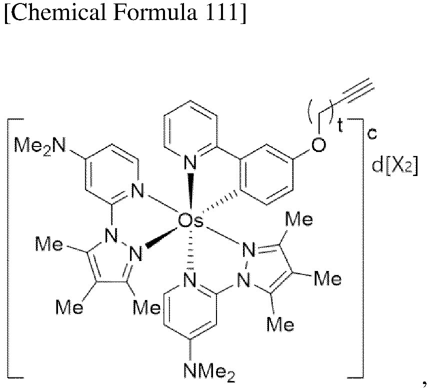 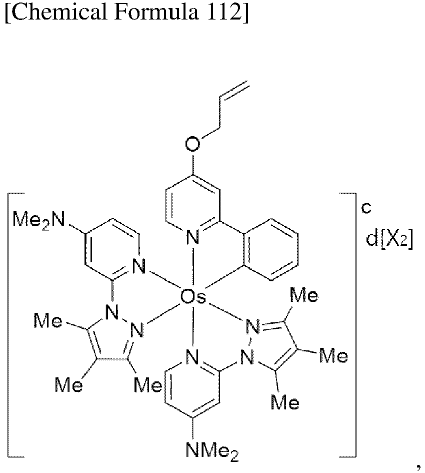 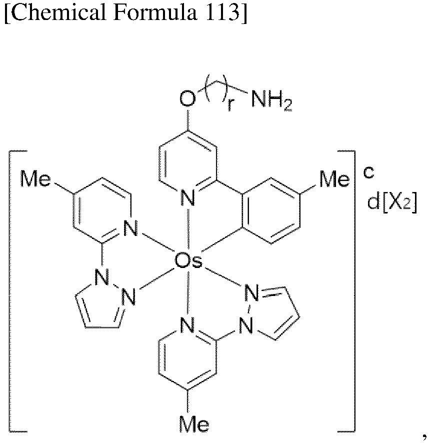 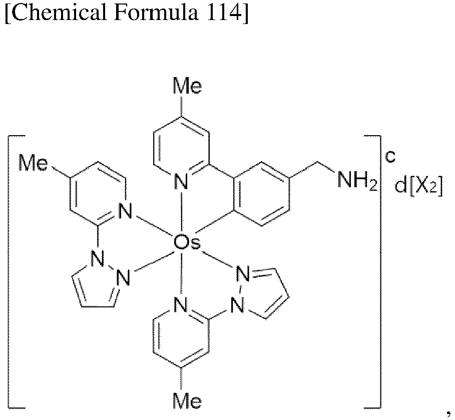 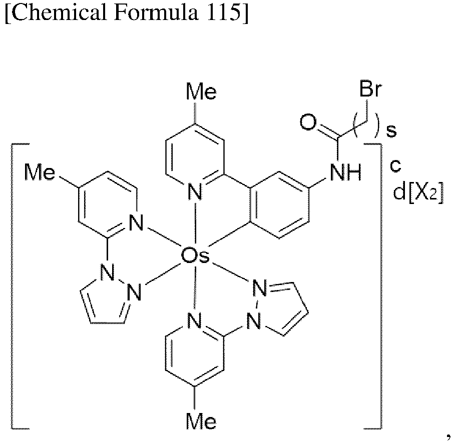 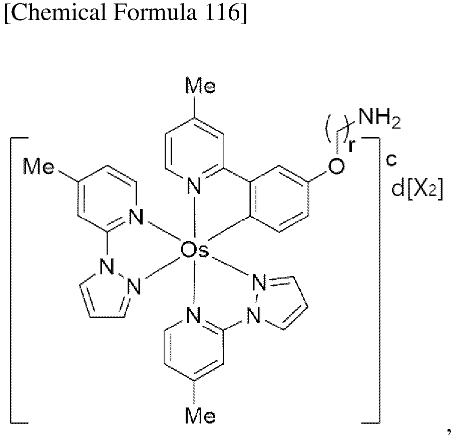 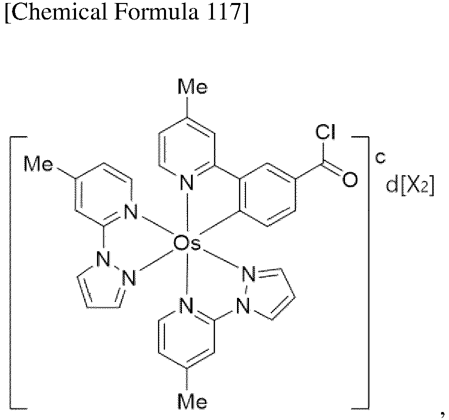 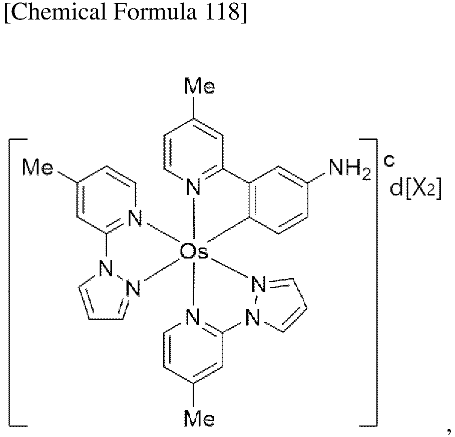 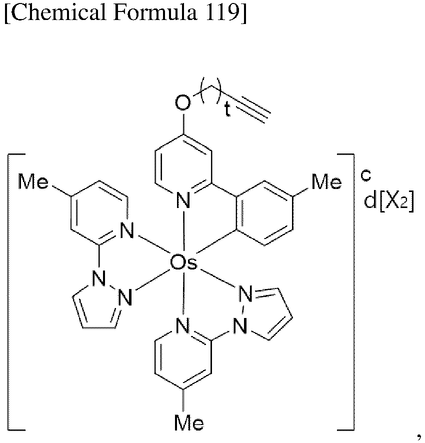 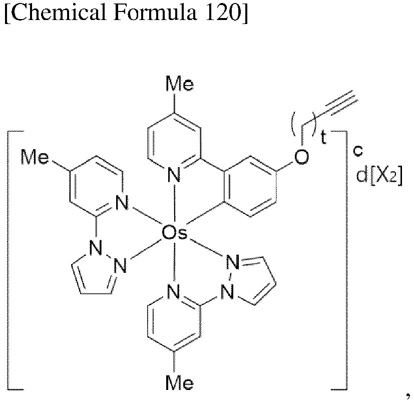 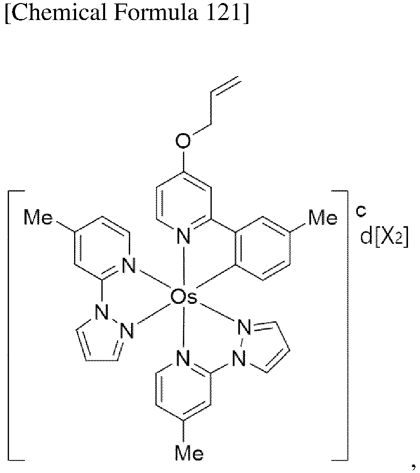 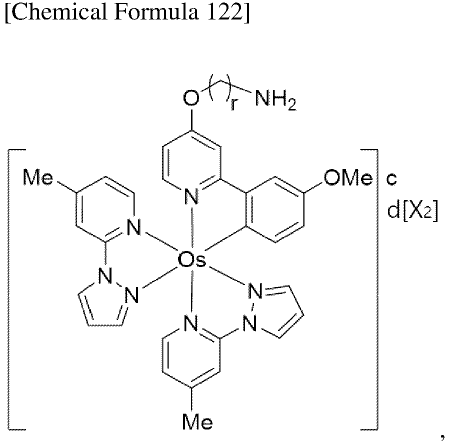 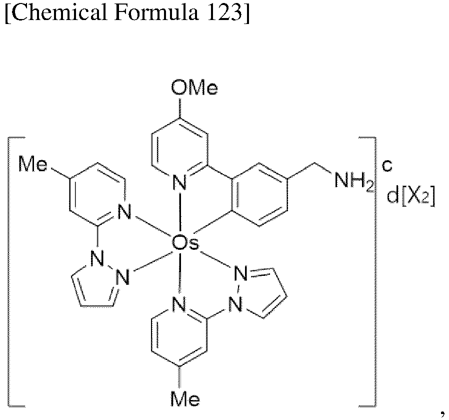 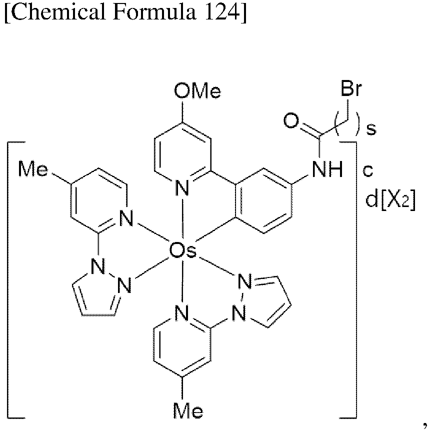 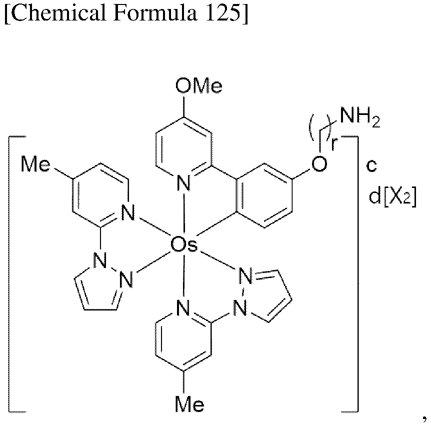 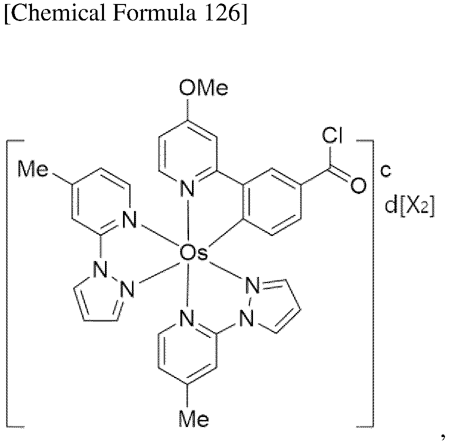 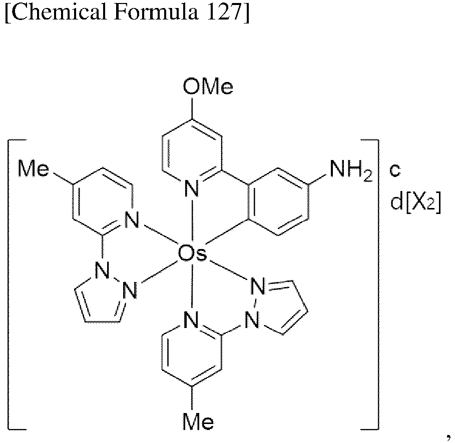 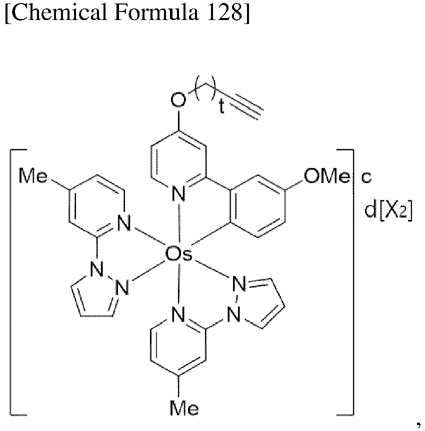 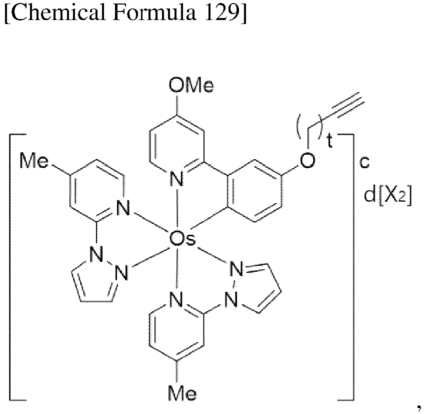 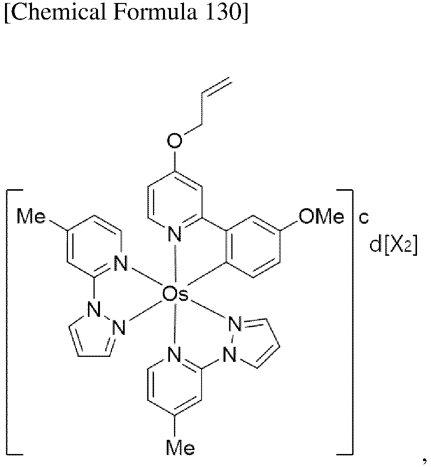 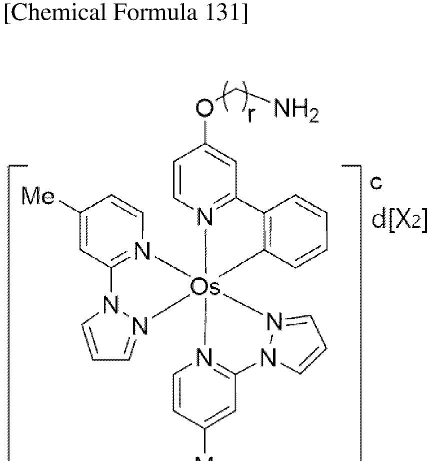 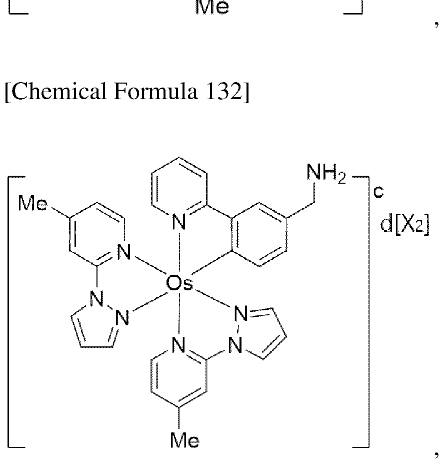 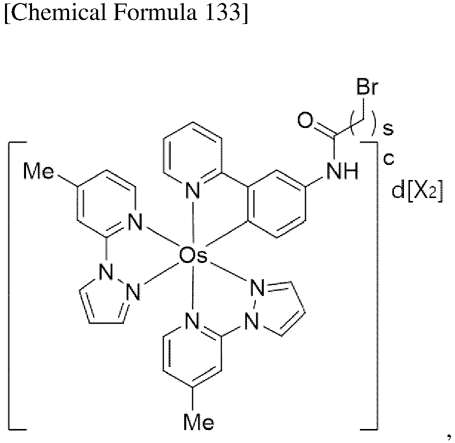 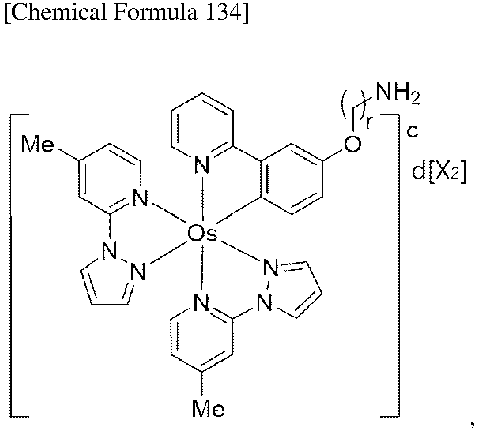 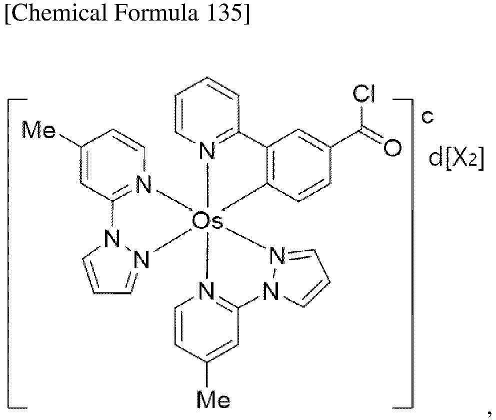 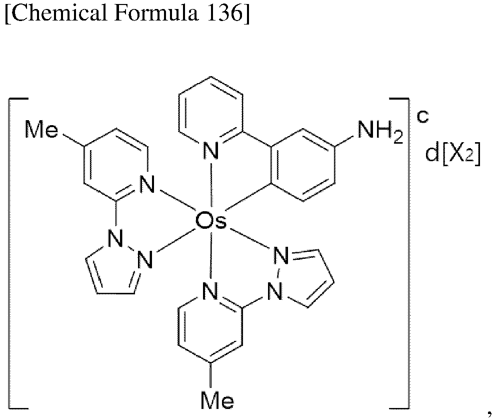 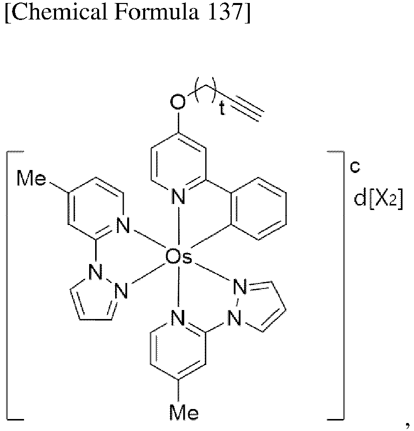 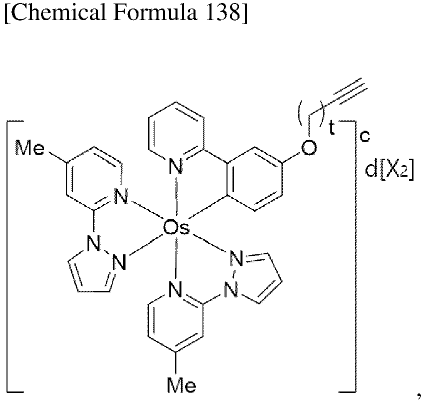 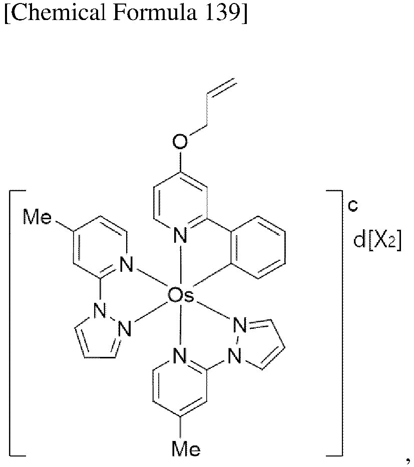 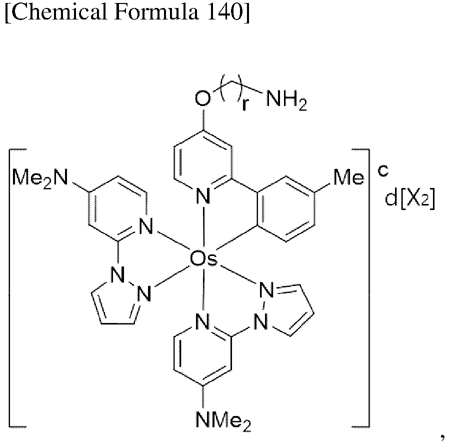 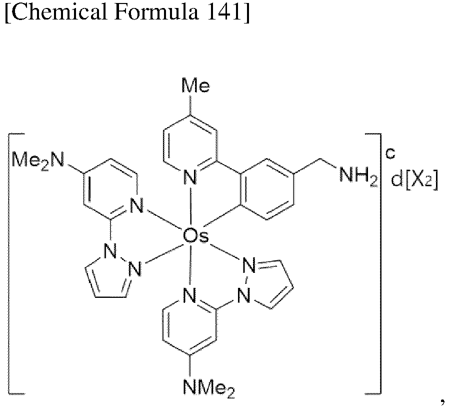 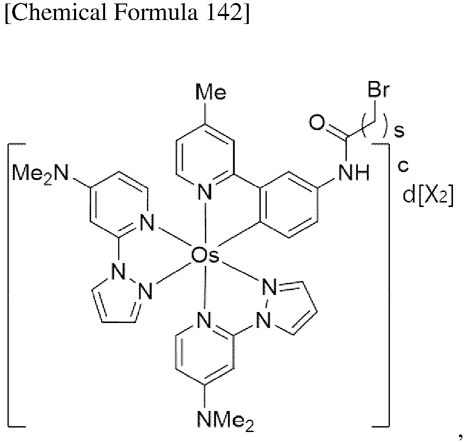 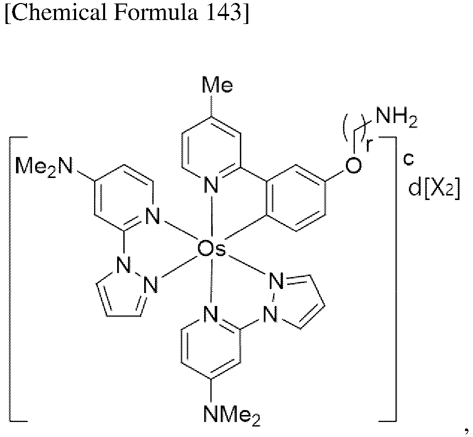 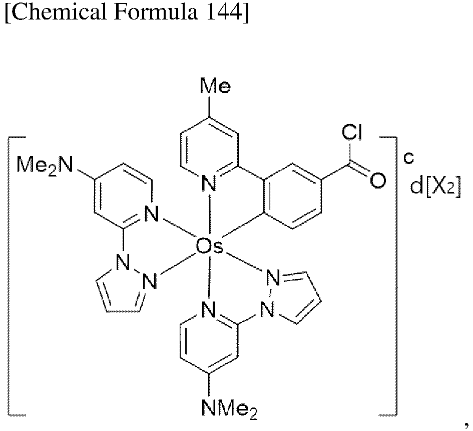 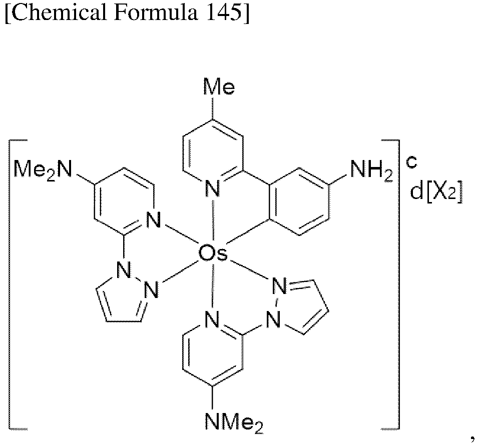 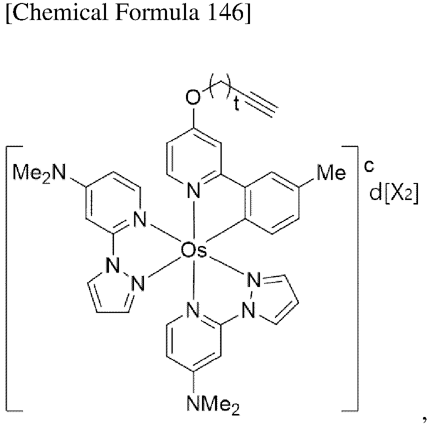 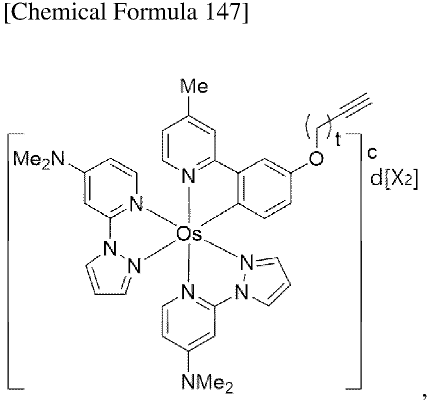 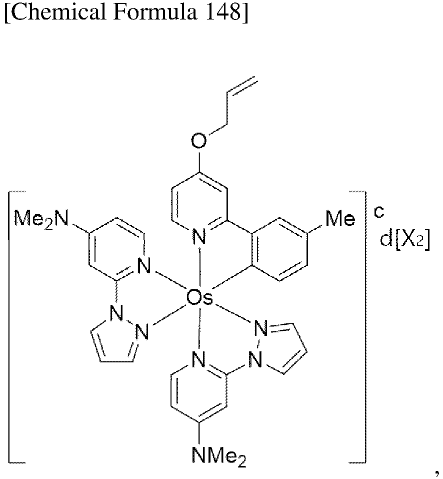 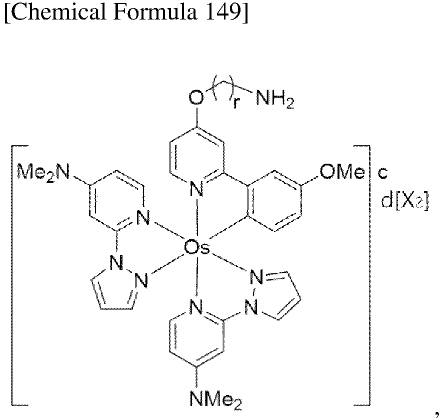 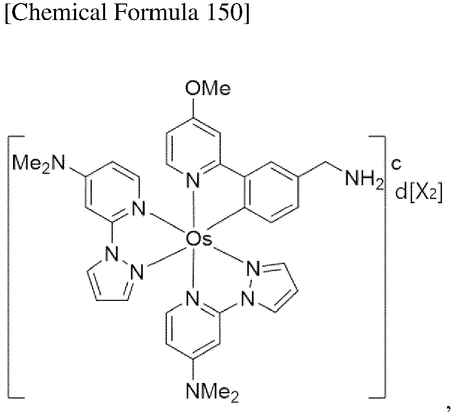 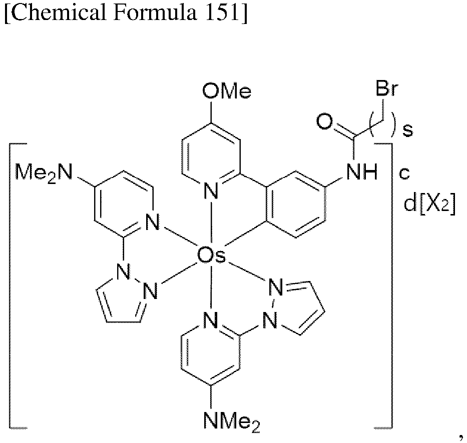 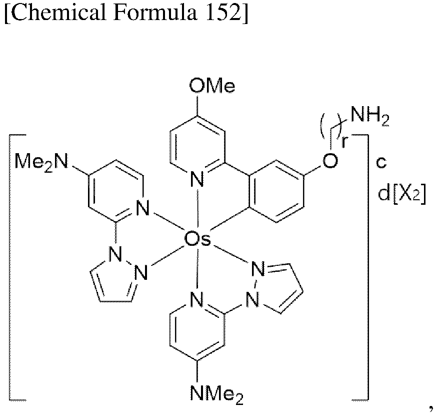 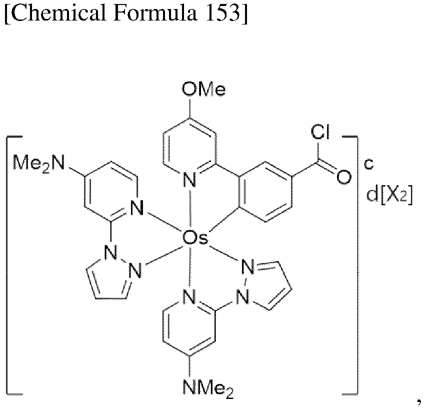 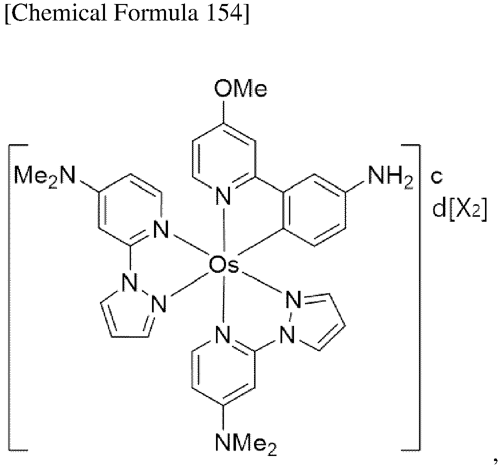 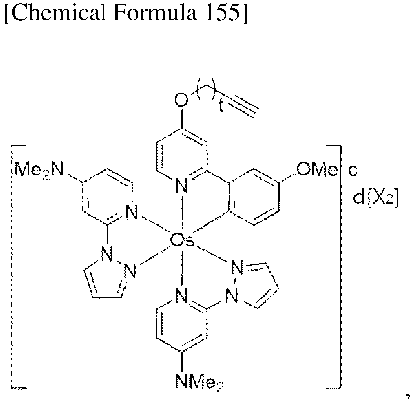 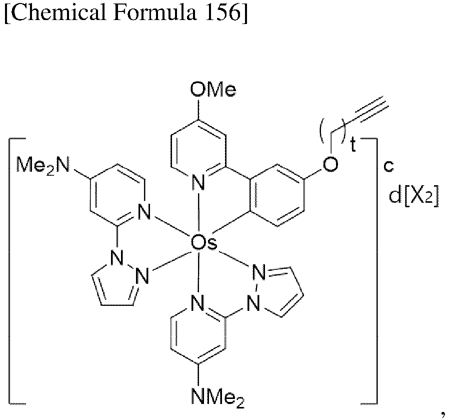 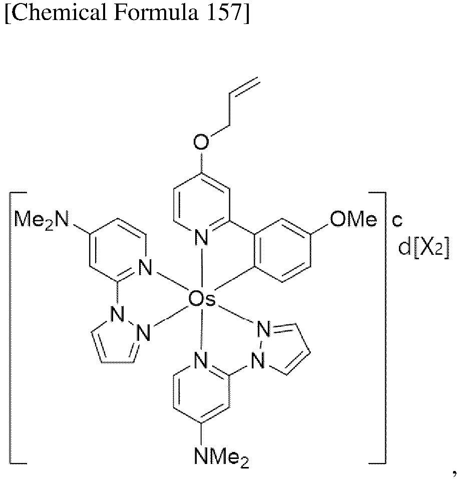 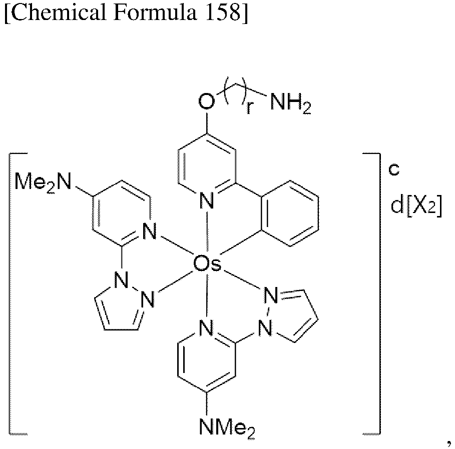 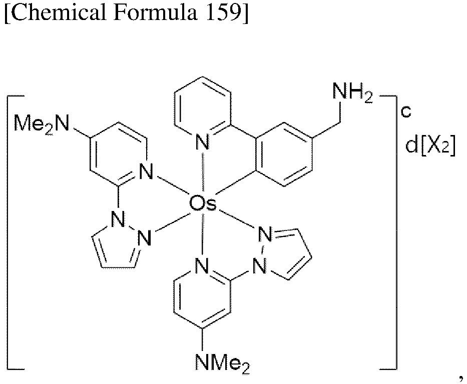 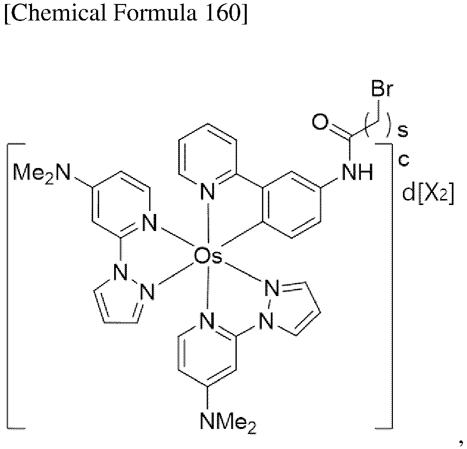 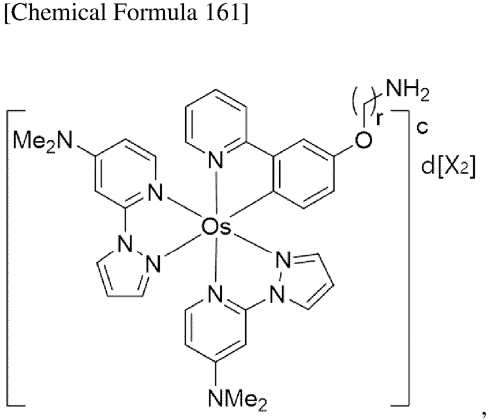 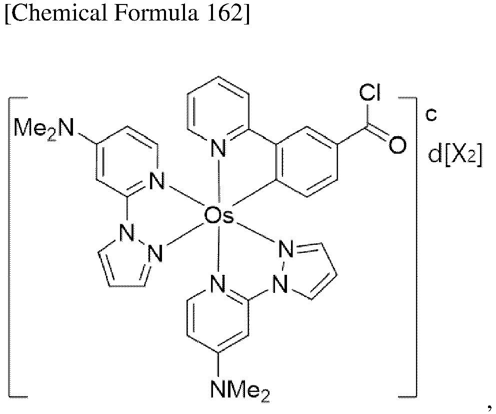 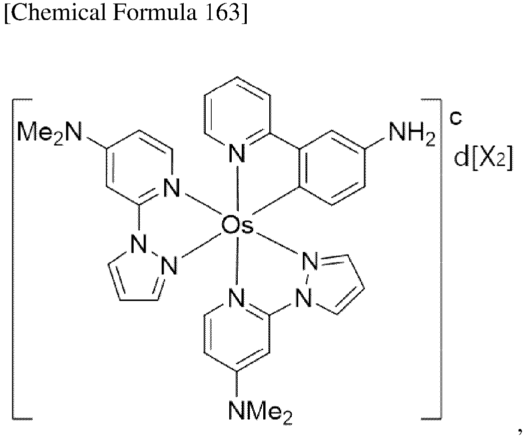 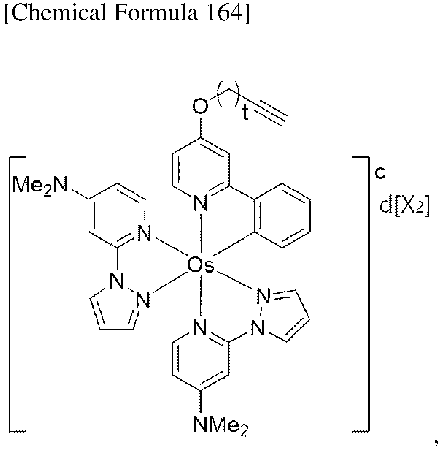 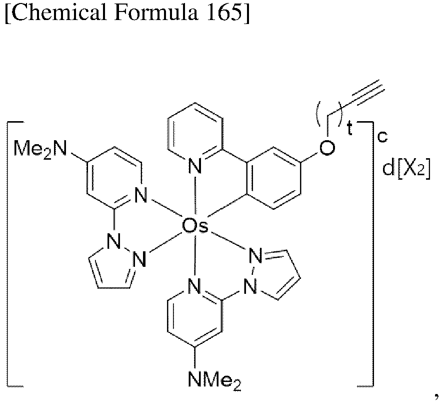 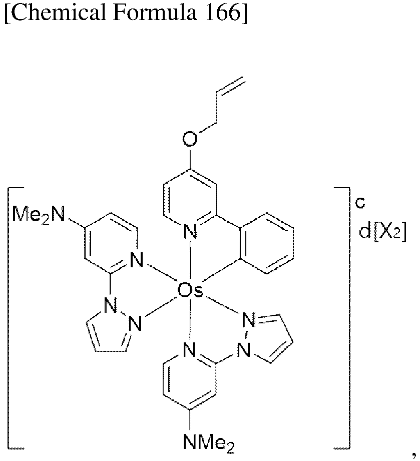 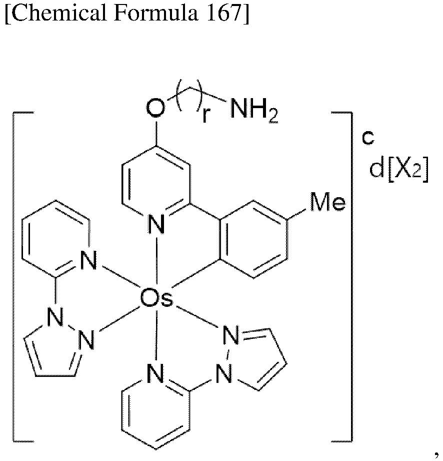 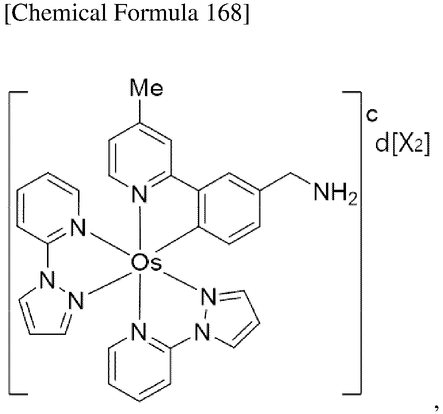 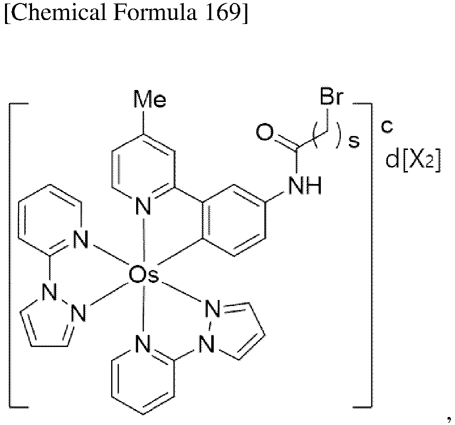 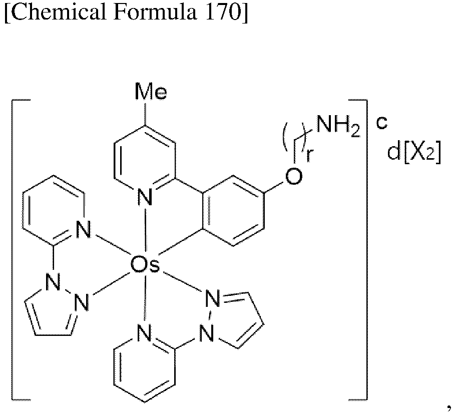 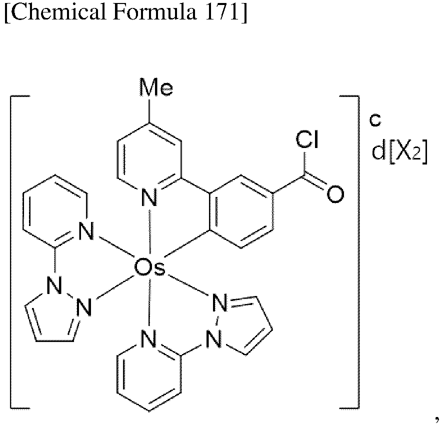 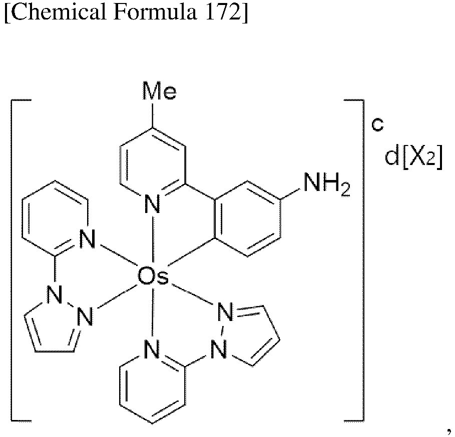 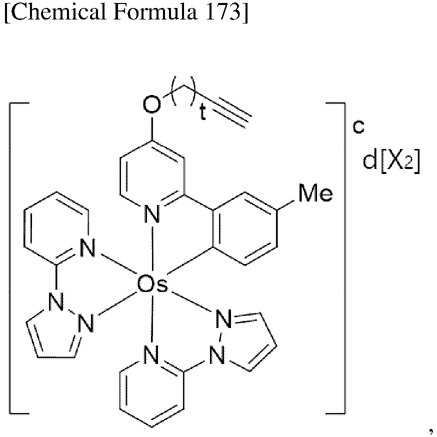 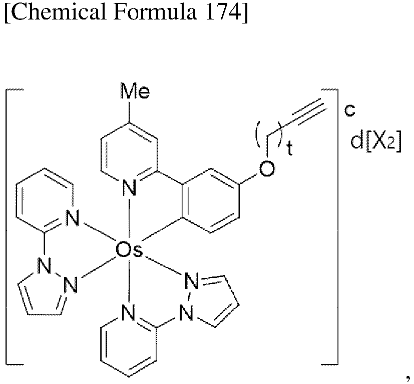 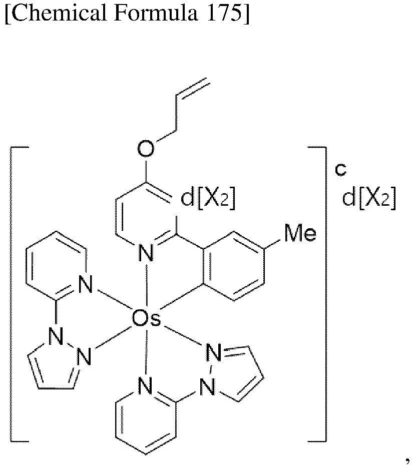 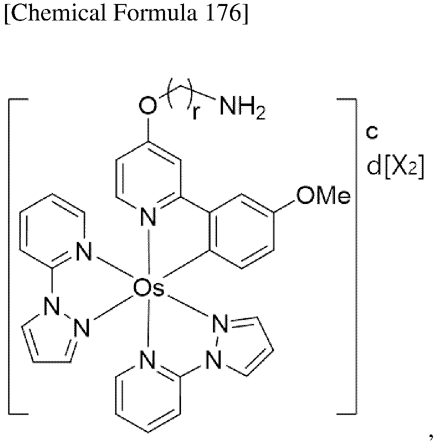 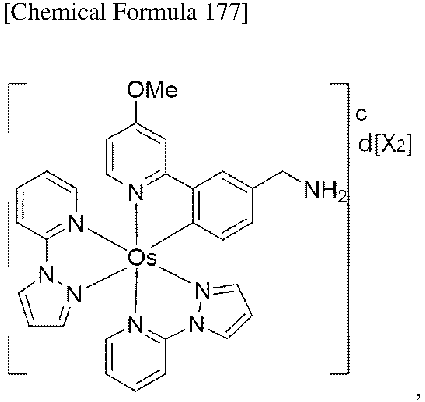 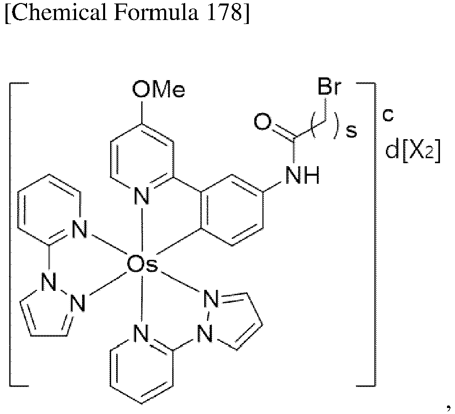 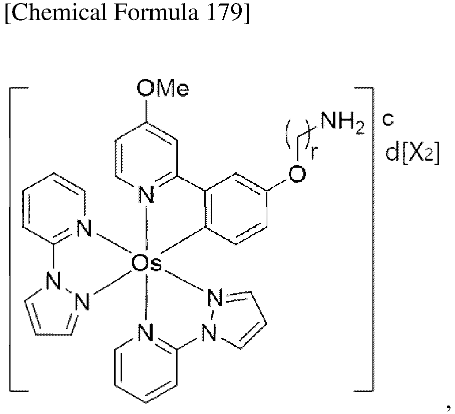 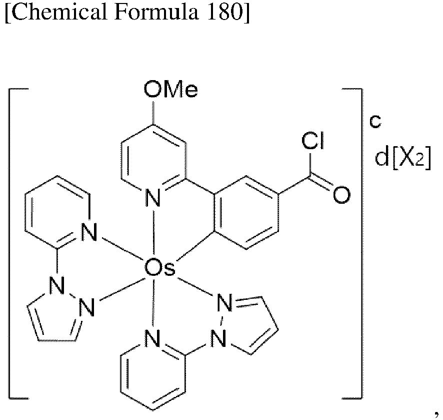 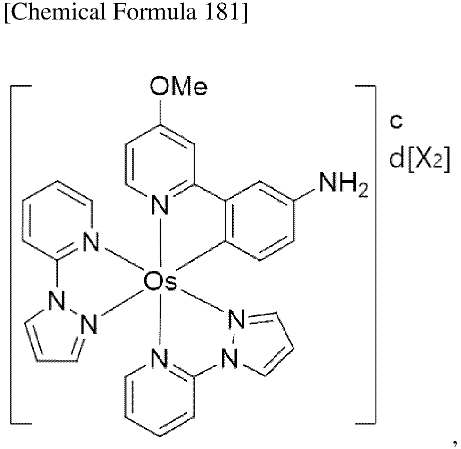 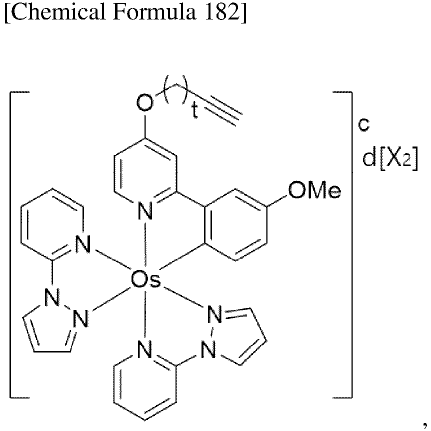 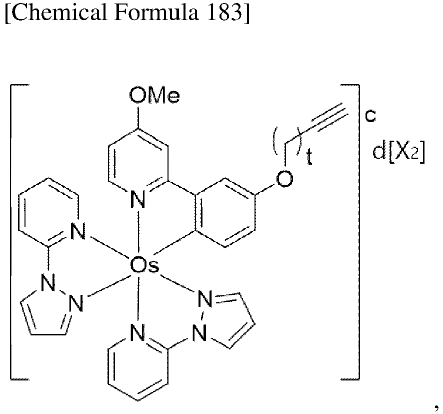 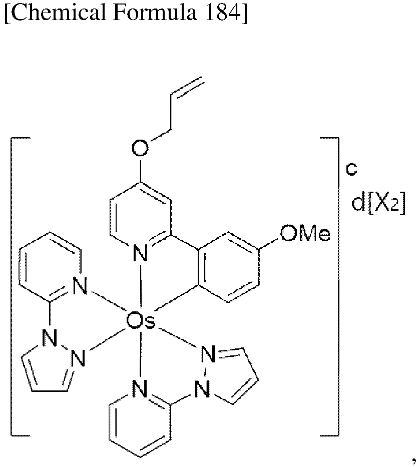 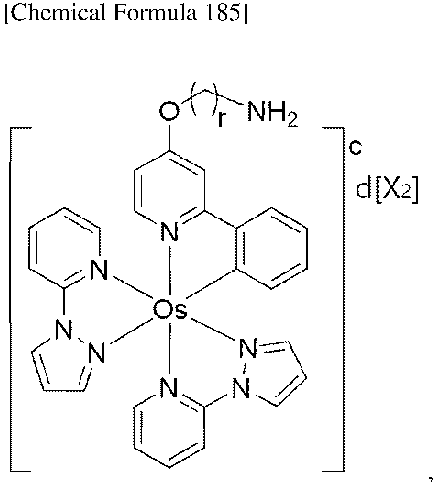 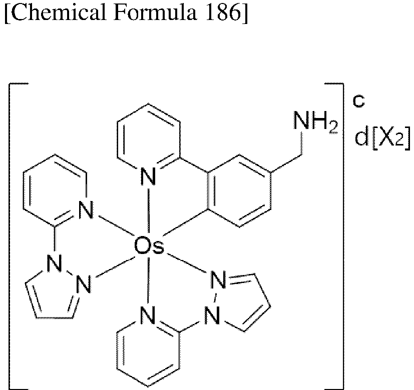 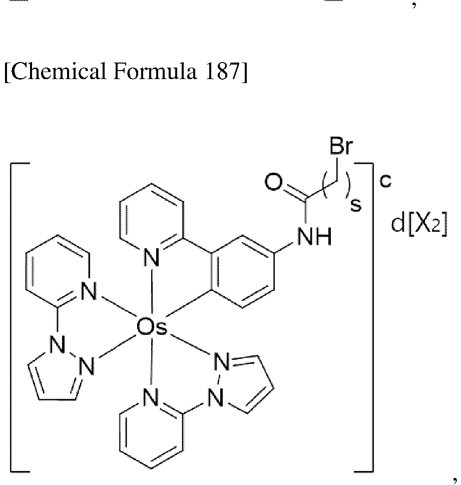 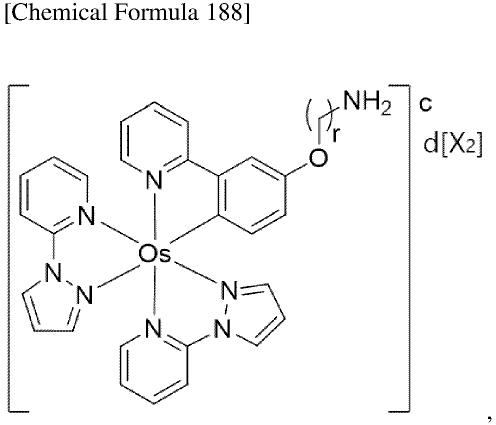 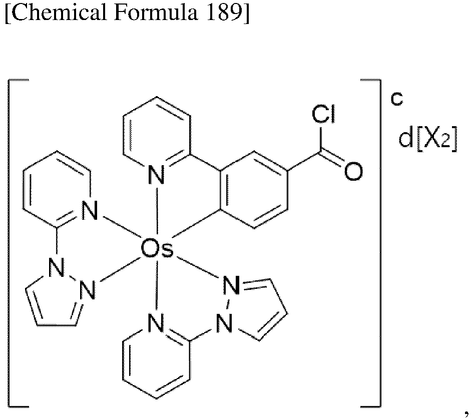 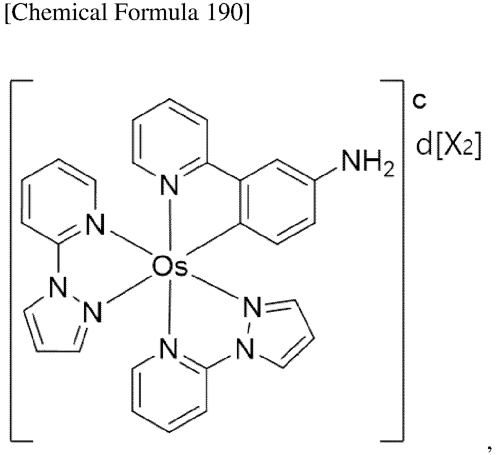 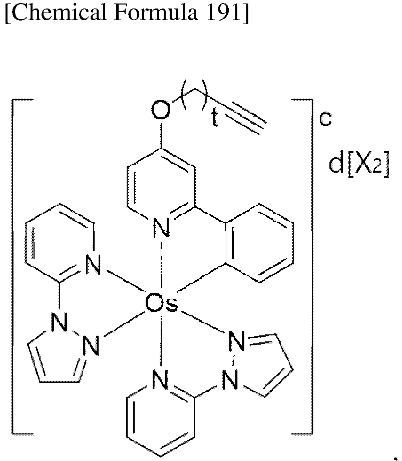 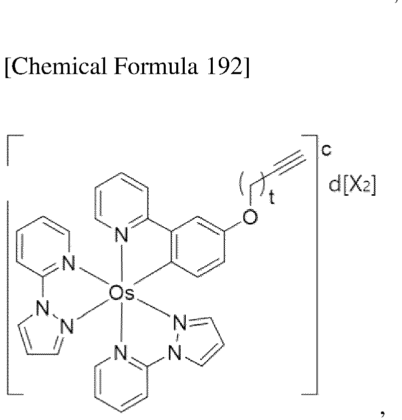 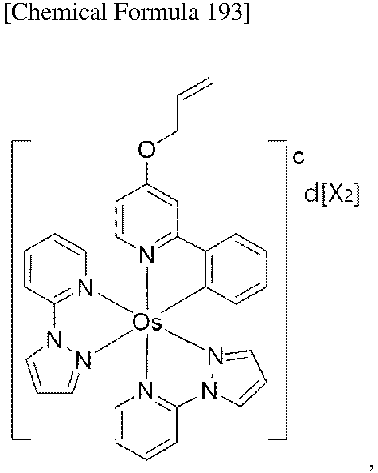 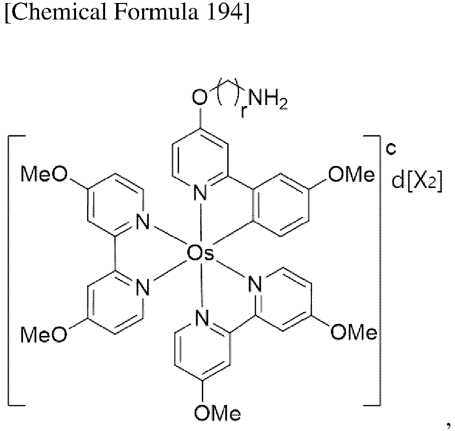 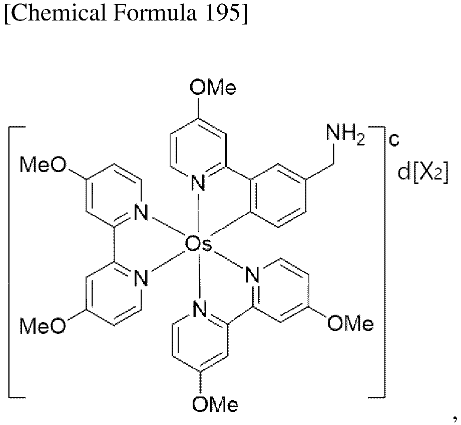 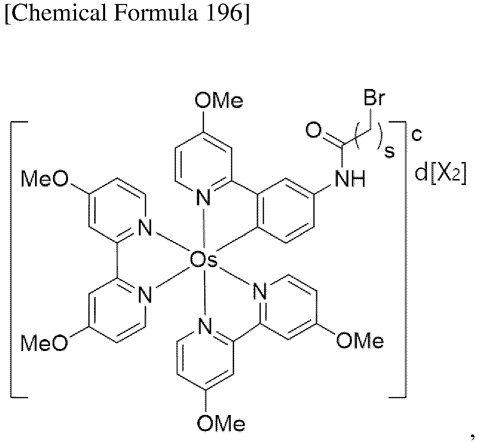 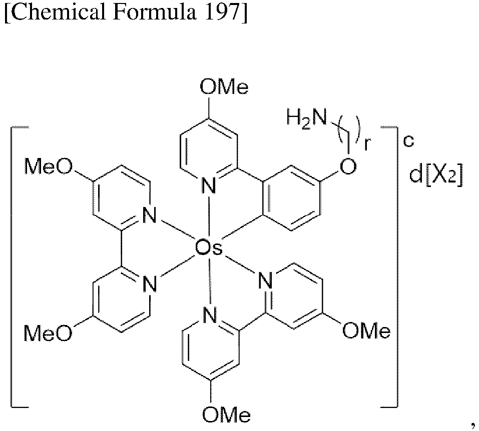 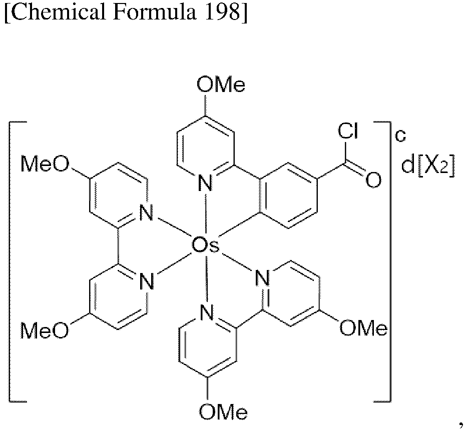 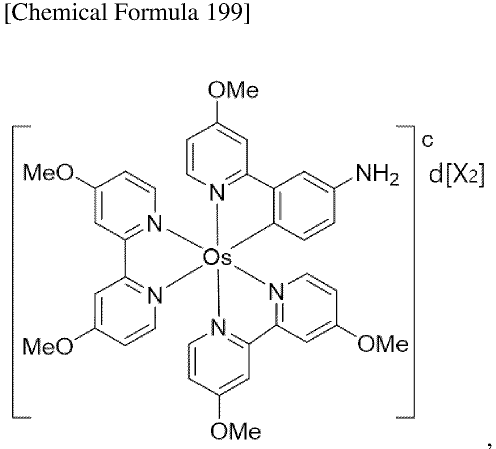 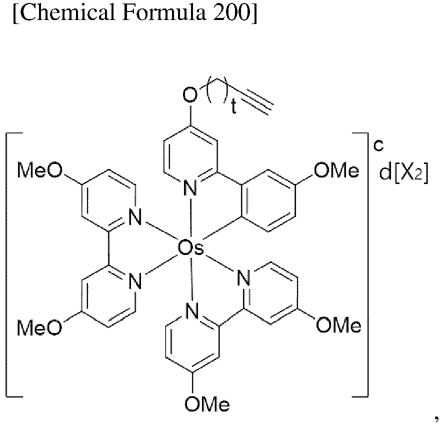 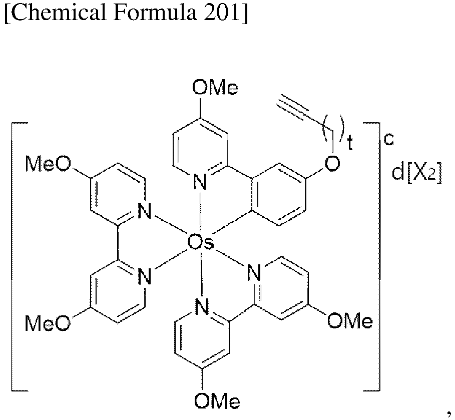 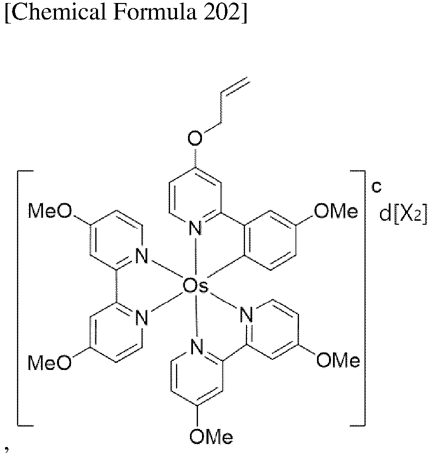 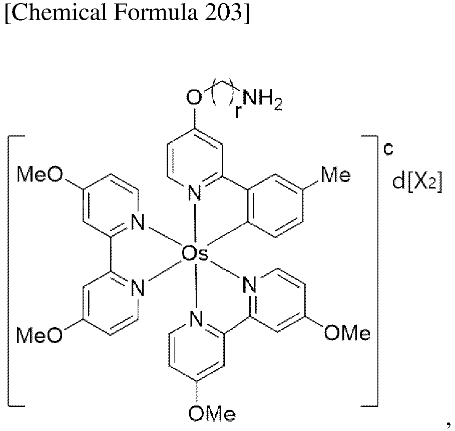 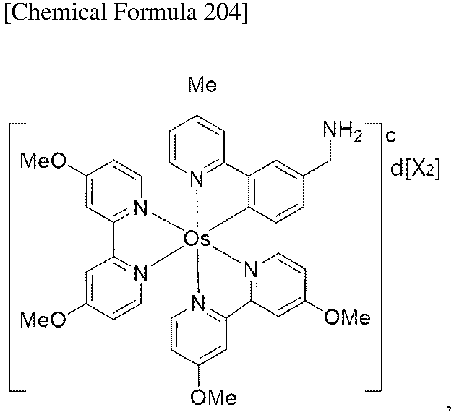 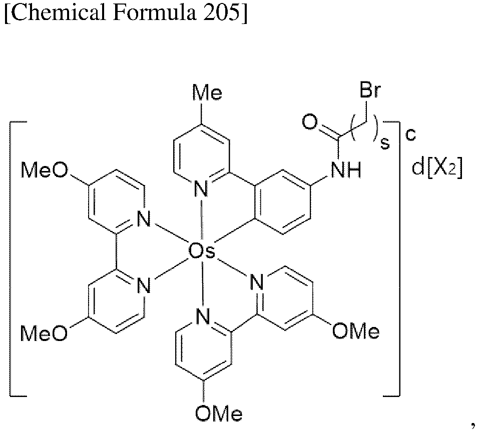 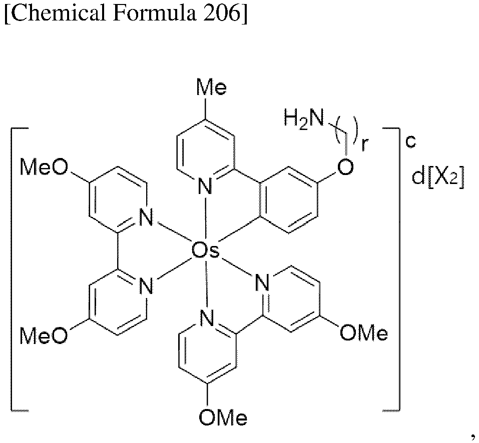 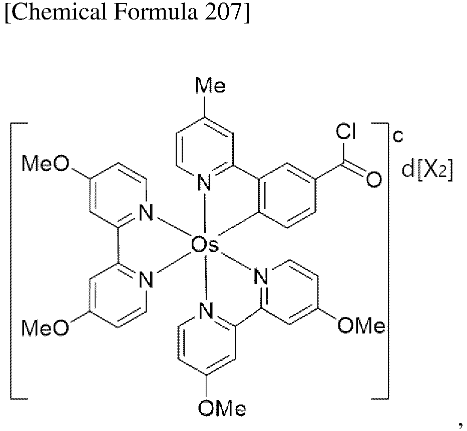 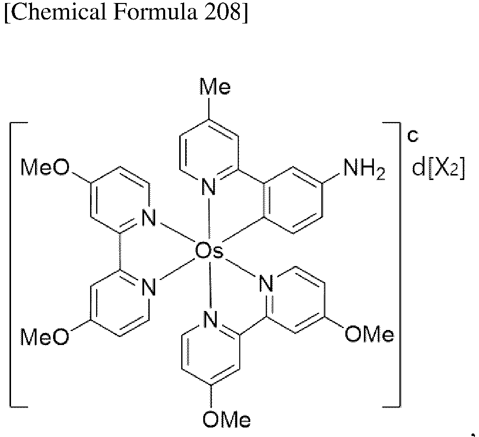 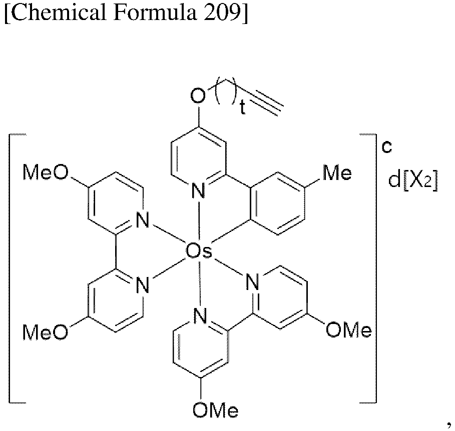 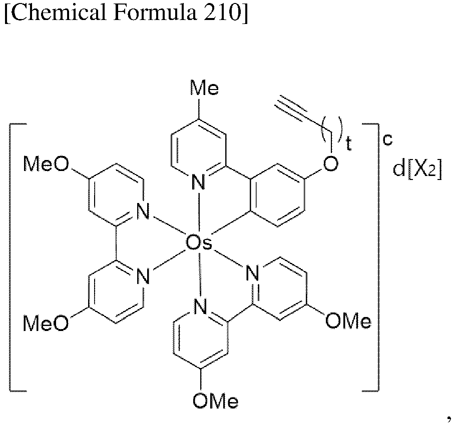 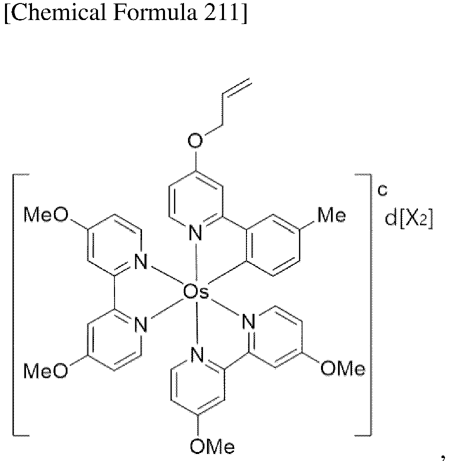 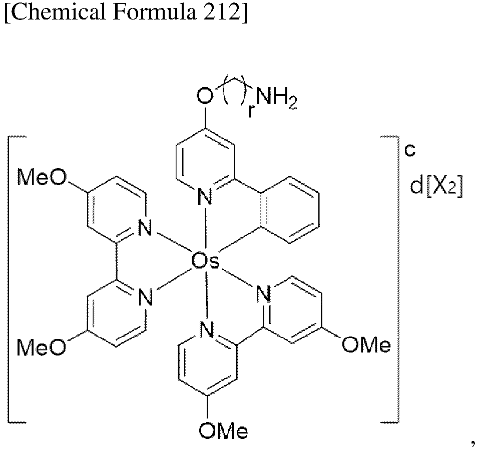 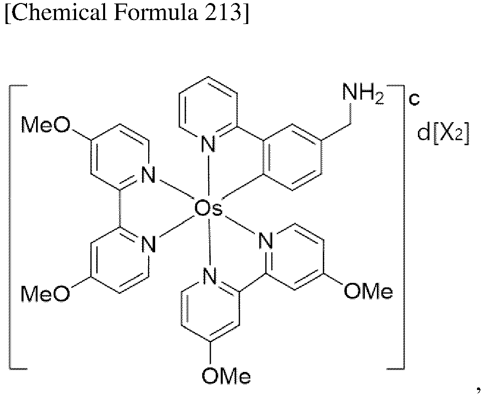 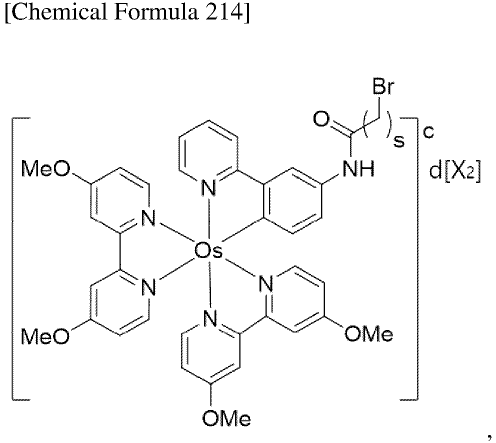 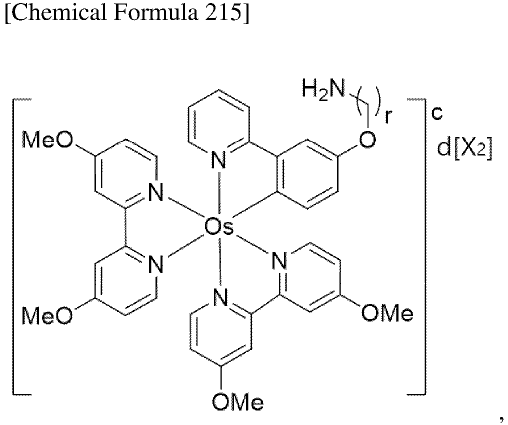 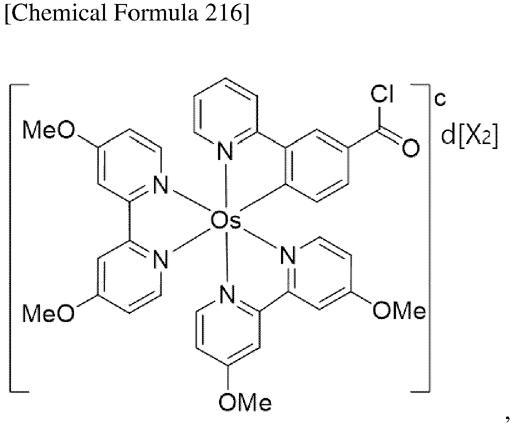 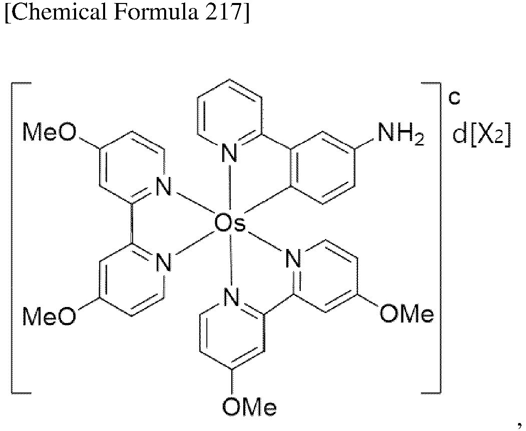 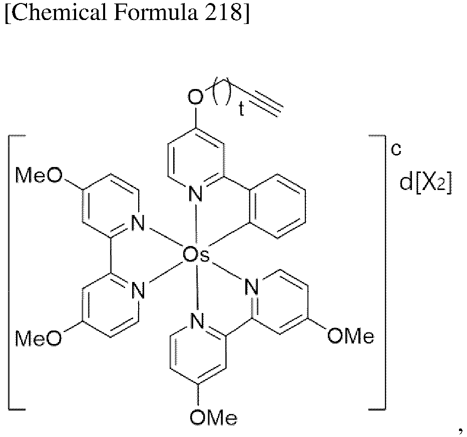 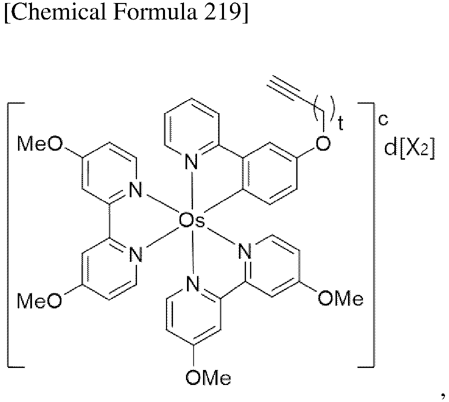 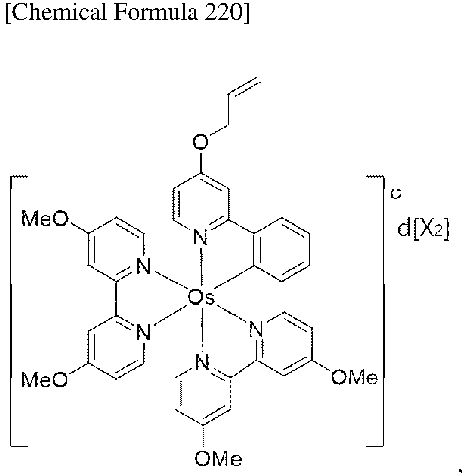 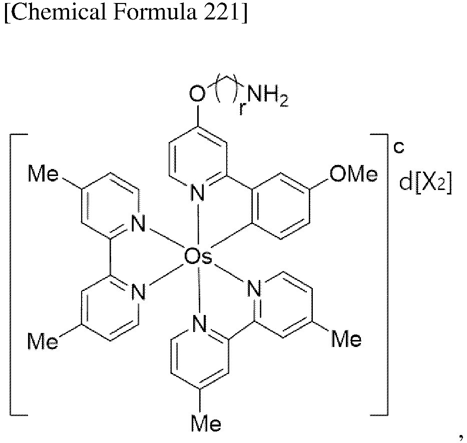 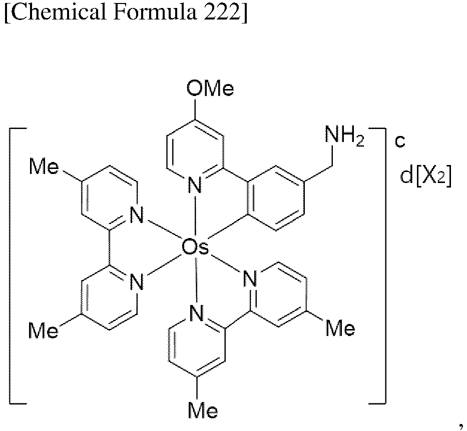 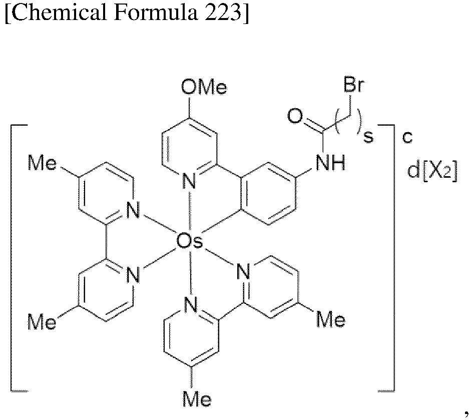 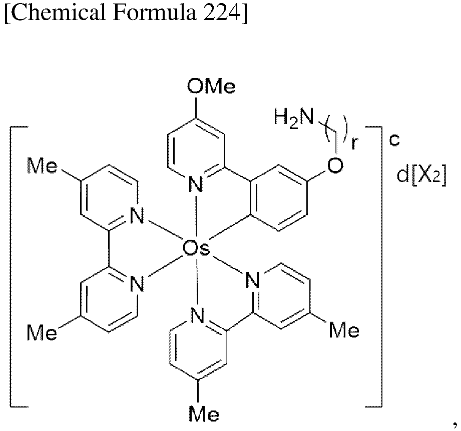 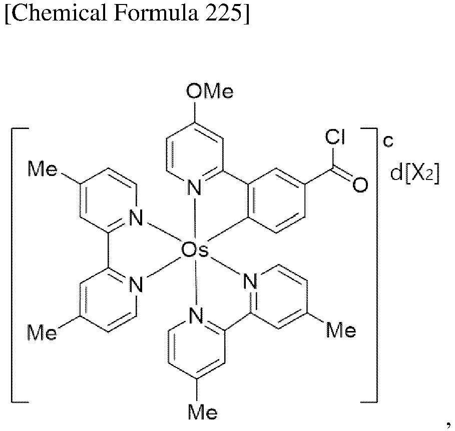 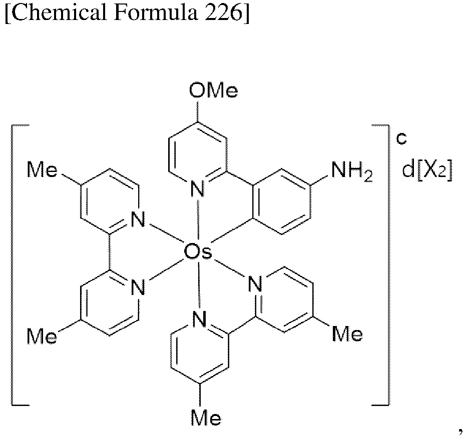 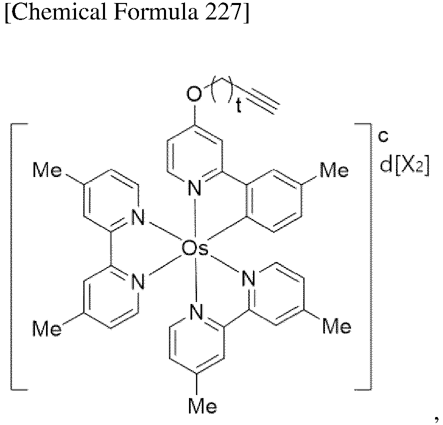 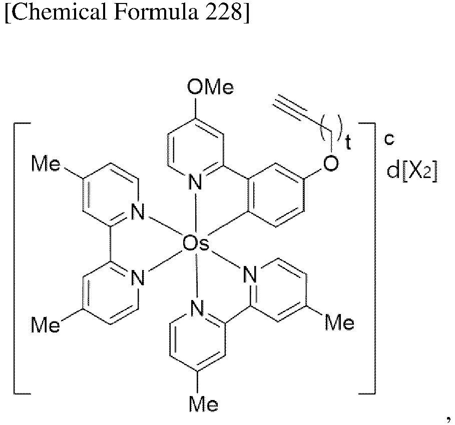 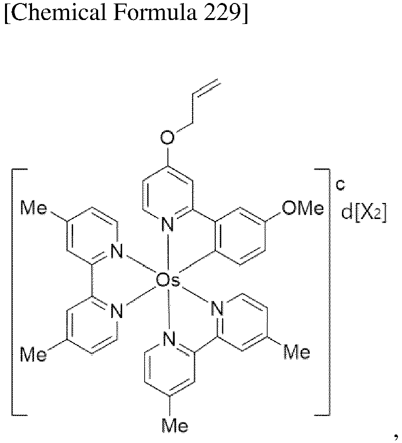 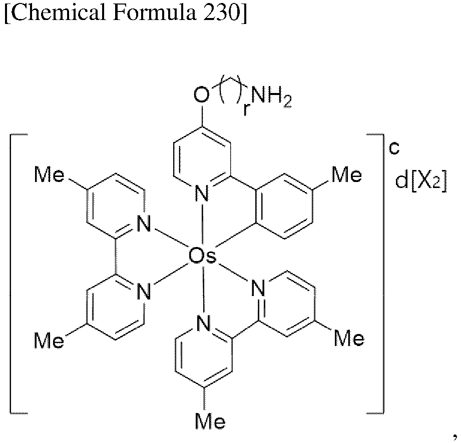 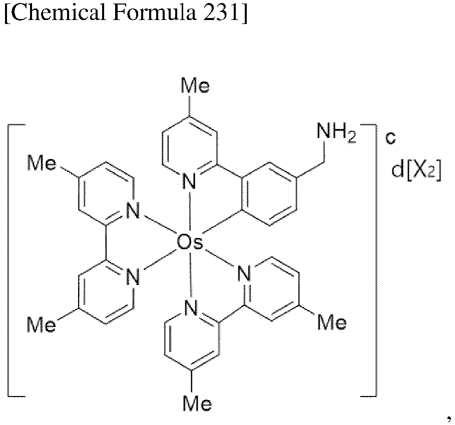 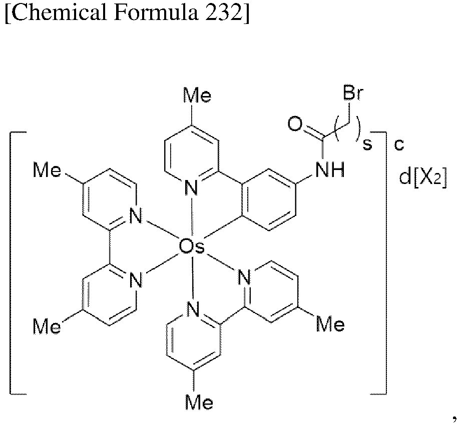 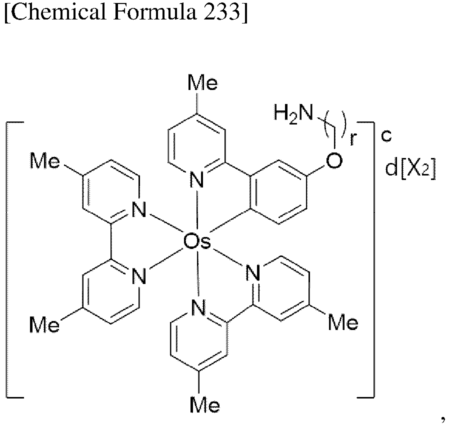 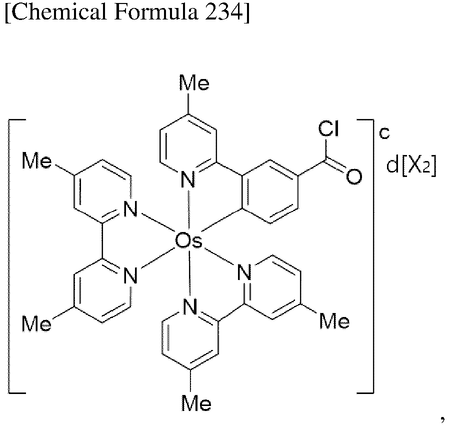 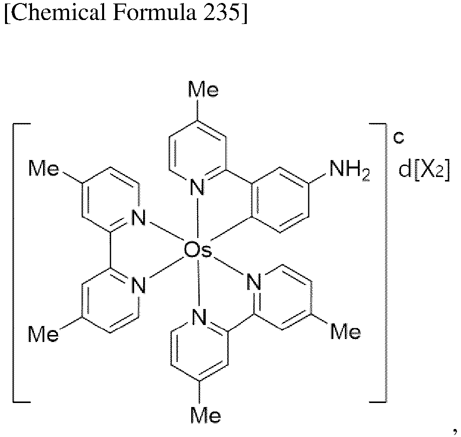 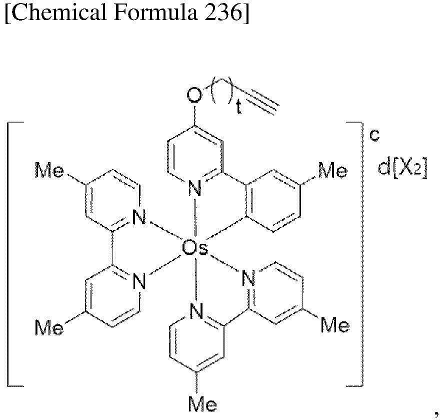 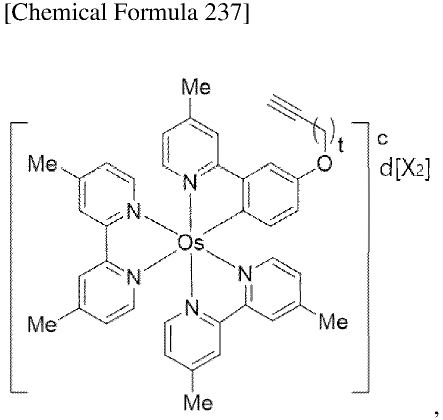 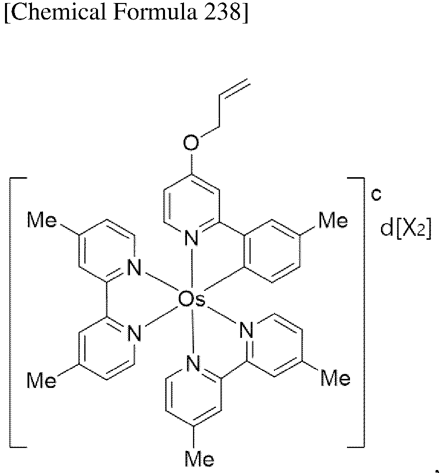 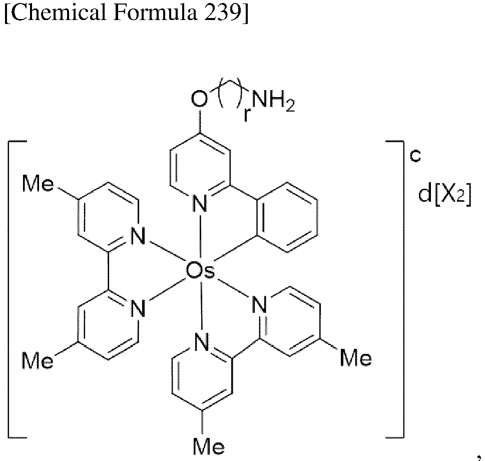 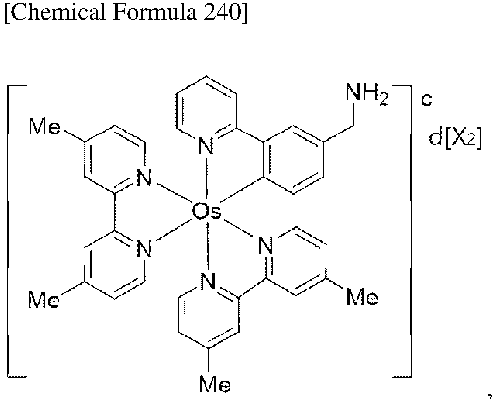 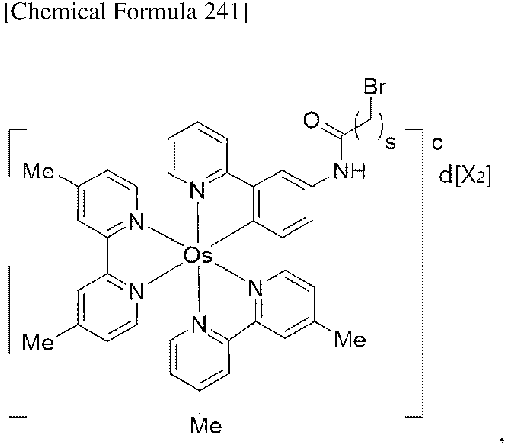 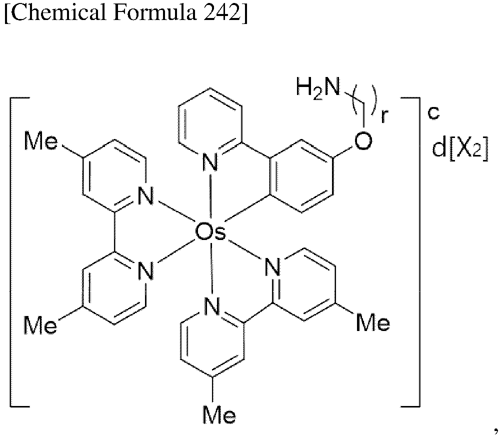 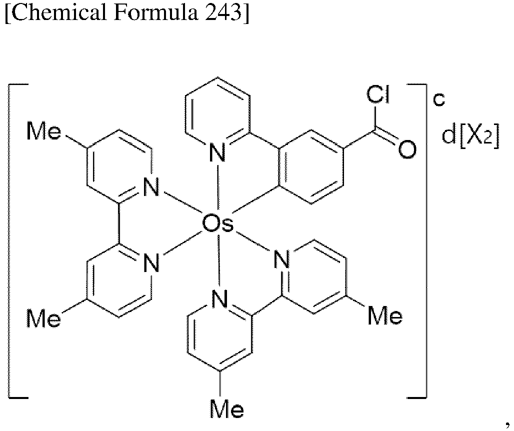 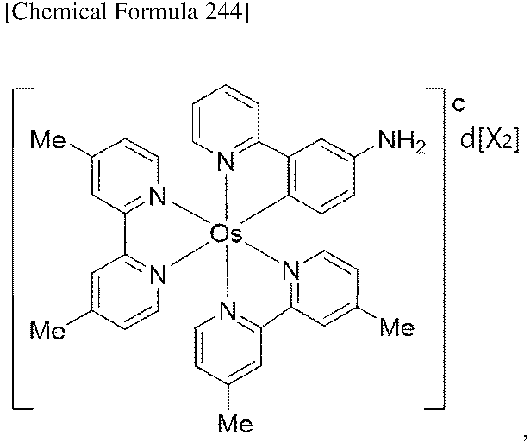 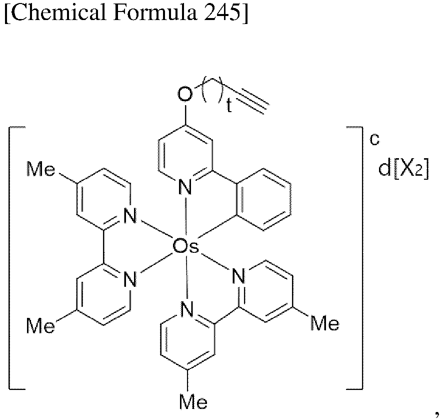 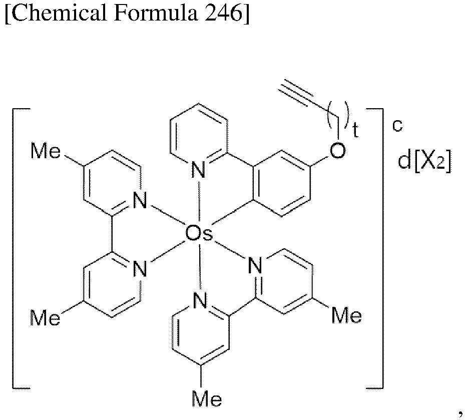 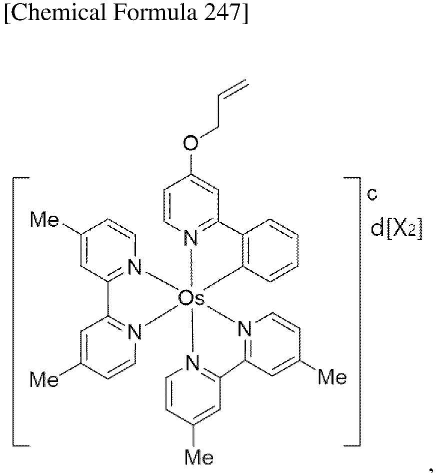 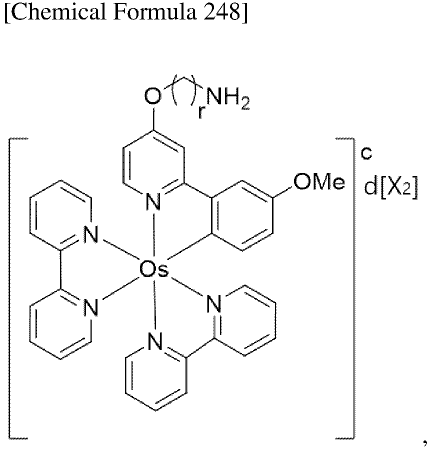 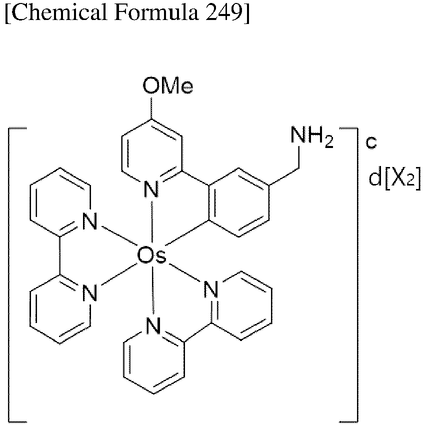 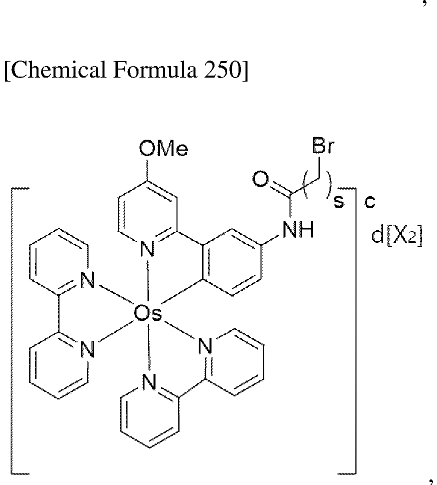 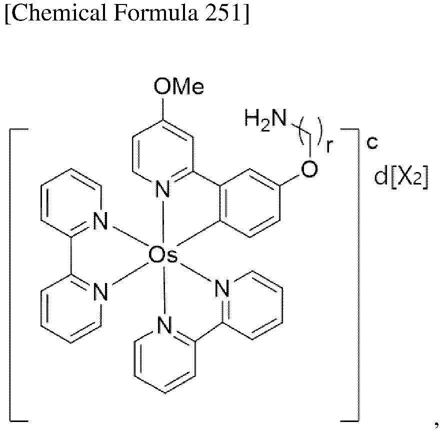 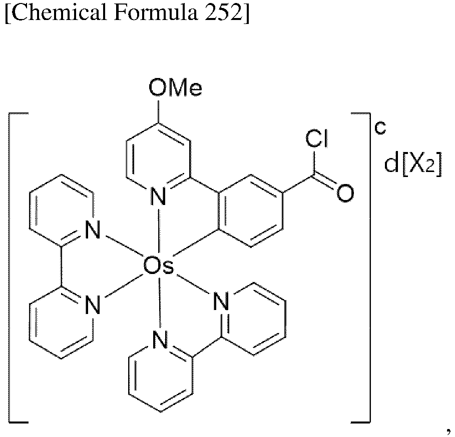 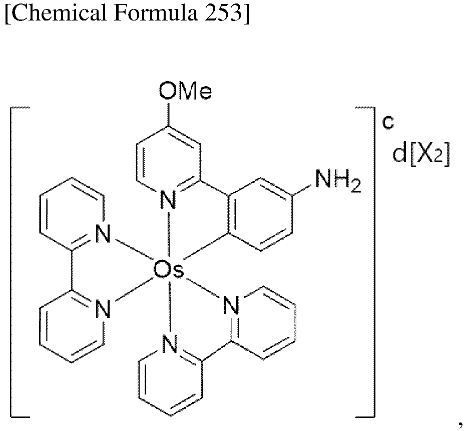 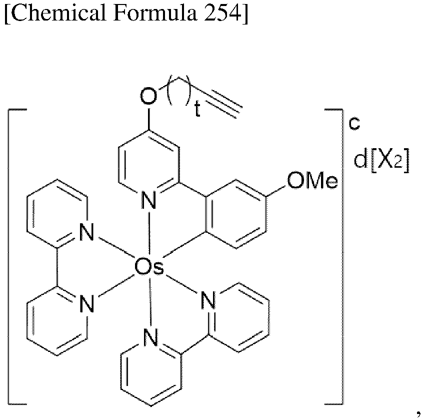 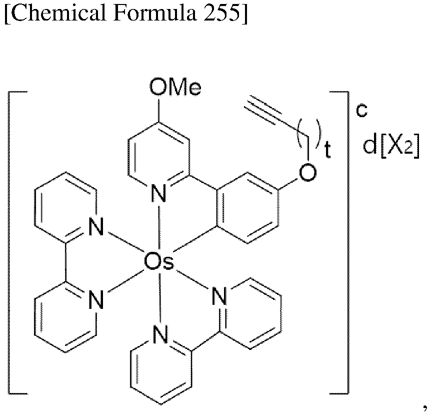 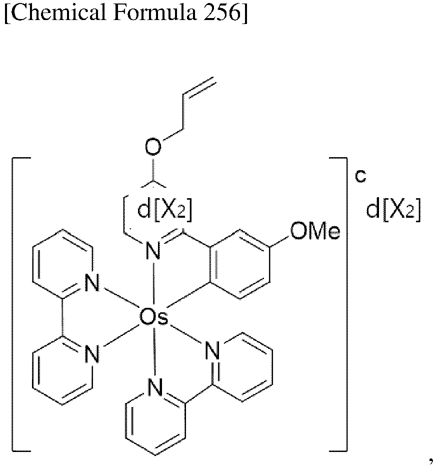 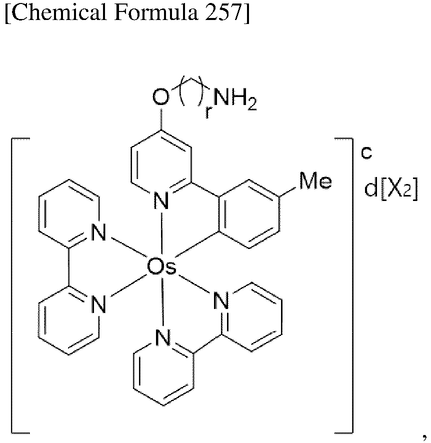 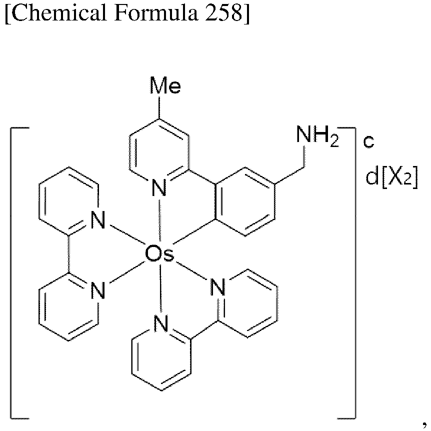 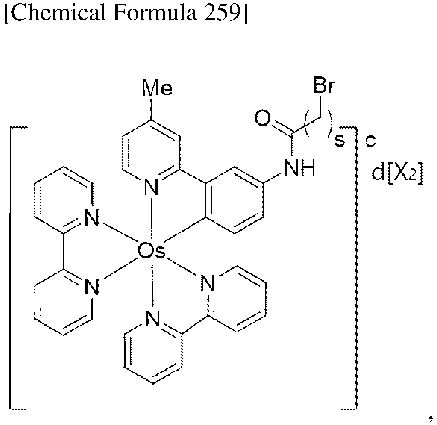 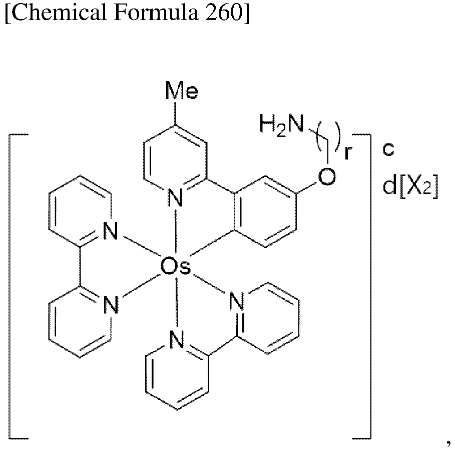 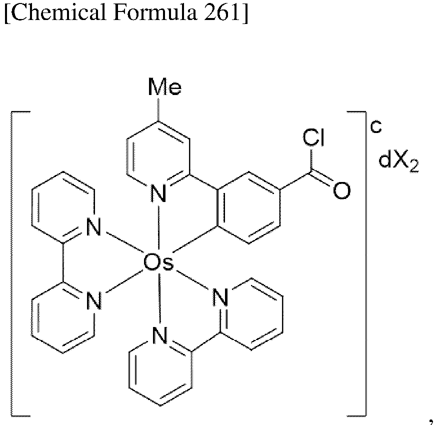 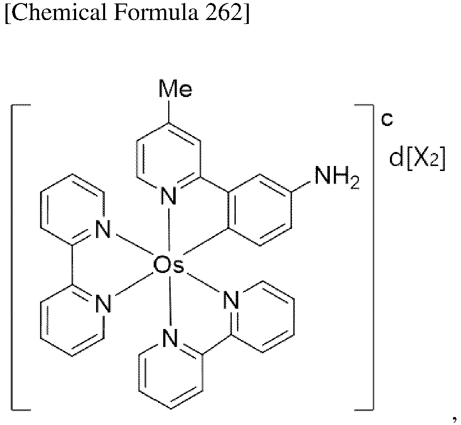 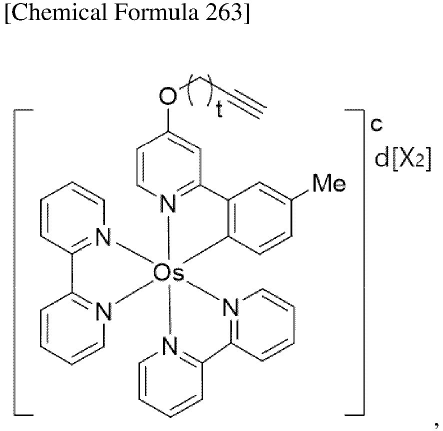 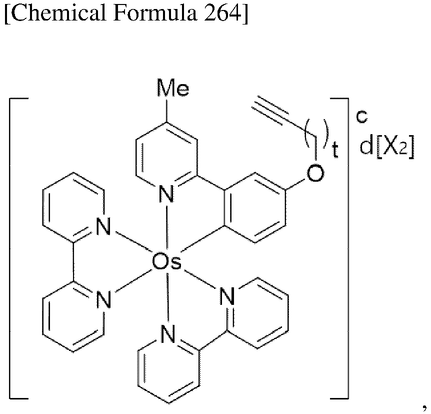 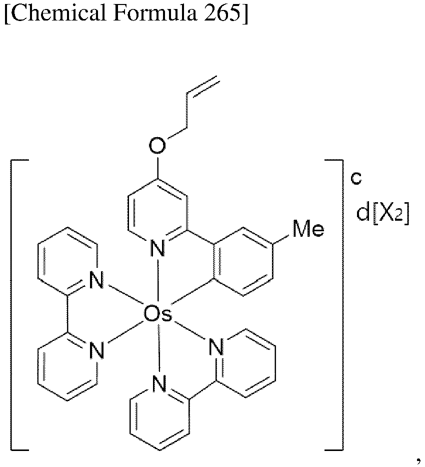 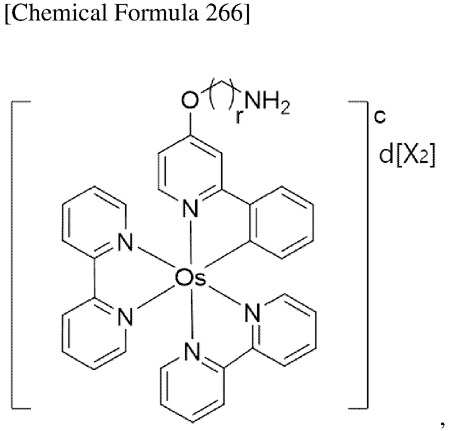 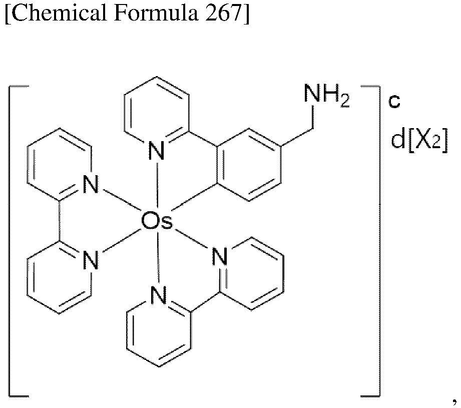 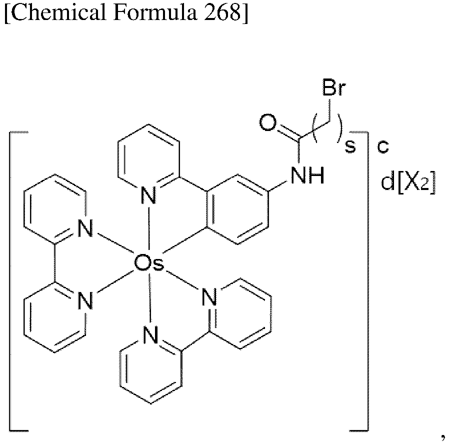 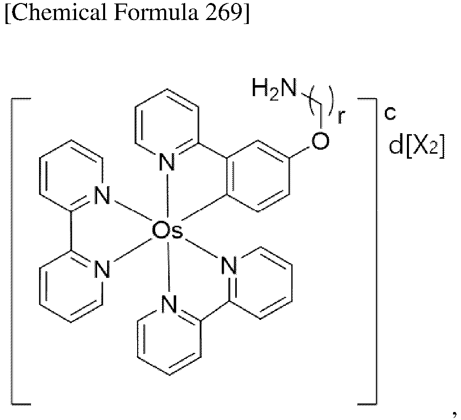 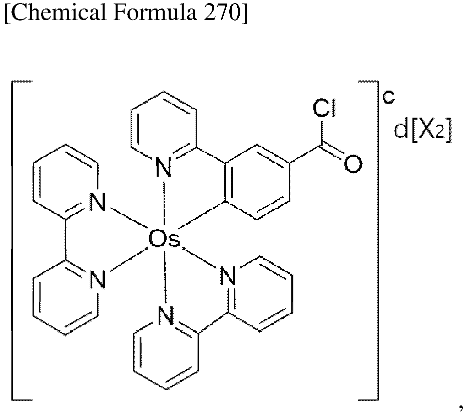 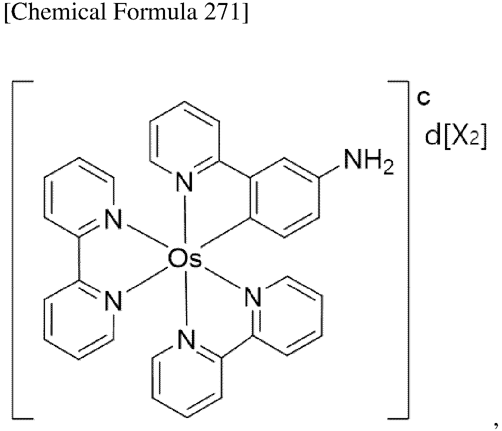 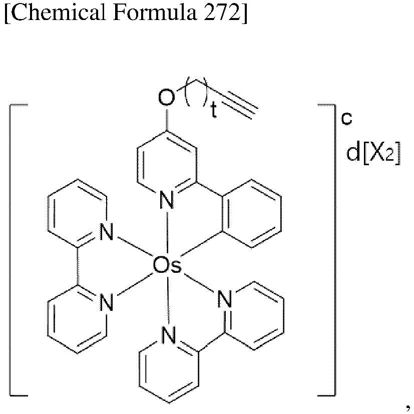 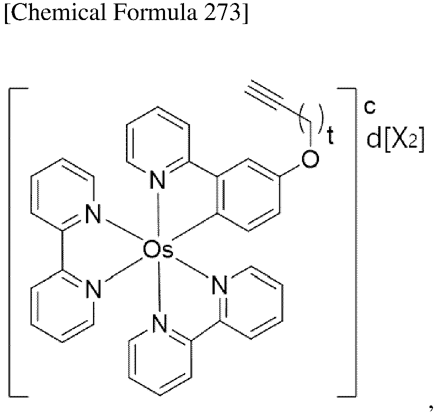 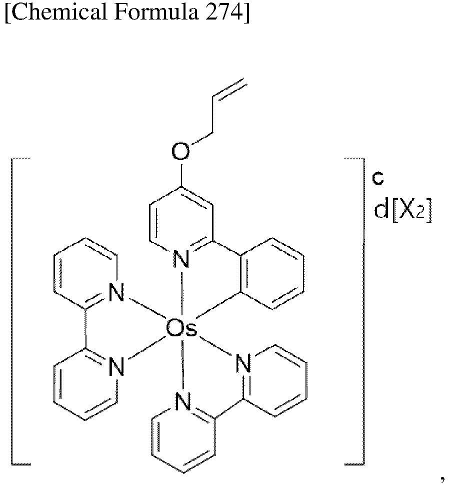
in Chemical Formulae 5 to 274,
c and d are each 1 or 2,
X₂ is one counter ion selected from the group consisting of F, Cl, Br, I, and PF₆,
r is an integer from 1 to 6,
s is an integer from 1 to 12, and
t is an integer from 1 to 4.

5. An oxidation-reduction polymer comprising a combination of a transition metal complex of Chemical Formula 1 or a salt compound thereof and a polymer,
wherein the polymer comprises one or more selected from the group consisting of polyvinylimidazole, polyvinylpyridine, poly(allyl glycidyl ether), poly(dimethylaminoethyl methacrylate), poly(pentafluorophenyl acrylate), poly(pentafluorophenyl methacrylate), a poly(pentafluorophenyl acrylate)-polyacrylamide copolymer, a poly(dimethylaminoethyl methacrylate)-poly(pentafluorophenyl methacrylate) copolymer, and polymers thereof.

6. The oxidation-reduction polymer of claim 5, wherein the oxidation-reduction polymer comprises one in which the polymer is covalently bonded to Adi of Chemical Formula 1.

7. The oxidation-reduction polymer of claim 5, wherein the polymer is one of the following Chemical Formulae 275 to 285: 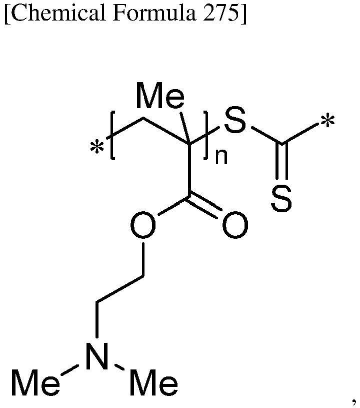 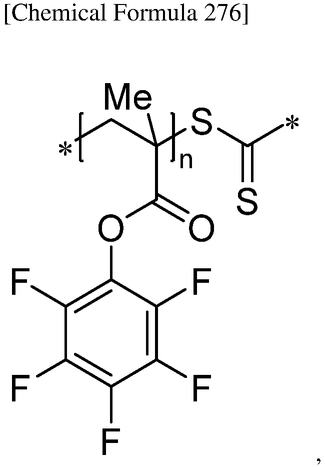 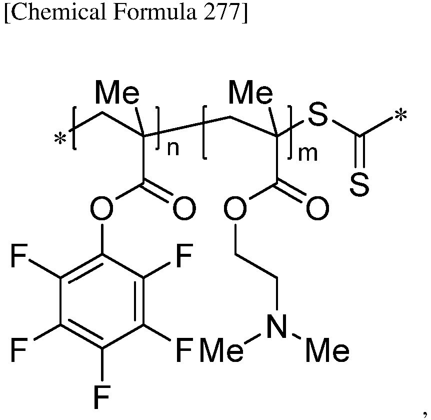 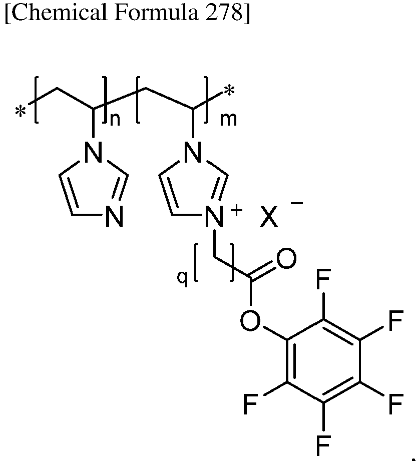 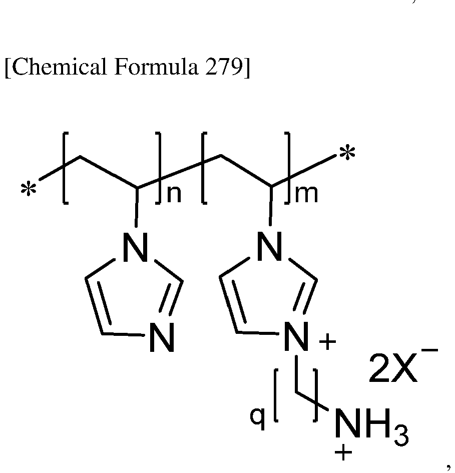 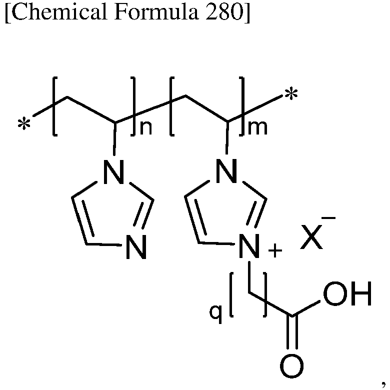 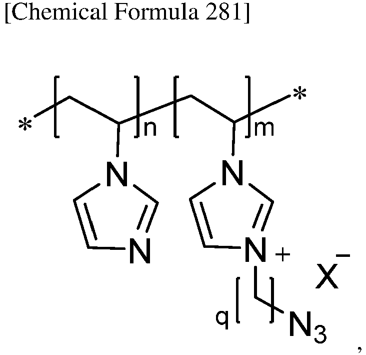 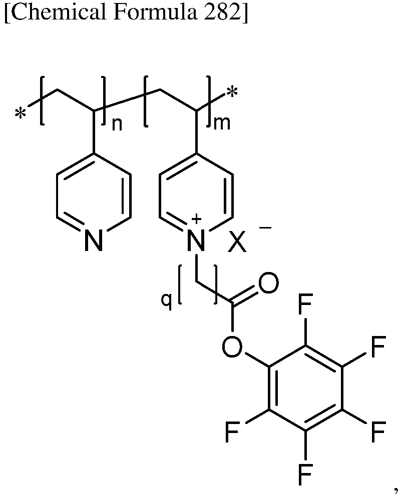 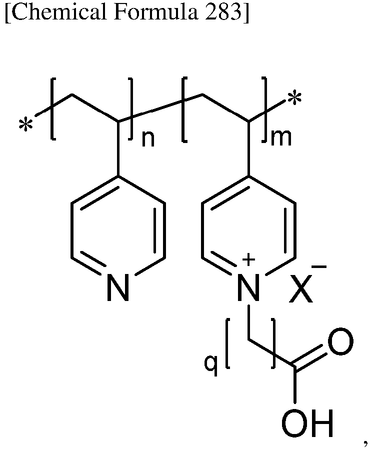 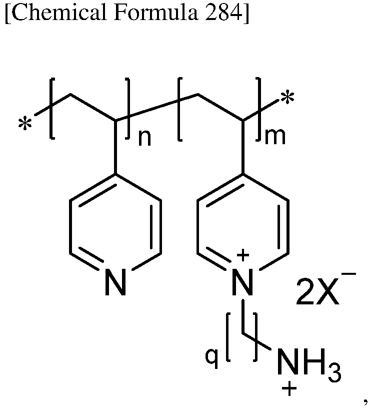 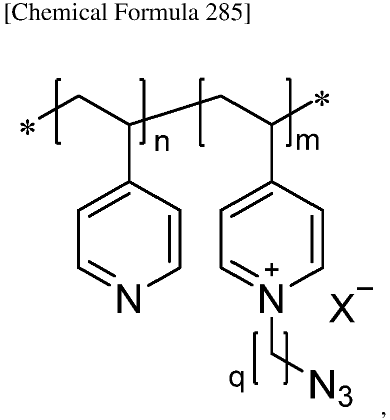
in Chemical Formulae 275 to 285,
n and m are each an integer from 3 to 300,
q is an integer from 1 to 12, and
X is one counter ion selected from the group consisting of F, Cl, Br, and I.

8. The oxidation-reduction polymer of claim 5, wherein the oxidation-reduction polymer comprises one or more compounds of the following Chemical Formulae 286 to 304: 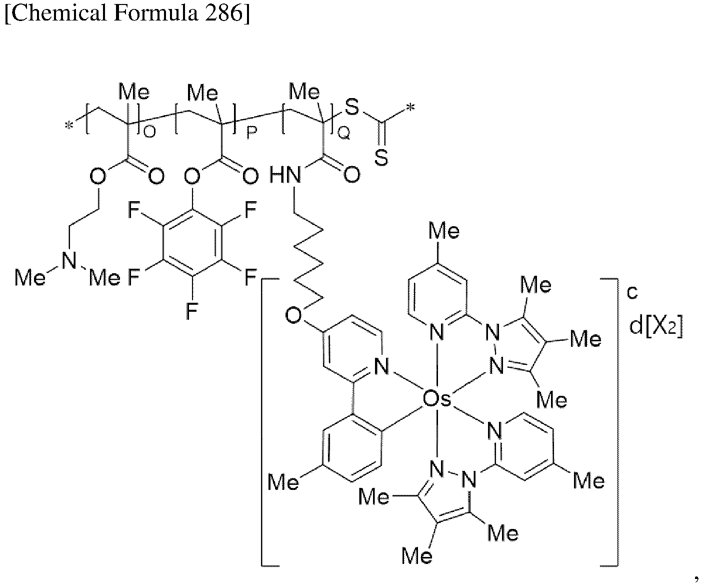 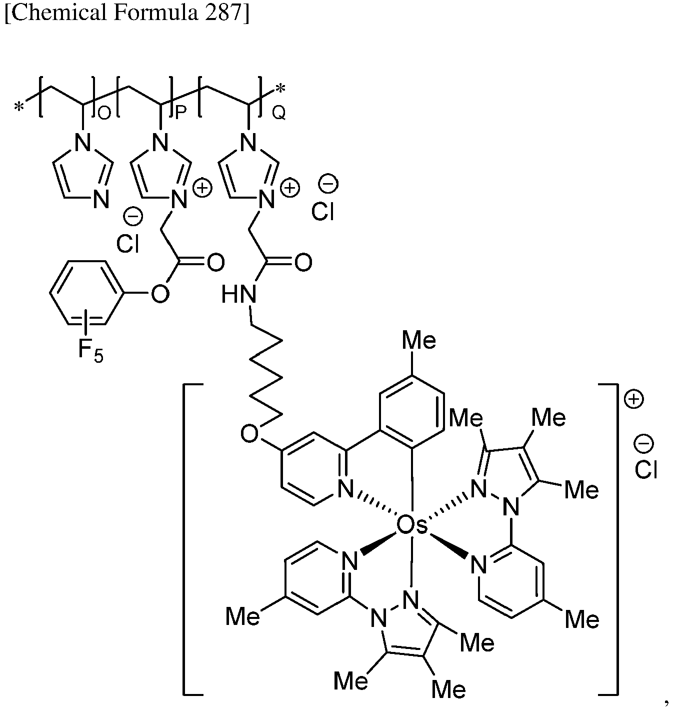 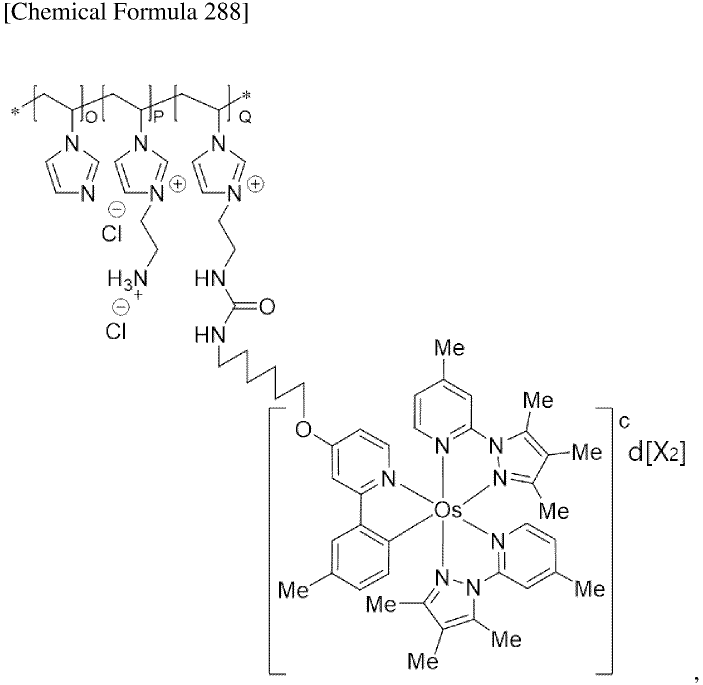 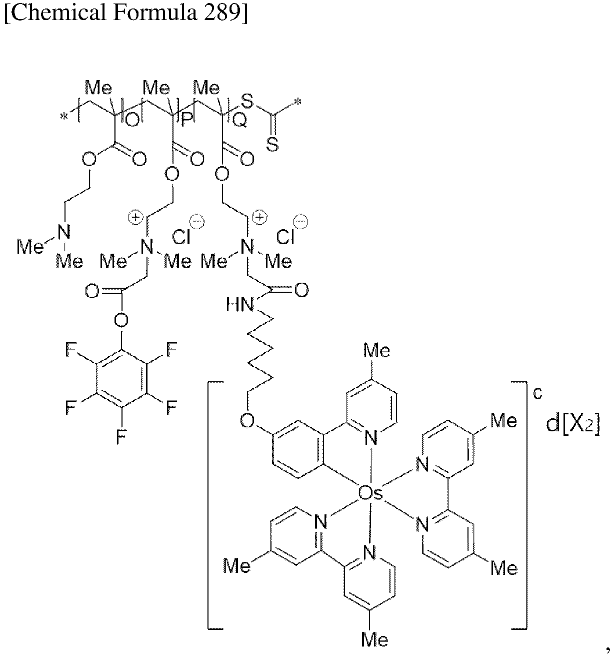 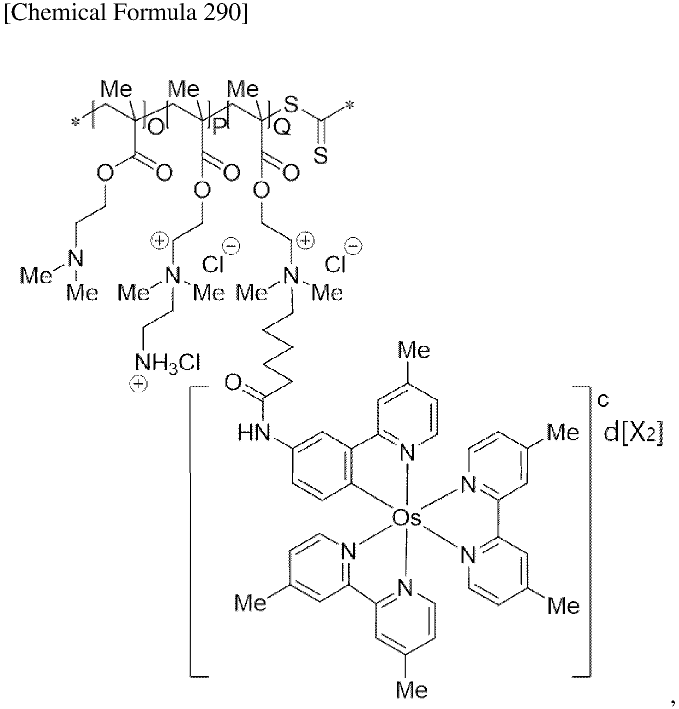 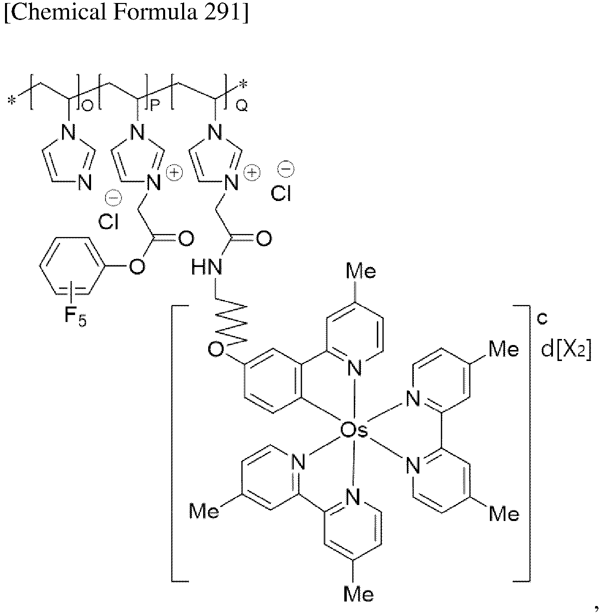 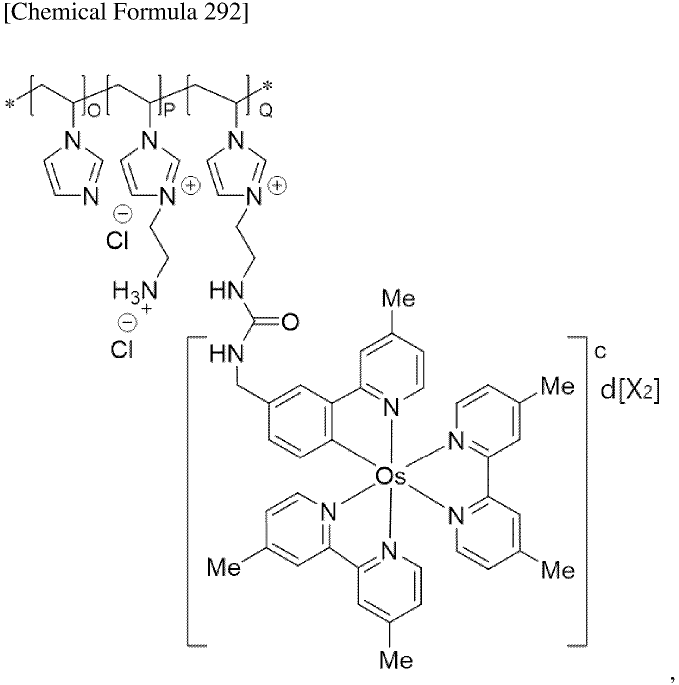 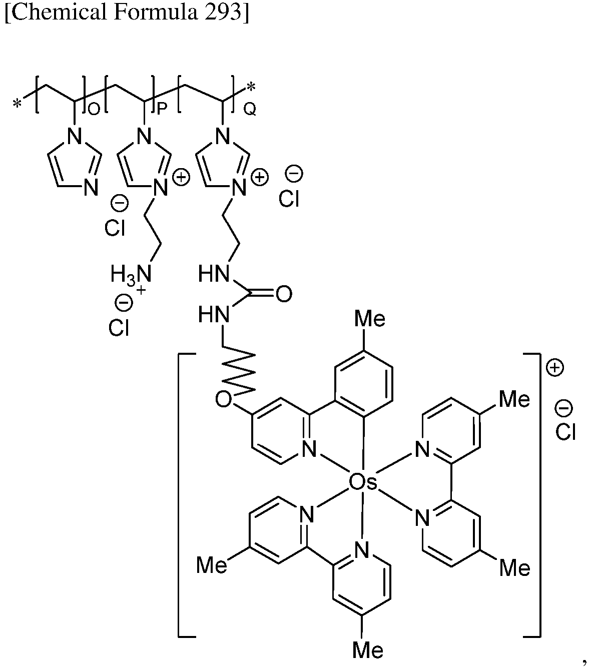 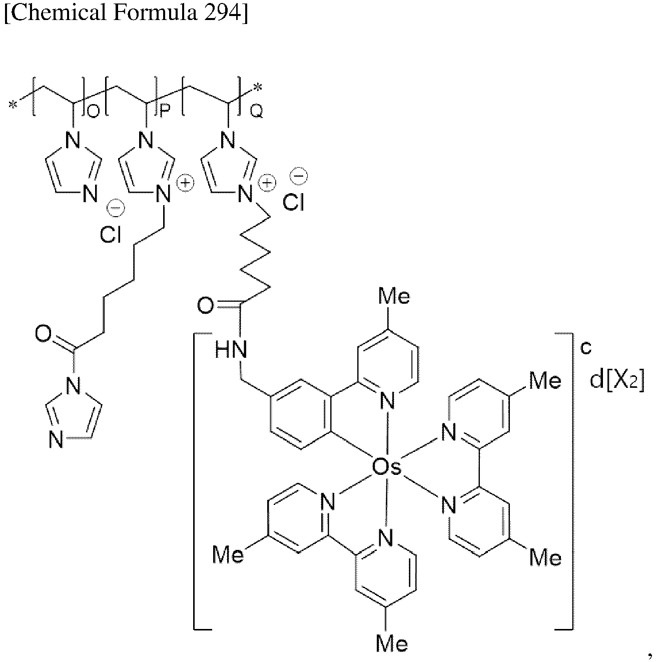 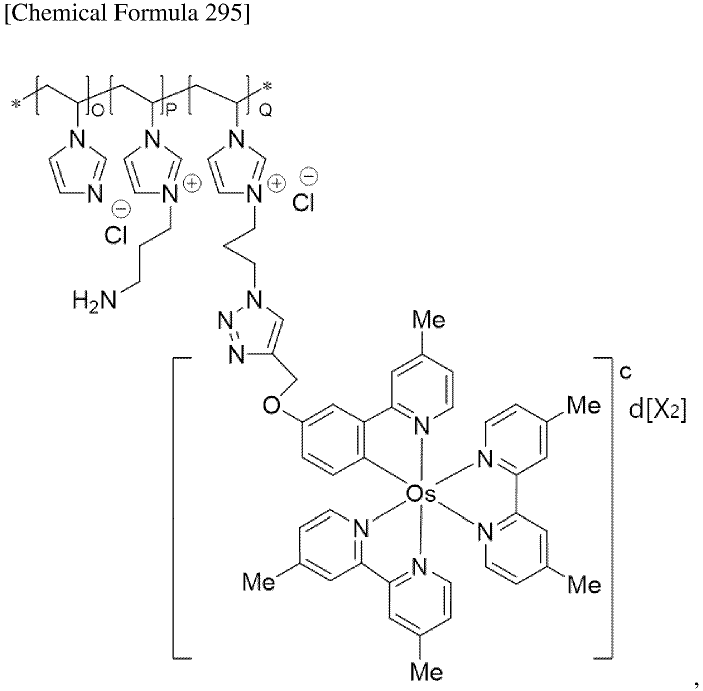 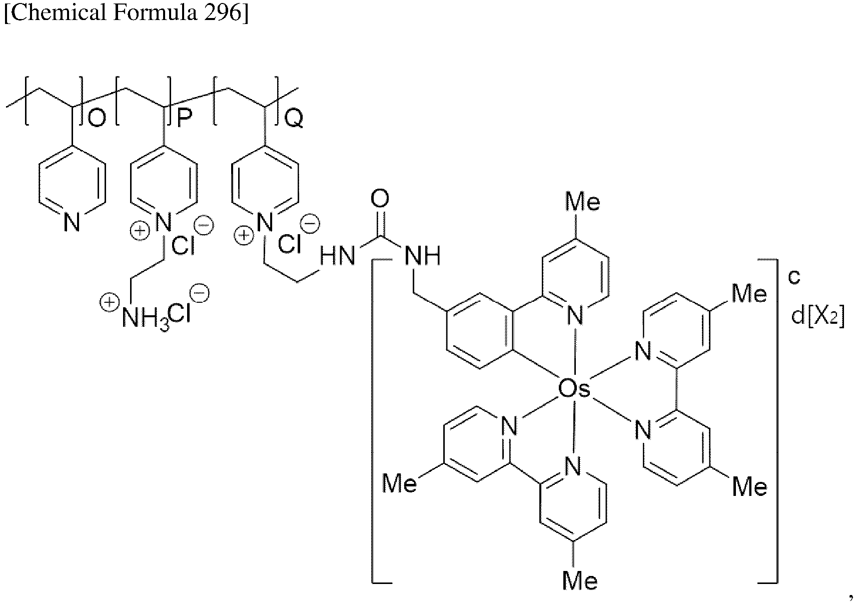 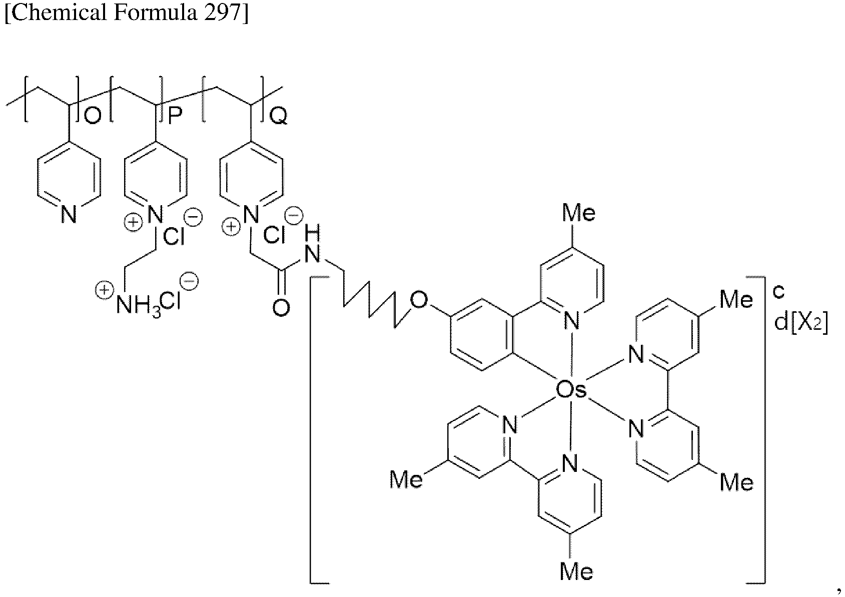 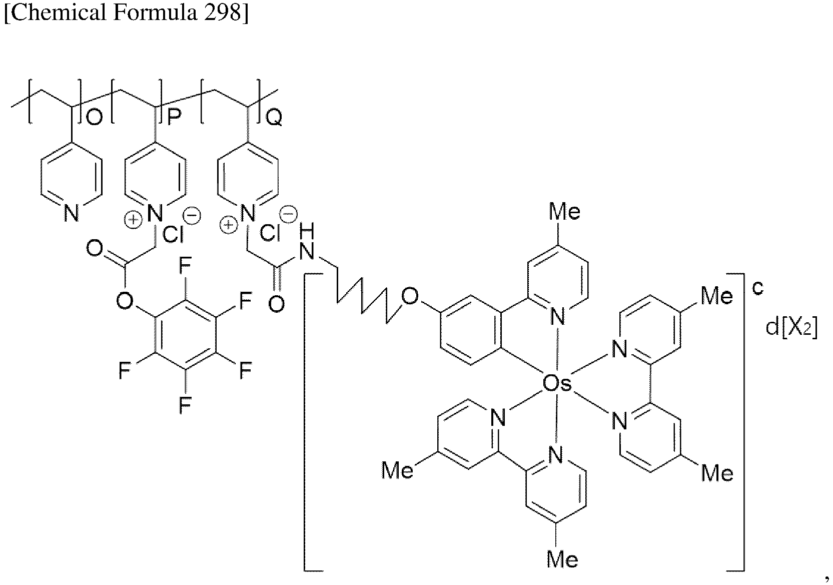 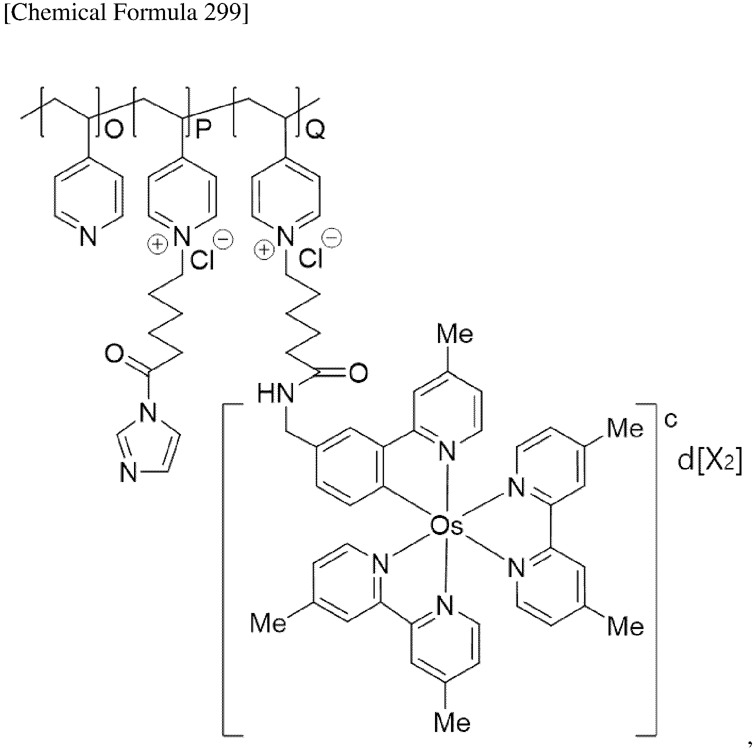 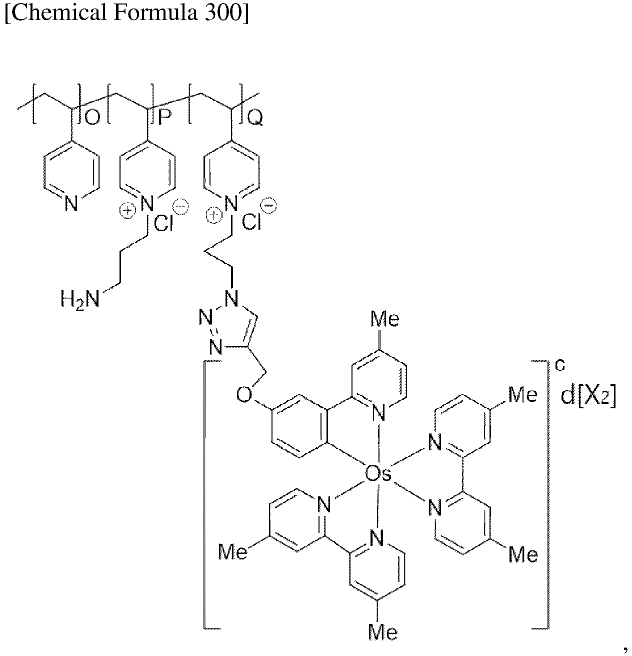 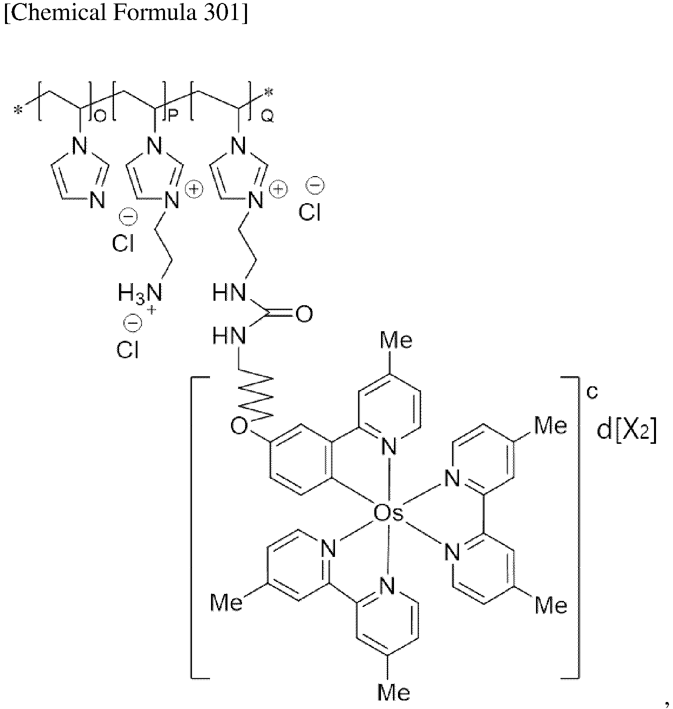 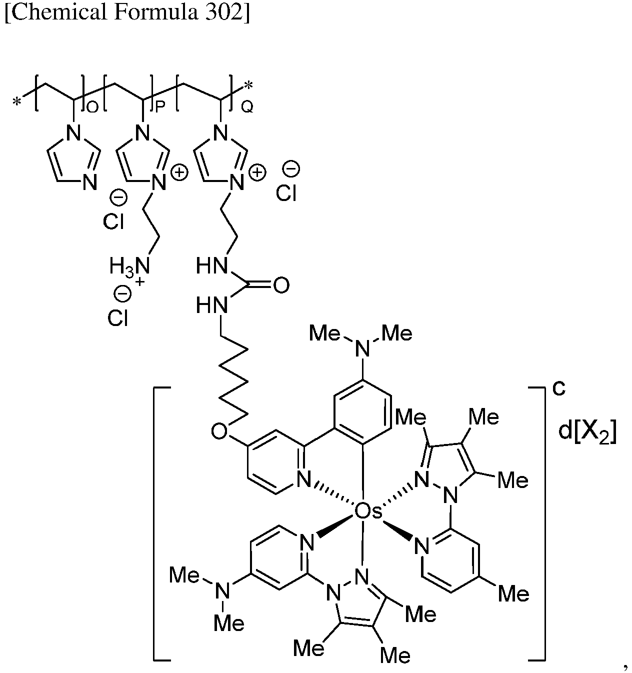 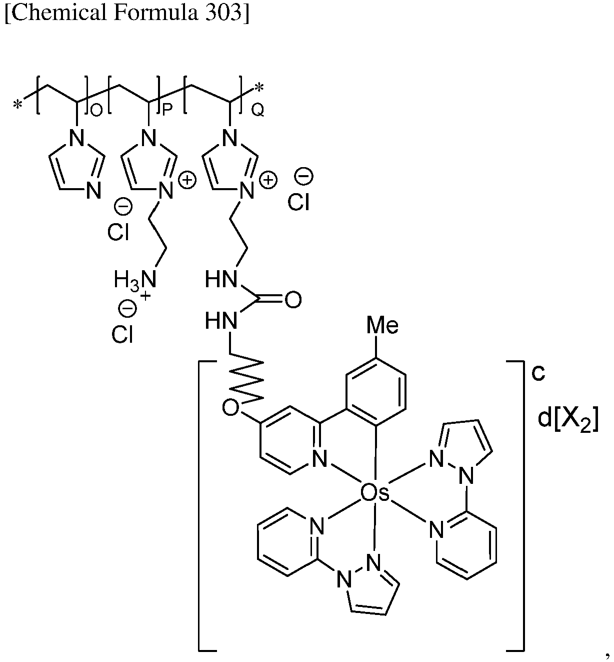 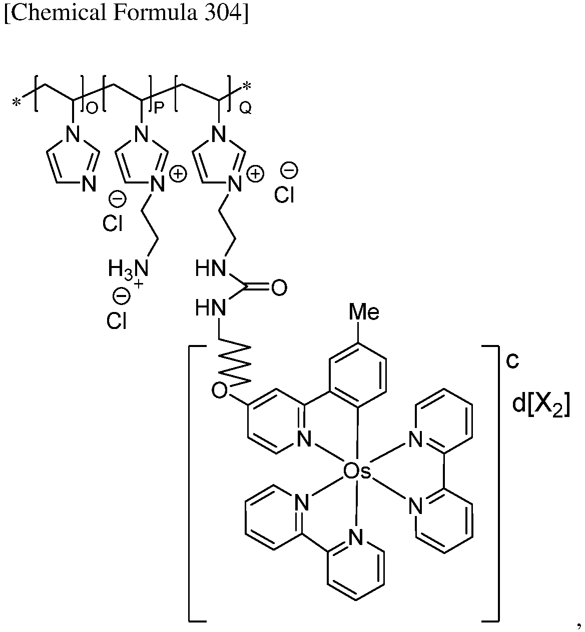
in Chemical Formulae 286 to 304,
c and d are each 1 or 2,
X is one counter ion selected from the group consisting of F, Cl, I, and PF₆, and
O, P, and Q are each an integer from 1 to 100.

9. The oxidation-reduction polymer of claim 5, wherein the oxidation-reduction polymer is for use in an electrochemical biosensor.

10. The oxidation-reduction polymer of claim 9, wherein the electrochemical biosensor comprises an electrochemical biosensor which is capable of being inserted into the body.

11. The oxidation-reduction polymer of claim 9, wherein the electrochemical biosensor comprises an electrochemical biosensor for measuring blood glucose.

12. A sensing layer for an electrochemical biosensor, comprising an enzyme capable of oxidizing-reducing a biological sample; and
the transition metal complex or the salt compound thereof of any one of claims 1 to 4, or the oxidation-reduction polymer of any one of claims 5 to 11 as an electron transfer mediator.

13. The sensing layer of claim 12, further comprising carbon nanotubes.

14. A continuous blood glucose monitoring sensor comprising the sensing layer of claim 12.
